(19) Europäisches Patentamt
European Patent Office
Office européen des brevets

(11) EP 3 449 923 A1

(12) EUROPEAN PATENT APPLICATION

(43) Date of publication:
**06.03.2019 Bulletin 2019/10**

(51) Int Cl.:
***A61K 31/4184*** $^{(2006.01)}$

(21) Application number: **18198417.0**

(22) Date of filing: **07.03.2008**

(84) Designated Contracting States:
**AT BE BG CH CY CZ DE DK EE ES FI FR GB GR HR HU IE IS IT LI LT LU LV MC MT NL NO PL PT RO SE SI SK TR**

(30) Priority: **07.03.2007 US 905293 P**

(62) Document number(s) of the earlier application(s) in accordance with Art. 76 EPC:
**08724338.2 / 2 131 840**

(71) Applicant: **MEI Pharma, Inc.**
**San Diego, CA 92130 (US)**

(72) Inventors:
- **GOH, Kay Lin**
  **689097 Singapore (SG)**
- **KHNG, Hwee Hoon**
  **128046 Singapore (SG)**
- **SABANAYAGAM, Vasantha, Malar**
  **81200 Johor Bahru (MY)**
- **SANGTHONGPITAQ, Kanda**
  **118172 Singapore (SG)**
- **STUNKEL, Walter**
  **127466 Singapore (SG)**
- **TAN, Yong Cheng**
  **148955 Singapore (SG)**
- **WOOD, Jeanette Marjorie**
  **238251 Singapore (SG)**

(74) Representative: **Campabadal i Monfà, Gemma**
**Wilson Sonsini Goodrich & Rosati LLP**
**Rue Montoyer 47**
**1000 Bruxelles (BE)**

Remarks:
This application was filed on 03-10-2018 as a divisional application to the application mentioned under INID code 62.

# (54) COMBINATION OF BENZIMIDAZOLE ANTI-CANCER AGENT AND A SECOND ANTI-CANCER AGENT

(57) The present invention relates to a pharmaceutical composition for the treatment of cancer as well as methods of treatment of cancer that are based on the finding that certain benzimidazole based anti-cancer agents can be used in combination with a second anti-cancer agent to achieve desirable therapeutic outcomes. More specifically the present invention relates to a pharmaceutical composition including a benzimidazole based anti-cancer agent and a second anti-cancer agent. The invention also relates to methods of treatment of cancer including administration of a benzimidazole based anti-cancer agent and a second anti-cancer agent to a patient in need thereof.



Printed by Jouve, 75001 PARIS (FR)

## Description

### FIELD OF THE INVENTION

The present invention relates to a pharmaceutical composition for the treatment of cancer as well as methods of treatment of cancer that are based on the finding that certain benzimidazole based anti-cancer agents can be used in combination with a second anti-cancer agent to achieve desirable therapeutic outcomes. More specifically the present invention relates to a pharmaceutical composition including a benzimidazole based anti-cancer agent and a second anti-cancer agent. The invention also relates to methods of treatment of cancer including administration of a benzimidazole based anticancer agent and a second anti-cancer agent to a patient in need thereof.

### BACKGROUND OF THE INVENTION

Cancer is one of the leading causes of death in the modern world. It has been observed that the death rates from cancer and cancer related illnesses is increasing presumably as other conditions that previously lead to mortality are being eliminated by advances in modern medicine. It is anticipated therefore that the need for further and improved treatment regimes for cancer will only increase as the death rates from cancer rise.

Cancer treatment can basically be divided into one of two strategies (or a combination thereof). In general the options available to a clinician are limited to either surgery aimed at removing the cancer or chemotherapy/targeted therapy aimed at killing the cancer in situ. With some cancer types a combination strategy is used in which as much of the cancerous tissue is removed by surgery as possible followed by a course or courses in chemotherapy/targeted therapy to eliminate any cancer cells that could not be surgically removed. In any circumstance the exact course of action taken will be determined on a case by case basis and will be based on such factors as the condition of the patient, the type of cancer, whether the cancer has spread from the initial site of cancer growth and the like. It is observed, however, that anti-cancer therapies such as targeted therapies or chemotherapies is a preferred approach as it will usually be able to be used with almost all cancer types.

Accordingly, there is a number of chemotherapy or targeted therapy regimes that are known and used throughout the world. These regimes may involve administration of a single active agent or a "cocktail" of active agents aimed at attacking the cancer cells from different aspects. Nevertheless, despite the plethora of treatment regimes available they are not effective for all patients. In addition many of the treatment regimes lead to undesirable side effects in the patients thus treated. There is therefore always the need to develop further treatment regimes and agents for use in cancer chemotherapy/targeted therapy which can be used either as stand alone treatments or as treatments to be used in combination with surgery.

### SUMMARY OF THE INVENTION

The present invention is based on the finding by the applicants that a benzimidazole based anti-cancer agent can be combined with a second anti-cancer agent agent to provide a pharmaceutical composition suitable for the treatment of cancer and can be used to provide therapeutically useful effects. In general there is a synergistic effect observed between the benzimidazole based anti-cancer agent and the second anti-cancer agent when used in the treatment of cancer whether used simultaneously or sequentially or a combination thereof. The benzimidazole anti-cancer agent and the second anti-cancer agent may be used with a further anti-cancer agent as discussed herein.

In one aspect the present invention provides a pharmaceutical composition for the treatment of cancer the composition including: (i) a benzimidazole based anti-cancer agent; and (ii) a second anti-cancer agent.

In another aspect the present invention provides a method for treating cancer including administering to a patient in need thereof: a first amount of a benzimidazole based anti-cancer agent and a second amount of a second anti-cancer agent; wherein the first amount and the second amount together comprise a therapeutically effective amount.

In an even further aspect the invention provides the use of a benzimidazole based anti-cancer agent in the manufacture of a medicament for the treatment of cancer by administration of (i) the benzimidazole based anti-cancer agent and (ii) a second anti-cancer agent.

In yet an even further aspect the invention provides a kit or kit-of-parts including (i) a benzimidazole based anti-cancer agent; and (ii) a second anti-cancer agent wherein said kit or kit-of-parts is suitable for use in a method for treating cancer.

### DETAILED DESCRIPTION OF THE INVENTION

In this specification a number of terms are used which are well known to a skilled addressee. Nevertheless for the purposes of clarity a number of terms will be defined.

As used herein, the term unsubstituted means that there is no substituent or that the only substituents are hydrogen.

The term "optionally substituted" as used throughout the specification denotes that the group may or may not be further substituted or fused (so as to form a condensed polycyclic system), with one or more substituent groups. Preferably the substituent groups are one or more groups independently selected from the group consisting of halogen, =O, =S, -CN, $-NO_2$, $-CF_3$, $-OCF_3$, alkyl, alkenyl, alkynyl, haloalkyl, haloalkenyl, haloalkynyl, heteroalkyl, cycloalkyl, cycloalkenyl, heterocycloalkyl, heterocycloalkenyl, aryl, heteroaryl, cycloalkylalkyl, heterocycloalkylalkyl, heteroarylalkyl, arylalkyl, cycloalkylalkenyl, heterocycloalkylalkenyl, arylalkenyl, heteroarylalkenyl, cycloalkylheteroalkyl, heterocycloalkylheteroalkyl, arylheteroalkyl, heteroarylheteroalkyl, hydroxy, hydroxyalkyl; alkoxy, alkoxyalkyl, alkoxycycloalkyl, alkoxyheterocycloalkyl, alkoxyaryl, alkoxyheteroaryl, alkoxycarbonyl, alkylaminocarbonyl, alkenyloxy, alkynyloxy, cycloalkyloxy, cycloalkenyloxy, heterocycloalkyloxy, heterocycloalkenyloxy, aryloxy, phenoxy, benzyloxy, heteroaryloxy, arylalkyloxy, arylalkyl, heteroarylalkyl, cycloalkylalkyl, heterocycloalkylalkyl, arylalkyloxy, amino, alkylamino, acylamino, aminoalkyl, arylamino, sulfonylamino, sulfinylamino, sulfonyl, alkylsulfonyl, arylsulfonyl, aminosulfonyl, sulfinyl, alkylsulfinyl, arylsulfinyl, aminosulfinylaminoalkyl, -COOH, $-COR^5$, $-C(O)OR^5$, $CONHR^5$, $NHCOR^5$, $NHCOOR^5$, $NHCONHR^5$, $C(=NOH)R^5$, -SH, $-SR^5$, $-OR^5$ and acyl.

"Alkyl" as a group or part of a group refers to a straight or branched aliphatic hydrocarbon group, preferably a $C_1$-$C_{14}$ alkyl, more preferably $C_1$-$C_{10}$ alkyl, most preferably $C_1$-$C_6$ unless otherwise noted. Examples of suitable straight and branched $C_1$-$C_6$ alkyl substituents include methyl, ethyl, n-propyl, 2-propyl, n-butyl, sec-butyl, t-butyl, hexyl, and the like. The group may be a terminal group or a bridging group.

"Alkylamino" includes both monoalkylamino and dialkylamino, unless specified. "Monoalkylamino" means a -NH-Alkyl group, in which alkyl is as defined above. "Dialkylamino" means a $-N(alkyl)_2$ group, in which each alkyl may be the same or different and are each as defined herein for alkyl. The alkyl group is preferably a $C_1$-$C_6$ alkyl group. The group may be a terminal group or a bridging group.

"Arylamino" includes both mono-arylamino and di-arylamino unless specified. Mono-arylamino means a group of formula aryl NH-, in which aryl is as defined herein. di-arylamino means a group of formula $(aryl_2)$ N- where each aryl may be the same or different and are each as defined herein for aryl. The group may be a terminal group or a bridging group.

"Acyl" means an alkyl-CO- group in which the alkyl group is as described herein. Examples of acyl include acetyl and benzoyl. The alkyl group is preferably a $C_1$-$C_6$ alkyl group. The group may be a terminal group or a bridging group.

"Alkenyl" as a group or part of a group denotes an aliphatic hydrocarbon group containing at least one carbon-carbon double bond and which may be straight or branched preferably having 2-14 carbon atoms, more preferably 2-12 carbon atoms, most preferably 2-6 carbon atoms, in the normal chain. The group may contain a plurality of double bonds in the normal chain and the orientation about each is independently E or Z. Exemplary alkenyl groups include, but are not limited to, ethenyl, propenyl, butenyl, pentenyl, hexenyl, heptenyl, octenyl and nonenyl. The group may be a terminal group or a bridging group.

"Alkoxy" refers to an -O-alkyl group in which alkyl is defined herein. Preferably the alkoxy is a $C_1$-$C_6$alkoxy. Examples include, but are not limited to, methoxy and ethoxy. The group may be a terminal group or a bridging group.

"Alkenyloxy" refers to an -O- alkenyl group in which alkenyl is as defined herein. Preferred alkenyloxy groups are $C_1$-$C_6$ alkenyloxy groups. The group may be a terminal group or a bridging group.

"Alkynyloxy" refers to an -O-alkynyl group in which alkynyl is as defined herein. Preferred alkynyloxy groups are $C_1$-$C_6$ alkynyloxy groups. The group may be a terminal group or a bridging group.

"Alkoxycarbonyl" refers to an -C(O)-O-alkyl group in which alkyl is as defined herein. The alkyl group is preferably a $C_1$-$C_6$ alkyl group. Examples include, but not limited to, methoxycarbonyl and ethoxycarbonyl. The group may be a terminal group or a bridging group.

"Akylsulfinyl" means a -S(O)-alkyl group in which alkyl is as defined above. The alkyl group is preferably a $C_1$-$C_6$ alkyl group. Exemplary alkylsulfinyl groups include, but not limited to, methylsulfinyl and ethylsulfinyl. The group may be a terminal group or a bridging group.

"Alkylsulfonyl" refers to a $-S(O)_2$-alkyl group in which alkyl is as defined above. The alkyl group is preferably a $C_1$-$C_6$ alkyl group. Examples include, but not limited to methylsulfonyl and ethylsulfonyl. The group may be a terminal group or a bridging group.

"Alkynyl as a group or part of a group means an aliphatic hydrocarbon group containing a carbon-carbon triple bond and which may be straight or branched preferably having from 2-14 carbon atoms, more preferably 2-12 carbon atoms, more preferably 2-6 carbon atoms in the normal chain. Exemplary structures include, but are not limited to, ethynyl and propynyl. The group may be a terminal group or a bridging group.

"Alkylaminocarbonyl" refers to an alkylamino-carbonyl group in which alkylamino is as defined above. The group may be a terminal group or a bridging group.

"Cycloalkyl" refers to a saturated or partially saturated, monocyclic or fused or spiro polycyclic, carbocycle preferably containing from 3 to 9 carbons per ring, such as cyclopropyl, cyclobutyl, cyclopentyl, cyclohexyl and the like,

unless otherwise specified. It includes monocyclic systems such as cyclopropyl and cyclohexyl, bicyclic systems such as decalin, and polycyclic systems such as adamantane. The group may be a terminal group or a bridging group.

"Cycloalkenyl" means a non-aromatic monocyclic or multicyclic ring system containing at least one carbon-carbon double bond and preferably having from 5-10 carbon atoms per ring. Exemplary monocyclic cycloalkenyl rings include cyclopentenyl, cyclohexenyl or cycloheptenyl. The cycloalkenyl group may be substituted by one or more substituent groups. The group may be a terminal group or a bridging group.

The above discussion of alkyl and cycloalkyl substituents also applies to the alkyl portions of other substituents, such as without limitation, alkoxy, alkyl amines, alkyl ketones, arylalkyl, heteroarylalkyl, alkylsulfonyl and alkyl ester substituents and the like.

"Cycloalkylalkyl" means a cycloalkyl-alkyl- group in which the cycloalkyl and alkyl moieties are as previously described. Exemplary monocycloalkylalkyl groups include cyclopropylmethyl, cyclopentylmethyl, cyclohexylmethyl and cycloheptylmethyl. The group may be a terminal group or a bridging group.

"Halogen" represents chlorine, fluorine, bromine or iodine.

"Heterocycloalkyl" refers to a saturated or partially saturated monocyclic, bicyclic, or polycyclic ring containing at least one heteroatom selected from nitrogen, sulfur, oxygen, preferably from 1 to 3 heteroatoms in at least one ring. Each ring is preferably from 3 to 10 membered, more preferably 4 to 7 membered. Examples of suitable heterocycloalkyl substituents include pyrrolidyl, tetrahydrofuryl, tetrahydrothiofuranyl, piperidyl, piperazyl, tetrahydropyranyl, morphilino, 1,3-diazapane, 1,4-diazapane, 1,4-oxazepane, and 1,4-oxathiapane. The group may be a terminal group or a bridging group.

"Heterocycloalkenyl" refers to a heterocycloalkyl as described above but containing at least one double bond. The group may be a terminal group or a bridging group.

"Heterocycloalkylalkyl" refers to a heterocycloalkyl-alkyl group in which the heterocycloalkyl and alkyl moieties are as previously described. Exemplary heterocycloalkylalkyl groups include (2-tetrahydrothiofuranyl)methyl. The group may be a terminal group or a bridging group.

"Heteroalkyl" refers to a straight- or branched-chain alkyl group preferably having from 2 to 14 atoms, more preferably 2 to 10 atoms in the chain, one or more of which is a heteroatom selected from S, O, and N. Exemplary heteroalkyls include alkyl ethers, secondary and tertiary alkyl amines, alkyl sulfides, and the like. The group may be a terminal group or a bridging group.

"Aryl" as a group or part of a group denotes (i) an optionally substituted monocyclic, or fused polycyclic, aromatic carbocycle (ring structure having ring atoms that are all carbon) preferably having from 5 to 12 atoms per ring. Examples of aryl groups include phenyl, naphthyl, and the like; (ii) an optionally substituted partially saturated bicyclic aromatic carbocyclic moiety in which a phenyl and a $C_{5-7}$ cycloalkyl or $C_{5-7}$ cycloalkenyl group are fused together to form a cyclic structure, such as tetrahydronaphthyl, indenyl or indanyl. The group may be a terminal group or a bridging group.

"Arylalkenyl" means an aryl-alkenyl- group in which the aryl and alkenyl are as previously described. Exemplary arylalkenyl groups include phenylallyl. The group may be a terminal group or a bridging group.

"Arylalkyl" means an aryl-alkyl- group in which the aryl and alkyl moieties are as previously described. Preferred arylalkyl groups contain a $C_1$-$_5$ alkyl moiety. Exemplary arylalkyl groups include benzyl, phenethyl and naphthelenemethyl. The group may be a terminal group or a bridging group.

"Arylacyl" means an aryl-acyl- group in which the aryl and acyl moieties are as previously described. In general the aryl moiety is attached to the alkyl portion of the acyl moiety, typically to the terminal carbon of the alkyl portion of the acyl moiety. Preferred arylacyl groups contain a $C_1$-$_5$ alkyl moiety in the acyl moiety. Exemplary arylacyl groups include 2-phenyl-acetyl. The group may be a terminal group or a bridging group.

"Heteroaryl" either alone or part of a group refers to groups containing an aromatic ring (preferably a 5 or 6 membered aromatic ring) having one or more heteroatoms as ring atoms in the aromatic ring with the remainder of the ring atoms being carbon atoms. Suitable heteroatoms include nitrogen, oxygen and sulphur. Examples of heteroaryl include thiophene, benzothiophene, benzofuran, benzimidazole, benzoxazole, benzothiazole, benzisothiazole, naphtho[2,3-b]thiophene, furan, isoindolizine, xantholene, phenoxatine, pyrrole, imidazole, pyrazole, pyridine, pyrazine, pyrimidine, pyridazine, indole, isoindole, 1H-indazole, purine, quinoline, isoquinoline, phthalazine, naphthyridine, quinoxaline, cinnoline, carbazole, phenanthridine, acridine, phenazine, thiazole, isothiazole, phenothiazine, oxazole, isooxazole, furazane, phenoxazine, 2-,3- or4- pyridyl, 2-, 3-, 4-, 5-, or 8- quinolyl, 1-, 3-, 4-, or 5- isoquinolinyl1-, 2-, or 3- indolyl, and 2-, or 3-thienyl. The group may be a terminal group or a bridging group.

"Heteroarylalkyl" means a heteroaryl-alkyl group in which the heteroaryl and alkyl moieties are as previously described. Preferred heteroarylalkyl groups contain a lower alkyl moiety. Exemplary heteroarylalkyl groups include pyridylmethyl. The group may be a terminal group or a bridging group.

"Lower alkyl" as a group means unless otherwise specified, an aliphatic hydrocarbon group which may be straight or branched having 1 to 6 carbon atoms in the chain, more preferably 1 to 4 carbons such as methyl, ethyl, propyl (n-propyl or isopropyl) or butyl (n-butyl, isobutyl or tertiarybutyl). The group may be a terminal group or a bridging group.

The term "Pharmaceutically acceptable salts" refers to salts that retain the desired biological activity of the above-identified compounds, and include pharmaceutically acceptable acid addition salts and base addition salts. Suitable pharmaceutically acceptable acid addition salts of compounds of Formula (I) may be prepared from an inorganic acid or from an organic acid. Examples of such inorganic acids are hydrochloric, sulfuric, and phosphoric acid. Appropriate organic acids may be selected from aliphatic, cycloaliphatic, aromatic, heterocyclic carboxylic and sulfonic classes of organic acids, examples of which are formic, acetic, propionic, succinic, glycolic, gluconic, lactic, malic, tartaric, citric, fumaric, maleic, alkyl sulfonic, arylsulfonic. Suitable pharmaceutically acceptable base addition salts of compounds of Formula (I) include metallic salts made from lithium, sodium, potassium, magnesium, calcium, aluminium, and zinc, and organic salts made from organic bases such as choline, diethanolamine, morpholine. Other examples of organic salts are: ammonium salts, quatemary salts such as tetramethylammonium salt; amino acid addition salts such as salts with glycine and arginine. Additional information on pharmaceutically acceptable salts can be found in Remington's Pharmaceutical Sciences, 19th Edition, Mack Publishing Co., Easton, PA 1995. In the case of agents that are solids, it is understood by those skilled in the art that the inventive compounds, agents and salts may exist in different crystalline or polymorphic forms, all of which are intended to be within the scope of the present invention and specified formulae.

**BENZIMIDAZOLE BASED ANTI-CANCER AGENTS**

The compositions, methods, uses and kits of the invention as described above utilise a benzimidazole based anti-cancer agent. As used herein the term "benzimidazole based anti-cancer agent" means a chemical compound or salt thereof that has activity against one or more cancer cell lines and which contains a benzimidazole moiety in its structure.

Any suitable benzimidazole based anti-cancer agent may be used in the invention. In one embodiment the benzimidazole based anti-cancer agent is a compound of the formula (1):

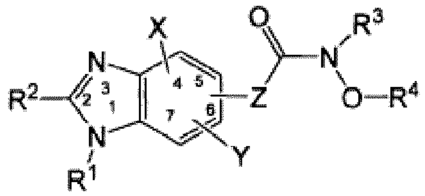

Formula (1)

wherein

$R^1$ is an optionally substituted heteroaryl group, an optionally substituted heterocycloalkyl group or a group of formula:

$$-(CR^{20}R^{21})_m-(cR^{22}R^{23})_n-(CR^{24}R^{25})_o-NR^{26}R^{27};$$

$R^2$ is selected from the group consisting of: H, alkyl, alkenyl, alkynyl, heteroalkyl, cycloalkyl, cycloalkenyl, cycloalkylalkyl, alkoxyalkyl, $R^{11}S(O)R^{13}$-, $R^{11}S(O)_2R^{13}$-, $R^{11}C(O)N(R^{12})R^{13}$-, $R^{11}SO_2N(R^{12})R^{13}$-, $R^{11}N(R^{12})C(O)R^{13}$-, $R^{11}N(R^{12})SO_2R^{13}$-, $R^{11}N(R^{12})C(O)N(R^{12})R^{13}$- and acyl, each of which may be optionally substituted;

$R^3$ is selected from the group consisting of H, $C_1$ -$C_6$ alkyl, and acyl, each of which may be optionally substituted;

X and Y are the same or different and are independently selected from the group consisting of: H, halogen, -CN, -$NO_2$, -$CF_3$, -$OCF_3$, alkyl, alkenyl, alkynyl, haloalkyl, haloalkenyl, haloalkynyl, heteroalkyl, cycloalkyl, cycloalkenyl, heterocycloalkyl, heterocycloalkenyl, aryl, heteroaryl, hydroxy, hydroxyaliryl, alkoxy, alkoxyalkyl, alkoxyaryl, alkoxyheteroaryl, alkenyloxy, alkynyloxy, cycloalkyloxy, cycloalkenyloxy, heterocycloalkyloxy, heterocycloalkenyloxy, aryloxy, heteroaryloxy, arylalkyl, heteroarylalkyl, arylalkyloxy, -amino, alkylamino, acylamino, aminoalkyl, arylamino, sulfonyl, alkylsulfonyl, arylsulfonyl, aminosulfonyl, aminoalkyl, alkoxyalky, -COOH -C(O)$OR^5$, -$COR^5$, -SH, -$SR^6$, -$OR^6$ acyl and -$NR^7R^8$, each of which may be optionally substituted;

each $R^4$ is selected from the group consisting of: H, alkyl, alkenyl, alkynyl, haloalkyl, heteroalkyl, cycloalkyl, heterocycloalkyl, aryl, heteroaryl, cycloalkylalkyl, heterocycloalkylalkyl, arylalkyl, heteroarylalkyl and acyl, each of which may be optionally substituted;

each $R^5$ is independently selected from the group consisting of: H, alkyl, alkenyl, alkynyl, haloalkyl, heteroalkyl,

cycloalkyl, heterocycloalkyl, aryl, heteroaryl, cycloalkylalkyl, heterocycloalkylalkyl, arylalkyl, heteroarylalkyl and acyl, each of which may be optionally substituted;

each $R^6$ is independently selected from the group consisting of: H, alkyl, alkenyl, alkynyl, haloalkyl, heteroalkyl, cycloalkyl, heterocycloalkyl, aryl, heteroaryl, cycloalkylalkyl, heterocycloalkylalkyl, arylalkyl, heteroarylalkyl and acyl; each of which may be optionally substituted;

each $R^7$ and $R^8$ is independently selected from the group consisting of: H, alkyl, alkenyl, alkynyl, haloalkyl, heteroalkyl, cycloalkyl, heterocycloalkyl, aryl, heteroaryl, cycloalkylalkyl, heterocycloalkylalkyl, arylalkyl, heteroarylalkyl and acyl, each of which may be optionally substituted;

each $R^{11}$ and $R^{12}$ is independently selected from the group consisting of H, alkyl, alkenyl, and alkynyl, each of which may be optionally substituted;

each $R^{13}$ is a bond or is independently selected from the group consisting of: alkyl, alkenyl, and alkynyl, each of which may be optionally substituted;

each $R^{20}$, $R^{21}$, $R^{22}$, $R^{23}$, $R^{24}$ and $R^{25}$ is independently selected from the group consisting of: H, halogen, -CN, $-NO_2$, $-CF_3$, $-OCF_3$, alkyl, alkenyl, alkynyl, haloalkyl, haloalkenyl, haloalkynyl, heteroalkyl, cycloalkyl, cycloalkenyl, heterocycloalkyl, heterocycloalkenyl, aryl, heteroaryl, cycloalkylalkyl, heterocycloalkylalkyl, arylalkyl, heteroarylalkyl, arylalkenyl, cycloalkylheteroalkyl, heterocycloalkylheteroalkyl, heteroarylheteroalkyl, arylheteroalkyl, hydroxy, hydroxyalkyl, alkoxy, alkoxyalkyl, alkoxyaryl, alkoxyheteroaryl, alkenyloxy, alkynyloxy, cycloalkylkoxy, heterocycloalkyloxy, aryloxy, arylalkyloxy, phenoxy, benzyloxy heteroaryloxy, amino, alkylamino, acylamino, aminoalkyl, arylamino, alkoxycarbonyl, alkylaminocarbonyl, sulfonyl, alkylsulfonyl, aminosulfonyl, arylsulfonyl, arylsulfinyl -COOH, $-C(O)OR^5$, $-COR^5$, -SH, $-SR^6$, $-OR^6$ and acyl, each of which may be optionally substituted; or
$R^{20}$ and $R^{21}$ when taken together may form a group of formula =O or =S, and/or
$R^{22}$ and $R^{23}$ when taken together may form a group of formula =O or =S, and/or
$R^{24}$ and $R^{25}$ when taken together may form a group of formula =O or =S;

each $R^{26}$ and $R^{27}$ is independently selected from the group consisting of is selected from the group consisting of: H, halogen, alkyl, alkenyl, alkynyl, haloalkyl, haloalkenyl, heteroalkyl, cycloalkyl, cycloalkenyl, heterocycloalkyl, heterocycloalkenyl, aryl, heteroaryl, cycloalkylalkyl, heterocycloalkylalkyl, arylalkyl, heteroarylalkyl, arylalkenyl, cycloalkylheteroalkyl, heterocycloalkylheteroalkyl, heteroarylheteroalkyl, arylheteroalkyl, hydroxy, hydroxyalkyl, alkoxy, alkoxyalkyl, alkoxyaryl, alkenyloxy, alkynyloxy, cycloalkylkoxy, heterocycloalkyloxy, aryloxy, arylalkyloxy, heteroaryloxy, amino, alkylamino, aminoalkyl, acylamino, arylamino, phenoxy, benzyloxy, COOH, alkoxycarbonyl, alkylaminocarbonyl, sulfonyl, alkylsulfonyl, alkylsulfinyl, arylsulfonyl, arylsulfinyl, aminosulfonyl, $SR^5$, and acyl, each of which may be optionally substituted, or $R^{26}$ and $R^{27}$ when taken together with the nitrogen atom to which they are attached form an optionally substituted heterocycloalkyl group;

Z is selected from the group consisting of $-CH_2-$, $-CH_2CH_2-$, -CH=CH-, $C_3$-$C_6$ alkylene, $C_3$-$C_6$ alkenylene, $C_3$-$C_6$ alkynylene, $C_3$-$C_6$ cycloalkyl, unsubstituted or substituted with one or more substituents independently selected from the group consisting of $C_1$-$C_4$ alkyl;

m, n and o are integers independently selected from the group consisting of 0, 1, 2, 3 and 4;

or a pharmaceutically acceptable salt or prodrug thereof.

In one embodiment of the compounds of formula (1) the Z moiety is at the 5 or 6 position. In a specific embodiment the Z moiety is at the 5 position. In one embodiment the Z moiety is a group of formula -CH=CH-. In one embodiment if the Z moiety is a group of this type it is in the "E" configuration.
In one embodiment of the compounds of formula (1) $R^3$ is H.
In one embodiment of the compounds of formula (1) $R^4$ is H.
In one embodiment of the compounds of formula (1) X is H.
In one embodiment of the compounds of formula (1) Y is H.
In one embodiment of the compounds of formula (1) the group $R^1$ is a group of formula:

$$-(CR^{20}R^{21})_m-(CR^{22}R^{23})_n-(CR^{24}R^{25})_o-NR^{26}R^{27};$$

in which m, n and o are integers independently selected from the group consisting of 0, 1, 2, 3 and 4.

As the values of m, n and o in the compounds of formula (1) are integers ranging from 0 to 4 the sum of m+n+o is an integer selected from the group consisting of 0, 1, 2, 3, 4, 5, 6, 7, 8, 9, 10, 11, and 12. In one embodiment the sum of m+n+o is an integer selected from the group consisting of 0, 1, 2, 3, 4, 5, 6, 7 and 8. In another embodiment the sum of m+n+o is an integer selected from the group consisting of 0, 1, 2, 3 and 4. In another embodiment the sum of m+n+o is an integer selected from the group consisting of 2 and 3.

In one embodiment of the compounds of formula (1) $R^1$ is selected from the group consisting of:

$-(CR^{20}R^{21})_2-NR^{26}R^{27}$;
$-(CR^{22}R^{23})_2-NR^{26}R^{27}$;
$-(CR^{24}R2^{25})_2-NR^{26}R^{27}$;
$-(CR^{20}R^{21})-(CR^{22}R^{23})-NR^{26}R^{27}$;
$-(CR^{20}R^{21})(CR^{24}R^{25})-NR^{26}R^{27}$;
$-(CR^{22}R^{23})-(CR^{24}R^{25})-NR^{26}R^{27}$,
$-(CR^{20}R^{21})_3-NR^{26}R^{27}$;
$-(CR^{22}R^{23})_3-NR^{26}R^{27}$;
$-(CR^{24}R^{25})_3-NR^{26}R^{27}$;
$-(CR^{20}R^{21})_2-(CR^{22}R^{23})-NR^{26}R^{27}$;
$-(CR^{20}R^{21})_2-(CR^{24}R^{25})-NR^{26}R^{27}$;
$-(CR^{20}R^{21})-(CR^{22}R^{23})_2-NR^{26}R^{27}$;
$-(CR^{22}R^{23})_2-(CR^{24}R^{25})-NR^{26}R^{27}$;
$-(CR^{20}R^{21})-(CR^{24}R^{25})_2-NR^{26}R^{27}$;
$-(CR^{22}R^{23})-(CR^{24}R^{25})_2-NR^{26}R^{27}$; and
$-(CR^{20}R^{21})-(CR^{22}R^{23})-(CR^{24}R^{25})-NR^{26}R^{27}$.

In one specific embodiment the compound of formula (1) has the formula:

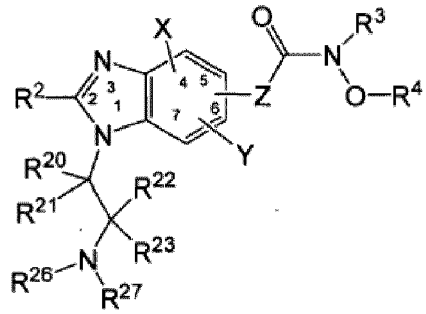

In another specific embodiment the compound of formula (1) has the formula:

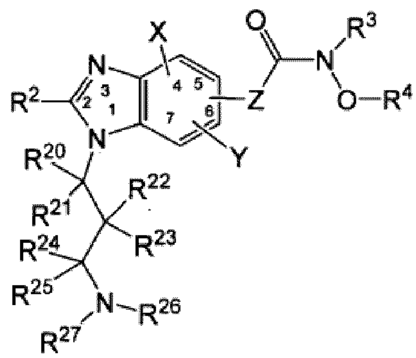

In each of the above embodiments of the compounds of formula (1) $R^{20}$ and $R^{21}$ may represent a number of different variables. In one embodiment $R^{20}$ and $R^{21}$ are independently selected from the group consisting of H, alkyl, alkenyl and alkynyl. In another embodiment $R^{20}$ and $R^{21}$ are independently selected from the group consisting of H and alkyl. In yet another embodiment $R^{20}$ and $R^{21}$ are independently selected from the group consisting of H, methyl, ethyl,

isopropyl, propyl, 2-ethyl-propyl, 3,3-dimethyl-propyl, butyl, isobutyl, 3,3-dimethyl-butyl, 2-ethyl-butyl, pentyl, 2-methyl, pentyl, pent-4-enyl, hexyl, heptyl and octyl. In a specific embodiment $R^{20}$ and $R^{21}$ are both H.

In each of the above embodiments of the compounds of formula (1) $R^{22}$ and $R^{23}$ may represent a number of different variables. In one embodiment $R^{22}$ and $R^{23}$ are independently selected from the group consisting of H, alkyl, alkenyl and alkynyl. In another embodiment $R^{22}$ and $R^{23}$ are independently selected from the group consisting of H and alkyl. In yet another embodiment $R^{22}$ and $R^{23}$ are independently selected from the group consisting of H, methyl, ethyl, isopropyl, propyl, 2-ethyl-propyl, 3,3-dimethyl-propyl, butyl, isobutyl, 3,3-dimethyl-butyl, 2-ethyl-butyl, pentyl, 2-methyl, pentyl, pent-4-enyl, hexyl, heptyl and octyl. In a further embodiment $R^{22}$ and $R^{23}$ are independently selected from the group consisting of alkyl. In a most specific embodiment $R^{22}$ and $R^{23}$ are both methyl.

In each of the above embodiments of the compounds of formula (1) $R^{24}$ and $R^{25}$ may represent a number of different variables. In one embodiment $R^{24}$ and $R^{25}$ are preferably independently selected from the group consisting of H, alkyl, alkenyl and alkynyl. In another embodiment $R^{24}$ and $R^{25}$ are independently selected from the group consisting of H and alkyl. In yet another embodiment $R^{24}$ and $R^{25}$ are independently selected from the group consisting of H, methyl, ethyl, isopropyl, propyl, 2-ethyl-propyl, 3,3-dimethyl-propyl, butyl, isobutyl, 3,3-dimethyl-butyl, 2-ethyl-butyl, pentyl, 2-methyl, pentyl, pent-4-enyl, hexyl, heptyl and octyl. In a specific embodiment $R^{24}$ and $R^{25}$ are both H.

In each of the above embodiments of the compounds of formula (1) there are a number of values for $R^{26}$ and $R^{27}$. In one embodiment $R^{26}$ and $R^{27}$ are independently selected from the group consisting of: H, alkyl, alkenyl, alkynyl, alkoxyalkyl, and acyl. In another embodiment $R^{26}$ and $R^{27}$ are independently selected from the group consisting of: H, alkyl and acyl. In a further embodiment $R^{26}$ and $R^{27}$ are independently selected from the group consisting of H, methyl, ethyl, isopropyl, propyl, 2-ethyl-propyl, 3,3-dimethyl-propyl, butyl, isobutyl, 3,3-dimethyl-butyl, 2-ethyl-butyl, pentyl, 2-methyl, pentyl, pent-4-enyl, hexyl, heptyl, octyl, acetyl and 2-methoxy-ethyl.

In another embodiment of the compounds of formula (1) $R^1$ is a group selected from the group consisting of:

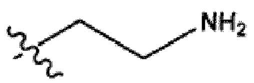
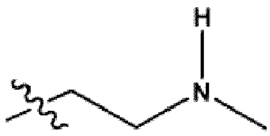
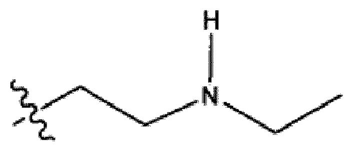
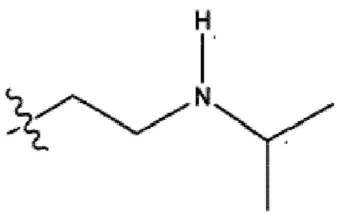
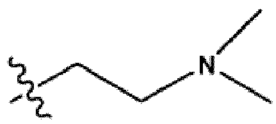
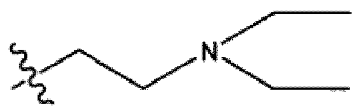
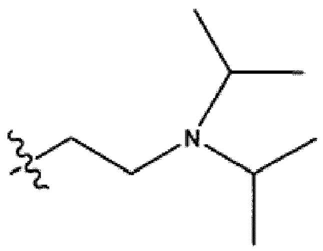
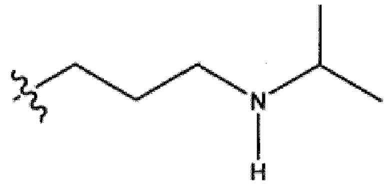
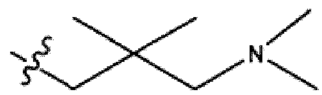
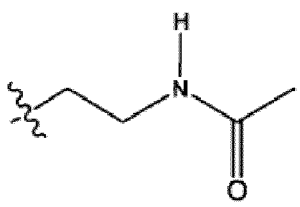
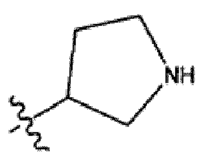
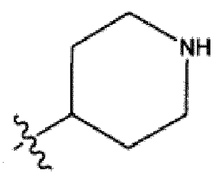
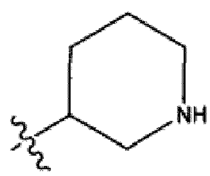

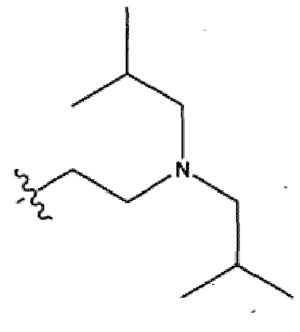

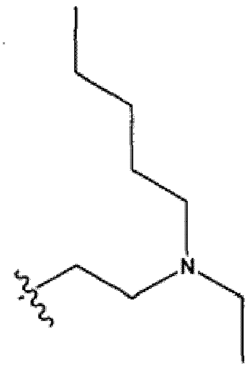

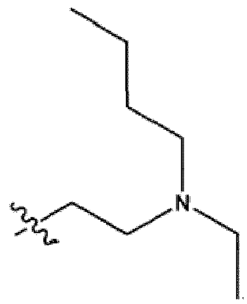

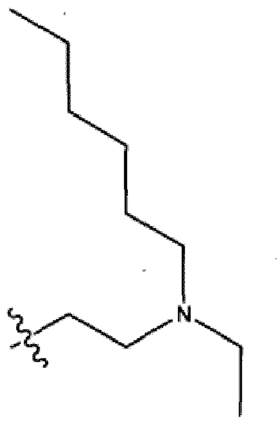

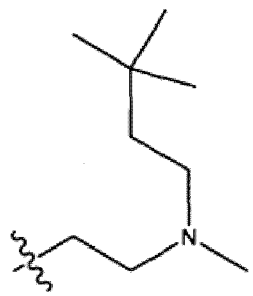

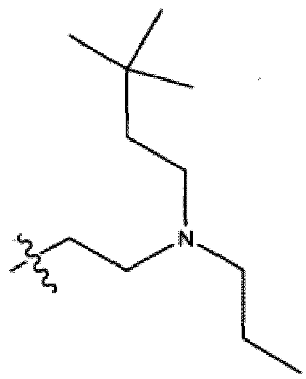

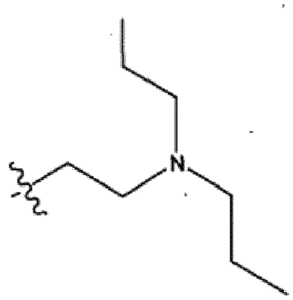

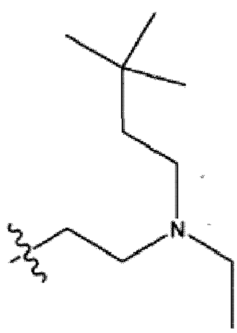

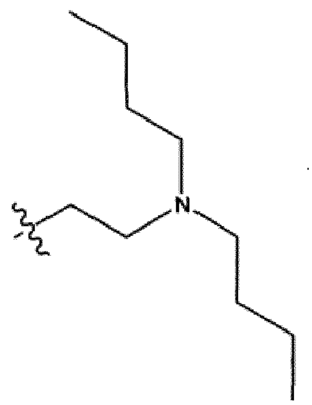

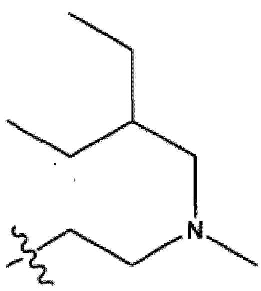

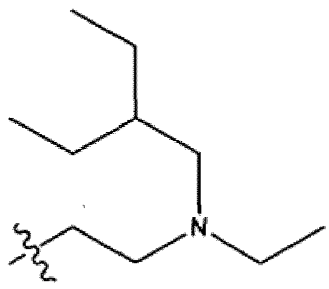

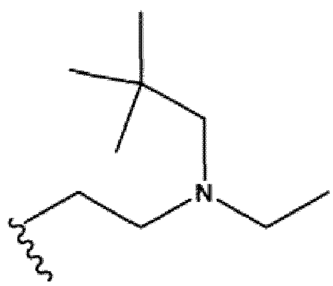

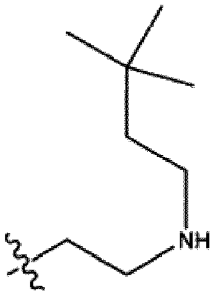

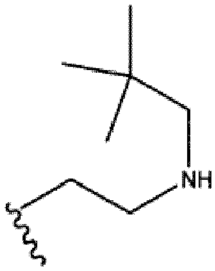

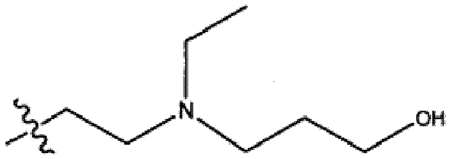

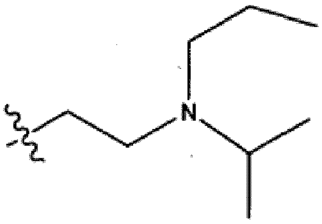

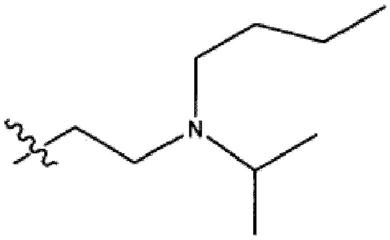

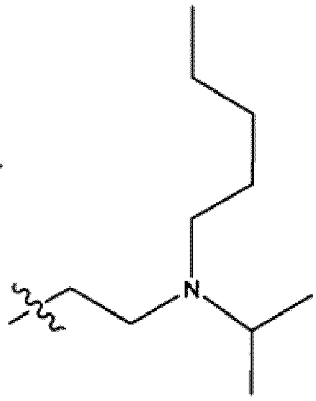

In one specific embodiment $R^1$ is a group of formula:

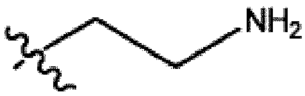

In another specific embodiment $R^1$ is a group of formula:

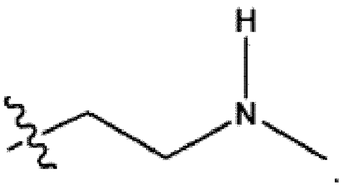

In another specific embodiment $R^1$ is a group of formula:

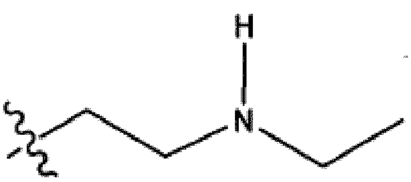

In yet another specific embodiment $R^1$ is a group of formula:

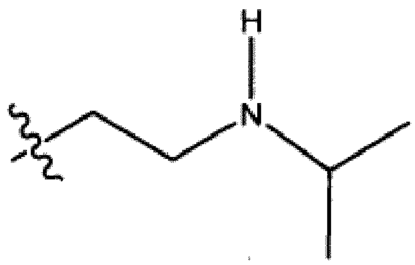

In another specific embodiment $R^1$ is a group of formula:

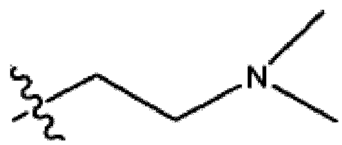

In another specific embodiment $R^1$ is a group of formula:

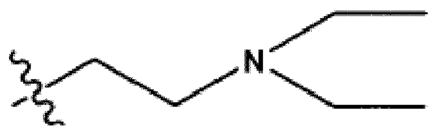

In another specific embodiment $R^1$ is a group of formula:

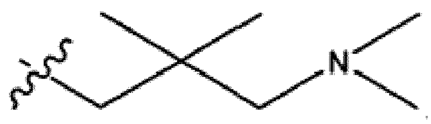

In another specific embodiment $R^1$ is a group of formula:

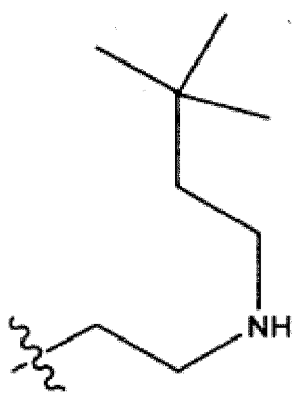

In another specific embodiment $R^1$ is a group of formula:

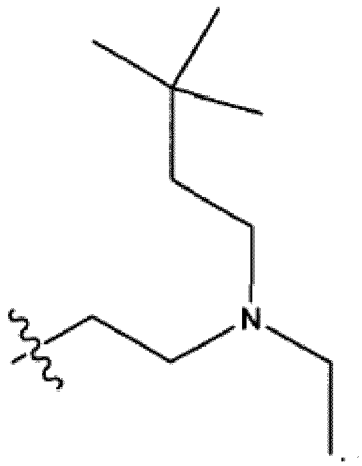

In one embodiment of the compounds of formula (1) $R^2$ is selected from the group consisting of H, alkyl, cycloalkyl, heteroalkyl, alkenyl, alkynyl, alkoxyalkyl and cycloalkylalkyl, each of which may be optionally substituted.

In one form of this embodiment $R^2$ is alkyl. In one embodiment the alkyl is a $C_1$-$C_{10}$ alkyl. In another form of this embodiment the alkyl is a $C_1$-$C_6$ alkyl group. In another form of this embodiment $R^2$ is selected from the group consisting of: methyl; ethyl; propyl; 2-methyl-propyl, 2-2-dimethyl-propyl; isopropyl; 3,3,3-triflouro-propyl; butyl; isobutyl; 3,3-dimethyl-butyl; pentyl; 2,4,4-trimethyl-pentyl; hexyl; heptyl, octyl, nonyl, and 2-methoxy nonyl.

In one form of this embodiment $R^2$ is alkenyl. In one form of this embodiment the alkenyl is a $C_1$-$C_{10}$ alkenyl. In another form of this embodiment the alkenyl is a $C_1$-$C_6$ alkenyl group. In another form of this embodiment $R^2$ is selected from the group consisting of: ethenyl, prop-1-enyl, prop-2-enyl, but-1-enyl, but-2-enyl but-3-enyl, pent-1-enyl, pent-2-enyl, pent-3-enyl, pent-4-enyl, hex-1-enyl, hex-2-enyl, hex-3-enyl, hex-4-enyl and hex-5-enyl.

In another embodiment of the compounds of formula (1) $R^2$ is selected from the group consisting of $R^{11}S(O)R^{13}$-, $R^{11}S(O)_2R^{13}$-, $R^{11}C(O)N(R^{12})R^{13}$-, $R^{11}SO_2N(R^{12})R^{13}$-, $R^{11}N(R^{12})C(O)R^{13}$-, $R^{11}N(R^{12})SO_2R^{13}$-, and $R^{11}N(R^{12})C(O)N(R^{12})R^{13}$-. In one form of this embodiment $R^2$ is a group of the formula $R^{11}C(O)N(R^{12})R^{13}$-. In one form of this embodiment $R^{13}$ is a $C_1$-$C_6$ alkyl. In a specific form of this embodiment $R^{13}$ is methyl or ethyl. In one form of this embodiment $R^{12}$ is H or $C_1$-$C_6$alkyl. A specific value for $R^{12}$ is H. In one form of this embodiment $R^{11}$ is $C_1$-$C_6$ alkyl group. Specific values for $R^{11}$ include t-butyl and propyl. Specific examples of groups of this type include: $(CH_3)_3CCH_2CONH(CH_2)_2$-; $(CH_3)_3CCONH(CH_2)_2$-; $(CH_3)_3CCONH(CH_2)$- and $CH_3(CH_2)_2CONH(CH_2)$-.

Specific values of $R^2$ are selected from the group consisting of: H; methyl; ethoxymethyl; [Bicyclo[2.2.1]2-ylmethyl; Adamantan-2-ylmethyl; 2-methansulfanyl-ethyl; 2,2,2-triflouro-ethyl; propyl; 2-2-dimethyl-propyl; isopropyl; 3,3,3-triflouro-propyl; butyl; isobutyl; 3,3-dimethyl-butyl; but-3-enyl; but-3-yny; pentyl; 2,4,4-trimethyl-pentyl; Bicyclo[2.2.1]hept-5-en-2yl; hexyl; hex-3-enyl; octyl; non-3-enyl; non-6-enyl; 2-methoxy-nonyl, 2-phenyl-cyclopropyl; cyclohexyl; $(CH_3)_3CCH_2CONH(CH_2)_2$-; $(CH_3)_3CCONH(CH_2)_2$-; $(CH_3)_3CCONH(CH_2)$- and $CH_3(CH_2)_2CONH(CH_2)$-.

In one embodiment of the compounds of formula (1) $R^5$ is $C_1$-$C_4$ alkyl, heteroalkyl, or acyl. A specific value for $R^5$ is methyl.

In one embodiment of the compounds of formula (1) $R^6$ is $C_1$-$C_4$ alkyl, heteroalkyl or acyl. A specific value for $R^6$ is $C_1$-$C_4$ alkyl.

In one embodiment of the compounds of formula (1) $R^7$ and $R^8$ are selected from the group consisting of H, $C_1$-$C_6$ alkyl, $C_4$-$C_9$cycloalkyl, $C_4$-$C_9$heterocycloalkyl, aryl, heteroaryl, arylalkyl, and heteroarylalkyl.

Many if not all of the variables discussed above for the compounds of formula (1) may be optionally substituted. If the variable is optionally substituted then in one embodiment the optional substituent is selected from the group consisting of: halogen, =O, =S, -CN, $-NO_2$, $-CF_3$, $-OCF_3$, alkyl, alkenyl, alkynyl, haloalkyl, haloalkenyl, haloalkynyl, heteroalkyl, cycloalkyl, cycloalkenyl, heterocycloalkyl, heterocycloalkenyl, aryl, heteroaryl, hydroxy, hydroxyalkyl, alkoxy, alkoxyalkyl, alkoxyaryl, alkoxyheteroaryl, alkenyloxy, alkynyloxy, cycloalkyloxy, cycloalkenyloxy, heterocycloalkyloxy, heterocycloalkenyloxy, aryloxy, heteroaryloxy, arylalkyl, heteroarylalkyl, arylalkyloxy, -amino, alkylamino, acylamino, aminoalkyl, arylamino, sulfonyl, alkylsulfonyl, arylsulfonyl, aminosulfonyl, aminoalkyl, alkoxyalky, -COOH, $-COR^5$, $-C(O)OR^5$, -SH, $-SR^5$, $-OR^6$and acyl.

In a further embodiment the optional substituents are selected from the group consisting of: halogen, =O, =S, -CN, $-NO_2$, alkyl, alkenyl, heteroalkyl, haloalkyl, alkynyl, aryl, cycloalkyl, heterocycloalkyl, heteroaryl, hydroxy, hydroxyalkyl, alkoxy, alkylamino, aminoalkyl, acylamino, phenoxy, alkoxyalkyl, benzyloxy, alkylsulfonyl, arylsulfonyl, aminosulfonyl, $-C(O)OR^5$, COOH, SH, and acyl.

In one embodiment the benzimidazole based anti-cancer agent is selected from the group consisting of:

- (E)-3-[1-(3-Dimethylamino-2,2-dimethyl-propyl)-2-isopropyl-1H-benzimidazol-5-yl]-N-hydroxy-acrylamide
- (E)-3-[2-Butyl-1-(3-dimethylamino-2,2-dimethyl-propyl)-1H-benzimidazol-5-yl]-N-hydroxy-acrylamide
- (E)-3-[1-(3-Dimethylamino-2,2-dimethyl-propyl)-2-(2-methylsulfanyl-ethyl)-1H-benzimidazol-5-yl]-N-hydroxy-acrylamide
- (E)-3-[1-(3-Dimethylamino-2,2-dimethyl-propyl)-2-ethoxymethyl-1H-benzimidazo1-5-yl]-N-hydroxy-acrylamide
- (E)-3-[1-(3-Dimethylamino-2,2-dimethyl-propyl)-2-isobutyl-1H-benzimidazol-5-yl]-N-hydroxy-acrylamide
- (E)-3-[1-(2-Diethylamino-ethyl)-2-isobutyl-1H-benzimidazo1-5-yl]-N-hydroxy-acrylamide
- (E)-3-[2-Butyl-1-(2-diethylamino-ethyl)-1H-benzimidazo1-5-yl]-N-hydroxy-acrylamide
- (E)-3-[2-But-3-ynyl-1-(3-dimethylamino-2,2-dimethyl-propyl)-1H-benzimidazol-5-yl]-N-hydroxy-acrylamide
- (E)-3-[2-But-3-enyl-1-(3-dimethylamino-2,2-dimethyl-propyl)-1H-benzimidazol-5-yl]-N-hydroxy-acrylamide
- (E)-3-[2-But-3-enyl-1-(2-diethylamino-ethyl)-1H-benzimidazol-5-yl]-N-hydroxy-acrylamide
- (E)-3-[2-But-3-ynyl-1-(2-diethylamino-ethyl)-1H-benzimidazol-5-yl]-N-hydroxy-acrylamide
- (E)-3-[1-(3-Dimethylamino-2,2-dimethyl-propyl)-2-(3,3,3-trifluoro-propyl)-1H-benzimidazol-5-yl]-N-hydroxy-acrylamide
- (E)-3-[1-(2-Diethylamino-ethyl)-2-(3,3,3-trifluoro-propyl)-1H-benzimidazol-5-yl]-N-hydroxy-acrylamide
- (E)-3-[1-(2-Diethylamino-ethyl)-2-ethoxymethyl-1H-benzimidazol-5-yl]-N-hydroxy-acrylamide
- (E)-3-[1-(3-Dimethylamino-2,2-dimethyl-propyl)-2-methyl-1H-benzimidazol-5-yl]-N-hydroxy-acrylamide
- (E)-3-[1-(2-Diethylamino-ethyl)-2-(2,2-dimethyl-propyl)-1H-benzimidazol-5-yl]-N-hydroxy-acrylamide
- (E)-N-Hydroxy-3-[1-(3-isopropylamino-propyl)-2-(3,3,3-trifluoro-propyl)-1H-benzimidazol-5-yl]-acrylamide
- (E)-3-[2-(2,2-Dimethyl-propyl)-1-(2-isopropylamino-ethyl)-1H-benzimidazol-5-yl]-N-hydroxy-acrylamide
- (E)-3-[1-(2-Diisopropylamino-ethyl)-2-(2,2-dimethyl-propyl)-1H-benzimidazol-5-yl]-N-hydroxy-acrylamide
- (E)-3-[1-(2-Diisopropylamino-ethyl)-2-isobutyl-1H-benzimidazol-5-yl]-N-hydroxy-acrylamide
- (E)-3-[1-(3-Dimethylamino-2,2-dimethyl-propyl)-2-hex-3-enyl-1H-benzimidazol-5-yl]-N-hydroxy-acrylamide
- (E)-3-[1-(3-Dimethylamino-2,2-dimethyl-propyl)-2-(2,4,4-trimethyl-pentyl)-1H-benzimidazol-5-yli-N-hydroxy-acrylamide
- (E)-3-[2-Cyclohexyl-1-(3-dimethylamino-2,2-dimethyl-propyl)-1H-benzimidazol-5-yl]-N-hydroxy-acrylamide
- (E)-3-[2-Bicyclo[2.2.1]hept-5-en-2-yl-1-(3-dimethylamino-2,2-dimethyl-propyl)-1H-benzimidazol-5-yl]-N-hydroxy-acrylamide
- (E)-3-[1-(2-Diethylamino-ethyl)-2-hex-3-enyl-1H-benzimidazol-5-yl]-N-hydroxy-acrylamide
- (E)-3-[1-(2-Diisopropylamino-ethyl)-2-hex-3-enyl-1H-benzimidazol-5-yl]-N-hydroxy-acrylamide
- (E)-3-[2-Hex-3-enyl-1-(2-isopropylamino-ethyl)-1H-benzimidazol-5-yl]-N-hydroxy-acrylamide
- (E)-3-[2-Hex-3-enyl-1-(3-isopropylamino-propyl)-1H-benzimidazol-5-yl]-N-hydroxy-acrylamide
- (E)-3-[1-(2-Ethylamino-ethyl)-2-hex-3-enyl-1H-benzimidazol-5-yl]-N-hydroxy-acrylamide
- (E)-3-[1-(2-Diethylamino-ethyl)-2-hexyl-1H-benzimidazol-5-yl]-N-hydroxy-acrylamide
- (E)-N-Hydroxy-3-[1-(3-isopropylamino-propyl)-2-(2,4,4-trimethyl-pentyl)-1H-benzimidazol-5-yl]-acrylamide
- (E)-3-[2-(2,2-Dimethyl-propyl)-1-(3-isopropylamino-propyl)-1H-benzimidazol-5-yl]-N-hydroxy-acrylamide
- (E)-3-[1-(2-Diisopropylamino-ethyl)-2-(3,3,3-trifluoro-propyl)-1H-benzimidazol-5-yl]-N-hydroxy-acrylamide
- (E)-N-Hydroxy-3-[2-isobutyl-1-(2-isopropylamino-ethyl)-1H-benzimidazol-5-yl]-acrylamide
- (E)-3-[2-(2,2-Dimethyl-propyl)-1-(2-ethylamino-ethyl)-1H-benzimidazol-5-yl]-N-hydroxy-acrylamide
- (E)-3-[1-(2-Ethylamino-ethyl)-2-isobutyl-1H-benzimidazol-5-yl]-N-hydroxy-acrylamide
- (E)-3-[1-(2-Diisopropylamino-ethyl)-2-(2,4,4-trimethyl-pentyl)-1H-benzimidazol-5-yl]-N-hydroxy-acrylamide
- (E)-N-Hydroxy-3-[1-(2-isopropylamino-ethyl)-2-(2,4,4-trimethyl-pentyl)-1H-benzimidazol-5-yl]-acrylamide
- (E)-3-[1-(2-Ethylamino-ethyl)-2-(2,4,4-trimethyl-pentyl)-1H-benzimidazol-5-yl]-N-hydroxy-acrylamide
- (E)-3-[1-(2-Diethylamino-ethyl)-2-(2,4,4-trimethyl-pentyl)-1H-benzimidazol-5-yl]-N-hydroxy-acrylamide
- (E)-3-[1-(2-Diethylamino-ethyl)-2-propyl-1H-benzimidazol-5-yl]-N-hydroxy-acrylamide
- (E)-3-[2-Butyl-1-(2-diisopropylamino-ethyl)-1H-benzimidazol-5-yl]-N-hydroxy-acrylamide
- (E)-3-[2-Butyl-1-(2-ethylamino-ethyl)-1H-benzimidazol-5-yl]-N-hydroxy-acrylamide
- (E)-3-[1-(2-Diethylamino-ethyl)-2-(2-methylsulfanyl-ethyl)-1H-benzimidazol-5-yl]-N-hydroxy-acrylamide
- (E)-3-[2-Butyl-1-(2-isopropylamino-ethyl)-1H-benzimidazol-5-yl]-N-hydroxy-acrylamide
- (E)-3-[2-Butyl-1-(3-isopropylamino-propyl)-1H-benzimidazol-5-yl]-N-hydroxy-acrylamide
- (E)-3-[1-(1-Benzyl-piperidin-4-yl)-2-butyl-1H-benzimidazol-5-yl]-N-hydroxy-acrylamide
- (E)-3-[2-But-3-enyl-1-(2-ethylamino-ethyl)-1H-benzimidazol-5-yl]-N-hydroxy-acrylamide
- (E)-3-[2-Hexyl-1-(2-isopropylamino-ethyl)-1H-benzimidazol-5-yl]-N-hydroxy-acrylamide
- (E)-3-[1-(2-Dimethylamino-ethyl)-2-(2,4,4-trimethyl-pentyl)-1H-benzimidazol-5-yl]-N-hydroxy-acrylamide
- (E)-3-[1-(2-Ethylamino-ethyl)-2-hexyl-1H-benzimidazol-5-yl]-N-hydroxy-acrylamide
- (E)-N-Hydroxy-3-[1-(2-isopropylamino-ethyl)-2-(3,3,3-trifluoro-propyl)-1H-benzimidazol-5-yl]-acrylamide
- (E)-3-[1-(2-Dimethylamino-ethyl)-2-hex-3-enyl-1H-benzimidazol-5-yl]-N-hydroxy-acrylamide
- (E)-3-[1-(2-Amino-ethyl)-2-(2,4,4-trimethyl-pentyl)-1H-benzimidazol-5-yl]-N-hydroxy-acrylamide

- (E)-3-[1-(2-Amino-ethyl)-2-(2-methoxy-nonyl)-1H-benzimidazol-5-yl]-N-hydroxy-acrylamide
- (E)-3-[2-Butyl-1-(2-dimethylamino-ethyl)-1H-benzimidazol-5-yl]-N-hydroxy-acrylamide
- (E)-3-[1-(2-Dimethylamino-ethyl)-2-hexyl-1H-benzimidazol-5-yl]-N-hydroxy-acrylamide
- (E)-N-{2-[1-(2-Diethylamino-ethyl)-5-(2-hydroxycarbamoyl-vinyl)-1H-benzimidazol-2-yl]-ethyl}-3,3-dimethyl-butyramide
- (E)-3-{1-(2-Diethylamino-ethyl)-2-[2-(2,2-dimethyl-propionylamino)-ethyl]-1H-benzimidazol-5-yl}-N-hydroxy-acrylamide
- (E)-3-{1-(2-Diethylamino-ethyl)-2-[(2,2-dimethyl-propionylamino)-methyl]-1H-benzimidazol-5-yl}-N-hydroxy-acrylamide
- (E)-N-[1-(2-Diethylamino-ethyl)-5-(2-hydroxycarbamoyl-vinyl)-1H-benzimidazol-2-ylmethyl]-butyramide
- (E)-3-[1-(2-ethylamino-ethyl)-2-(3,3-dimethyl-butyl)-1H-benzimidazol-5-yl]-N-hydroxy-acrylamide
- (E)-3-[2-(3,3-Dimethyl-butyl)-1-(2-Dimethylamino-ethyl)-1H-benzimidazol-5-yl]-N-hydroxy-acrylamide
- (E)-3-[1-(2-Dimethylamino-ethyl)-2-pentyl-1H-benzimidazol-5-yl]-N-hydroxy-acrylamide
- (E)-3-[1-(2-Dimethylamino-ethyl)-2-(2,2,2-trifluoro-ethyl)-1H-benzimidazol-5-yl]-N-hydroxy-acrylamide
- (E)-N-Hydroxy-3-[1-(5-methyl-1H-pyrazol-3-yl)-2-(2,4,4-trimethyl-pentyl)-1H-benzimidazol-5-yl]-acrylamide
- (E)-3-[1-(2-Ethylamino-ethyl)-2-pentyl-1H-benzimidazol-5-yl]-N-hydroxy-acrylamide
- (E)-3-(2-Butyl-1-pyrrolidin-3-yl-1H-benzimidazol-5-yl)-N-hydroxy-acrylamide
- (E)-3-(2-Butyl-1-piperidin-4-yl-1H-benzimidazol-5-yl)-N-hydroxy-acrylamide
- (E)-N-Hydroxy-3-[1-(2-isopropylamino-ethyl)-2-pentyl-1H-benzimidazol-5-yl]-acrylamide
- (E)-N-Hydroxy-3-[1-(2-methylamino-ethyl)-2-non-3-enyl-1H-benzimidazol-5-yl]-acrylamide
- (E)-N-Hydroxy-3-[1-(2-methylamino-ethyl)-2-non-6-enyl-1H-benzimidazol-5-yl]-acrylamide
- (E)-3-[2-Hexyl-1-(2-methylamino-ethyl)-1H-benzimidazol-5-yl]-N-hydroxy-acrylamide
- (E)-N-Hydroxy-3-[1-(2-methylamino-ethyl)-2-pentyl-1H-benzimidazol-5-yl]-acrylamide
- (E)-N-Hydroxy-3-[1-(2-methylamino-ethyl)-2-octyl-1H-benzimidazol-5-yl]-acrylamide
- (E)-3-[1-(2-Amino-ethyl)-2-octyl-1H-benzimidazol-5-yl]-N-hydroxy-acrylamide
- (E)-3-{2-Butyl-1-[2-(isopropyl-methyl-amino)-ethyl]-1H-benzimidazol-5-yl}-N-hydroxy-acrylamide
- (E)-3-{1-[2-(Ethyl-methyl-amino)-ethyl]-2-pentyl-1H-benzimidazol-5-yl}-N-hydroxy-acrylamide
- (E)-3-(2-Hexyl-1-pyrrolidin-3-yl-1H-benzimidazol-5-yl)-N-hydroxy-acrylamide
- (E)-3-[2-Butyl-1-(1-methyl-pyrrolidin-3-yl)-1H-benzimidazol-5-yl]-N-hydroxy-acrylamide
- (E)-3-(2-Butyl-1-piperidin-3-yl-1H-benzimidazol-5-yl)-N-hydroxy-acrylamide
- (E)-3-(2-Hexyl-1-piperidin-3-yl-1H-benzimidazol-5-yl)-N-hydroxy-acrylamide
- (E)-3-(1-{2-[Ethyl-(2-methoxy-ethyl)-amino]-ethyl}-2-pentyl-1H-benzimidazol-5-yl)-N-hydroxy-acrylamide
- (E)-3-{2-Butyl-1-[2-(ethyl-methyl-amino)-ethyl]-1H-benzimidazol-5-yl}-N-hydroxy-acrylamide
- (E)-N-Hydroxy-3-[1-(1-methyl-piperidin-3-yl)-2-pentyl-1H-benzimidazol-5-yl]-acrylamide
- (E)-3-{1-[2-(Ethyl-hexyl-amino)-ethyl]-1H-benzimidazol-5-yl}-N-hydroxy-acrylamide
- (E)-3-{1-[2-(Ethyl-pentyl-amino)-ethyl]-1H-benzimidazol-5-yl}-N-hydroxy-acrylamide
- (E)-3-{1-[2-(Ethyl-heptyl-amino)-ethyl]-1H-benzimidazol-5-yl}-N-hydroxy-acrylamide
- (E)-3-{2-Hexyl-1-[1-(2-hydroxy-ethyl)-piperidin-3-yl]-1H-benzimidazol-5-yl}-N-hydroxy-acrylamide
- (E)-3-(2-Butyl-1-{2-[ethyl-(3-hydroxy-propyl)-amino]-ethyl}-1H-benzimidazol-5-yl)-N-hydroxy-acrylamide
- (E)-3-(1-{2-[Ethyl-(3-hydroxy-propyl)-amino]-ethyl}-2-pentyl-1H-benzimidazol-5-yl)-N-hydroxy-acrylamide
- (E)-N-hydroxy-3-(1-(1-phenethylpyrrolidin-3-yl)-1H-benzo[d]imidazol-5-yl)acrylamide
- (E)-N-hydroxy-3-(1-(1-pentylpiperidin-3-yl)-1H-benzo[d]imidazol-5-yl)acrylamide
- (E)-3-{1-[2-(Butyl-ethyl-amino)-ethyl]-1H-benzimidazol-5-yl}-N-hydroxy-acrylamide
- (E)-N-hydroxy-3-(1-(1-phenethylpiperidin-3-yl)-1H-benzo[d]imidazol-5-yl)acrylamide
- (E)-N-hydroxy-3-(1-(1-(3-phenylpropyl)piperidin-3-yl)-1H-benzo[d]imidazol-5-yl)acrylamide
- (E)-N-hydroxy-3-(1-(1-(3-phenylpropyl)pyrrolidin-3-yl)-1H-benzo[d]imidazol-5-yl)acrylamide
- (E)-3-{1-(3,3-Dimethyl-butyl)-pyrrolidin-3-yl]-1H-benzimidazol-5-yl}-N-hydroxy-acrylamide
- (E)-3-(1-(2-(diethylamino)ethyl)-)ethyl)-1H-benzo[d]imidazol-5-yl)-N-hydroxyacrylamide
- (E)-3-[2-(4-Cyano-butyl)-1-(2-diethylamino-ethyl)-1H-benzimidazol-5-yl]-N-hydroxy-acrylamide
- (E)-3-(1-(1-butylpiperidin-3-yl)-1H-benzo[d]imidazol-5-yl)-N-hydroxyacrylamide
- (E)-N-hydroxy-3-(1-(1-(pent-4-enyl)piperidin-3-yl)-1H-benzo[d]imidazol-5-yl)acrylamide
- (E)-3-(1-(1-(3,3-dimethylbutyl)piperidin-4-yl)-1H-benzo[d]imidazol-5-yl)-N-hydroxyacrylamide
- (E)-3-[1-(2-Diethylamino-ethyl)-2-propylamino-1H-benzimidazol-5-yl]-N-hydroxy-acrylamide
- (E)-N-hydroxy-3-(1-(2-(isopropyl(propyl)amino)ethyl)-1H-benzo[d]imidazol-5-yl)acrylamide
- (E)-3-{1-[2-(Butyl-isopropyl-amino)-ethyl]-1H-benzimidazol-5-yl}-N-hydroxy-acrylamide
- N-Hydroxy-3-{1-[2-(isopropyl-pentyl-amino)-ethyl]-1H-benzimidazol-5-yl}-acrylamide
- (E)-3-[2-(5-Cyano-pentyl)-1-(2-diethylamino-ethyl)-1H-benzimidazol-5-yl]-N-hydroxy-acrylamide
- (E)-3-(1-{2-[(3,3-Dimethyl-butyl)-ethyl-amino]-ethyl}-1H-benzimidazol-5-yl)-N-hydroxy-acrylamide

- (E)-3-{1-[2-(Ethyl-propyl-amino)-ethyl]-1H-benzimidazol-5-yl}-N-hydroxy-acrylamide
- (E)-N-Hydroxy-3-(1-{2-[isopropyl-(2-methyl-pentyl)-amino]-ethyl}-1H-benzimidazol-5-yl)-acrylamide
- (E)-N-hydroxy-3-(1-(2-(isopropyl(4,4,4-trifluorobutyl)amino)ethyl)-1H-benzo[d]imidazol-5-yl)acrylamide
- (E)-3-[1-(3-Dimethylamino-2,2-dimethyl-propyl)-2-propylamino-1H-benzimidazol-5-yl]-N-hydroxy-acrylamide
- (E)-3-{1-[2-(Ethyl-hexyl-amino)-ethyl]-2-methyl-1H-benzimidazol-5-yl}-N-hydroxy-acrylamide
- (E)-3-{1-[2-(Butyl-ethyl-amino)-ethyl]-2-trifluoromethyl-1H-benzimidazol-5-yl}-N-hydroxy-acrylamide
- (E)-3-{1-[2-(Ethyl-hexyl-amino)-ethyl]-2-trifluoromethyl-1H-benzimidazol-5-yl}-N-hydroxy-acrylamide
- (E)-3-(1-(2-(dibutylamino)ethyl)-2-propyl-1H-benzo[d]imidazol-5-yl)-N-hydroxyacrylamide
- (E)-3-[1-(2-Dipropylamino-ethyl)-1H-benzimidazol-5-yl]-N-hydroxy-acrylamide
- (E)-N-Hydroxy-3-(1-{2-[isopropyl-(3-methyl-butyl)-amino]-ethyl}-1H-benzimidazol-5-yl)-acrylamide
- (E)-3-(1-{2-[(3,3-Dimethyl-butyl)-methyl-amino]-ethyl}-1H-benzimidazol-5-yl)-N-hydroxy-acrylamide
- (E)-3-(1-{2-[(2-Ethyl-butyl)-methyl-amino]-ethyl}-1H-benzimidazol-5-yl)-N-hydroxy-acrylamide
- (E)-3-(1-(2-(bis(3,3-dimethylbutyl)amino)ethyl)-1H-benzo[d]imidazol-5-yl)-N-hydroxyacrylamide
- (E)-3-(1-(2-(diisobutylamino)ethyl)-1H-benzo[d]imidazol-5-yl)-N-hydroxyacrylamide
- (E)-3-{1-[2-(3,3-Dimethyl-butylamino)-ethyl]-1H-benzimidazol-5-yl}-N-hydroxy-acrylamide
- (E)-N-Hydroxy-3-{1-[2-(methyl-pent-4-enyl-amino)-ethyl]-1H-benzimidazol-5-yl}-acrylamide
- (E)-3-(1-{2-[(3,3-Dimethyl-butyl)-propyl-amino]-ethyl}-1H-benzimidazol-5-yl)-N-hydroxy-acrylamide
- (E)-3-[1-(3-Dimethylamino-2,2-dimethyl-propyl)-2-methylsulfanyl-1H-benzimidazol-5-yl]-N-hydroxy-acrylamide
- (E)-3-{1-[2-(3,3-Dimethyl-butylamino)-ethyl]-2-propyl-1H-benzimidazol-5-yl}-N-hydroxy-acrylamide
- (E)-3-[1-[2-(3,3-Dimethyl-butylamino)-ethyl]-2-(2,2-dimethyl-propyl)-1H-benzimidazol-5-yl]-N-hydroxy-acrylamide
- (E)-3-[1-{2-[Bis-(3,3-dimethyl-butyl)-amino]-ethyl}-2-(2,2-dimethyl-propyl)-1H-benzimidazol-5-yl]-N-hydroxy-acrylamide
- (E)-3-{1-[2-(2,2-Dimethyl-propylamino)-ethyl]-1H-benzimidazol-5-yl}-N-hydroxy-acrylamide
- (E)-3-(1-{2-[(2,2-Dimethyl-propyl)-propyl-amino]-ethyl}-1H-benzimidazol-5-yl)-N-hydroxy-acrylamide
- (E)-3-{1-[2-(3,3-Dimethyl-butylamino)-ethyl]-2-ethyl-1H-benzimidazol-5-yl)-N-hydroxy-acrylamide
- (E)-3-(1-{2-[(3,3-Dimethyl-butyl)-methyl-amino]-ethyl}-2-propyl-1H-benzimidazol-5-yl)-N-hydroxy-acrylamide
- (E)-3-(1-{2-[(3,3-Dimethyl-butyl)-(2,2,2-trifluoro-ethyl)-amino]-ethyl}-1H-benzimidazol-5-yl)-N-hydroxy-acrylamide
- (E)-3-(1-{2-[Butyl-(2,2,2-trifluoro-ethyl)-amino]-ethyl}-1H-benzimidazol-5-yl)-N-hydroxy-acrylamide

or a pharmaceutically acceptable salt thereof.

In one embodiment of the invention the benzimidazole based anti-cancer agent used in the compositions, methods uses and kits of the invention is a compound of the formula:

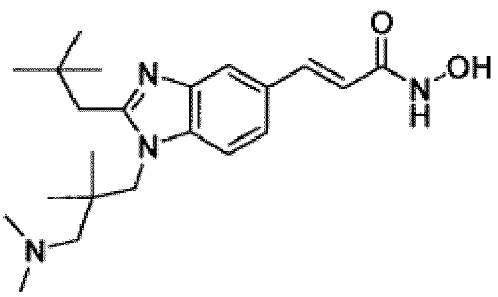

or a pharmaceutically acceptable salt thereof.

In another embodiment of the invention the benzimidazole based anti-cancer agent used in the compositions, methods uses and kits of the invention is a compound of the formula:

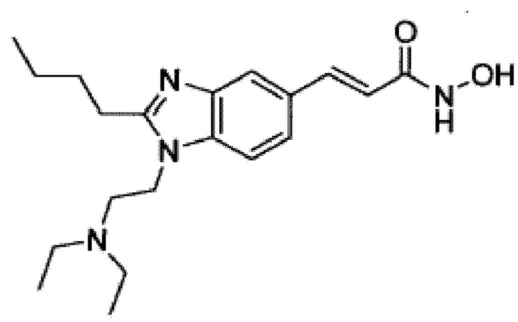

or a pharmaceutically acceptable salt thereof.

In another embodiment of the invention the benzimidazole based anti-cancer agent used in the compositions, methods uses and kits of the invention is a compound of the formula:

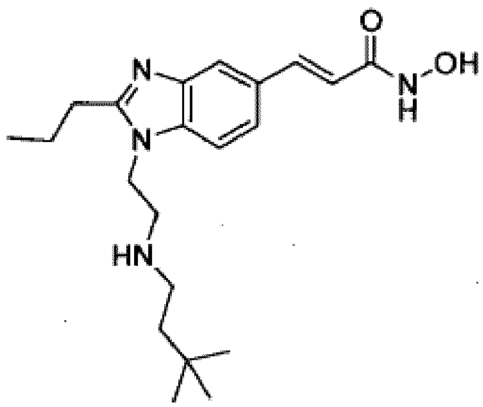

or a pharmaceutically acceptable salt thereof.

In another embodiment of the invention the benzimidazole based anti-cancer agent used in the compositions, methods uses and kits of the invention is a compound of the formula:

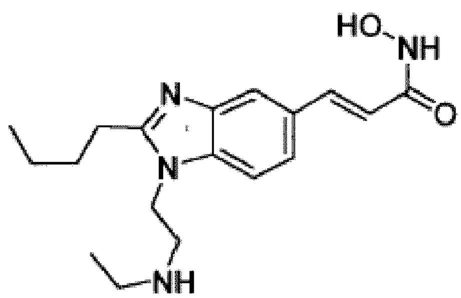

or a pharmaceutically acceptable salt thereof.

**ANTI-CANCER AGENTS**

In addition to the benzimidazole based anti-cancer agents as described above the compositions, methods, uses and kits of the invention utilise a second anti-cancer agent and may also utilse a further anti-cancer agent. In general the second anti-cancer agent and the further anti-cancer agent may be any substance that has anti-cancer activity against one or more cancer cell lines. A number of anti-cancer agents that demonstrate this activity are known in the art and a skilled addressee would readily be able to identify a large number of potential anti-cancer agents that could be used as the second or further anti-cancer agents.

Examples of suitable anti-cancer agents may be selected from the group consisting of alkylating agents, antimetabolites, anti-tumour antibiotics, mitotic spindle agents, topoisomerase inhibitors, steroid and hormonal agents, monoclonal antibodies, proteasome inhibitors, thalidomide and its analogs, death receptor ligands, kinase inhibitors,

anti-angiogenesis agents, HDAC inhibitors, synthetic zinc finger transcription factors, interferons, platelet growth factors, photosensitizers, hypomethylating agents, rapalogs and mTor inhibitors, analogs thereof, derivatives thereof, and pharmaceutically acceptable salts thereof.

Examples of suitable anti-cancer agents which may be used in the present invention as the second anti-cancer agent or the further anti-cancer agent include agents selected from the following groups and subgroups:

Alkylating agents, for example:

- ➢ Platinum based compounds such as Cisplatin, Oxaliplatin, Carboplatin, Satraplatin
- ➢ General alkylators such as Amsacrine, Busulfan, Chlorambucil and Cyclophosphamide

Antimetabolites, for example:

- ➢ Thymidylate synthetase inhibitors such as Fluorouracil (5-FU)
- ➢ Ribonucleotide reductase inhibitors such as Gemcitabine
- ➢ Dihydrofolate reductase inhibitors such as Methotrexate
- ➢ 5'Adenosylmethionine decarboxylase inhibitors such as Mitoguazone ➢ Adenine deaminase inhibitors such as Pentostatin
- ➢ Purine antimetabolites such as Thioguanine

Antitumor-antibiotics, for example:

- ➢ DNA intercalators such as Doxorubicin (Adriamycin), Actinomycin D, Daunorubicin, Bleomycin and Epirubicin

Mitotic spindle agents, for example:

- ➢ Vinca alkaloids such as Vinblastine, Vincristine, Vinorelbin and Vindesine
- ➢ Taxane derived compounds such as Paclitaxel and Docetaxel

Topoisomerase inhibitors, for example:

- ➢ Topoisomerase I inhibitors such as Irinotecan and Topotecan
- ➢ Topoisomerase II inhibitors such as Etoposide and Teniposide

Steroid and Hormonal agents, for example:

- ➢ Aromatase inhibitors such as Anastrozole and Letrozole
- ➢ Anti-estrogens such as Tamoxifen and Toremifene
- ➢ Estrogens such as Diethylstilbestrol (DES)
- ➢ Androgens such as Methyltestosterone and Fluoxymesterone
- ➢ Anti-androgens such as Flutamide, Nilutamide and Bicalutamide
- ➢ Corticosteroids such as prednisone

Monoclonal antibodies, for example:

- ➢ Anti-CD29 antibodies such as Rituximab (Rituxan) and Tositumomab
- ➢ Anti-Her2 antibodies such as Trastuzumab (Herceptin)
- ➢ Anti-EGFR antibodies such as Cetuximab and Bevacizumab

- Anti-CD33 antibodies such as Gemtuzumab

Proteasome inhibitors such as Bortezomib (Velcade)
Thalidomide and analogs such as lenalidomide (Revlimid)
Death receptor ligands such as Fas and TRAIL
Kinase inhibitors, for example:

- EGFR inhibitors such as Gefitinib (Iressa), Erlotinib (Tarceva)
- RAF kinase inhibitors such as Sorafenib (Nexavar)
- MEK kinase inhibitors such as CI-1040
- C-Abl inhibitors such as Imatinib (Gleevac)
- CDK2 inhibitors such as Flavopyridol
- VGFR inhibitors such as ZD6474, Sorafenib (Nexavar)

Anti-angiogenesis agents such as Sorafenib, Sunitinib (Sutent), bevacizumab (Avastin) HDAC inhibitors such as SAHA, LBH589 and PXD101
Synthetic Zinc-finger transcription factors
Interferons
Platelet growth factors
Photosensitizers such as Aminolevulinic acid
Hypomethylating agents such as decitabine (Dacogen) and 5-azacitidine (Vidaza) Rapalogs and mTor inhibitors such as Temsirolimus (Torisel) and Everolimus

Further examples of suitable anti-cancer agents from various therapeutic groups are selected from the group consisting of Cyclophosphamide, Ifosfamide, Melphalan, Carmustine, Fotemustine, Lomustine, Streptozocin, Carboplatin, Oxaliplatin, Satraplatin, Busulfan, Dacarbazine, Mechlorethamine, Procarbazine, Temozolomide, ThioTepa, Uramustine, Aminopterin, Methotrexate, Pemetrexed (Alimta), Raltitrexed, Cladribine, Clofarabine, Fludarabine, Mercaptopurine, Thioguanine, Capecitabine, Cytarabine, Gemcitabine, docetaxel, Paclitaxel, Vincristine, Vindesine, Vinorelbine, Dexamethasone, Daunorubicin, Doxorubicin, Epirubicin, Idararubicin, Mitoxantrone, Valrubicin, Bleomycin, Hydroxyurea, Mitomycin, Actinomycin D, Irinotecan, Topotecan, Etoposide, adrenocorticosteroids, androgens, Tamoxifen, Alemtuzumab, Bevacizumab, Cetuximab, Gemtuzumab, Panitumab, Tositumomab, Trastuzumab, Dasatinib, Erlotinib, Gefitinib, Imatinib, Lapatinib, Nilotinib, Sorafenib, Sunitinib, Vandetanib, Alitretinoin, Altretamine, Anagrelide, Asparaginase, Bexarotene, Bortezomib, Estramustine, Hydroxycarbamide, Angiostatin, Endostatin, Pentostatin, Masoprocol, Mitotane, Pegasaparase, Estramustine, Levamisole, Octreotide, Suramin, Hexamethylmelamine, Anagrelide, Anastrozole, Letrozole, L-Asparaginase, Levamisole, 5-Azadeoxycytidine, decitabine, Fas, TRAIL, Velcade, Tretoin, derivatives thereof, analogs thereof, pharmaceutically acceptable salts thereof and combinations thereof.

Specific examples of suitable anti-cancer agents include agents selected from the group consisting of Adriamycin, avastin, Cisplatin, Oxaliplatin, Carboplatin, Satroplatin, Gemcitabine, Dasatinib, decitabine (Dacogen), Docetaxel, Doxorubicin, hydroxydaunarubicin, Dexamethason, Chlorambucil, Fludarabine, Erlotinib (Tarceva), Iressa, Alimta, Melphalan, 5-Azacytidine (Vidaza), 5-FU (5-Fluorouracil), Actinomycin D, Paclitaxel, prednisone, Vinblastine, lenalidomide (Revlimid), Irinotecan, Topotecan, Etoposide, Tamoxifen, Rituximab (Rituxan), Herceptin, Cetuximab, sorafenib, sunitinib, Angiostatin, Endostatin, temsirolimus, everolimus, Bevacizumab (Avastin), derivatives thereof, analogs thereof, pharmaceutically acceptable salts thereof and combinations thereof.

In one specific embodiment of the method the second anti-cancer agent is Dasatinib or a pharmaceutically acceptable salt thereof. In one specific embodiment of the method the second anti-cancer agent is Erlotinib or a pharmaceutically acceptable salt thereof. In one specific embodiment of the method the second anti-cancer agent is Doxorubicin or a pharmaceutically acceptable salt thereof. In one specific embodiment of the method the second anti-cancer agent is 5-Fluorouracil or a pharmaceutically acceptable salt thereof. In one specific embodiment of the method the second anti-cancer agent is Oxaliplatin. In one specific embodiment of the method the second anti-cancer agent is Rituximab or a pharmaceutically acceptable salt thereof. In one specific embodiment the second anti-cancer agent is Satraplatin. In one specific embodiment the second anti-cancer agent is Vinblastine or a pharmaceutically acceptable salt thereof. In one specific embodiment the second anti-cancer agent is irinotecan or a pharmaceutically acceptable salt thereof. In one specific embodiment the second anti-cancer agent is docetaxel or a pharmaceutically acceptable

salt thereof. In one specific embodiment the anti-cancer agent is lenalidomide or a pharmaceutically acceptable salt thereof. In one specific embodiment the second anti-cancer agent is sorafenib or a pharmaceutically acceptable salt thereof. In one specific embodiment the second anti-cancer agent is decitabine or a pharmaceutically acceptable salt thereof. In one specific embodiment the second anti-cancer agent is 5-azacytidine or a pharmaceutically acceptable salt thereof. In one specific embodiment the second anti-cancer agent is temsirolemus or a pharmaceutically acceptable salt thereof. In one specific embodiment the second anti-cancer agent is everolimus or a pharmaceutically acceptable salt thereof. In one specific embodiment the second anti-cancer agent is sunitinib (Sutent) or a pharmaceutically acceptable salt thereof. In one specific embodiment the second anti-cancer agent is gemcitabine or a pharmaceutically acceptable salt thereof. In one specific embodiment the second anti-cancer agent is prednisone or a pharmaceutically acceptable salt thereof. In one specific embodiment the second anti-cancer agent is gefitinib (Iressa) or a pharmaceutically acceptable salt thereof. In one specific embodiment the second anti-cancer agent is erlotinib (Tarceva) or a pharmaceutically acceptable salt thereof. In one specific embodiment the second anti-cancer agent is avastin or a pharmaceutically acceptable salt thereof. In one specific embodiment the second anti-cancer agent is irinotecan or a pharmaceutically acceptable salt thereof.

In addition to the benzimidazole based anti-cancer agents and the second anti-cancer agents as described above the compositions, methods, uses and kits of the invention may utilise a further anti-cancer agent. This may be the case, for example, where the pharmaceutical compositions contains three active anti-cancer agents or where the treatment regime involves administration of a benzimidazole based anti-cancer agent and a plurality of other anti-cancer agents.

The further anti-cancer agent may be selected from any suitable group of anti-cancer agents as discussed above. In one embodiment the further anti-cancer agent is selected from the group consisting of Adriamycin, avastin, Cisplatin, Oxaliplatin, Carboplatin, Satraplatin, Gemcitabine, Dasatinib, decitabine (Dacogen), Docetaxel, Doxorubicin, hydroxydaunarubicin, Dexamethasone, Chlorambucil, Fludarabine, Erlotinib (Tarceva), Iressa, Alimta, Melphalan, 5-Azacytidine (Vidaza), 5-FU (5-Fluorouracil), Actinomycin D, Paclitaxel, prednisone, Vinblastine, lenalidomide (Revlimid), Irinotecan, Topotecan, Etoposide, Tamoxifen, Rituximab (Rituxan), Herceptin, Cetuximab, sorafenib, Angiostatin, Endostatin, temsirolimus, everolimus, Bevacizumab (Avastin), derivatives thereof, analogs thereof, combinations thereof and pharmaceutically acceptable salt thereof. In cases where a further anti-cancer agent is used it is typically chosen such that its mode of action is complementary to both the benzimidazole based anti-cancer agent and the second anti-cancer agent.

In general it is considered that a skilled worker in the field would be familiar with the structure and potential uses of the anti-cancer agents discussed above. Nevertheless in order to remove any uncertainty the base structure (i.e the non-salt form) of many of the agents is given below in order to assist the reader. In general the structures shown depict the basic structure of the anti-cancer agent. In many instances these are administered in the salt form (not shown). A skilled reader will readily be able to determine from the literature the appropriate salt form to use with any particular anti-cancer agent.

Chlorambucil

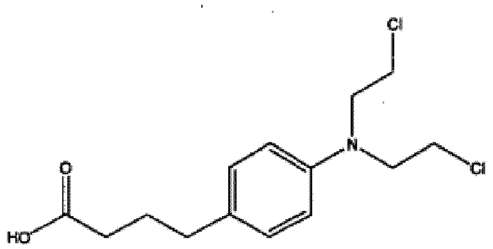

Tretoin

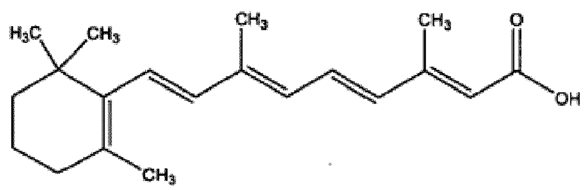

Cyclophosphamide

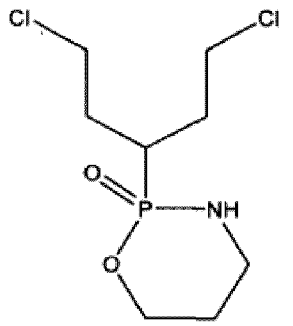

Ifosfamide

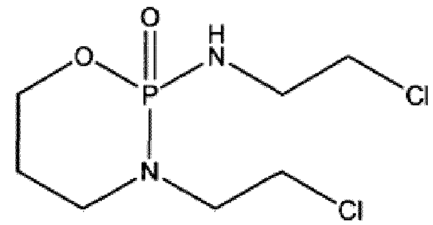

Melphalan

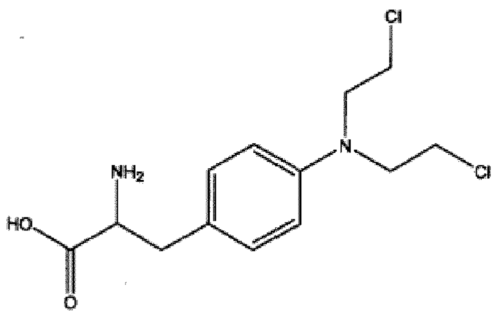

Carmustine

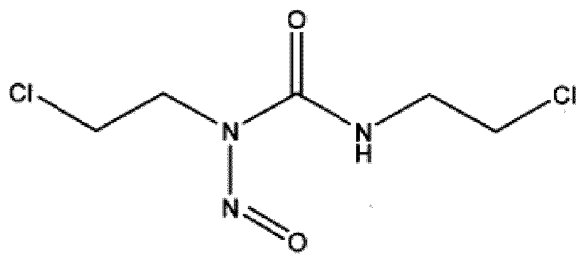

Fotemustine

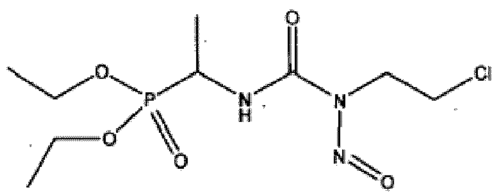

Lomustine

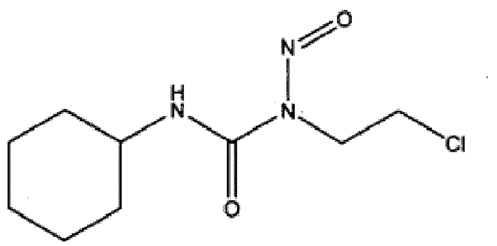

Streptozocin

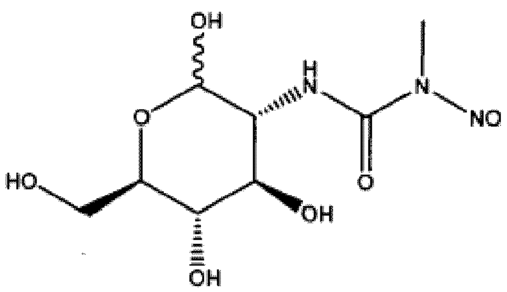

Carboplatin

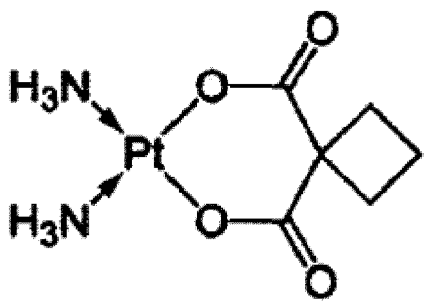

Cisplatin

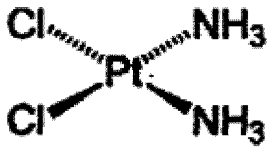

Oxaliplatin

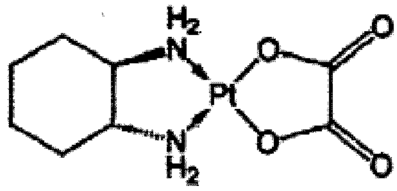

Busulfan

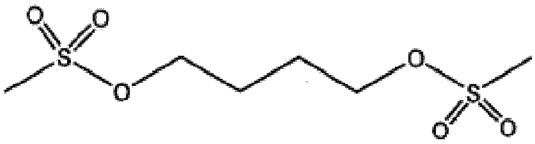

Dacarbazine

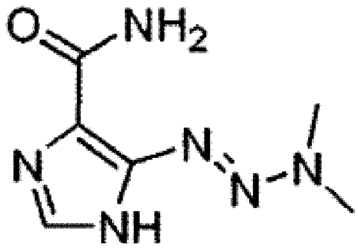

Mechlorethamine

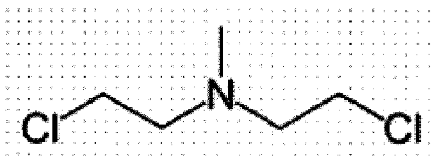

Procarbazine

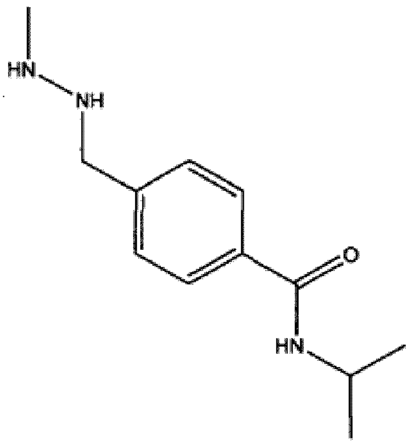

Temozolomide

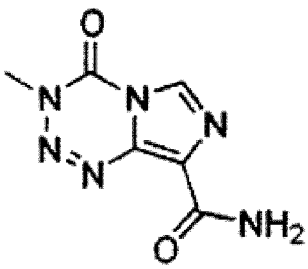

ThioTepa

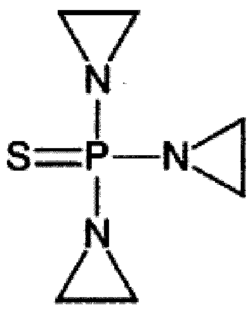

Uramustine

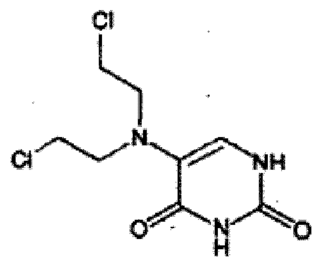

Aminopterin

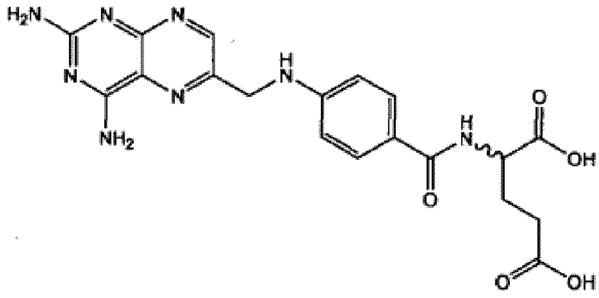

Methotrexate

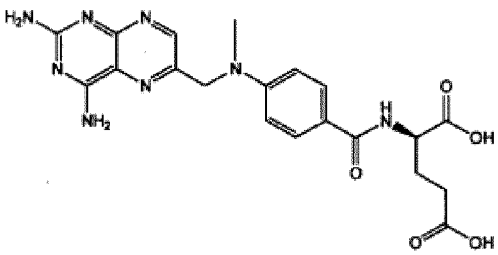

Pemetrexed

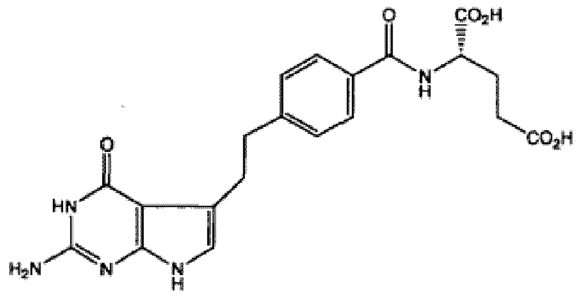

Raltitrexed

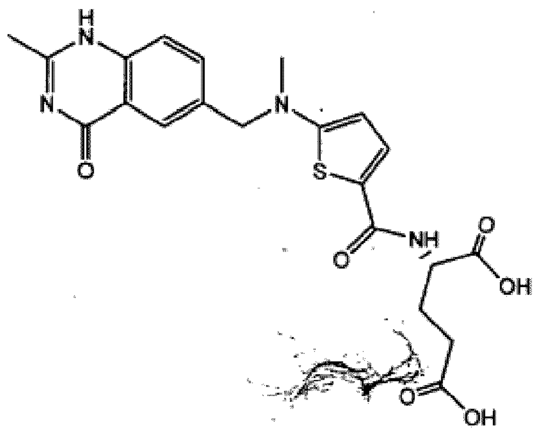

Cladribine

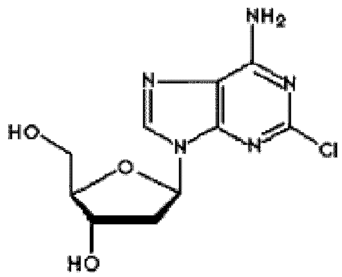

Clofarabine

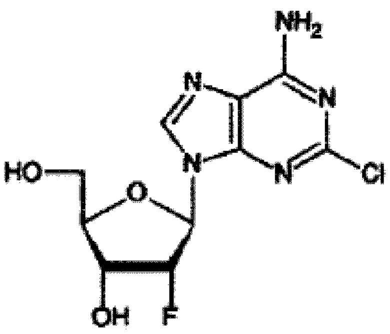

Fludarabine

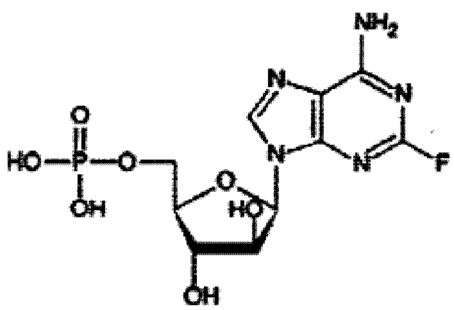

**Mercaptopurine**

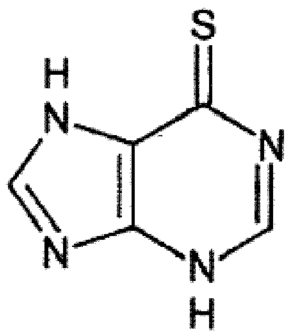

**Thioguanine**

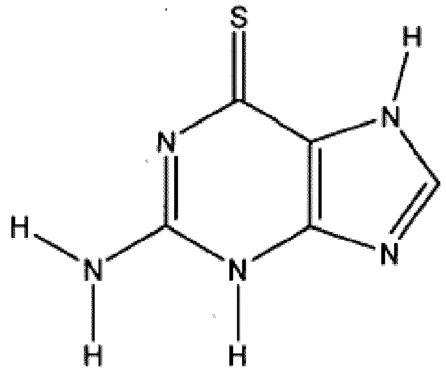

**Capecitabine**

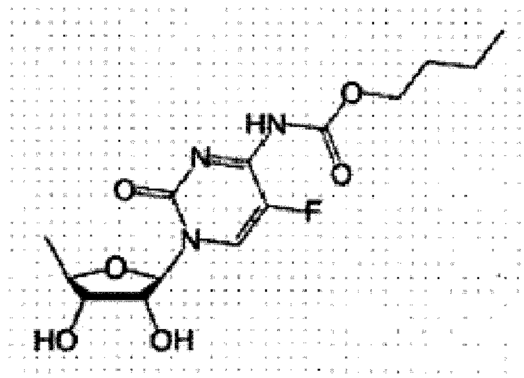

**Cytarabine**

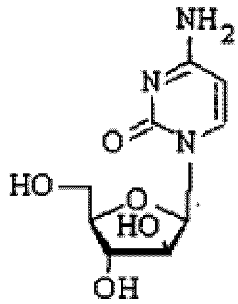

**Fluorouracil**

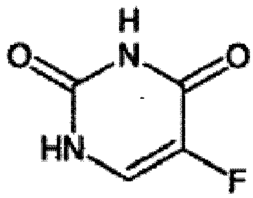

**Gemcitabine**

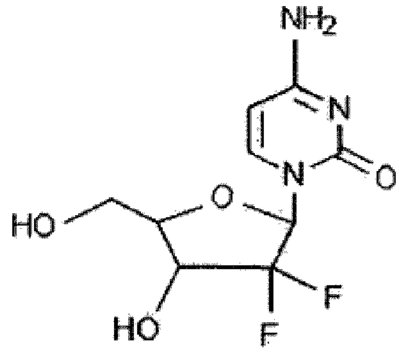

**Docetaxel**

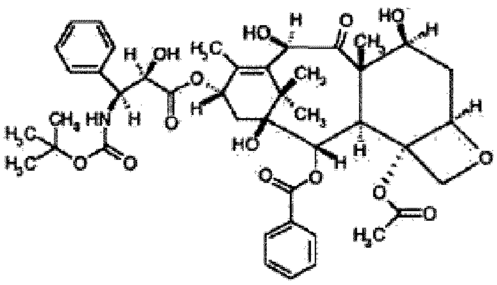

**Paclitaxel**

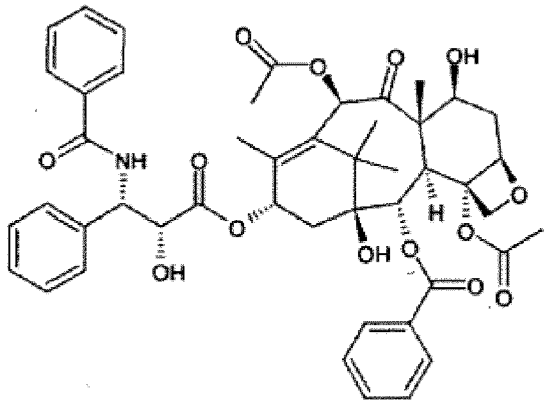

**Vindesine**

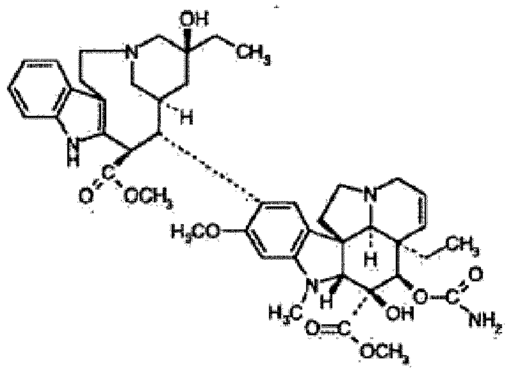

**Vinorelbine**

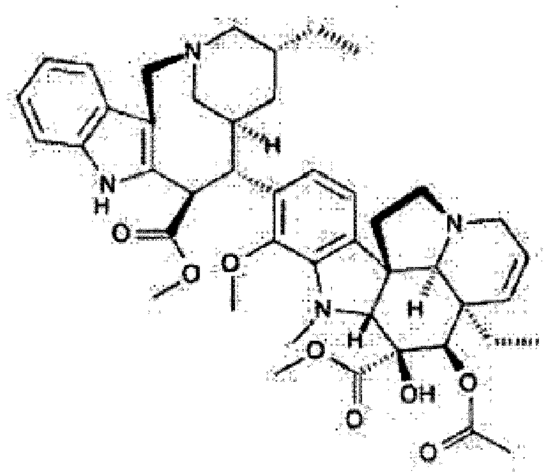

**Daunorubicin**

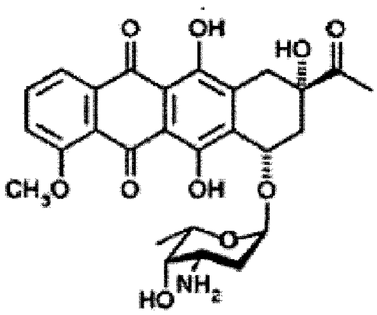

**Doxorubicin**

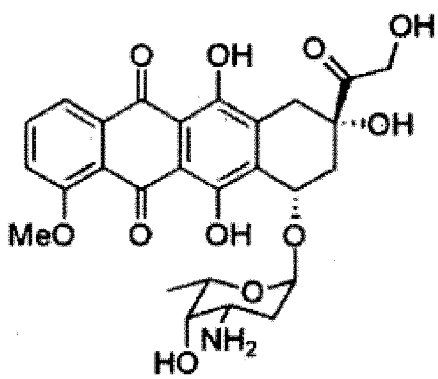

Epirubicin

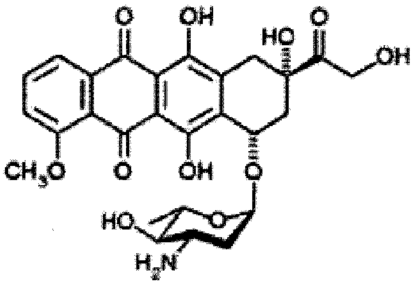

Idarubicin

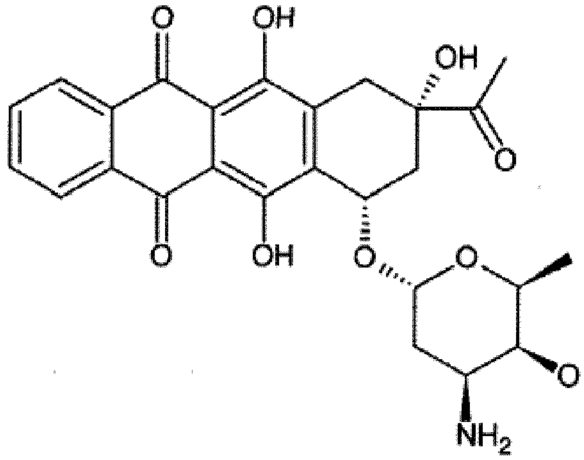

Mitoxantrone

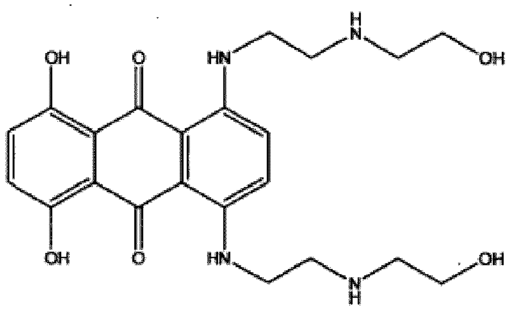

Valrubicin

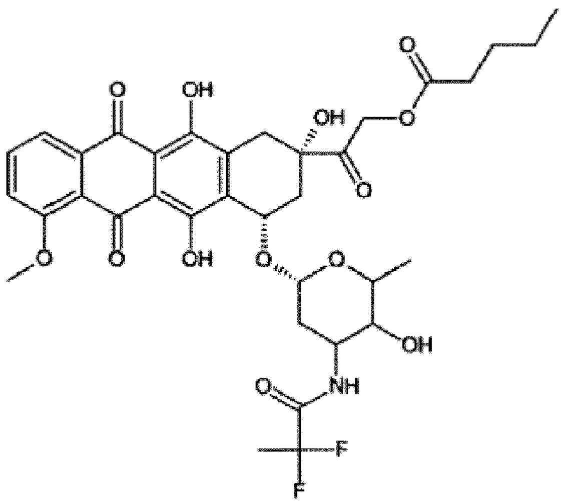

Bleomycin

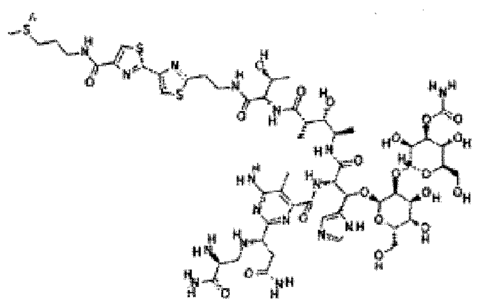

Mitomycin

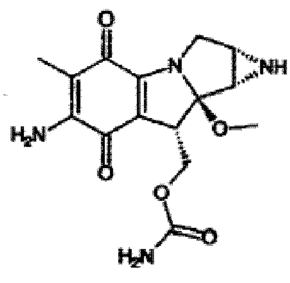

Hydroxyurea

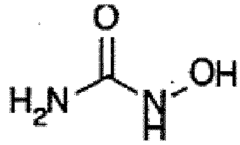

Satraplatin

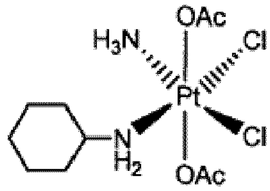

5-Azacytidine

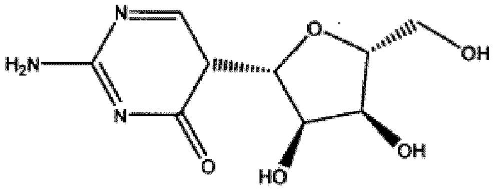

Dexamethasone

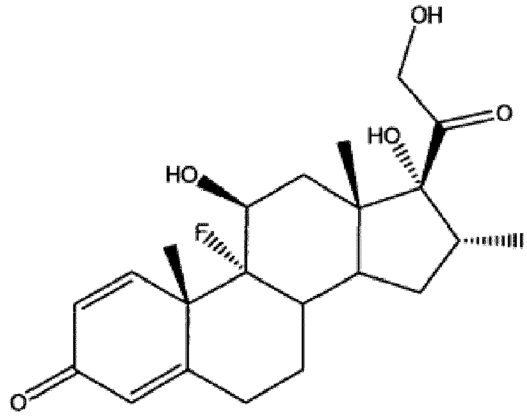

Actinomycin

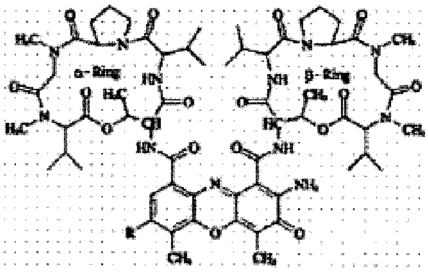

Irinotecan

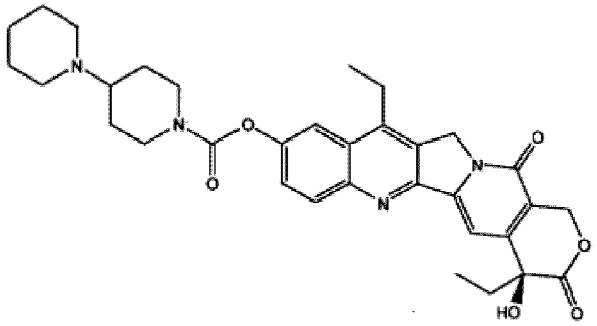

**Topotecan**

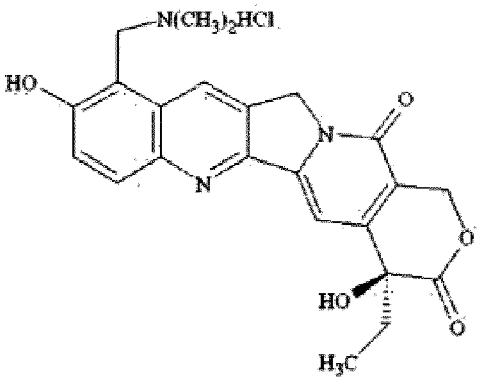

**Etoposide,**

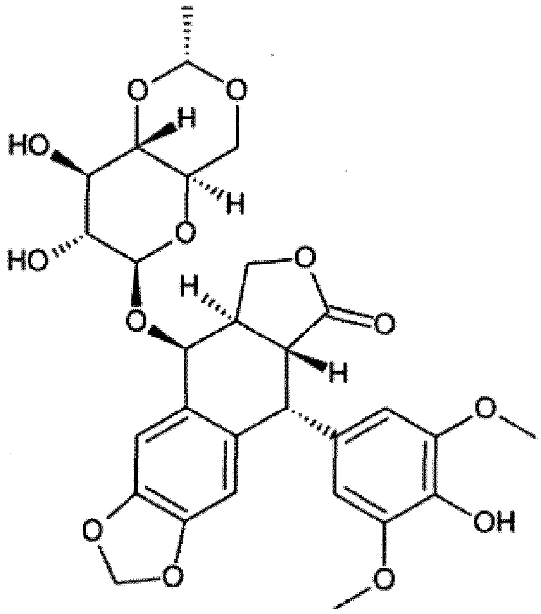

**Tamoxifen**

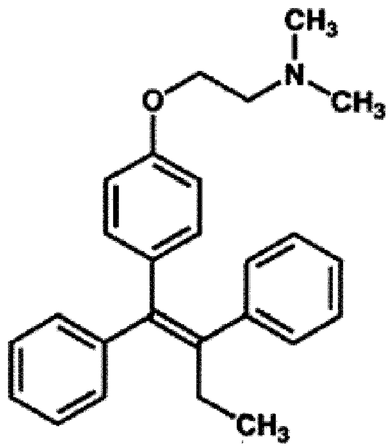

**Dasatinib**

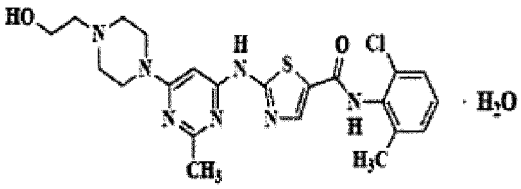

**Erlotinib**

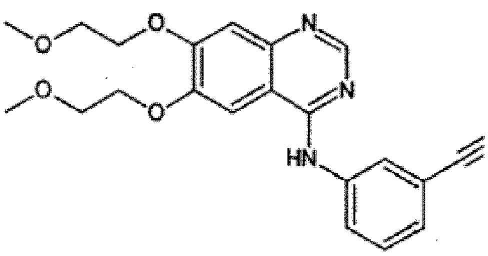

**Gefitinib**

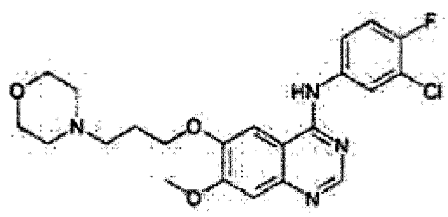

**Imatinib**

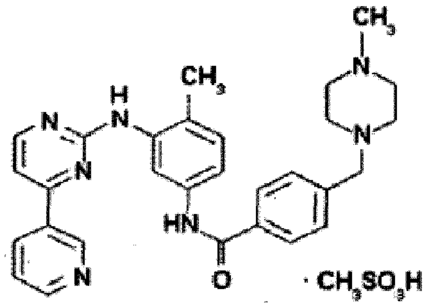

**Lapatinib**

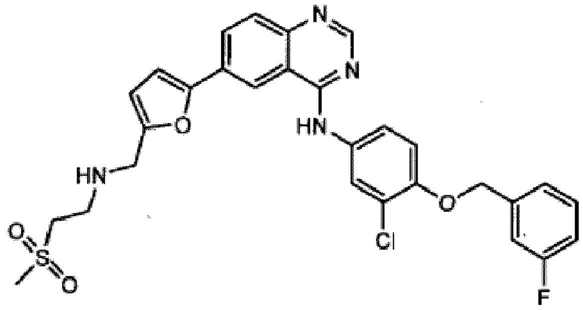

**Nilotinib**

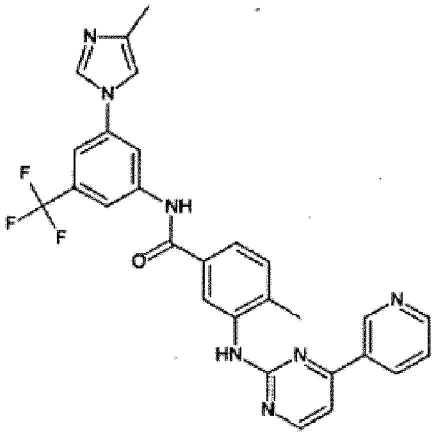

**Sorafenib**

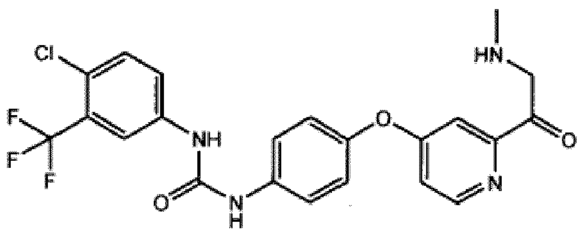

**Sunitinib**

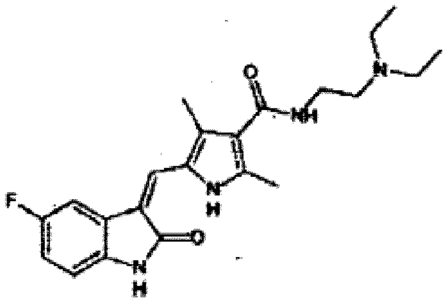

**Vandetanib**

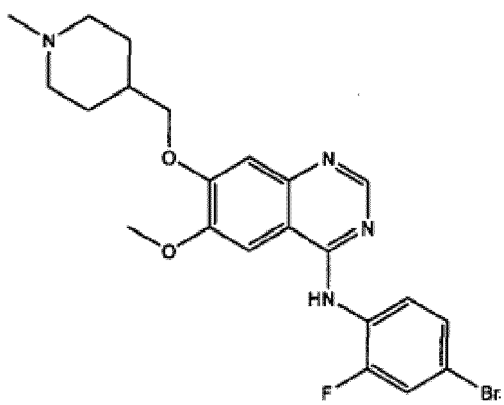

**Alitretinoin**

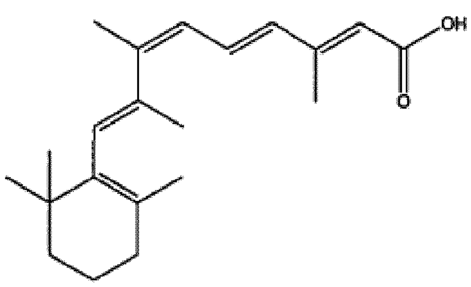

**Altretamine**

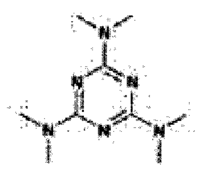

**Amsacrine**

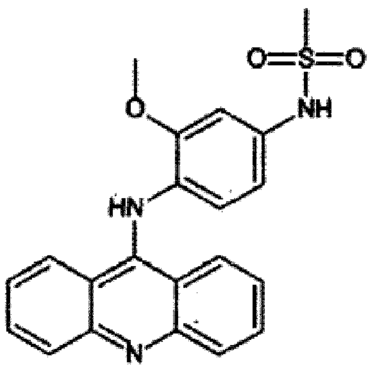

**Anagrelide**

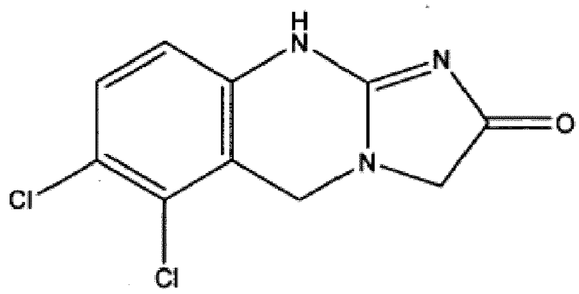

**Bexarotene**

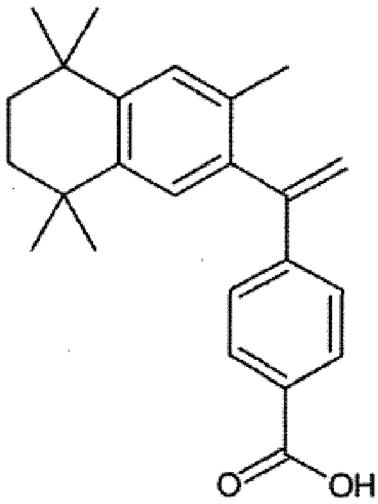

**Bortezomib**

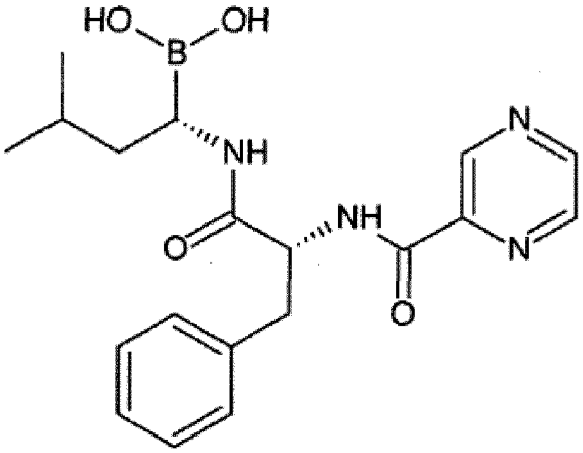

**Estramustine**

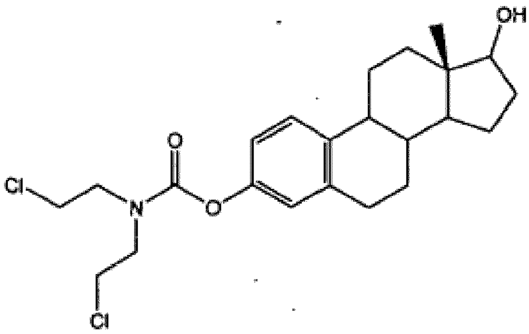

**Pentostatin**

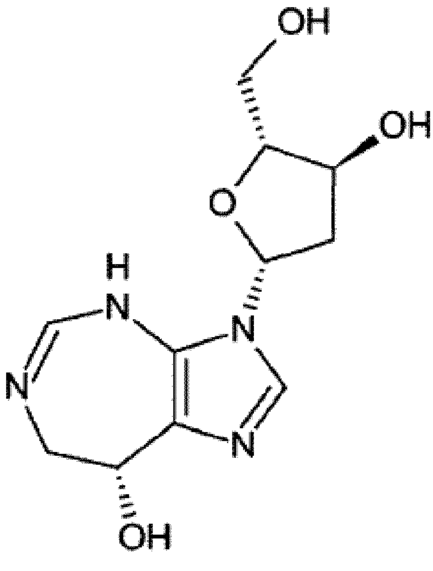

**Masoprocol**

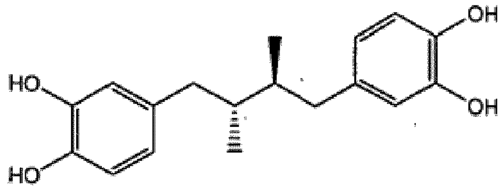

**Mitotane**

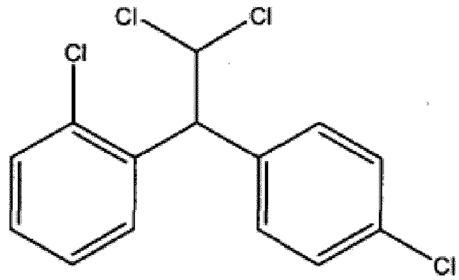

**Revlimid**

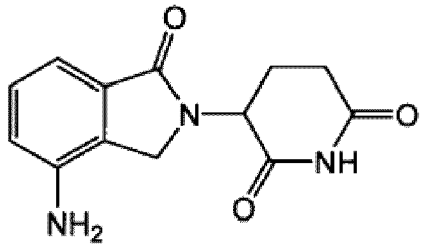

**Prednisone**

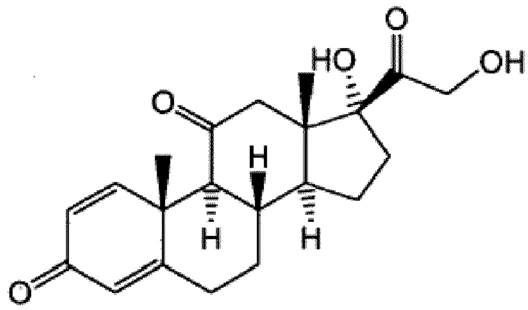

**Temsirolimus**

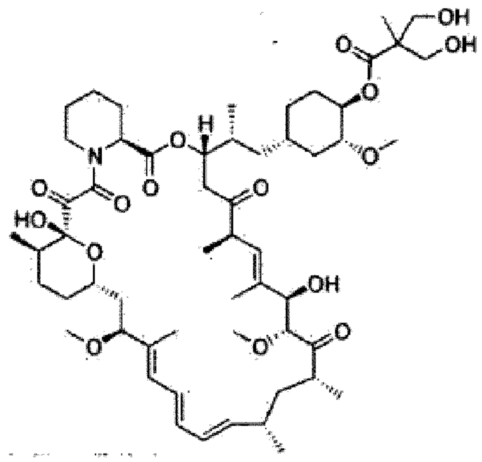

**Everolimus**

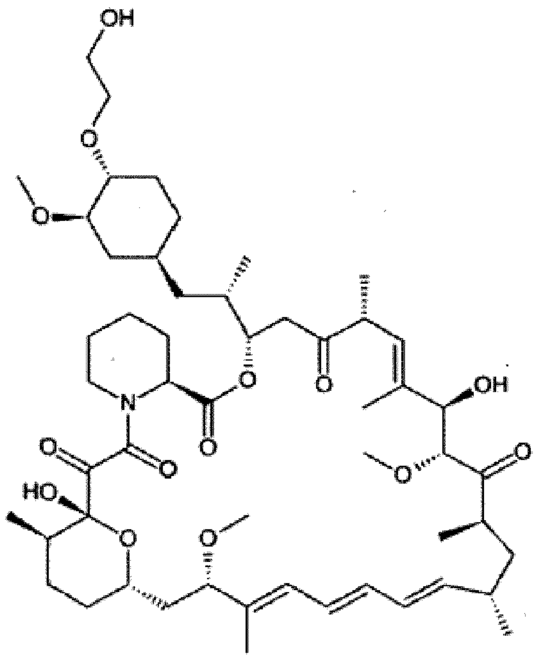

In selecting an appropriate anti-cancer agent to use in the compositions and methods of the invention a clinician will make the determination based on a number of factors such as the condition to be treated and the side effects profile of the anti-cancer agent versus the condition of the patient. In particular it is envisaged that a skilled worker will readily be able to select the required second and further (if required) therapeutic agent(s).

**PHARMACEUTICAL COMPOSITIONS**

As stated above the present invention provides a pharmaceutical composition for the treatment of cancer the composition including: (i) a benzimidazole based anti-cancer agent; and (ii) a second anti-cancer agent.

In the compositions of the invention the benzimidazole based anti-cancer agent and the second anti-cancer agent are as described above. It is envisaged that the second anti-cancer agent will not be a benzimidazole based anti-cancer agent. The pharmaceutical compositions of the invention may also include a further anti-cancer agent as described above. It is also envisaged that this further anti-cancer agent will not be a benzimidazole based anti-cancer agent. In addition it is envisaged that the further anti-cancer agent will be different to the second anti-cancer agent.

Pharmaceutical compositions of this invention for parenteral injection comprise pharmaceutically acceptable sterile aqueous or nonaqueous solutions, dispersions, suspensions or emulsions as well as sterile powders for reconstitution into sterile injectable solutions or dispersions just prior to use. Examples of suitable aqueous and nonaqueous carriers, diluents, solvents or vehicles include water, ethanol, polyols (such as glycerol, propylene glycol, polyethylene glycol, and the like), and suitable mixtures thereof, vegetable oils (such as olive oil), and injectable organic esters such as ethyl oleate. Proper fluidity can be maintained, for example, by the use of coating materials such as lecithin, by the maintenance of the required particle size in the case of dispersions, and by the use of surfactants.

These compositions may also contain adjuvants such as preservative, wetting agents, emulsifying agents, and dispersing agents. Prevention of the action of microorganisms may be ensured by the inclusion of various antibacterial and antifungal agents, for example, paraben, chlorobutanol, phenol sorbic acid, and the like. It may also be desirable to include isotonic agents such as sugars, sodium chloride, and the like. Prolonged absorption of the injectable pharmaceutical form may be brought about by the inclusion of agents that delay absorption such as aluminium monostearate and gelatin.

If desired, and for more effective distribution, the active agents can be incorporated into slow release or targeted delivery systems such as polymer matrices, liposomes, and microspheres.

The injectable formulations can be sterilized, for example, by filtration through a bacterial-retaining filter, or by incorporating sterilizing agents in the form of sterile solid compositions that can be dissolved or dispersed in sterile water or other sterile injectable medium just prior to use.

Solid dosage forms for oral administration include capsules, tablets, pills, powders, and granules. In such solid dosage forms, the active agents are mixed with at least one inert, pharmaceutically acceptable excipient or carrier such as sodium citrate or dicalcium phosphate and/or a) fillers or extenders such as starches, lactose, sucrose, glucose, mannitol, and silicic acid, b) binders such as, for example, carboxymethylcellulose, alginates, gelatin, polyvinylpyrrolidone, sucrose, and acacia, c) humectants such as glycerol, d) disintegrating agents such as agar-agar, calcium carbonate, potato or tapioca starch, alginic acid, certain silicates, and sodium carbonate, e) solution retarding agents such as paraffin, f) absorption accelerators such as quaternary ammonium compounds, g) wetting agents such as, for example, cetyl alcohol and glycerol monostearate, h) absorbents such as kaolin and bentonite clay, and i) lubricants such as talc, calcium stearate, magnesium stearate, solid polyethylene glycols, sodium lauryl sulfate, and mixtures thereof. In the case of capsules, tablets and pills, the dosage form may also comprise buffering agents.

Solid compositions of a similar type may also be employed as fillers in soft and hard-filled gelatin capsules using such excipients as lactose or milk sugar as well as high molecular weight polyethylene glycols and the like.

The solid dosage forms of tablets, dragees, capsules, pills, and granules can be prepared with coatings and shells such as enteric coatings and other coatings well known in the pharmaceutical formulating art. They may optionally contain opacifying agents and can also be of a composition that they release the active ingredient(s) only, or preferentially, in a certain part of the intestinal tract, optionally, in a delayed manner.

Examples of embedding compositions which can be used include polymeric substances and waxes.

If desired, and for more effective distribution, the active agents can be incorporated into slow release or targeted delivery systems such as polymer matrices, liposomes, and microspheres.

The active agents can also be in microencapsulated form, if appropriate, with one or more of the above-mentioned excipients.

Liquid dosage forms for oral administration include pharmaceutically acceptable emulsions, solutions, suspensions, syrups and elixirs. In addition to the active agents, the liquid dosage forms may contain inert diluents commonly used in the art such as, for example, water or other solvents, solubilizing agents and emulsifiers such as ethyl alcohol, isopropyl alcohol, ethyl carbonate, ethyl acetate, benzyl alcohol, benzyl benzoate, propylene glycol, 1,3-butylene glycol, dimethyl formamide, oils (in particular, cottonseed, groundnut, corn, germ, olive, castor, and sesame oils), glycerol, tetrahydrofurfuryl alcohol, polyethylene glycols and fatty acid esters of sorbitan, and mixtures thereof.

Besides inert diluents, the oral compositions can also include adjuvants such as wetting agents, emulsifying and suspending agents, sweetening, flavoring, and perfuming agents.

Suspensions, in addition to the active agents, may contain suspending agents as, for example, ethoxylated isostearyl alcohols, polyoxyethylene sorbitol and sorbitan esters, microcrystalline cellulose, aluminium metahydroxide,

bentonite, agar-agar, and tragacanth, and mixtures thereof.

Compositions for rectal or vaginal administration are preferably suppositories which can be prepared by mixing the active agents with suitable non-irritating excipients or carriers such as cocoa butter, polyethylene glycol or a suppository wax which are solid at room temperature but liquid at body temperature and therefore melt in the rectum or vaginal cavity and release the active agents.

Dosage forms for topical administration of the therapeutic agents include powders, patches, sprays, ointments and inhalants. The active agents are mixed under sterile conditions with a pharmaceutically acceptable carrier and any needed preservatives, buffers, or propellants which may be required.

One skilled in the art of preparing pharmaceutical compositions can readily select the proper form and mode of administration depending upon the particular characteristics of the active agents selected, the condition to be treated, the stage of the condition to be treated and other relevant circumstances. We refer the reader to Remingtons Pharmaceutical Sciences, 19th edition, Mack Publishing Co. (1995) for further information.

The pharmaceutical compositions of the invention typically contain a benzimidazole based anti-cancer agent and a second anti-cancer agent and may also contain a further anti-cancer agent. These components typically constitute all the active anti-cancer agents present in the composition however the composition may also contain one or more other active agents with different biological activity. The amount of each of the active agents present in the composition will vary widely depending upon the desired activity of the final composition and the identity of each of the active agents in the specific composition. Thus for example a skilled worker in the field of formulation chemistry will be able to determine from the relative activity of the active agents to be incorporated in any specific composition the relative amounts of each of the agents to be incorporated. In formulating these compositions the absolute amount of each active ingredient in the formulation is not as important as the relative amount of each ingredient. This is because for compositions containing a lower absolute amount of active ingredient the dose rate will be higher in order to deliver an effective dose. In compositions with a relatively high absolute amount of active ingredient the dose rate will be lower in order to deliver the same effective dose. As such the absolute amounts of the ingredients to be incorporated in the pharmaceutical compositions will depend upon the desired final dose size and the potency or activity of the active agent(s).

The pharmaceutical compositions of the invention will typically contain from 0.01% to 99.99% of the benzimidazole based anti cancer agent by weight of the total weight of the composition. In a further embodiment the composition will contain from 0.1% to 99.9% of the benzimidazole based anti cancer agent by weight of the total weight of the composition. In a further embodiment the composition will contain from 1% to 99% of the benzimidazole based anti cancer agent by weight of the total weight of the composition.

The pharmaceutical compositions of the invention will typically contain from 0.01% to 99.99% of the second anti cancer agent by weight of the total weight of the composition. In a further embodiment the composition will contain from 0.1% to 99.9% of the second anti cancer agent by weight of the total weight of the composition. In a further embodiment the composition will contain from 1% to 99% of the second anti cancer agent by weight of the total weight of the composition.

The pharmaceutical compositions of the invention may also contain a further anti-cancer agent. If the composition contains a further anti-cancer agent it will typically contain from 0.01% to 99.99% of the further anti cancer agent by weight of the total weight of the composition. In a further embodiment the composition will contain from 0.1% to 99.9% of the further anti-cancer agent by weight of the total weight of the composition. In a further embodiment the composition will contain from 1% to 99% of the further anti cancer agent by weight of the total weight of the composition.

**Methods of Treatment**

The present invention also provides a method for treating cancer including administering to a patient in need thereof: a first amount of a benzimidazole based anti-cancer agent and a second amount of a second anti-cancer agent; wherein the first amount and the second amount together comprise a therapeutically effective amount.

In the method of the invention the benzimidazole based anti-cancer agent and the second anti-cancer agent are as described above.

In the method of the invention the benzimidazole based anti-cancer agent and the anti-cancer agent may be administered simultaneously or sequentially in any order or a combination thereof.

In relation to simultaneous administration the benzimidazole based anti-cancer agent and the second anti-cancer agent may be administered as a combined preparation or composition (such as the composition of the invention as described above) or as separate preparations that are administered to the patient at the same time. Thus for example if the administration is by oral administration the two agents could be administered in the form of two tablets or the like at the same time. In a similar manner where the agents are to be administered intravenously the patient can have two drip lines inserted wherein the two agents are administered simultaneously through two separate drip lines.

The administration of the agents may also be by way of sequential administration. If the administration is sequential the two agents may be administered in any order. Thus for example the benzimidazole based anti-cancer

agent may be administered followed by administration of the second anti-cancer agent. Alternatively the second anti-cancer agent may be administered followed by the benzimidazole based anti-cancer agent. In relation to sequential administration the period of time between administrations of the two agents can vary considerably and the agent that is administered second may be administered as little as a few minutes up to a significant number of days (eg, 1, 7, 14 or 21 days) after administration of the agent that is administered first.

Administration of the agents may also be such that there is a partial concurrent administration. Thus for example one of the agents may be delivered slowly such as by infusion and before completion of this administration the administration of the other agent is commenced such that for a period of time there is concurrent administration such that both agents are being administered at the same time.

The method of the invention may also include administration of a further anti-cancer agent. The further anti-cancer agent may be as discussed above. The further anti-cancer agent may be administered either simultaneously or sequentially with either the benzimidazole based anti-cancer agent and/or the second anti-cancer agent.

Administration of the agents to humans can be by any of the accepted modes for enteral administration such as oral or rectal, or by parenteral administration such as subcutaneous, intramuscular, intravenous and intradermal routes. Injection can be bolus or via constant or intermittent infusion. The agent is typically included in a pharmaceutically acceptable carrier or diluent and in an amount sufficient to deliver to the patient a therapeutically effective dose. In various embodiments the agent may be selectively toxic or more toxic to rapidly proliferating cells, e.g. cancerous tumors, than to normal cells.

The term "therapeutically effective amount" or "effective amount" is an amount sufficient to effect beneficial or desired results. An effective amount can be administered in one or more administrations. An effective amount is typically sufficient to palliate, ameliorate, stabilize, reverse, slow or delay the progression of the disease state. A therapeutically effective amount can be readily determined by an attending diagnostician by the use of conventional techniques and by observing results obtained under analogous circumstances. In determining the therapeutically effective amount a number of factors are to be considered including but not limited to, the species of animal, its size, age and general health, the specific condition involved, the severity of the condition, the response of the patient to treatment, the particular agent administered, the mode of administration, the bioavailability of the preparation administered, the dose regime selected, the use of other medications and other relevant circumstances. In relation to the combined treatments described above the first amount and the second amount are selected upon the basis of the respective substances to be administered such that the total amount of agent administered is an effective amount.

A typical dosage of the benzimidazole-based agent will be a range from about 0.01 to 300 mg per kilogram of body weight per day. A more preferred dosage will be in the range from 0.1 to 100 mg per kilogram of body weight per day, more preferably from 0.2 to 80 mg per kilogram of body weight per day, even more preferably 0.2 to 50 mg per kilogram of body weight per day. A suitable dose can be administered in multiple sub-doses per day.

In relation to the typical dosage of the second and further anti-cancer agents (if used) the dosage will be able to be readily determined for each agent based on the accepted dose rate for the agent in question and will vary widely depending upon the identity of the specific agent. In general the dosage will be either the accepted clinical dose for the agent in question or will be a dose lower than the accepted clinical dose. This will be able to be readily determined by a skilled addressee in the art in each instance once the exact second and/ or further anti-cancer agent has been selected.

In one embodiment of the invention the method includes administration of a first amount of a benzimidazole based anti-cancer agent or a pharmaceutically acceptable salt thereof and a second amount of Dasatinib or a pharmaceutically acceptable salt thereof wherein the first amount and the second amount together comprise a therapeutically effective amount. In one form of this embodiment the method includes simultaneous administration of a first amount of a benzimidazole based anti-cancer agent or a pharmaceutically acceptable salt thereof and a second amount of Dasatinib or a pharmaceutically acceptable salt thereof wherein the first amount and the second amount together comprise a therapeutically effective amount. In one form of this embodiment the method includes administration of a first amount of a benzimidazole based anti-cancer agent or a pharmaceutically acceptable salt thereof followed by administration of a second amount of Dasatinib or a pharmaceutically acceptable salt thereof wherein the first amount and the second amount together comprise a therapeutically effective amount. In another form of this embodiment the method includes administration of a second amount of Dasatinib or a pharmaceutically acceptable salt thereof followed by administration of a first amount of a benzimidazole based anti-cancer agent or a pharmaceutically acceptable salt thereof wherein the first amount and the second amount together comprise a therapeutically effective amount.

In one embodiment of the invention the method includes administration of a first amount of a benzimidazole based anti-cancer agent or a pharmaceutically acceptable salt thereof and a second amount of Erlotinib or a pharmaceutically acceptable salt thereof wherein the first amount and the second amount together comprise a therapeutically effective amount. In one form of this embodiment the method includes simultaneous administration of a first amount of a benzimidazole based anti-cancer agent or a pharmaceutically acceptable salt thereof and a second amount of Erlotinib or a pharmaceutically acceptable salt thereof wherein the first amount and the second amount together comprise a therapeutically effective amount. In one form of this embodiment the method includes administration of a first amount

of a benzimidazole based anti-cancer agent or a pharmaceutically acceptable salt thereof followed by administration of a second amount of Erlotinib or a pharmaceutically acceptable salt thereof wherein the first amount and the second amount together comprise a therapeutically effective amount. In another form of this embodiment the method includes administration of a second amount of Erlotinib or a pharmaceutically acceptable salt thereof followed by administration of a first amount of a benzimidazole based anti-cancer agent or a pharmaceutically acceptable salt thereof wherein the first amount and the second amount together comprise a therapeutically effective amount. In one form of this embodiment the cancer is non small cell lung cancer.

In one embodiment of the invention the method includes administration of a first amount of a benzimidazole based anti-cancer agent or a pharmaceutically acceptable salt thereof and a second amount of Doxorubicin or a pharmaceutically acceptable salt thereof wherein the first amount and the second amount together comprise a therapeutically effective amount. In one form of this embodiment the method includes simultaneous administration of a first amount of a benzimidazole based anti-cancer agent or a pharmaceutically acceptable salt thereof and a second amount of Doxorubicin or a pharmaceutically acceptable salt thereof wherein the first amount and the second amount together comprise a therapeutically effective amount. In one form of this embodiment the method includes administration of a first amount of a benzimidazole based anti-cancer agent or a pharmaceutically acceptable salt thereof followed by administration of a second amount of Doxorubicin or a pharmaceutically acceptable salt thereof wherein the first amount and the second amount together comprise a therapeutically effective amount. In another form of this embodiment the method includes administration of a second amount of Doxorubicin or a pharmaceutically acceptable salt thereof followed by administration of a first amount of a benzimidazole based anti-cancer agent or a pharmaceutically acceptable salt thereof wherein the first amount and the second amount together comprise a therapeutically effective amount.

In one embodiment of the invention the method includes administration of a first amount of a benzimidazole based anti-cancer agent or a pharmaceutically acceptable salt thereof and a second amount of 5-Fluorouracil or a pharmaceutically acceptable salt thereof wherein the first amount and the second amount together comprise a therapeutically effective amount. In one form of this embodiment the method includes simultaneous administration of a first amount of a benzimidazole based anti-cancer agent or a pharmaceutically acceptable salt thereof and a second amount of 5-Fluorouracil or a pharmaceutically acceptable salt thereof wherein the first amount and the second amount together comprise a therapeutically effective amount. In one form of this embodiment the method includes administration of a first amount of a benzimidazole based anti-cancer agent or a pharmaceutically acceptable salt thereof followed by administration of a second amount of 5-Fluorouracil or a pharmaceutically acceptable salt thereof wherein the first amount and the second amount together comprise a therapeutically effective amount. In another form of this embodiment the method includes administration of a second amount of 5-Fluorouracil or a pharmaceutically acceptable salt thereof followed by administration of a first amount of a benzimidazole based anti-cancer agent or a pharmaceutically acceptable salt thereof wherein the first amount and the second amount together comprise a therapeutically effective amount. In one form of this embodiment the cancer is colorectal cancer.

In one embodiment of the invention the method includes administration of a first amount of a benzimidazole based anti-cancer agent or a pharmaceutically acceptable salt thereof and a second amount of Oxaliplatin wherein the first amount and the second amount together comprise a therapeutically effective amount. In one form of this embodiment the method includes simultaneous administration of a first amount of a benzimidazole based anti-cancer agent or a pharmaceutically acceptable salt thereof and a second amount of Oxaliplatin wherein the first amount and the second amount together comprise a therapeutically effective amount. In one form of this embodiment the method includes administration of a first amount of a benzimidazole based anti-cancer agent or a pharmaceutically acceptable salt thereof followed by administration of a second amount of Oxaliplatin wherein the first amount and the second amount together comprise a therapeutically effective amount. In another form of this embodiment the method includes administration of a second amount of Oxaliplatin followed by administration of a first amount of a benzimidazole based anti-cancer agent or a pharmaceutically acceptable salt thereof wherein the first amount and the second amount together comprise a therapeutically effective amount. In one form of this embodiment the cancer is non small cell lung cancer. In one form of this embodiment the cancer is colorectal cancer.

In one embodiment of the invention the method includes administration of a first amount of a benzimidazole based anti-cancer agent or a pharmaceutically acceptable salt thereof and a second amount of Rituximab or a pharmaceutically acceptable salt thereof wherein the first amount and the second amount together comprise a therapeutically effective amount. In one form of this embodiment the method includes simultaneous administration of a first amount of a benzimidazole based anti-cancer agent or a pharmaceutically acceptable salt thereof and a second amount of Rituximab or a pharmaceutically acceptable salt thereof wherein the first amount and the second amount together comprise a therapeutically effective amount. In one form of this embodiment the method includes administration of a first amount of a benzimidazole based anti-cancer agent or a pharmaceutically acceptable salt thereof followed by administration of a second amount of Rituximab or a pharmaceutically acceptable salt thereof wherein the first amount and the second amount together comprise a therapeutically effective amount. In another form of this embodiment the method includes administration of a second amount of Rituximab or a pharmaceutically acceptable salt thereof followed by administration

of a first amount of a benzimidazole based anti-cancer agent or a pharmaceutically acceptable salt thereof wherein the first amount and the second amount together comprise a therapeutically effective amount. In one form of this embodiment the cancer is lymphoma.

In one embodiment of the invention the method includes administration of a first amount of a benzimidazole based anti-cancer agent or a pharmaceutically acceptable salt thereof and a second amount of Satraplatin wherein the first amount and the second amount together comprise a therapeutically effective amount. In one form of this embodiment the method includes simultaneous administration of a first amount of a benzimidazole based anti-cancer agent or a pharmaceutically acceptable salt thereof and a second amount of Satraplatin wherein the first amount and the second amount together comprise a therapeutically effective amount. In one form of this embodiment the method includes administration of a first amount of a benzimidazole based anti-cancer agent or a pharmaceutically acceptable salt thereof followed by administration of a second amount of Satraplatin wherein the first amount and the second amount together comprise a therapeutically effective amount. In another form of this embodiment the method includes administration of a second amount of Satraplatin followed by administration of a first amount of a benzimidazole based anti-cancer agent or a pharmaceutically acceptable salt thereof wherein the first amount and the second amount together comprise a therapeutically effective amount. In one form of this embodiment the cancer is colorectal cancer.

In one embodiment of the invention the method includes administration of a first amount of a benzimidazole based anti-cancer agent or a pharmaceutically acceptable salt thereof and a second amount of Vinblastine or a pharmaceutically acceptable salt thereof wherein the first amount and the second amount together comprise a therapeutically effective amount. In one form of this embodiment the method includes simultaneous administration of a first amount of a benzimidazole based anti-cancer agent or a pharmaceutically acceptable salt thereof and a second amount of Vinblastine or a pharmaceutically acceptable salt thereof wherein the first amount and the second amount together comprise a therapeutically effective amount. In one form of this embodiment the method includes administration of a first amount of a benzimidazole based anti-cancer agent or a pharmaceutically acceptable salt thereof followed by administration of a second amount of Vinblastine or a pharmaceutically acceptable salt thereof wherein the first amount and the second amount together comprise a therapeutically effective amount. In another form of this embodiment the method includes administration of a second amount of Vinblastine or a pharmaceutically acceptable salt thereof followed by administration of a first amount of a benzimidazole based anti-cancer agent or a pharmaceutically acceptable salt thereof wherein the first amount and the second amount together comprise a therapeutically effective amount.

In one embodiment of the invention the method includes administration of a first amount of a benzimidazole based anti-cancer agent or a pharmaceutically acceptable salt thereof and a second amount of lenalidomide or a pharmaceutically acceptable salt thereof wherein the first amount and the second amount together comprise a therapeutically effective amount. In one form of this embodiment the method includes simultaneous administration of a first amount of a benzimidazole based anti-cancer agent or a pharmaceutically acceptable salt thereof and a second amount of lenalidomide or a pharmaceutically acceptable salt thereof wherein the first amount and the second amount together comprise a therapeutically effective amount. In one form of this embodiment the method includes administration of a first amount of a benzimidazole based anti-cancer agent or a pharmaceutically acceptable salt thereof followed by administration of a second amount of lenalidomide or a pharmaceutically acceptable salt thereof wherein the first amount and the second amount together comprise a therapeutically effective amount. In another form of this embodiment the method includes administration of a second amount of lenalidomide or a pharmaceutically acceptable salt thereof followed by administration of a first amount of a benzimidazole based anti-cancer agent or a pharmaceutically acceptable salt thereof wherein the first amount and the second amount together comprise a therapeutically effective amount. In one form of this embodiment the cancer is myelodysplastic syndrome.

In one embodiment of the invention the method includes administration of a first amount of a benzimidazole based anti-cancer agent or a pharmaceutically acceptable salt thereof and a second amount of decitabine or a pharmaceutically acceptable salt thereof wherein the first amount and the second amount together comprise a therapeutically effective amount. In one form of this embodiment the method includes simultaneous administration of a first amount of a benzimidazole based anti-cancer agent or a pharmaceutically acceptable salt thereof and a second amount of decitabine or a pharmaceutically acceptable salt thereof wherein the first amount and the second amount together comprise a therapeutically effective amount. In one form of this embodiment the method includes administration of a first amount of a benzimidazole based anti-cancer agent or a pharmaceutically acceptable salt thereof followed by administration of a second amount of decitabine or a pharmaceutically acceptable salt thereof wherein the first amount and the second amount together comprise a therapeutically effective amount. In another form of this embodiment the method includes administration of a second amount of decitabine or a pharmaceutically acceptable salt thereof followed by administration of a first amount of a benzimidazole based anti-cancer agent or a pharmaceutically acceptable salt thereof wherein the first amount and the second amount together comprise a therapeutically effective amount. In one form of this embodiment the cancer is myelodysplastic syndrome.

In one embodiment of the invention the method includes administration of a first amount of a benzimidazole based anti-cancer agent or a pharmaceutically acceptable salt thereof and a second amount of 5-azacytidine or a

pharmaceutically acceptable salt thereof wherein the first amount and the second amount together comprise a therapeutically effective amount. In one form of this embodiment the method includes simultaneous administration of a first amount of a benzimidazole based anti-cancer agent or a pharmaceutically acceptable salt thereof and a second amount of 5-azacytidine or a pharmaceutically acceptable salt thereof wherein the first amount and the second amount together comprise a therapeutically effective amount. In one form of this embodiment the method includes administration of a first amount of a benzimidazole based anti-cancer agent or a pharmaceutically acceptable salt thereof followed by administration of a second amount of 5-azacytidine or a pharmaceutically acceptable salt thereof wherein the first amount and the second amount together comprise a therapeutically effective amount. In another form of this embodiment the method includes administration of a second amount of 5-azacytidine or a pharmaceutically acceptable salt thereof followed by administration of a first amount of a benzimidazole based anti-cancer agent or a pharmaceutically acceptable salt thereof wherein the first amount and the second amount together comprise a therapeutically effective amount. In one form of this embodiment the cancer is myelodysplastic syndrome.

In one embodiment of the invention the method includes administration of a first amount of a benzimidazole based anti-cancer agent or a pharmaceutically acceptable salt thereof and a second amount of sorafenib or a pharmaceutically acceptable salt thereof wherein the first amount and the second amount together comprise a therapeutically effective amount. In one form of this embodiment the method includes simultaneous administration of a first amount of a benzimidazole based anti-cancer agent or a pharmaceutically acceptable salt thereof and a second amount of sorafenib or a pharmaceutically acceptable salt thereof wherein the first amount and the second amount together comprise a therapeutically effective amount. In one form of this embodiment the method includes administration of a first amount of a benzimidazole based anti-cancer agent or a pharmaceutically acceptable salt thereof followed by administration of a second amount of sorafenib or a pharmaceutically acceptable salt thereof wherein the first amount and the second amount together comprise a therapeutically effective amount. In another form of *this* embodiment the method *includes* administration of a second amount of sorafenib or a pharmaceutically acceptable salt thereof followed by administration of a first amount of a benzimidazole based anti-cancer agent or a pharmaceutically acceptable salt thereof wherein the first amount and the second amount together comprise a therapeutically effective amount. In one form of this embodiment the cancer is renal cell carcinoma. In one form of this embodiment the cancer is hepatocarcinoma.

In one embodiment of the invention the method includes administration of a first amount of a benzimidazole based anti-cancer agent or a pharmaceutically acceptable salt thereof and a second amount of temsirolimus or a pharmaceutically acceptable salt thereof wherein the first amount and the second amount together comprise a therapeutically effective amount. In one form of this embodiment the method includes simultaneous administration of a first amount of a benzimidazole based anti-cancer agent or a pharmaceutically acceptable salt thereof and a second amount of temsirolimus or a pharmaceutically acceptable salt thereof wherein the first amount and the second amount together comprise a therapeutically effective amount. In one form of this embodiment the method includes administration of a first amount of a benzimidazole based anti-cancer agent or a pharmaceutically acceptable salt thereof followed by administration of a second amount of temsirolimus or a pharmaceutically acceptable salt thereof wherein the first amount and the second amount together comprise a therapeutically effective amount. In another form of this embodiment the method includes administration of a second amount of temsirolimus or a pharmaceutically acceptable salt thereof followed by administration of a first amount of a benzimidazole based anti-cancer agent or a pharmaceutically acceptable salt thereof wherein the first amount and the second amount together comprise a therapeutically effective amount. In one form of this embodiment the cancer is renal cell carcinoma. In one form of this embodiment the cancer is lymphoma.

In one embodiment of the invention the method includes administration of a first amount of a benzimidazole based anti-cancer agent or a pharmaceutically acceptable salt thereof and a second amount of everolimus or a pharmaceutically acceptable salt thereof wherein the first amount and the second amount together comprise a therapeutically effective amount. In one form of this embodiment the method includes simultaneous administration of a first amount of a benzimidazole based anti-cancer agent or a pharmaceutically acceptable salt thereof and a second amount of everolimus or a pharmaceutically acceptable salt thereof wherein the first amount and the second amount together comprise a therapeutically effective amount. In one form of this embodiment the method includes administration of a first amount of a benzimidazole based anti-cancer agent or a pharmaceutically acceptable salt thereof followed by administration of a second amount of everolimus or a pharmaceutically acceptable salt thereof wherein the first amount and the second amount together comprise a therapeutically effective amount. In another form of this embodiment the method includes administration of a second amount of everolimus or a pharmaceutically acceptable salt thereof followed by administration of a first amount of a benzimidazole based anti-cancer agent or a pharmaceutically acceptable salt thereof wherein the first amount and the second amount together comprise a therapeutically effective amount. In one form of this embodiment the cancer is renal cell carcinoma. In one form of this embodiment the cancer is lymphoma.

In one embodiment of the invention the method includes administration of a first amount of a benzimidazole based anti-cancer agent or a pharmaceutically acceptable salt thereof and a second amount of sunitinib or a pharmaceutically acceptable salt thereof wherein the first amount and the second amount together comprise a therapeutically effective amount. In one form of this embodiment the method includes simultaneous administration of a first amount of

a benzimidazole based anti-cancer agent or a pharmaceutically acceptable salt thereof and a second amount of sunitinib or a pharmaceutically acceptable salt thereof wherein the first amount and the second amount together comprise a therapeutically effective amount. In one form of this embodiment the method includes administration of a first amount of a benzimidazole based anti-cancer agent or a pharmaceutically acceptable salt thereof followed by administration of a second amount of sunitinib or a pharmaceutically acceptable salt thereof wherein the first amount and the second amount together comprise a therapeutically effective amount. In another form of this embodiment the method includes administration of a second amount of sunitinib or a pharmaceutically acceptable salt thereof followed by administration of a first amount of a benzimidazole based anti-cancer agent or a pharmaceutically acceptable salt thereof wherein the first amount and the second amount together comprise a therapeutically effective amount. In one form of this embodiment the cancer is renal cell carcinoma.

In one embodiment of the invention the method includes administration of a first amount of a benzimidazole based anti-cancer agent or a pharmaceutically acceptable salt thereof and a second amount of docetaxel or a pharmaceutically acceptable salt thereof wherein the first amount and the second amount together comprise a therapeutically effective amount. In one form of this embodiment the method includes simultaneous administration of a first amount of a benzimidazole based anti-cancer agent or a pharmaceutically acceptable salt thereof and a second amount of docetaxel or a pharmaceutically acceptable salt thereof wherein the first amount and the second amount together comprise a therapeutically effective amount. In one form of this embodiment the method includes administration of a first amount of a benzimidazole based anti-cancer agent or a pharmaceutically acceptable salt thereof followed by administration of a second amount of docetaxel or a pharmaceutically acceptable salt thereof wherein the first amount and the second amount together comprise a therapeutically effective amount. In another form of this embodiment the method includes administration of a second amount of docetaxel or a pharmaceutically acceptable salt thereof followed by administration of a first amount of a benzimidazole based anti-cancer agent or a pharmaceutically acceptable salt thereof wherein the first amount and the second amount together comprise a therapeutically effective amount. In one form of this embodiment the cancer is prostate cancer. In one form in this embodiment the prostate cancer is hormone refractory prostate cancer.

In one embodiment of the invention the method includes administration of a first amount of a benzimidazole based anti-cancer agent or a pharmaceutically acceptable salt thereof and a second amount of gemcitabine or a pharmaceutically acceptable salt thereof wherein the first amount and the second amount together comprise a therapeutically effective amount. In one form of this embodiment the method includes simultaneous administration of a first amount of a benzimidazole based anti-cancer agent or a pharmaceutically acceptable salt thereof and a second amount of gemcitabine or a pharmaceutically acceptable salt thereof wherein the first amount and the second amount together comprise a therapeutically effective amount. In one form of this embodiment the method includes administration of a first amount of a benzimidazole based anti-cancer agent or a pharmaceutically acceptable salt thereof followed by administration of a second amount of gemcitabine or a pharmaceutically acceptable salt thereof wherein the first amount and the second amount together comprise a therapeutically effective amount. In another form of this embodiment the method includes administration of a second amount of gemcitabine or a pharmaceutically acceptable salt thereof followed by administration of a first amount of a benzimidazole based anti-cancer agent or a pharmaceutically acceptable salt thereof wherein the first amount and the second amount together comprise a therapeutically effective amount. In one form of this embodiment the cancer is pancreatic cancer. In one form of this embodiment the cancer is non small cell lung cancer.

In one embodiment of the invention the method includes administration of a first amount of a benzimidazole based anti-cancer agent or a pharmaceutically acceptable salt thereof and a second amount of prednisone or a pharmaceutically acceptable salt thereof wherein the first amount and the second amount together comprise a therapeutically effective amount. In one form of this embodiment the method includes simultaneous administration of a first amount of a benzimidazole based anti-cancer agent or a pharmaceutically acceptable salt thereof and a second amount of prednisone or a pharmaceutically acceptable salt thereof wherein the first amount and the second amount together comprise a therapeutically effective amount. In one form of this embodiment the method includes administration of a first amount of a benzimidazole based anti-cancer agent or a pharmaceutically acceptable salt thereof followed by administration of a second amount of prednisone or a pharmaceutically acceptable salt thereof wherein the first amount and the second amount together comprise a therapeutically effective amount. In another form of this embodiment the method includes administration of a second amount of prednisone or a pharmaceutically acceptable salt thereof followed by administration of a first amount of a benzimidazole based anti-cancer agent or a pharmaceutically acceptable salt thereof wherein the first amount and the second amount together comprise a therapeutically effective amount. In one form of this embodiment the cancer is prostate cancer. In yet another form of this embodiment the prostate cancer is hormone refractory prostate cancer. In one form of this embodiment the method further includes administration of a third amount of docetaxel or a pharmaceutically acceptable salt thereof. The third amount may be administered either simultaneously with the first amount or the second amount or sequentially with either the first amount or the second amount.

In one embodiment of the invention the method includes administration of a first amount of a benzimidazole based anti-cancer agent or a pharmaceutically acceptable salt thereof and a second amount of Gefitinib (iressa) or a pharmaceutically acceptable salt thereof wherein the first amount and the second amount together comprise a therapeutically effective amount. In one form of this embodiment the method includes simultaneous administration of a first amount of a benzimidazole based anti-cancer agent or a pharmaceutically acceptable salt thereof and a second amount of Gefitinib (iressa) or a pharmaceutically acceptable salt thereof wherein the first amount and the second amount together comprise a therapeutically effective amount. In one form of this embodiment the method includes administration of a first amount of a benzimidazole based anti-cancer agent or a pharmaceutically acceptable salt thereof followed by administration of a second amount of Gefitinib (iressa) or a pharmaceutically acceptable salt thereof wherein the first amount and the second amount together comprise a therapeutically effective amount. In another form of this embodiment the method includes administration of a second amount of Gefitinib (iressa) or a pharmaceutically acceptable salt thereof followed by administration of a first amount of a benzimidazole based anticancer agent or a pharmaceutically acceptable salt thereof wherein the first amount and the second amount together comprise a therapeutically effective amount. In one form of this embodiment the cancer is non small cell lung cancer.

In one embodiment of the invention the method includes administration of a first amount of a benzimidazole based anti-cancer agent or a pharmaceutically acceptable salt thereof and a second amount of irinotecan or a pharmaceutically acceptable salt thereof wherein the first amount and the second amount together comprise a therapeutically effective amount. In one form of this embodiment the method includes simultaneous administration of a first amount of a benzimidazole based anti-cancer agent or a pharmaceutically acceptable salt thereof and a second amount of irinotecan or a pharmaceutically acceptable salt thereof wherein the first amount and the second amount together comprise a therapeutically effective amount. In one form of this embodiment the method includes administration of a first amount of a benzimidazole based anti-cancer agent or a pharmaceutically acceptable salt thereof followed by administration of a second amount of irinotecan or a pharmaceutically acceptable salt thereof wherein the first amount and the second amount together comprise a therapeutically effective amount. In another form of this embodiment the method includes administration of a second amount of irinotecan or a pharmaceutically acceptable salt thereof followed by administration of a first amount of a benzimidazole based anti-cancer agent or a pharmaceutically acceptable salt thereof wherein the first amount and the second amount together comprise a therapeutically effective amount. In one form of this embodiment the cancer is colorectal cancer. In one form of this embodiment the cancer is ovarian cancer.

In one embodiment of the invention the method includes administration of a first amount of a benzimidazole based anti-cancer agent or a pharmaceutically acceptable salt thereof and a second amount of avastin or a pharmaceutically acceptable salt thereof wherein the first amount and the second amount together comprise a therapeutically effective amount. In one form of this embodiment the method includes simultaneous administration of a first amount of a benzimidazole based anti-cancer agent or a pharmaceutically acceptable salt thereof and a second amount of avastin or a pharmaceutically acceptable salt thereof wherein the first amount and the second amount together comprise a therapeutically effective amount. In one form of this embodiment the method includes administration of a first amount of a benzimidazole based anti-cancer agent or a pharmaceutically acceptable salt thereof followed by administration of a second amount of avastin or a pharmaceutically acceptable salt thereof wherein the first amount and the second amount together comprise a therapeutically effective amount. In another form of this embodiment the method includes administration of a second amount of avastin or a pharmaceutically acceptable salt thereof followed by administration of a first amount of a benzimidazole based anti-cancer agent or a pharmaceutically acceptable salt thereof wherein the first amount and the second amount together comprise a therapeutically effective amount. In one form of this embodiment the cancer is colorectal cancer. In one form of this embodiment the cancer is non-small cell lung cancer. In one form of this embodiment the cancer is breast cancer.

## KITS

The invention in other embodiments provides a kit or kit-of-parts including (i) a benzimidazole based anti-cancer agent; and (ii) a second anti-cancer agent wherein said kit or kit-of-parts is suitable for use in a method for treating cancer. The kit may also contain a further anti-cancer agent.

In such a kit or kit of parts can be found a container having a unit dosage of the agent (s). The kits can include a composition comprising an effective agent either as concentrates (including lyophilized compositions), which can be diluted further prior to use or they can be provided at the concentration of use, where the vials may include one or more dosages. Conveniently, in the kits, single dosages can be provided in sterile vials so that the physician can employ the vials directly, where the vials will have the desired amount and concentration of agent(s). Associated with such container(s) can be various written materials such as instructions for use (such as instructions for the administration of the two drugs), or a notice in the form prescribed by a governmental agency regulating the manufacture, use or sale of pharmaceuticals or biological products, which notice reflects approval by the agency of manufacture, use or sale for human administration. In one embodiment the kit or kit of parts includes further reagents such as buffers, solvents and the like as well as medical devices (such as syringes, tubing etc) which can be assembled and used in the administration of the drugs in the kit to the patient.

**CANCERS**

As used herein the term 'cancer' is a general term intended to encompass a number of conditions that are characterised by uncontrolled abnormal growth of cells.

It is anticipated that the compositions and methods of the invention will be useful in treating various cancers including but not limited to bone cancers including Ewing's sarcoma, osteosarcoma, chondrosarcoma and the like, brain and CNS tumours including acoustic neuroma, neuroblastomas, glioma and other brain tumours, spinal cord tumours, breast cancers including ductal adenocarcinoma, metastatic ductal breast carcinoma, colorectal cancers, advanced colorectal adenocarcinomas, colon cancers, endocrine cancers including adenocortical carcinoma, pancreatic cancer, pituitary cancer, thyroid cancer, parathyroid cancer, thymus cancer, multiple endocrine neoplasma, gastrointestinal cancers including stomach cancer, esophageal cancer, small intestine cancer, liver cancer, extra hepatic bile duct cancer, gastrointestinal carcinoid tumour, gall bladder cancer, genitourinary cancers including testicular cancer, penile cancer, prostate cancer, gynaecological cancers including cervical cancer, ovarian cancer, vaginal cancer, uterus/endometrium cancer, vulva cancer, gestational trophoblastic cancer, fallopian tube cancer, uterine sarcoma, head and neck cancers including oral cavity cancer, lip cancer, salivary gland cancer, larynx cancer, hypopharynx cancer, orthopharynx cancer, nasal cancer, paranasal cancer, nasopharynx cancer, leukemias including childhood leukemia, acute lymphocytic leukemia, acute myeloid leukemia, chronic lymphocytic leukemia, chronic myeloid leukemia, hairy cell leukemia, acute promyelocytic leukemia, plasma cell leukemia, erythroleukemia,myelomas, haematological disorders including myelodysplastic syndromes, myeloproliferative disorders, aplastic anemia, Fanconi anemia, Waldenstroms Macroglobulinemia, lung cancers including small cell lung cancer, non-small cell lung cancer, mesothelioma, lymphomas including Hodgkin's disease, non-Hodgkin's lymphoma, cutaneous T-cell lymphoma, peripheral T-cell lymphoma, AIDS related Lymphoma, B-cell lymphoma, Burkitt's lymphoma, mantle cell lymphoma, eye cancers including retinoblastoma, intraocular melanoma, skin cancers including melanoma, non-melanoma skin cancer, squamous cell carcinoma, merkel cell cancer, soft tissue sarcomas such as childhood soft tissue sarcoma, adult soft tissue sarcoma, Kaposi's sarcoma, gastrointestinal stromal tumors, urinary system cancers including kidney cancer, Wilms tumour, bladder cancer, urethral cancer, and transitional cell cancer.

Exemplary cancers that may be treated by the compositions and methods of the present invention are breast cancer, lung cancer, ovarian cancer, prostate cancer, head and neck cancer, renal cancer (e.g. renal cell carcinoma), gastric cancer, colon cancer, colorectal cancer and brain cancer.

Exemplary cancers that may be treated by the compositions and methods of the present invention include but are not limited to leukemias such as erythroleukemia, acute promyelocytic leukemia, acute myeloid leukemia, acute lymophocytic leukemia, acute T-cell leukemia and lymphoma such as B-cell lymphoma (e.g. Burkitt's lymphoma), mantle cell lymphoma, cutaneous T-cell lymphoma (CTCL), and peripheral T-cell lymphoma. In another embodiment, the cancer is Hodgkin's lymphoma. In yet another embodiment, the cancer is non-Hodgkin's lymphoma.

Exemplary cancers that may be treated by the compositions and methods of the present invention include solid tumors and hematologic malignancies. In another embodiment, preferred cancers that may be treated with the compounds of the present invention are colon cancer, prostate cancer, hepatoma and ovarian cancer. In another embodiment the cancer is hormone refractory prostate cancer.

In another embodiment, exemplary cancers that may be treated with the compositions and methods of the present invention are non small cell lung cancer, small cell lung cancer and mesothelioma.

In another embodiment, exemplary cancers that may be treated with the compositions and methods of the present invention are clear cell carcinoma/mesonephroma, intestinal cancer and pancreatic cancer.

In another embodiment the disorder that may be treated with the compositions and methods of the present invention is a proliferative disorder. In one embodiment the proliferative disorder is cancer.

The embodiments disclosed also relate to compositions of the present invention being used to treat cellular proliferative ailments, e.g., inhibition of proliferation of malignant cancer cells, benign tumor cells or other proliferative cells.

In another embodiment, exemplary proliferative disorders that may be treated with the compositions and methods of the present invention are pre-cancer conditions or hyperplasia including familial adenomatous polyposis, colonic adenomatous polyps, myeloid dysplasia (myelodysplastic syndrome), endometrial dysplasia, endometrial hyperplasia with atypia, cervical dysplasia, vaginal intraepithelial neoplasia, benign prostatic hyperplasia, papillomas of the larynx, actinic and solar keratosis, seborrheic keratosis and keratoacanthoma. In one specific embodiment, the proliferative disorder is myeloid dysplasia. In another preferred embodiment, the myelodysplastic syndrome can be high risk myelodysplastic syndrome or low risk myelodysplastic syndrome.

The compositions and methods of present invention as discussed above can also be used to also treat relapsed, metastatic or refractory forms of the cancers discussed above.

## SYNTHESIS OF BENZIMIDAZOLE BASED ANTI-CANCER AGENTS OF FORMULA 1

The benzimidazole based anti-cancer agents of formula (1) may be synthesised using any technique well known in the art and a skilled addressee in the field could contemplate a number of potential synthetic routes that may be applicable and which would work in a satisfactory manner. Nevertheless for the purposes of instruction we provide herein a number of different synthetic schemes which illustrate the synthesis of compound of this type.

The compounds of formula (1) may be prepared using the reaction routes and synthesis schemes as described below, employing the techniques available in the art using starting materials that are readily available. In addition the preparation of particular compounds of formula (1) is described in detail in the following examples, but the artisan will recognize that the chemical reactions described may be readily adapted to prepare a number of other agents of the various embodiments.

For example, the synthesis of non-exemplified compounds may be successfully performed by modifications apparent to those skilled in the art, e.g. by appropriately protecting interfering groups, by changing to other suitable reagents known in the art, or by making routine modifications of reaction conditions. A list of suitable protecting groups in organic synthesis can be found in T.W. Greene's Protective Groups in Organic Synthesis, 3rd Edition, John Wiley & Sons, 1991. Alternatively, other reactions disclosed herein or known in the art will be recognized as having applicability for preparing other compounds of the various embodiments.

Reagents useful for synthesizing compounds may be obtained or prepared according to techniques known in the art.

In the examples described below, unless otherwise indicated, all temperatures in the following description are in degrees Celsius and all parts and percentages are by weight, unless indicated otherwise.

Various starting materials and other reagents were purchased from commercial suppliers, such as Aldrich Chemical Company or Lancaster Synthesis Ltd., and used without further purification, unless otherwise indicated. Tetrahydrofuran (THF) and N,N-dimethylformamide (DMF) were purchased from Aldrich in SureSeal bottles and used as received. All solvents were purified by using standard methods in the art, unless otherwise indicated.

The reactions set forth below were performed under a positive pressure of nitrogen, argon or with a drying tube, at ambient temperature (unless otherwise stated), in anhydrous solvents, and the reaction flasks are fitted with rubber septa for the introduction of substrates and reagents via syringe. Glassware was oven-dried and/or heat-dried. Analytical thin-layer chromatography was performed on glass-backed silica gel 60 F 254 plates (E Merck (0.25 mm)) and eluted with the appropriate solvent ratios (v/v). The reactions were assayed by TLC and terminated as judged by the consumption of starting material.

The TLC plates were visualized by UV absorption or with a p-anisaldehyde spray reagent or a phosphomolybdic acid reagent (Aldrich Chemical, 20wt% in ethanol) which was activated with heat, or by staining in iodine chamber. Work-ups were typically done by doubling the reaction volume with the reaction solvent or extraction solvent and then washing with the indicated aqueous solutions using 25% by volume of the extraction volume (unless otherwise indicated). Product solutions were dried over anhydrous sodium sulfate prior to filtration, and evaporation of the solvents was under reduced pressure on a rotary evaporator and noted as solvents removed in vacuo. Flash column chromatography [Still et al, J. Org. Chem., 43, 2923 (1978)] was conducted using Silica gel 60 (Merck KGaA, 0.040-0.063 mm, 230-400 mesh ASTM) and a silica gel:crude material ratio of about 20:1 to 50:1, unless otherwise stated. Hydrogenolysis was done at the pressure indicated or at ambient pressure.

## SYNTHESIS

Schemes I and II illustrate the procedures used for preparing compounds of formula Ib, wherein X and Y are hydrogens, compounds **(VII)** of formula Ia can be prepared by analogous procedure, for example, by the choice of appropriate starting material. For example, in the case of Z is -CH=CH- and attached to $C_5$-position in Formula Ib, such compound(s) can be synthesized by analogous method illustrated in Scheme I and II starting with a substituted cinnamic acid (e.g. *trans*-3-nitro-4-chloro-cinnamic acid), appropriate amine component ($R^1NH_2$), carboxylic acid component ($R^2CO_2H$, Scheme I) or aldehyde ($R^2CHO$, Scheme II), and appropriate hydroxylamine or N-alkyl hydroxylamine ($NHR^3OH$ where $R^3$ is defined as above in Formula Ia).

Specifically, the hydroxamate compounds Formula Ib can be synthesized by the synthetic route shown in Scheme I. The reaction of *trans*-4-chloro-3-nitrocinnamic acid (**I**) with an amine $R^1NH_2$ in the presence of a base (e.g. triethylamine) in an appropriate solvent (e.g. dioxane) gave (**II**). Treatment of (**II**) in methanol under acid catalysis (e.g. sulfuric acid) resulted in esterification providing (**III**). Alternatively, the carboxylic acid (**I**) may be esterified to the methyl ester (**Ia**) and then the chloride was replaced by the appropriate amine component $R^1NH_2$ to give compound (III). The nitro group of (**III**) can be reduced by appropriate reducing agent (e.g. tin (**II**) chloride) and the resulting phenylenediamine (**IV**) was coupled with an acid $R^2CO_2H$ to give amide (**V**) which was subsequently cyclized in an appropriate solvent (e.g. acetic acid) to give benzimidazole (**VI**) (J. Med. Chem. 2001, 44, 1516-1529). The hydroxamate compounds (**VI**)

were obtained from methyl ester (**VI**) by a known synthesis method (J. Med. Chem., 2002, 45, 753-757).

**Scheme I**

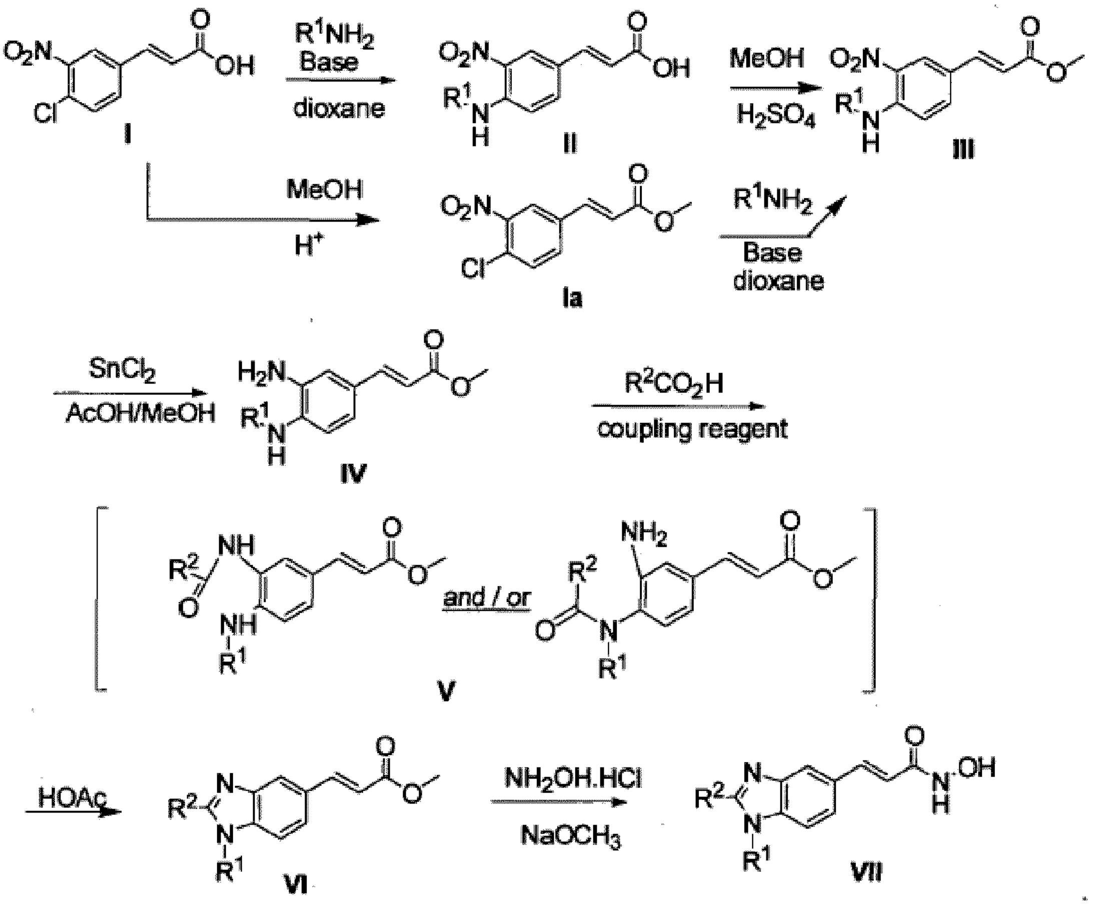


Alternatively, as depicted in Scheme **II,** compound **(VI)** was prepared by reacting with an appropriate aldehyde component $R^2CHO$ in the presence of a reducing agent of nitro group (e.g. tin (II) chloride, or zinc powder) in one-pot (Tetrahedron Letters, 2000, 41, 9871-9874). Formic acid was used to prepare compound (**VI**) when $R^2$ = H.

**Scheme II**

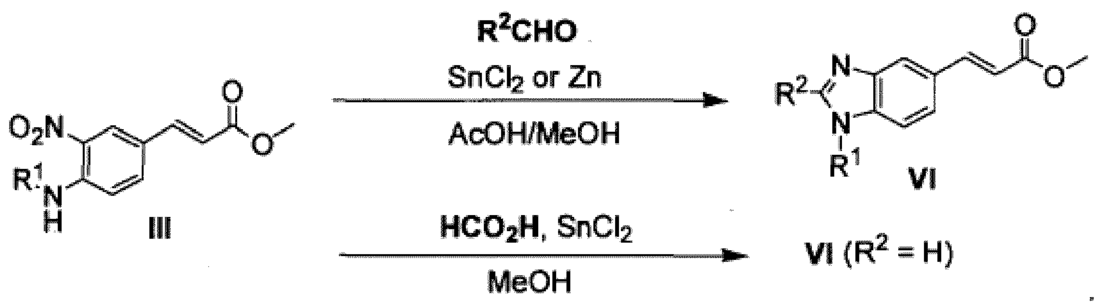


In both Schemes I & II, the benzimidazole ring may be constructed by a cyclization step involving either an aldehyde or a carboxylic acid. The following reaction steps 1-4 refer to the use of carboxylic acid for the cyclization of (**IV**) via (**V**) to form benzimidazole derivatives (**VI**), followed by the conversion of ester (**VI**) to the hydroxamate (**VII**). For one-pot cycylization of (**III**) to (**VI**), see the procedures under Example 1.

**Step 1: Reduction of nitro group**

To a pre-stirred solution of starting material (**III**, 1.0 mmol) in 50 mL of co-solvent (glacial acetic acid: methanol= 2:8), Tin chloride was added (5.0 mmol). The resulting solution was heated to 55°C overnight and then cooled to room temperature. The solvent was removed and the mixture was neutralized with sodium bicarbonate to pH 8. The crude product was extracted with dichloromethane (20 mL) for three times. The organic extracts were combined and washed with water (15 mL) twice and brine (15 mL) once and further dried over $Na_2SO_4$ for 1 hour. It was filtered and concentrated; the diamino product (**IV**) was purified by flash chromatography.

**Step 2: Amide formation**

To a pre-stirred solution of carboxylic acid (1.1 mmol), diamino product (**IV**, 1.0 mmol) and PyBOP (1.1 mmol) in 10 mL of dried dichloromethane, was added DIEA (3.0 mmol) via a syringe. The resulting mixture was stirred at room temperature for 4 hours. The amide product (**V**) was purified by silica gel column chromatography.

**Step 3: Cyclization**

The amide product (**V**), obtained in Step 2, was treated with 5 mL of glacial acetic acid, the resulting solution was heated to 75°C for 24 hours. After cooling down to rt, the solvent was removed under vacuum to give product (**VI**) near quantitatively.

**Step 4: Hydroxamic acid formation**

To a stirred solution of ester (**VI**) and $NH_2OH$•HCl (10 equiv.) in MeOH (0.5 M) was added NaOMe solution (20 equiv.) at - 78°C. The reaction mixture was then allowed to warm up slowly to room temperature. The reaction was monitored by LC/MS and was completed in around 15~60 min. 1*N* HCl was then added slowly into the reaction mixture at 0°C. The desired product was separated by reverse-phase preparative HPLC and the fractions containing the desired product were freeze-dried. The hydroxamate product (**VI**) was obtained as TFA salt (isolated yield varies between 40 - 70%).

Scheme III illustrates another alternative procedure used for preparing compounds of formula Ib, where X and Y are hydrogens and $R^2$ is selected from the group $R^{11}S(O)R^{13}$-, $R^{11}S(O)_2R^{13}$-, $R^{11}C(O)N(R^{12})R^{13}$-, $R^{11}SO_2N(R^{12})R^{13}$-, $R^{11}N(R^{12})C(O)R^{13}$-, $R^{11}N(R^{12})SO_2R^{13}$-, $R^{11}N(R^{12})C(O)N(R^{12})R^{13}$- and heteroalkyl. For example, in the case of Z is -CH=CH- and attached to $C_5$-position in Formula Ib, such compound(s) (**XIII**) can be synthesized by analogous method illustrated in Schemes I & starting with appropriate (**III**), appropriate Fmoc. protected amino acids, appropriate acid chlorides or aldehydes, and hydroxylamine.

**Scheme III**

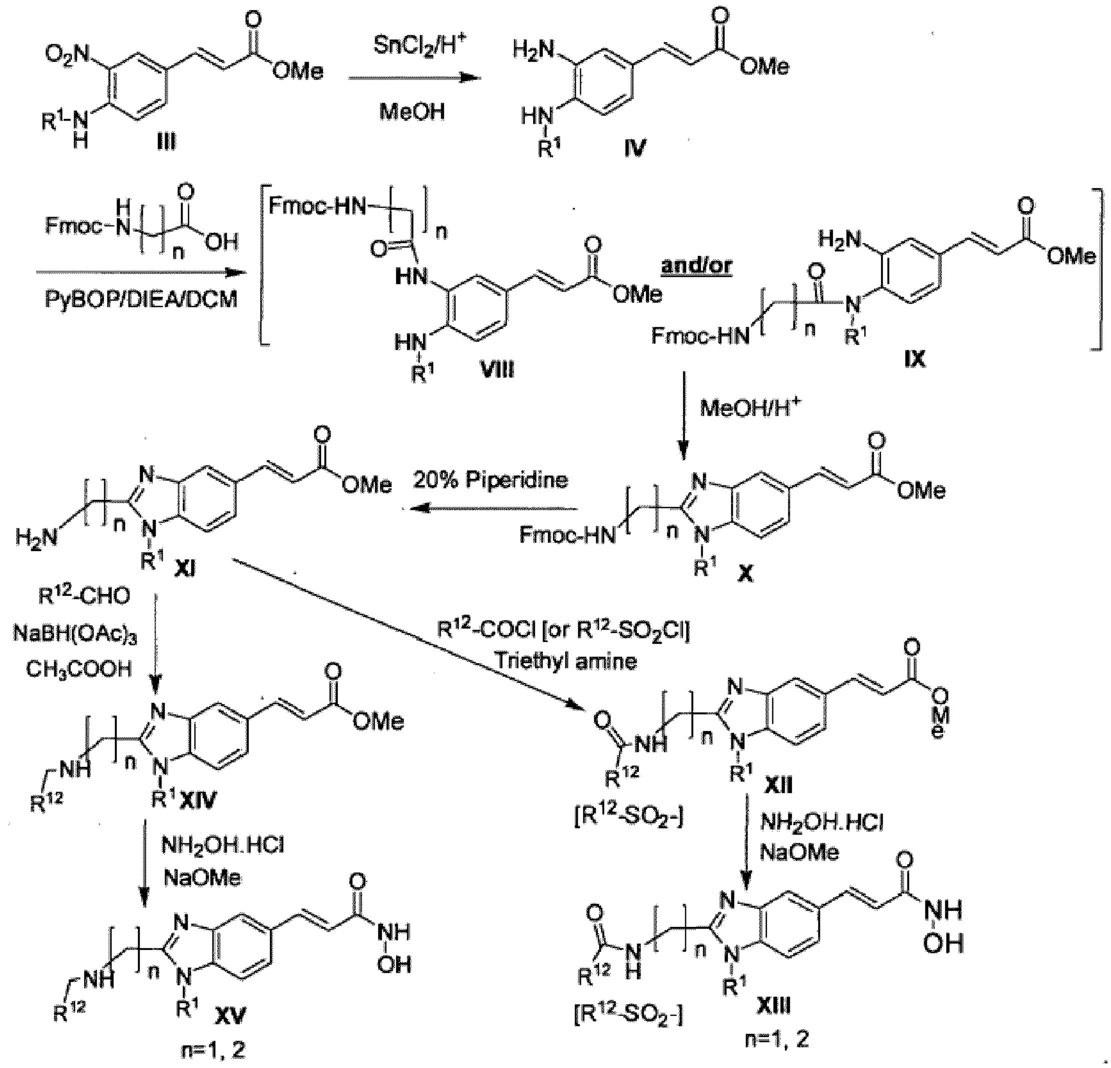


More specifically, for example, the hydroxamate compounds Formula Ib, where X and Y are hydrogens, $R^2$ is selected from the group $R^{11}S(O)R^{13}$-, $R^{11}S(O)_2R^{13}$-, $R^{11}C(O)N(R^{12})R^{13}$-, $R^{11}SO_2N(R^{12})R^{13}$-, $R^{11}N(R^{12})C(O)R^{13}$-, $R^{11}N(R^{12})SO_2R^{13}$-, $R^{11}N(R^{12})C(O)N(R^{12})R^{13}$- and heteroalkyl; and Z is attached to $C_5$-position, can be synthesized by the synthetic route shown in Scheme III. Appropriate intermediate (**III**) was reduced with tin chloride to the corresponding diamines (**IV**). The coupling reaction with appropriate Fmoc protected amino acids in the presence of PyBOP gave coupling product(s) (**VIII**) and/or (**IX**). Without further separation, (**VIII**) and/or (**IX**) were subjected to cyclization under acid conditions and yielded benzimdiazole (**X**). The key intermediate (**XI**) can be obtained by treating (**X**) with 20% piperidine. Treatment of (**XI**) with an appropriate acid chloride or an appropriate sulfonyl chloride gave (**XII**) and the target compounds (**XIII**) were obtained by using similar method described in Scheme I.

When (**XI**) was reacted with an appropriate aldehyde under reduction conditions ($NaBH(OAc)_3/CH_3CO_2H$), (**XIV**) was obtained and can be transformed to corresponding hydroxamate derivatives (**XV**) by the same methods described above.

Scheme IV illustrates some reactions to further modify $R^1$ side chain. If the $R^1$ side chain contained a protecting group such as Boc in compound **(VIa1)**, it could be removed before converting to the final hydroxamic acid **(VIIa).** The intermediate **(VIa)** could be modified by acylation, reductive alkylation, alkylation or sulfonylation to form new analogs **(VIIb, VIIc, VIId** and **VIIe)** through new intermediates **(VIb, VIc, VId** and **VIe).** The above described methods were also applied to $R^1$ = heterocycles, e.g., $R^1$ = *N*-Boc-piperidin-3-yl, *N*-Boc-piperidin-4-yl and *N*-Boc-pyrrolidin-3-yl.

**Scheme IV**

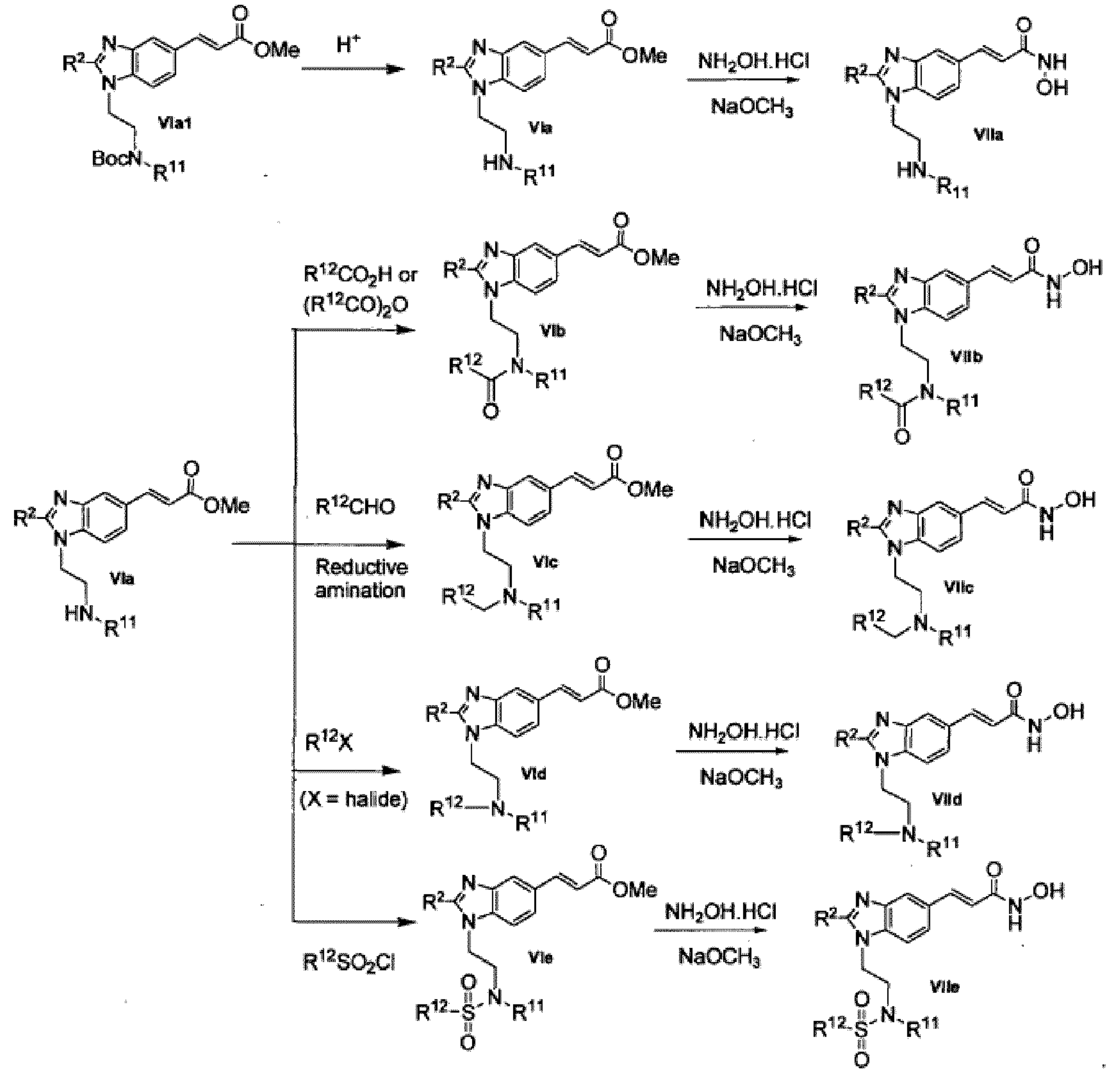


Scheme V illustrates some alternative method to prepare **(VIa)** and **(VIc)**. The primary amine **(IIIa2)** was prepared either from (**Ia**) or via **(IIIa1)**. The derivertization of the amino group (e.g., reductive amination) could be performed either from **(IIIa2)** or **(VIa2).** The products, i.e., **(IIIa2-1)** and **(VIa2-1)**, could be further derivertized (e.g., reductive amination of the secondary amine).

**Scheme V**

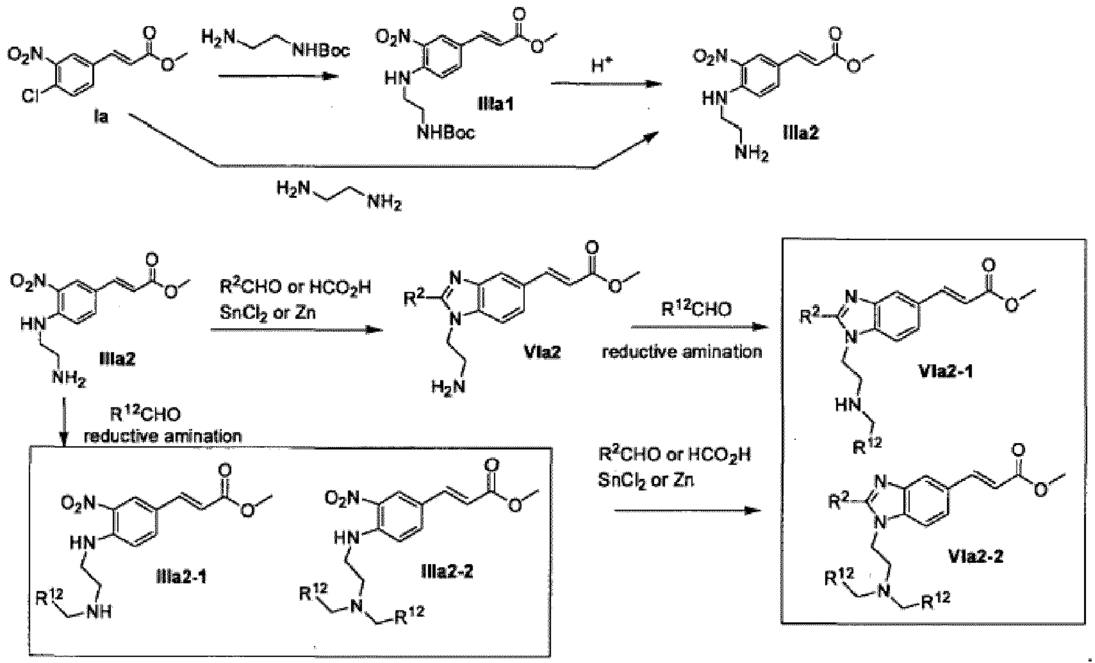


Scheme VI and VII illustrate some alternative methods to prepare (**VI**) by forming the benzimidazole ring first and introducing the double bond later.

In Scheme VI, compound (**XVI**) was reacted with an amine $R^1NH_2$ in the presence of a base (e.g. triethylamine) in an appropriate solvent (e.g. dioxane) to give (**XVII**). Benzimidazole (**XVIII**) ring was formed by reacting compound (**XVII**) with aldehyde $R^2CHO$ in the presence of a reducing agent of nitro group (e.g. tin (II) chloride, zinc powder or other appropriate reducing agent) in one-pot. The ester (**XVIII**) was converted to the aldehyde (**XX**) via a reduction and oxidation process. Finally, (**VI**) was obtained by reacting aldehyde (**XX**) with a Wittig or Wittig-Horner reagent.

**Scheme VI**

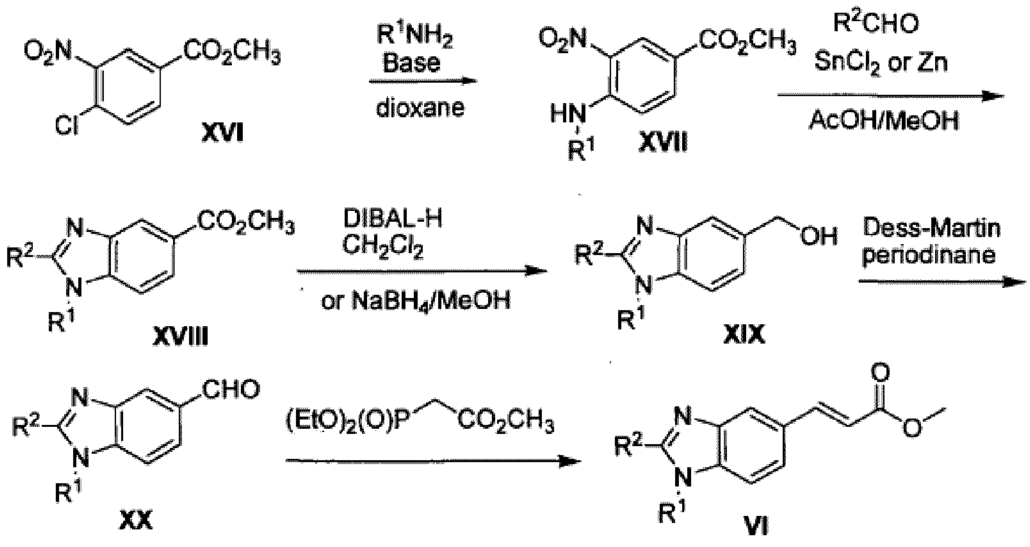


In Scheme VII, compound (**XXI**) was reacted with an amine $R^1NH_2$ in the presence of a base (e.g. triethylamine) in an appropriate solvent (e.g. dioxane) to give (**XXII**). Benzimidazole (**XXIII**) ring was formed by reacting compound (**XXII**) with aldehyde $R^2CHO$ in the presence of a reducing agent of nitro group (e.g. tin (**II**) chloride, zinc powder or other appropriate reducing agent) in one-pot. Finally, the bromide (**XXIII**) was converted to (**VI**) under Heck reaction condition.

**Scheme VII**

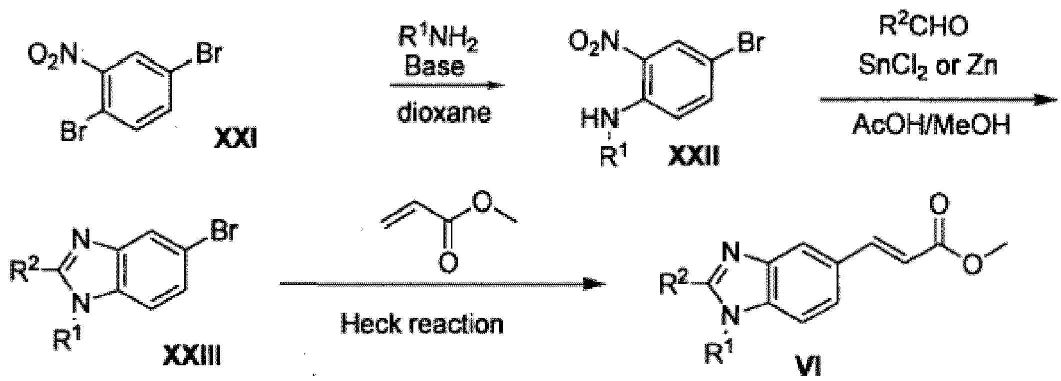


In order to further assist the reader the following preparations and examples are given to enable those skilled in the art synthesis a compound of formula 1.

**Preparation of intermediates III**

Compound **(III)** was prepared either from **(I)** via **(II)** or from **(I)** via **(Ia)** (Scheme I and V). The following are examples of **(III).**

**Intermediate**

**3-[4-(2-Dimethylamino-ethylamino)-3-nitro-phenyl]-acrylic acid methyl ester**

A mixture of 3-(4-chloro-3-nitro-phenyl)-acrylic acid methyl ester (**Ia,** 0.658 g, 2.72 mmol), N,N-dimethylethylenediamine (0.90 mL, 8.20 mmol) and triethylamine (1.2 mL, 8.6 mmol) in dioxane (20 mL) was heated at 80°C for 5h. The solution was evaporated and the residue was added DCM and aqueous $Na_2CO_3$. The DCM (x3) extracts were concentrated and the residue was added EtOAc-hexane. The resulting red solid was filtered to give the titled compound (0.672 g, 84.2%). HPLC purity at 254 nm: 99.2%, $t_R$ = 1.59 min. LCMS (ESI) m/z: 294 ([M + H]$^+$). $^1$H NMR ($CDCl_3$ + $CD_3OD$) δ 8.21 (1H, d, J = 2.1 Hz), 7.56 (1H, dd, J = 9.0, 2.1 Hz), 7.48 (1H, d, J = 16.0 Hz), 6.81 (1H, d, J = 9.0 Hz), 6.20 (1H, d, J = 15.9 Hz), 3.70 (3H, s), 3.34 (2H, t, J = 6.5 Hz), 2.56 (2H, t, J = 6.4 Hz), 2.23 (6H, s); $^{13}$C NMR ($CDCl_3$ + $CD_3OD$) δ 167.3, 145.4, 142.6, 134.0, 131.1, 127.1, 121.3, 114.8, 114.0, 56.7, 51.1, 44.6, 40.1.

**Intermediate 2**

**3-[4-(2-Diethylamino-ethylamino)-3-nitro-phenyl]-acrylic acid methyl ester.**

Yellow solid. LCMS (ESI) m/z: 322 ([M + H]$^+$). $^1$H NMR ($CDCl_3$) δ 8.73 (1H, t-like, J = 4.3 Hz), 8.32 (1H, d, J = 2.0 Hz), 7.62 (1H, dd, J = 9.2, 2.0 Hz), 7.58 (1H, d, J = 15.9 Hz), 6.85 (1H, d, J = 9.0 Hz), 6.29 (1H, d, J = 15.9 Hz), 3.80 (3H, s), 3.35 (2H, td, J = 5.4, 6.0 Hz), 2.77 (2H, t, J = 6.2 Hz), 2.59 (4H, q, J = 7.1 Hz), 1.07 (6H, t, J = 7.1 Hz).

**Intermediate 3**

**3-[4-(2-Ethylamino-ethylamino)-3-nitro-phenyl]-acrylic acid methyl ester**

Red solid. LCMS (ESI) m/z: 294 ([M + H]$^+$). $^1$H NMR (DMSO-$d_6$) δ 8.49 (1H, t, J = 6.1 Hz), 8.35 (1H, d, J = 2.0 Hz), 7.96 (1H, dd, J = 9.1, 1.9 Hz), 7.62 (1H, d, J = 16.0 Hz), 7.20 (1H, d, J = 9.1 Hz), 6.52 (1H, d, J = 16.0 Hz), 3.75 (2H, td, J = 6.5, 6.2 Hz), 3.70 (3H, s), 3.08 (2H, t, J = 6.5 Hz), 2.93 (4H, q, J = 7.2 Hz), 1.17 (6H, t, J = 7.2 Hz).

**Intermediate 4**

**3-[4-(2-Isopropylamino-ethylamino)-3-nitro-phenyl]-acrylic acid methyl ester**

Red solid. LCMS (ESI) m/z: 308 ([M + H]$^+$). $^1$H NMR (DMSO-$d_6$) δ 8.58 (1H, t, J = 5.6 Hz), 8.33 (1H, d, J = 2.0

Hz), 7.94 (1H, dd, J = 9.1, 1.9 Hz), 7.60 (1H, d, J = 16.0 Hz), 7.14 (1H, d, J = 9.2 Hz), 6.49 (1H, d, J = 16.0 Hz), 3.70 (3H, s), 3.56 (2H, masked by water peak, identified by COSY), 3.10 (1H, septet, J = 6.4 Hz), 2.94 (2H, t, J = 6.2 Hz), 1.10 (6H, d, J = 6.4 Hz).

**Intermediate 5**

**3-[4-(3-Dimethylamino-2,2-dimethyl-propylamino)-3-nitro-phenyl]-acrylic acid methyl ester.**

Red solid. LCMS (ESI) m/z: 336 ($[M + H]^+$). $^1H$ NMR ($CDCl_3$) $\delta$ 9.73 (1H, br s or t), 8.33 (1H, d, J = 2.0 Hz), 7.60 (1H, dd, J = 8.9, 2.0 Hz), 7.59 (1H, d, J = 16.1 Hz), 6.88 (1H, d, J = 9.1 Hz), 6.28 (1H, d, J = 15.9 Hz), 3.80 (3H, s), 3.21 (2H, d, J = 4.6 Hz), 2.36 (2H, s), 2.34 (6H, s), 1.04 (6H, s).

**Intermediate 6**

**3-[4-(2-Diisopropylamino-ethylamino)-3-nitro-phenyl]-acrylic acid methyl ester**

Yellow solid. LCMS (ESI) m/z: 350 ($[M + H]^+$). $^1H$ NMR ($CDCl_3$) $\delta$ 8.76 (1H, t-like, J = 4.3 Hz), 8.32 (1H, d, J = 2.0 Hz), 7.61 (1H, dd, J = 8.3, 2.7 Hz), 7.58 (1H, d, J = 15.8 Hz), 6.85 (1H, d, J = 9.0 Hz), 6.29 (1H, d, J = 15.9 Hz), 3.79 (3H, s), 3.31 (2H, td, J = 5.3, 6.1 Hz), 3.08 (2H, septet, J = 6.6 Hz), 2.84 (2H, t, J = 6.2 Hz), 1.07 (12H, d, J = 6.6 Hz).

**Intermediate 7**

**3-[4-(2-Methylamino-ethylamino)-3-nitro-phenyl]-acrylic acid methyl ester**

Red solid. LCMS (ESI) m/z: 280 ($[M + H]^+$). $^1H$ NMR ($CDCl_3$) $\delta$ 8.54 (1H, t-like, J = 4.2 Hz), 8.33 (1H, d, J = 2.1 Hz), 7.63 (1H, dd, *J* = 9.0, 2.2 Hz). 7.59 (1H, d, *J* = 16.0 Hz), 6.90 (1H, d, *J* = 9.0 Hz), 6.31 (1H, d, *J* = 15.9 Hz), 3.80 (3H, s), 3.45 (2H, td, *J* = 5.8, 5.6 Hz), 2.96 (2H, t, *J* = 6.2 Hz), 2.50 (3H, s).

**Intermediate 8**

**3-[4-(2-tert-Butoxycarbonylamino-ethylamino)-3-nitro-phenyl]-acrylic acid methyl ester (IIIa1)**

Step 1:

A suspension of *trans*-4-chloro-3-nitrocinnamic acid **(I,** 5.057 g, 22.22 mmol) in MeOH (40 mL) and DCM (20 mL) was stirred and cooled in a dry-ice/acetone bath. $SOCl_2$ (1.0 mL, 13.8 mmol) was added to the above mixture. Dry-ice bath was removed, then the mixture was warmed to room temperature and stirred at 40 °C till the reaction completed. The solution was evaporated to dryness to a pale yellow solid (5.364 g, 99.9%). HPLC purity at 254 nm: 99.5%; $t_R$ = 2.96 min. LCMS (ESI) m/z: 210 and 212 (very weak signal, $[M+H-MeOH]^+$).

Step 2:

A mixture of 3-(4-chloro-3-nitro-phenyl)-acrylic acid methyl ester **(Ia,** 0.243 g, 1.00 mmol), N-Boc-ethylenediamine (0.316 mL, 2.0 mmol) and triethylamine (0.50 mL, 3.59 mmoL) in dioxane (7 mL) was heated at 80°C for about 80 h. The solution was evaporated and the residue was added MeOH. The resulting solid was filtered and washed with MeOH. 3-[4-(2-tert-Butoxycarbonylamino-ethylamino)-3-nitro-phenyl]-acrylic acid methyl ester **(IIIa1)** was obtained as bright yellow solid (0.193 g, 52.6%). HPLC purity at 254 nm: 96.0~98.1%; $t_R$ = 3.27 min. LCMS (ESI) m/z: 366 ($[M + H]^+$), 310 (M+H-56), 266 (M+H-Boc). $^1H$ NMR ($CDCl_3$) $\delta$ 8.41 (1H, br t like, NHAr), 8.31 (1H, d, J = 1.8 Hz), 7.63 (1H, dd, J = 9.0, 1.7 Hz), 7.57 (1H, d, J = 16.0 Hz), 6.98 (1H, d, J = 8.9 Hz), 6.30 (1H, d, J = 15.9 Hz), 3.80 (3H, s), 3.52 (2H, m), 3.45 (2H, m), 1.45 (9H, s); $^{13}C$ NMR ($CDCl_3$) $\delta$ 166.9, 155.7, 145.8, 142.3, 134.1, 131.5, 127.1, 121.8, 115.4, 113.9, 79.5, 51.2, 42.7, 39.1, 27.9.

**Intermediate 9**

**3-[4-(2-Amino-ethylamino)-3-nitro-phenyl]-acrylic acid methyl ester (IIIa2)**

Method 1:

Remove Boc protecting group from **(IIIa1)** under acidic condition: 1) HCl/MeOH; 2) TFA/DCM.

Method 2:

To the ester **(Ia,** 2.47 g, 10.2 mmol) in dioxane (102 mL, 0.1 M) was added ethylenediamine (Merck. Product no. 8.00947, 2.04 mL, 30.6 mmol) followed by triethylamine (2.8 mL, 20.47 mmol). The resulting mixture was heated to 90 ❑ C and stirred for 20 hours. The completion of reaction was confirmed by using HPLC (where the product **IIIa2** $\mathbf{t_R}$ = 1.6 min, starting material **Ia** $t_R$ = 3.1 min). Upon completion, solvent was removed and the crude was dissolved in DCM. The solution was washed with water, brine, dried over $Na_2SO_4$ and filtered. The filtrate after removal of the solvent gave the titled compound **IIIa2.** Yield = 98 %, LCMS m/z: 266 ($[M+H]^+$).

**Synthesis 1**

**Preparation of 3-[1-(3-Dimethylamino-2,2-dimethyl-propyl)-2-(2,2-dimethyl-propyl)-1H-benzimidazol-5-yl]-N-hydroxy-acrylamide (1)**

The titled compound (1) was prepared according to Scheme I and II, by using appropriate starting materials.

Step 1:

To a pre-stirred solution of *trans*-4-chloro-3-nitrocinnamic acid **(I,** 11g, 48 mmol) in dioxane (200 mL) was added triethylamine (20 mL, 126 mmol), followed by 3-dimethylamino-2,2-dimethylpropylamine (20 mL, 143 mmol). The reaction mixture was allowed to stir at 100 °C for 1-2 days till all starting material was fully converted. Then, the solvent was removed under vacuum followed by the addition of $H_2O$ (250 mL) to dissolve the residue. Conc. HCl was added till pH ≈ 1 with orange precipitation. The suspension was filtered and residue was washed with $H_2O$ several times to obtain (**II**) as orange solid (13 g, 84%). LCMS (ESI) m/z: 322 ($[M+H]^+$).

Step 2:

Compound (II, 13 g, 40.5 mmol) was dissolved in MeOH (250 mL) followed by the addition of conc. $H_2SO_4$ (5 mL). The reaction mixture was allowed to stir at 80 °C for 18 h. Solvent was removed under vacuum and $H_2O$ (250 mL) was added to dissolve the residue. $Na_2CO_3$ was added till pH ≈ 8-9, subsequently, MeOH was added and stirred for 1 hour. Then, the suspension was filtered under vacuo and the residue was washed with $H_2O$ several times to obtain ester (**III**) as orange solid (10 g, 74%). LCMS (ESI) m/z: 336 ($[M+H]^+$).

Step 3:

To a stirred solution of ester (**III**, 1 equiv) and $SnCl_2$•$2H_2O$ (5 equiv) in AcOH and MeOH (0.2 M, 1:9 mixture) was added 3,3-dimethyl butyraldehyde (1.5 equiv). The resulting mixture was heated to 45 °C with stirring. The progress of the reaction was monitor by LC/MS. When the reaction was completed, solvent was removed under reduced pressure at 30-35°C. To the resulting residue, 20 mL of water and 20 mL of ethyl acetate were added at room temperature, the pH value of the mixture was carefully adjusted to 9-10 by addition of conc. $NH_3$•$H_2O$. The mixture was stirred for half an hour, followed by centrifuge if necessary to separate the organic layer. The organic layer was collected. The aqueous phase and residue (oily-solid precipitate) were extracted another 3 times more with ethyl acetate as described above. The combined organic contents were dried over sodium sulphate, filtered and evaporated to dryness. The resulting oily residue was purified by flash column chromatography (isolated yield of cyclized product (**VI**) varies between 50-90%). LCMS (ESI) m/z: 386 ($[M+H]^+$).

Step 4:

To a stirred solution of ester (**VI**) and $NH_2OH.HCl$ (10 equiv.) in MeOH (0.5M) was added NaOMe (20 equiv.) at - 78 °C. The reaction mixture was then allowed to warm up slowly to room temperature. The reaction was monitored by LC/MS and was completed in around 15 min. 1*N* HCl was then added slowly into the reaction mixture at 0 °C. The desired product was separated by prep-HPLC and the fractions containing the desired product were freeze-dried. Product (**VII**) was obtained as TFA salt (isolated yield varies between 40 - 70%). HPLC purity at 254 nm: 100%, $t_R$ = 0.78 min. LCMS (ESI) m/z: 387 ($[M+H]^+$). $^1H$ NMR (DMSO-$d_6$) δ 1.05 (15H, s), 2.91 (6H, s), 2.92 (2H, s), 3.32 (2H, bs), 4.35 (2H, s), 6.49 (1H, d, J = 15.8 Hz), 7.56 (1H, d, J = 9.0 Hz), 7.61 (1H, d, J = 15.76 Hz), 7.83 (1H, d, J = 9.0 Hz)., 7.85 (1H, s), 9.22 (1H, bs), 10.72 (1H, bs); $^{13}C$ NMR (DMSO-$d_6$) δ 162.6, 154.2, 138.0, 135.3 (br), 134.7, 131.5, 122.8, 119.2, 115.2, 114.0, 66.5, 51.1, 46.7, 38.4, 38.3, 33.6, 29.1, 22.8.

**Synthesis 2**

**Preparation of 3-[1-(3-Dimethylamino-2,2-dimethyl-propyl)-2-isopropyl-1H-benzimidazol-5-yl]-N-hydroxy-acrylamide (2)**

The titled compound **(2)** was prepared according to the procedures described in Synthesis 1, by using appropriate starting materials. HPLC purity at 254 nm: 100%, $t_R$ = 0.54 min. LCMS (ESI) m/z: 359 ($[M+H]^+$). $^1H$ NMR (DMSO-$d_6$) δ 1.05 (6H, s), 1.40 (6H, d, J = 6.36 Hz), 2.92 (6H, s), 3.36 (2H, s), 3.58 (1H, m, J = 6.4 Hz), 4.44 (2H, s), 6.55 (1H, d, J = 15.8 Hz), 7.63 (1H, d, J = 15.8 Hz), 7.66 (1H, d, J = 8.7 Hz), 7.95 (1H, d, J = 8.7 Hz), 7.90 (1H, s), 9.71 (1H, bs), 10.80 (1H, bs).

**Synthesis 3**

**Preparation of 3-[2-Butyl-1-(3-dimethylamino-2,2-dimethyl-propyl)-1H-benzimidazol-5-yl]-N-hydroxy-acrylimide (3)**

The titled compound **(3)** was prepared according to the procedures described in Synthesis 1, by using appropriate starting materials. Yield: 74 mg as TFA salt. HPLC purity at 254 nm: 99.0%, $t_R$ = 0.89 min. LCMS (ESI) m/z: 373 ($[M + H]^+$). $^1H$ NMR ($CD_3OD$) δ 7.99 (1H, d, J = 8.8 Hz), 7.84 (1H, s), 7.72 (1H, d, J = 8.7 Hz), 7.55 (1H, d, J = 15.8 Hz), 6.53 (1H, d, J = 15.7 Hz), 4.55 (2H, s), 3.43 (2H, s), 3.24 (2H, overlapped with $CD_2HOD$), 3.00 (6H, s), 1.90 (2H, quintet, J = 7.2 Hz), 1.49 (2H, m), 1.21 (6H, s), 0.98 (3H, t, J = 7.3 Hz); $^{13}C$ NMR ($CD_3OD$) δ 165.5 (br), 158.2, 139.8, 135.3, 135.1, 132.4, 126.4, 120.6 (br), 115.6, 114.3, 68.7, 53.5, 47.8 (Mex2), 39.5, 29.9, 27.2, 23.6 (Mex2), 23.3, 13.9.

**Synthesis 4**

**Preparation of 3-[1-(3-Dimethylamino-2,2-dimethyl-propyl)-2-(2-methylsulfanyl-ethyl)-1H-benzimidazol-5-yl]-N-hydroxy-acrylamide (4)**

The titled compound **(4)** was prepared according to the procedures described in Synthesis 1, by using appropriate starting materials. Yield: 17 mg as TFA salt. HPLC purity at 254 nm: 96.2%, $t_R$ = 0.75 min. LCMS (ESI) m/z: 391 ($[M+H]^+$). $^1H$ NMR ($CD_3OD$) δ 8.02 (1H, d, *J*= 8.3 Hz), 7.92 (1H, s), 7.80 (1H, d, *J* = 8.7 Hz), 7.69 (1H, d, *J* = 15.8 Hz), 6.60 (1H, d, *J* = 15.8 Hz), 4.49 (2H, s), 3.50 (2H, t, *J* = 7.2 Hz), 3.37 (2H, s), 3.03 (2H, t, *J* = 7.2 Hz), 2.95 (6H, s), 2.18 (3H, s), 1.25 (6H, s); $^{13}C$ NMR ($CD_3OD$) δ 163.7, 154.6, 138.2, 133.9, 132.8, 132.5, 124.1, 118.2, 113.3, 113.2, 66.7, 51.5, 45.9 (Mex2), 37.6, 29.9, 26.2, 21.7 (Mex2), 13.7.

**Synthesis 5**

**Preparation of 3-[1-(3-Dimethylamino-2,2-dimethyl-propyl)-2-isobutyl-1H-benzimidazol-5-yl]-N-hydroxy-acrylamide (6)**

The titled compound (**6**) was prepared according to the procedures described in Synthesis 1, by using appropriate starting materials. HPLC purity at 254 nm: 96.2%, $t_R$ = 0.82 min. LCMS (ESI) m/z: 373 ($[M+H]^+$). $^1H$ NMR (DMSO-$d_6$): δ 10.80 (1H, s), 9.47 (1H, s), 7.93 (1H, s), 7.90 (1H, d, *J* = 6.6 Hz), 7.64 (1H, d, *J* = 7.4 Hz), 7.62 (1H, d, *J* = 15.5 Hz), 6.54 (1H, d, *J* = 15.8 Hz), 4.39 (2H, s), 3.33 (2H, s), 2.97 (2H, d, *J* = 7.26 Hz), 2.92 (6H, s), 2.35 (1H, qn), 1.09 (6H, s), 0.97 (6H, d, *J* = 6.6 Hz).

**Synthesis 6**

**Preparation of 3-[1-(2-Diethylamino-ethyl)-2-isobutyl-1H-benzimidazol-5-yl]-N-hydroxy-acrylamide (7)**

The titled compound (7) was prepared according to the procedures described in Synthesis 1, by using appropriate starting materials. HPLC purity at 254 nm: 99.0%, $t_R$ = 0.56 min. LCMS (ESI) m/z: 359 ($[M+H]^+$). $^1H$ NMR (DMSO-$d_6$): δ 10.81 (1H, s), 10.13 (1H, s), 7.90 (1H, s), 7.81 (1H, d, *J* = 8.5 Hz), 7.66 (1H, d, *J* = 8.6 Hz), 7.61 (1H, d, *J* = 15.8 Hz), 6.53 (1H, d, *J* = 15.8 Hz), 4.72 (2H, t, *J* = 7.8 Hz), 3.30 (2H, d), 2.93 (2H, d, *J* = 7.2 Hz), 2.27 (1H, m), 1.24 (6H, t, *J* = 7.2 Hz), 0.97 (6H, d, *J* = 6.6 Hz) $^{13}C$ NMR (DMSO-$d_6$) δ 162.7, 158.5, 158.2, 155.2, 138.4, 133.9, 131.0, 123.0, 118.6, 116.0, 111.6, 48.8, 46.8, 34.1, 27.1, 22.2, 8.5.

**Synthesis 7**

**Preparation of 3-(2-Butyl-1-(2-diethylamino-ethyl)-1H-benzimidazol-5-yl]-N-hydroxy-acrylamide (8)**

The titled compound **(8)** was prepared according to the procedures described in Synthesis 1, by using appropriate starting materials. Yield: 61 mg (20% in two steps) as TFA salt. HPLC purity at 254 nm: 98.1%, $t_R$ = 0.59 min. LCMS (ESI) m/z: 359 ($[M + H]^+$). $^1H$ NMR ($CD_3OD$) δ 7.94 (1H, d, J = 8.6 Hz), 7.85 (1H, s), 7.76 (1H, d, J = 8.5 Hz), 7.50 (1H, d, J = 15.7 Hz), 6.49 (1H, d, J = 15.7 Hz), 4.96 (2H, overlapped with DHO, identified by COSY), 3.69 (2H, t-like, J = 7.6 Hz), 3.44 (4H, q, J = 7.6 Hz), 3.26 (2H, t, J = 7.9 Hz), 1.94 (2H, quintet, J = 7.5 Hz), 1.57 (2H, m), 1.40 (6H, t, J = 7.2 Hz), 1.05 (3H, t, J = 7.3 Hz); $^{13}C$ NMR ($CD_3OD$) δ 165.5, 157.7, 140.0, 134.8, 134.0, 133.8, 126.5, 119.9, 115.1, 113.6, 50.2, 48.7 (2C), 40.5, 29.4, 26.6, 23.3, 13.9, 8.9 (2C). (TFA peak 163.4. 163.0, 162.7, 162.3; 122.3, 119.5, 116.6).

Dihydrochloride salt of **8** was prepared according to the procedures described below: methyl (2*E*)-342-butyl-1[2-(diethylamino)ethyl]-1*H*-benzimidazol-5-yl}acrylate (3.4g, 9.6 mmol, which was prepared according to the procedures described in Synthesis 1, step 3, by using appropriate starting materials) was mixed with hydroxylamine hydrochloride (6.7g, 96 mmol) in MeOH (~3.0 mL) with stirring at room temperature. The reaction mixture was cooled over dry ice, followed by the addition of sodium methoxide (30% in MeOH, ~5.4 M, 44 mL, 193 mmol). The mixture was then allowed to warm up slowly to room temperature. The reaction was monitored by LCMS and was completed in ~30 min. The reaction mixture was cooled in a ice water bath and slowly acidified with 3M hydrochloric acid to pH ~5. Deionized water was added until a clear solution was obtained. The pH value of the aqueous solution was carefully adjusted to ~8 using 1N NaOH, the precipitate that was formed was collected by filtration. The solid residue was washed with water 3 times. The above solid was suspended in methanol and water again and was treated with 6N HCl until all dissolved, the pH value was carefully adjusted to ~8 using 1N NaOH. The precipitate that was formed was again collected by filtration. The freebase was suspended in methanol and was treated with 6N HCl (3eq) (pH ~2). The solution became clear and was concentrated on a rotovap. The residue was further dried by co-evaporating with toluene to dryness. The hydrochloride salt was recrystallized from methanol/ethyl acetate (3:7 v/v) and obtained as white solid or powder, LC-MS m/z 359 ($[M+H]^+$). $^1H$ NMR (DMSO-$d_6$) δ 11.79 (brs, 1H), 10.92 (very br s, 1H), 8.18 (1H, d, J = 8.6 Hz), 7.97 (1H, s), 7.79 (1H, d, J = 8.6 Hz), 7.64 (1H, d, J = 15.8 Hz), 6.65 (1H, d, J = 15.8 Hz), 5.01 (2H, t-like, J = 7.7 Hz), 3.48 (2H, m), 3.30-3.19 (6H, m), 1.87 (2H, quintet, J = 7.8 Hz), 1.47 (2H, sextet, J = 7.5 Hz), 1.29 (6H, t, J = 7.2 Hz), 0.97(3H, t, J = 7.3 Hz); $^{13}C$ NMR (DMSO-$d_6$) δ 162.3, 156.0, 137.3 (CH), 132.8, 132.3, 132.0 (br, identified by HMBC), 124.7 (CH), 120.2 (CH), 113.1 (2xCH), 48.2, 46.3, 39.0, 28.1, 25.0, 21.7, 13.6, 8.3.

**Synthesis 8**

**Preparation of 3-{1-[2-(3,3-Dimethyl-butylamino)-ethyl]-2-propyl-1H-benzimidazol-5-yl)-N-hydroxy-acrylamide (9)**

The titled compound (**9**) was prepared according to the synthetic route described in Scheme V and procedures described in Synthesis 1, by using appropriate starting materials.

TFA salt of **9**: HPLC purity: 99.9%; LCMS m/z: 373 ($[M+H]^+$). $^1H$ NMR ($CD_3OD$) δ 7.89 (d, 1H, *J* = 8.6 Hz), 7.81 (s, 1H), 7.76 (d, 1H, *J* = 8.6 Hz), 7.44 (d, 1H, *J* = 15.7 Hz), 6.44 (d, 1H, *J* = 15.7 Hz), 4.81 (t, 2H, *J* = 7.0 Hz), 3.65 (t, 2H, *J* = 6.4 Hz), 3.23 - 3.19 (m, 2H), 3.16 - 3.12 (m, 2H), 2.01 - 1.94 (m, 2H), 1.65 -1.61 (m, 2H), 1.16 (t, 3H, 7.3 Hz), 0.96 (s, 9H).

Dihydrochloride salt of **9** was prepared according to the procedures described in Synthesis 7, by using appropriate starting materials. HPLC purity: 98.1 %; LCMS m/z: 373 ($[M+H]^+$). $^1H$ NMR (DMSO-$d_6$) δ 10.89 (1H, br s), 9.77 (2H, b, $-NH_2^+-$), 8.12 (1H, d, J = 8.6 Hz), 7.97 (1H, s), 7.78 (1H, d, J = 8.5 Hz), 7.64 (1H, d, J = 15.8 Hz), 6.64 (1H, d, J = 15.8 Hz), 4.88 (2H, t, J = 5.8 Hz), 3.41 (2H, m), 3.26 (2H, t, J = 7.6 Hz), 2.91 (2H, m), 1.90 (2H, sextet, J = 7.6 Hz), 1.56 (2H, m), 1.05 (3H, t, J = 7.3 Hz), 0.88 (9H, s); $^{13}C$ NMR (DMSO-$d_6$) δ 162.4, 155.9, 137.4 (CH), 132.8, 132.4, 131.8 (br),

124.6 (CH), 120.2 (CH), 113.2 (CH), 113.0 (CH), 44.9, 44.0, 41.1, 38.6, 29.4 (Cq), 28.9, 27.1, 19.9, 13.5.

The procedures described provide the following compounds. Modification of the procedures or of the starting materials provides access to a wide range of benzimidazole compounds of this type.

| Compound No | Structure | m/z [M+H]+ | NAME |
|---|---|---|---|
| 1 | 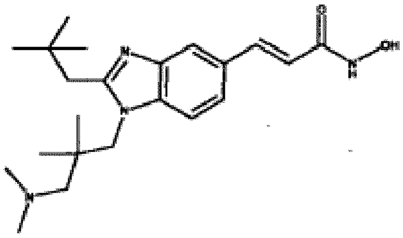 | 387 | 3-[1-(3-Dimethylamino-2,2-dimethyl-propyl)-2-(2,2-dimethyl-propyl)-1H-benzimidazol-5-yl]-N-hydroxy-acrylamide |
| 2 | 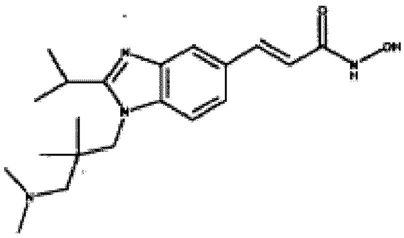 | 359 | 3-[1-(3-Dimethylamino-2,2-dimethyl-propyl)-2-isopropyl-1H-benzimidazol-5-yl]-N-hydroxy-acrylamide |
| 3 | 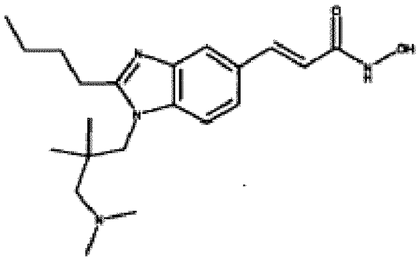 | 373 | 3-[2-Butyl-1-(3-dimethylamino-2,2-dimethyl-propyl)-1H-benzimidazol-5-yl]-N-hydroxy-acrylamide |
| 4 | 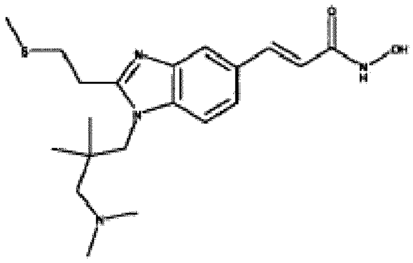 | 391 | 3-[1-(3-Dimethylamino-2,2-dimethyl-propyl)-2-(2-methylsulfanyl-ethyl)-1H-benzimidazol-5-yl]-N-hydroxy-acrylamide |
| 5 | 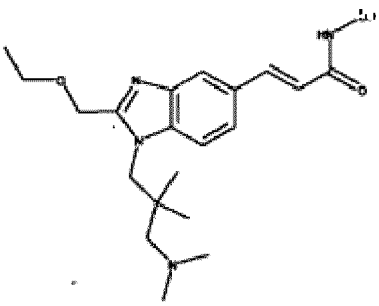 | 375 | 3-[1-(3-Dimethylamino-2,2-dimethyl-propyl)-2-ethoxymethyl-1H-benzimidazol-5-yl]-N-hydroxy-acrylamide |

(continued)

| Compound No | Structure | m/z [M+H] + | NAME |
|---|---|---|---|
| **6** | 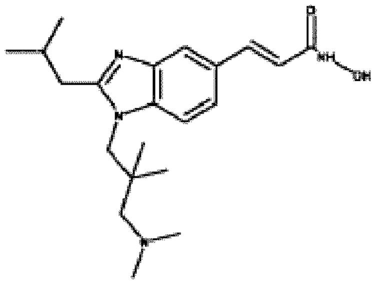 | **373** | 3-[1-(3-Dimethylamino-2,2-dimethyl-propyl)-2-isobutyl-1H-benzimidazol-5-yl]-N-hydroxy-acrylamide |
| **7** | 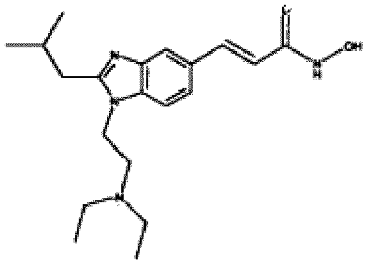 | **359** | 3-[1-(2-Diethylamino-ethyl)-2-isobutyl-1H-benzimidazol-5-yl]-N-hydroxy-acrylamide |
| **8** | 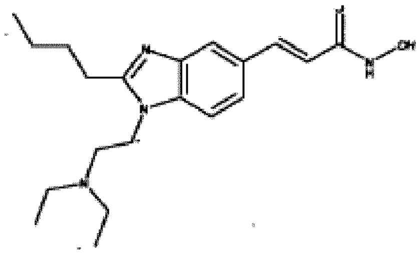 | **359** | 3-[2-Butyl-1-(2-diethylamino-ethyl)-1H-benzimidazol-5-yl]-N-hydroxy-acrylamide |
| **9** | 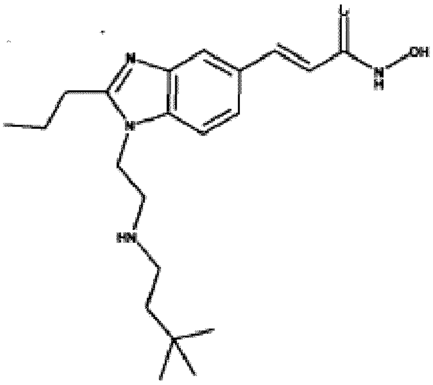 | **373** | 3-{1-[2-(3,3-Dimethyl-butylamino)-ethyl]-2-propyl-1H-benzimidazol-5-yl}-N-hydroxy-acrylamide |

**EXAMPLES**

The present invention will now be described with reference to a number of examples, which demonstrate the efficacy of the invention. The examples illustrate preferred embodiments of the invention and in no way should they be construed, however, as limiting the broad scope of the invention described herein.

In order to show the efficacy of the combination of a benzimidazole based anti-cancer agent and another anti-cancer agent the dihydrochloride salt of compound (8) (hereinafter called Compound A8) was chosen. This has been evaluated in a number of cancer cell lines using different methods (simultaneous or sequential), concentrations, ratios and/or schedules of treatments as well as *in vivo.*

### 1.1 Anti-cancer agents

The anti-cancer agents used in combination studies with Compound (A8) were as shown in Table 1.

**Table 1: Class of anti-cancer agents**

| Class of anti-cancer agents | Name |
|---|---|
| Antitumor antibiotics | Adriamycin/Doxorubicin/Hydroxydaunarubicin |
| Anti-metabolite | Fluorouracil [5FU], Gemcitabine |
| Alkylating agent | Oxaliplatin, Carboplatin, Satraplatin |
| HDAC inhibitors | SAHA |
| Kinase inhibitors | Iressa, Erlotinib (Tarceva) |
| Antibody | Rituximab |
| Topoisomerase inhibitors | Irinotecan |

### 1.2 Cancer cells

The human cancer cells used for the combination experiments included leukemia cell lines (K562, Hut78, HL60), lymphoma cell line (Ramos), multiple myeloma cell line (IM-9) lung cancer cell lines (H1975, H1650, H460, A549), liver cancer cell lines (Hep3B, SK-Hep1), gastric cancer cell lines (AGS, SNU-5, KATO-III), prostate cancer cell lines (PC3, DU145), colon cancer cell lines (HCT116), breast cancer cell line (MCF7). They were obtained from either ATCC or ECACC and were cultivated according to the manufacturer's instructions.

### 1.3 Identification of a constant molar ratio and concentration for a combined drug using the checker box method

After sub-confluence growth, DU145 and PC3 were seeded in 96-well plates at 1000 cells/well. AGS, A549, KATO-III and MCF7 were seeded in 96-well plates at 2000 cells/well. K562, Hep3B, SK-Hep1 and H460 were seeded in 96-well plates at 4000 cells/well. HCT116, HL60 and Ramos were seeded in 96-well plates at 3000, 8000 and 20,000 cells/well respectively. The plates were incubated at 37°C, 5% $CO_2$, for 24h. Cells were simultaneous treated with compounds at 4 different concentrations for 96h. For sequential treatment, cells were treated with the first y agent for 24h and subsequently treated with the second agent for 72h. Each compound and each concentration were tested in duplicates. The non-viable cells were monitored using the fluorescent YO-PRO® assay format (Idziorek, et al 1995) and/or subsequently confirmation using the standard flow cytometry based Annexin V assay following the instructions of the manufacturer (Becton Dickinson).

A good combined constant molar ratio and concentration was selected when the percentage of non-viable of the combined drugs, drug 1 and drug 2, shall be at least twice better than that of either drug 1 or drug 2. A dose response based on the selected constant molar ratio and concentration of the combined drugs was subsequently performed.

### 1.4 Determination of cell death and/or cell growth inhibition

Compound (A8) was used as an example of a benzimidazole based anti-cancer agent. Cell death following treatment with Compound (A8), anti-cancer agent and/or in combination was determined using YO-PRO® assay, which is based on the increased permeability of the cell membrane during the course of apoptosis. The YO-PRO® dye is a green fluorescent DNA staining probe, which can selectively enter apoptotic cells.

Briefly, after the treatment incubation, 25 $\mu$l of the YO-PRO® dye solution, containing 100 mM sodium citrate, pH4.0, 134 mM sodium chloride and 12.4 $\mu$M fluorescent YO-PRO® dye (YO-PRO®-1 iodide, Molecular probe) was added to the 100 $\mu$l medium in the treated cell well of the 96-well plate. The plate was incubated at room temperature in the dark with gently shaking for 10 min. The uptake of the fluorescent YO-PRO® dye into the cells was assessed by a first measurement using the Wallac Victor2 plate reader (Perkin Elmer Pte Ltd) at the excitation (nm) 485/20 and emission (nm) 530/25. After the first reading, 25 ul of the lysis buffer containing 20 mM sodium citrate, pH 4.0, 26.8 mM sodium chloride, 0.4% NP-40, 20 mM EDTA and 20 mM EGTA was added to each well. The plate was incubated subsequently for 30 min at room temperature in the dark with gently shaking. The total amount of the fluorescent YO-PRO® bound DNA was determined by a second measurement using the plate reader as described previously. The second readout leads to assessment of cell proliferation based on the intercalation of to YO-PRO® dye into the DNA.

The percentage of non-viable cell at each concentration was determined as follow;

$$\{[(\text{Treatment-1}^{st}\text{-read - blank})/(\text{Treatment-2}^{nd}\text{-read - blank})]*100)- \{[(\text{Control-1}^{st}\text{-read - blank})/(\text{Control-2}^{nd}\text{-read - blank})]*100\}.$$

Alternatively, the confirmation of cell death following the treatment could be determined by the Annexin V method (BD Pharmingen). Briefly, Annexin V (Mr 36-kDa), a member of the annexin family of calcium-dependent phospholipid binding proteins, has a high affinity for PS-containing phospholipid bilayers. When PS is exposed on the extracellular face of a cell membrane, annexin V binds with an affinity and can be used to monitor changes in cell membrane phospholipid asymmetry, thereby providing a convenient tool for detection of apoptotic cells. Since externalization of PS occurs earlier than the nuclear changes associated with apoptosis, the Annexin V Apoptosis Kit can be used for detection of cells earlier in the apoptotic pathway than do DNA-based assays.

**1.5 Dose response based on the constant molar ratio of a combined drug**

The cancer cell lines used for a dose response were prepared. They were simultaneous - and/or sequential treated with drug 1 and drug 2. For each constant molar ratio, 9 different concentrations of the two drugs (2 fold dilution) were used. Each single drug is also tested in the same manner. Each treatment was done in triplicates. After 96h of treatment incubation, the non-viable cells were monitored using the fluorescent YO-PRO® assay and the percentage of non-viable cells was calculated as previously described. Subsequently the data of percentage of non-viable cells at each concentration point *were* converted into the fraction affected by the dose ($f_a$; %non-viable cells/100). These data were then transported to CalcuSyn software (BIOSOFT Pte Ltd) for further combined dose-effect data analysis.

**1.6 Determination of combination index (CI) values using the software CalcuSyn (Biosoft, Cambridge, UK) and data analyses**

Combination index values were determined by the Calcusyn software according to the manufactures instructions. Different mathematical models are used for this evaluation of drug interactions. The CI method is based on the median-effect equation correlating biological effects such as cytotoxicity with the drug dose.

Drug combination effects were studied using the more widely used multiple drug-effect equation of Chou and Talalay (1984). The CI according to Chou and Talalay (Chou and Hayball, 1996) is defined as:

$$CI = (D_1/D_{x1}) + (D_2/D_{x2}) + [(D_1)(D_2)/ (D_{x1})( D_{x2})$$

when combined drugs have different modes-of-action. D1, D2: the concentration of drug 1, drug 2 in the combination experiment that gives an inhibition of x %. Dx1, Dx2: the concentration of drug1, drug2 alone giving an inhibition of x%. The interaction is considered synergistic if CI < 1, additive if CI = 1, antagonistic if CI > 1. The synergism here is defined as a more than expected additive effect, and antagonism is defined as a less than expected additive effect. The CI values for the 2 drugs were determined using CalcuSyn™ (BIOSOFT). The software calculates CI values at each drug concentration.

**Example 1: Combination of a Benzimidazole Based Anti-Cancer Agent and an Anti-Cancer Monoclonal Antibody**

In order to determine whether Compound (A8), an example of a benzimidazole-based anti-cancer agent, has the potential in combination therapies with the anti-cancer monoclonal antibody, its combination with Rituximab was studied. Compound (A8) and Rituximab, when simultaneously combined at the concentrations of 100 nM and 3 μg/ml respectively, (Table 3) results in synergistic effect on Ramos cell.

**Table 3:** Example of a synergistic combination of Compound (A8) and an anti-cancer monoclonal antibody (Rituximab)

| Cell line | Cell type | Anti-cancer agents | Methods of treatment and concentrations of treatment | Results of combination | CI* at $ED_{50}$ |
|---|---|---|---|---|---|
| Ramos | Lymphoma | (A8) and Rituximab | Simultaneous treatment at the concentrations of 100 nM Compound (A8) and 3 μg/ml Rituximab | Synergistic effect | 0.11 -0.62 (n=2) |

Ramos lymphoma cells were co-treated with 100 nM of Compound (A8) and 3 μg/ml of Rituximab for 96h. The non-viable Ramos cells were then monitored using the YO-PRO® assay format. The co-treatment of these two compounds resulted in synergistic effect.

In order to evaluate whether the schedule of treatment of Compound (A8) and Rituximab had significant effect on Ramos cells, the Ramos cells (1 x $10^5$ cells/ml) were pretreated with 125 nM of Compound (A8) for 24 h, then treated with 6 μg/ml of Rituximab for 72h. The non-viable cells were monitored using the Annexin V assay format as described previously. A good synergistic effect was observed for this schedule with the CI value of 0.35.

**Example 2: Combination of Benzimidazole Based Anti-Cancer Agent and A Number of Known Anti-Cancer Agents *In Vitro***

In order to determine the potential clinical use of benzimidazole-based anti-cancer agent, an example of such an agent, Compound (A8) is used in combination with a number of anti-cancer agents in different cancer cell lines.

The results of the studies carried out are shown in table 4.

**Table 4:** Examples of synergistic combinations of Compound (A8) and different anti-cancer agents in different cancer cell lines

| Cancer type | Cell line | Treatment | Regime | Ratio of compounds | CI at $ED_{50}$ |
|---|---|---|---|---|---|
| Leukemia | K562 (CML) | (A8) and Dasatinib | Simultaneous | 1:2222 Dasatinib: (A8) | 0.57 ± 0.07 (n = 3) |
| | | | (A8) prior to Dasatinib | 2222:1 (A8): Dasatinib | 0.78 ± 0.08 (n = 3) |
| Lymphoma | IM-9 (Lympho ma) | (A8) and Vinblastine | * Simultaneous | Vinblastine: (A8) 40:1 | 0.7-0.9 (n=2) |
| Lung cancer | H1975 (Lung) | (A8) and Tarceva (Erlotinib) | Simultaneous | 5.37:1 (A8): Tarceva | 0.66 ± 0.04 (n = 3) |
| | | | (A8) prior to Tarceva | 0.084:1 (A8): Tarceva | 0.75 ± 0.09 (n=3) |
| | H1650 (Lung) | (A8) and Tarceva (Erlotinib) | Simultaneous | 0.36:1 (A8): Tarceva | 0.56 ± 0.22 (n =3) |
| | | | (A8) prior to Tarceva | 0.36:1 (A8): Tarceva | 0.42 ± 0.24 (n = 3) |
| | | (A8) and Irresa | Simultaneous | 0.216:1 (A8): Irresa | 0.56 ± 0.10 (n = 3) |
| | | | (A8) prior to Irresa | 0.216:1 (A8): Irresa | 0.43 ± 0.06 (n = 3) |
| | | | Irresa prior to (A8) | 1:0.216 Irresa: (A8) | 0.65 ± 0.28 (n = 3) |
| Liver Cancer | Hep3B (Liver) | (A8) and Doxorubicin | Simultaneous | 1:0.5 Doxorubicin: (A8) | 0.6 ± 0.26 (n = 3) |
| | | | (A8) prior to Doxorubicin | 0.5:1 (A8): Doxorubicin | 0.30 ± 0.10 (n = 3) |
| | | | Doxorubicin prior to (A8) | 1:0.5 Doxorubicin: (A8) | 0.63 ± 0.21 (n = 3) |

(continued)

| Cancer type | Cell line | Treatment | Regime | Ratio of compounds | CI at $ED_{50}$ |
|---|---|---|---|---|---|
| Gastric cancer | AGS (Gastric) | (A8) and 5FU | Simultaneous | 1:80 (A8):5-FU | 0.38 ± 0.08 (n = 3) |
| | | | (A8) prior to 5FU | 1:80 (A8):5-FU | 0.54 ± 0.21 (n = 3) |
| | | | 5FU prior to (A8) | 80:1 5-FU:(A8) | 0.45 ± 0.26 (n = 3) |
| | | (A8) and Oxaliplatin | SimOtaneous | 1:20 (A8): Oxaliplatin | 0.43 ± 0.23 (n = 4) |
| | | | (A8) prior to Oxaliplatin | 1:20 (A8): Oxaliplatin | 0.54 ± 0.35 (n = 4) |
| | | | Oxaliplatin prior to (A8) | 20:1 Oxaliplatin: (A8) | 0.26 ± 0.14 (n = 4) |
| | KATO-III (Gastric) | (A8) and 5-FU | Simultaneous | 1:25.5 (A8):5-FU | 0.93 ± 0.15 (n = 3) |
| | | | 5FU prior to (A8) | 25.5:1 5-FU:(A8) | 0.57 ± 0.09 (n = 3) |
| | | (A8) and Oxaliplatin | (A8) prior to Oxaliplatin | 1:97 (A8): Oxaliplatin | 0.84 ± 0.11 (n = 3) |
| | | | Oxaliplatin prior to (A8) | 97:1 Oxaliplatin: (A8) | 0.28 ± 0.24 (n = 3) |
| | | (A8) and Irinotecan | Simultaneous | 1:97 (A8): Irinotecan | 0.90 ± 0.10 (n = 3) |
| | | | (A8) prior to Irinotecan | 1:97 (A8): Irinotecan | 0.69 ± 0.23 (n = 3) |
| | | | Irinotecan prior to (A8) | 97:1 Irinotecan: (A8) | 0.60 ± 0.10 (n = 3) |
| Prostate cancer | PC3 (Prostate) | (A8) and Satraplatin | Simultaneous | 1:2.3 Satraplatin: (A8) | 0.43 ± 0.10 (n = 4) |
| | | | (A8) prior to Satraplatin | 2.3:1 (A8): Satraplatin | 0.43 ± 0.10 (n = 4) |
| | | | Satraplatin prior to (A8) | 1:2.3 Satraplatin: (A8) | 0.43 ± 0.13 (n = 4) |
| Colon Cancer | HCT116 (Colon) | (A8) and 5FU | Simultaneous | 1:15 (A8):5FU | 0.56 ± 0.08 (n = 4) |
| | | | (A8) prior to 5FU | 1:15 (A8):5FU | 0.79 ± 0.20 (n = 4) |
| | | | 5FU prior to (A8) | 15:1 5FU:(A8) | 0.65 ± 0.15 (n = 4) |
| *For IM-9 cells ($8x10^4$ cells/ml), after co-treatment of Compound (A8) and Vinblastine for 96 h, the non-viable IM-9 cells were monitored using Annexin V assay format. | | | | | |

Compound (A8) was combined with different anti-cancer drugs and tested in both hematologic malignancies and solid tumor. A constant molar ratio of COMPOUND (A8) and drug 2 (9 different concentrations) was investigated. Synergistic - additive apoptotic induction was evaluated using YOPRO® dye assay. The CI values were calculated using

CalcuSyn[Tm] software. The data for CI values are given as a range (if n=2) or as mean ± SD (if n >2). The combinations were either treated simultaneously or sequentially. The simultaneous treatment was performed for 96h, while the sequential treatment was performed by treating drug 1 for 24h then followed by drug 2 for 72h. After treatment, the non-viable cancer cells were monitored using YO-PRO® assay format.

As seen from the Table 4 above, synergistic effects were observed for Compound A8, an example of a benzimidazole based anti-cancer agent, with a large number of anti-cancer agents over a wide range of cancer cell types.

**Example 3: Combination of Benzimidazole Based Anti-Cancer Agent and a Number of Known Anti-Cancer Agents in Vivo**

To assess *in vivo* antitumor activity in human xenograft models, female athymic nude mice, 12 to 14 weeks of age, were implanted subcutaneously in the flank with human cancer cells. When the tumors reached approximately 100 $mm^3$ in size, the mice were pair-matched into various treatment groups. For oral administration, compounds were dissolved in a mixture of 0.5% methylcellulose and 0.1% Tween 80 and administered by gavage to the tumor-bearing mice. For i.v.andi.p..administration, compounds were dissolved in 0.9% NaCl and for the i.v. administration, the compound was administered via the tail vein.

The TGI assessment, the treatment-induced decreases in tumor volume on day 21 of various treatment groups were compared to controls. Percent tumor growth inhibition (%TGI) was calculated as

$$\%TGI = (C_{day\ a} - T_{day\ a})/(C_{day\ a} - C_{day\ 1}) \times 100$$

Where C is the median tumor volume of the control group, T is the median tumor volume of the treatment group, and day a is the day of assessment.

Table 5 shows the results obtained by combining Compound (A8) with three different anti-cancer agents from different classes. It is evident that the combination of a benzimidazole based anti-cancer agent such as Compound (A8) with the different anti-cancer agents enhanced the anti-tumor effect as compared to administering either of the drug on its own.

**Table 5:** Examples of combination of Compound (A8) and anti-cancer agents in *in vivo* cancer models

| Cancer Cell type) | Compound(s) | Schedule | Dose (mg/ kg) | Route | %TGI at Day 15 | p-value* |
|---|---|---|---|---|---|---|
| HCT 116 (colon) | vehicle (Tw/MC)<br>+ vehicle (saline) | qd x 14 + iv<br>D1,8,15 | ---<br>--- | po<br>iv | --- | --- |
| | vehicle (Tw/MC)<br>+ 5FU | qd x 14 + iv<br>D1,8, 15 | ---<br>50 | po<br>iv | 34 | --- |
| | A8<br>+ vehicle (saline) | qdx 14 + iv<br>D1,8, 15 | 50<br>--- | po<br>iv | 64 | --- |
| | A8<br>+ 5FU | qdx 14 + iv<br>D1,8, 15 | 50<br>50 | po<br>iv | 98 | <0.01[A] |
| H460 (lung) | no treatment | --- | --- | --- | --- | --- |
| | A8 | D1-3,D8-10 | 75 | po | 14 | --- |
| | A8 | qd x 14d | 50 | po | 33 | --- |
| | gemcitabine<br>+ vehicle | D1,5,9,13 qd<br>x14d | 80<br>--- | ip<br>po | 36 | --- |

(continued)

| Cancer Cell type) | Compound(s) | Schedule | Dose (mg/ kg) | Route | %TGI at Day 15 | p-value* |
|---|---|---|---|---|---|---|
| | gemcitabine +A8 | D1,5,9,13 qd x 14 | 80 50 | ip po | 58 | <0.05$^{B}$ <0.01$^{C}$ |
| | gemcitabine +A8 | D1,5,9,13 D2-4, 6-8, 10-12 | 80 50 | ip po | 54 | <0.05$^{B}$ <0.01$^{C}$ |
| | carboplatin | D1,8 | 30 | ip | 29 | -- |
| | A8 + carboplatin | qd x 14d D1,8 | 50 30 | po ip | 60 | <0.01$^{D}$ |

* One-way ANOVA followed by Dunnett's Multiple Comparison test was used to determine the statistical significance of any difference in median tumor volume on Day 15 between a single treatment group and the combination group.
A: compared to vehicle, 5FU, and A8 groups
B: compared to gemcitabine group
C: compared to non-treated and A8 groups
D: compared to non-treated, A8, and carboplatin groups

The details of specific embodiments described in this invention are not to be construed as limitations. Various equivalents and modifications may be made without departing from the essence and scope of this invention, and it is understood that such equivalent embodiments are part of this invention.

The present invention relates to the following numbered embodiments:

1. A pharmaceutical composition for the treatment of cancer the composition including:

(i) a benzimidazole based anti-cancer agent; and

(ii) a second anti-cancer agent.

2. A pharmaceutical composition according to embodiment 1 wherein the benzimidazole based anti-cancer agent is a compound of the formula (1):

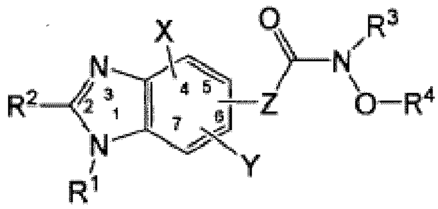

Formula (1)

wherein

$R^1$ is an optionally substituted heteroaryl group, an optionally substituted heterocycloalkyl group or a group of formula:
$-(CR^{20}R^{21})_m-(CR^{22}R^{23})_n-(CR^{24}R^{25})_o-NR^{26}R^{27}$;

$R^2$ is selected from the group consisting of: H, alkyl, alkenyl, alkynyl, heteroalkyl, cycloalkyl, cycloalkenyl, cycloalkylalkyl, alkoxyalkyl, $R^{11}S(O)R^{13}$-, $R^{11}S(O)_2R^{13}$-, $R^{11}C(O)N(R^{12})R^{13}$-, $R^{11}SO_2N(R^{12})R^{13}$-, $R^{11}N(R^{12})C(O)R^{13}$-, $R^{11}N(R^{12})SO_2R^{13}$-, $R^{11}N(R^{12})C(O)N(R^{12})R^{13}$- and acyl, each of which may be optionally substituted;

$R^3$ is selected from the group consisting of H, $C_1$-$C_6$ alkyl, and acyl, each of which may be optionally substituted;

X and Y are the same or different and are independently selected from the group consisting of: H, halogen, -CN, $-NO_2$, $-CF_3$, $-OCF_3$, alkyl, alkenyl, alkynyl, haloalkyl, haloalkenyl, haloalkynyl, heteroalkyl, cycloalkyl, cycloalkenyl, heterocycloalkyl, heterocycloalkenyl, aryl, heteroaryl, hydroxy, hydroxyalkyl, alkoxy, alkoxyalkyl, alkoxyaryl, alkoxyheteroaryl, alkenyloxy, alkynyloxy, cycloalkyloxy, cycloalkenyloxy, heterocycloalkyloxy, heterocycloalkenyloxy, aryloxy, heteroaryloxy, arylalkyl, heteroarylalkyl, arylalkyloxy, -amino, alkylamino, acylamino, aminoalkyl, arylamino, sulfonyl, alkylsulfonyl, arylsulfonyl, aminosulfonyl, aminoalkyl, alkoxyalky, -COOH $-C(O)OR^5$, $-COR^5$, -SH, $-SR^6$, $-OR^6$ acyl and $-NR^7R^8$, each of which may be optionally substituted;

each $R^4$ is selected from the group consisting of: H, alkyl, alkenyl, alkynyl, haloalkyl, heteroalkyl, cycloalkyl, heterocycloalkyl, aryl, heteroaryl, cycloalkylalkyl, heterocycloalkylalkyl, arylalkyl, heteroarylalkyl and acyl, each of which may be optionally substituted;

each $R^5$ is independently selected from the group consisting of: H, alkyl, alkenyl, alkynyl, haloalkyl, heteroalkyl, cycloalkyl, heterocycloalkyl, aryl, heteroaryl, cycloalkylalkyl, heterocycloalkylalkyl, arylalkyl, heteroarylalkyl and acyl, each of which may be optionally substituted;

each $R^6$ is independently selected from the group consisting of: H, alkyl, alkenyl, alkynyl, haloalkyl, heteroalkyl, cycloalkyl, heterocycloalkyl, aryl, heteroaryl, cycloalkylalkyl, heterocycloalkylalkyl, arylalkyl, heteroarylalkyl and acyl; each of which may be optionally substituted;

each $R^7$ and $R^8$ is independently selected from the group consisting of: H, alkyl, alkenyl, alkynyl, haloalkyl, heteroalkyl, cycloalkyl, heterocycloalkyl, aryl, heteroaryl, cycloalkylalkyl, heterocycloalkylalkyl, arylalkyl, heteroarylalkyl and acyl, each of which may be optionally substituted;

each $R^{11}$ and $R^{12}$ is independently selected from the group consisting of H, alkyl, alkenyl, and alkynyl, each of which may be optionally substituted;

each $R^{13}$ is a bond or is independently selected from the group consisting of: alkyl, alkenyl, and alkynyl, each of which may be optionally substituted;

each $R^{20}$, $R^{21}$, $R^{22}$, $R^{23}$, $R^{24}$ and $R^{25}$ is independently selected from the group consisting of: H, halogen, -CN, $-NO_2$, $-CF_3$, $-OCF_3$, alkyl, alkenyl, alkynyl, haloalkyl, haloalkenyl, haloalkynyl, heteroalkyl, cycloalkyl, cycloalkenyl, heterocycloalkyl, heterocycloalkenyl, aryl, heteroaryl, cycloalkylalkyl, heterocycloalkylalkyl, arylalkyl, heteroarylalkyl, arylalkenyl, cycloalkylheteroalkyl, heterocycloalkylheteroalkyl, heteroarylheteroalkyl, arylheteroalkyl, hydroxy, hydroxyalkyl, alkoxy, alkoxyalkyl, alkoxyaryl, alkoxyheteroaryl, alkenyloxy, alkynyloxy, cycloalkylkoxy, heterocycloalkyloxy, aryloxy, arylalkyloxy, phenoxy, benzyloxy heteroaryloxy, amino, alkylamino, acylamino, aminoalkyl, arylamino, alkoxycarbonyl, alkylaminocarbonyl, sulfonyl, alkylsulfonyl, aminosulfonyl, arylsulfonyl, arylsulfinyl -COOH, $-C(O)OR^5$, $-COR^5$, -SH, $-SR^6$, $-OR^6$ and acyl, each of which may be optionally substituted; or

$R^{20}$ and $R^{21}$ when taken together may form a group of formula =O or =S, and/or

$R^{22}$ and $R^{23}$ when taken together may form a group of formula =O or =S, and/or

$R^{24}$ and $R^{25}$ when taken together may form a group of formula =O or =S;

each $R^{26}$ and $R^{27}$ is independently selected from the group consisting of is selected from the group consisting of: H, halogen, alkyl, alkenyl, alkynyl, haloalkyl, haloalkenyl, heteroalkyl, cycloalkyl, cycloalkenyl, heterocycloalkyl, heterocycloalkenyl, aryl, heteroaryl, cycloalkylalkyl, heterocycloalkylalkyl, arylalkyl, heteroarylalkyl, arylalkenyl, cycloalkylheteroalkyl, heterocycloalkylheteroalkyl, heteroarylheteroalkyl, arylheteroalkyl, hydroxy, hydroxyalkyl, alkoxy, alkoxyalkyl, alkoxyaryl, alkenyloxy, alkynyloxy, cycloalkylkoxy, heterocycloalkyloxy, aryloxy, arylalkyloxy, heteroaryloxy, amino, alkylamino, aminoalkyl, acylamino, arylamino, phenoxy, benzyloxy, COOH, alkoxycarbonyl, alkylaminocarbonyl, sulfonyl, alkylsulfonyl, alkylsulfinyl, arylsulfonyl, arylsulfinyl, aminosulfonyl, $SR^5$, and acyl, each of which may be optionally substituted, or $R^{26}$ and $R^{27}$ when taken together with the nitrogen atom to which they are attached form an optionally substituted heterocycloalkyl group;

Z is selected from the group consisting of $-CH_2-$, $-CH_2CH_2-$, -CH=CH-, $C_3$-$C_6$ alkylene, $C_3$-$C_6$ alkenylene, $C_3$-$C_6$ alkynylene, $C_3$-$C_6$ cycloalkyl, unsubstituted or substituted with one or more substituents independently selected

from the group consisting of $C_1$-$C_4$ alkyl;

m, n and o are integers independently selected from the group consisting of 0, 1, 2, 3 and 4;

or a pharmaceutically acceptable salt or prodrug thereof.

3. A pharmaceutical composition according to embodiment 2 wherein in the compound of formula (1) Z is -CH=CH-, and is attached at ring position 5.

4. A pharmaceutical composition according to embodiment 2 or 3 wherein in the compound of formula (1) $R^3$, $R^4$. X and Y = H.

5. A pharmaceutical composition according to any one of embodiments 2 to 4 wherein in the compound of formula (1) $R^1$ is selected from the group consisting of:

$-(CR^{20}R^{21})_2-NR^{26}R^{27}$;

$-(CR^{22}R^{23})_2-NR^{26}R^{27}$;

$-(CR^{24}R^{25})_2-NR^{26}R^{27}$;

$-(CR^{20}R^{21})-(CR^{22}R^{23})-NR^{26}R^{27}$;

$-(CR^{20}R^{21})-(CR^{24}R^{25})-NR^{26}R^{27}$;

$-(CR^{22}R^{23})-(CR^{24}R^{25})-NR^{26}R^{27}$,

$-(CR^{20}R^{21})_3-NR^{26}R^{27}$;

$-(CR^{22}R^{23})_3-NR^{26}R^{27}$;

$-(CR^{24}R^{25})_3-NR^{26}R^{27}$;

$-(CR^{20}R^{21})_2-(CR^{22}R^{23})-NR^{26}R^{27}$;

$-(CR^{20}R^{21})_2-(CR^{24}R^{25})-NR^{26}R^{27}$;

$-(CR^{20}R^{21})-(CR^{22}R^{23})_2-NR^{26}R^{27}$;

$-(CR^{22}R^{23})_2-(CR^{24}R^{25})-NR^{26}R^{27}$;

$-(CR^{20}R^{21})-(CR^{24}R^{25})_2-NR^{26}R^{27}$;

$-(CR^{22}R^{23})-(CR^{24}R^{25})_2-NR^{26}R^{27}$; and

$-(CR^{20}R^{21})-(CR^{22}R^{23})-(CR^{24}R^{25})-NR^{26}R^{27}$.

6. A pharmaceutical composition according to embodiment 2 wherein the compound of formula (1) has the formula:

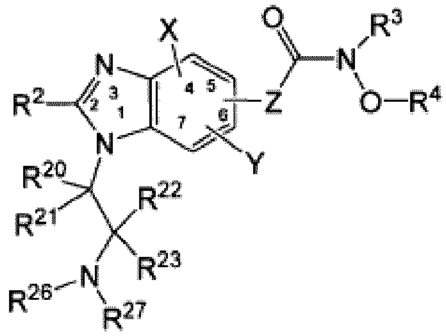

7. A pharmaceutical composition according to embodiment 2 wherein the compound of formula (1) has the formula:

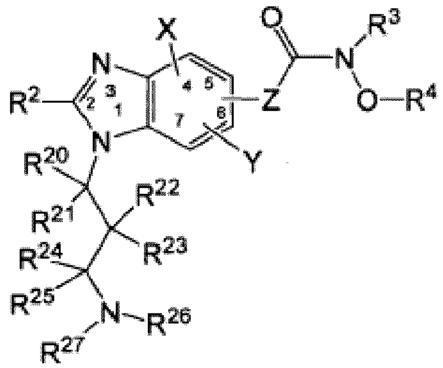

8. A pharmaceutical composition according to embodiment 6 or 7 wherein in the compound of formula (1) $R^{20}$, $R^{21}$, $R^{22}$, $R^{23}$, $R^{24}$ and $R^{25}$ are each independently selected from the group consisting of H, alkyl, alkenyl and alkynyl.

9. A pharmaceutical composition according to embodiment 6 wherein in the compound of formula (1) $R^{20}$, $R^{21}$, $R^{22}$, and $R^{23}$, are each H.

10. A pharmaceutical composition according to embodiment 6 or 7 wherein in the compound of formula (1) $R^{20}$, $R^{21}$, and $R^{24}$ and $R^{25}$ are each H, and $R^{22}$ and $R^{23}$ are each methyl.

11. A pharmaceutical composition according to any one of embodiments 2 to 10 wherein in the compound of formula (1) $R^{26}$ and $R^{27}$ are independently selected from the group consisting of: H, alkyl, alkenyl, alkynyl, alkoxyalkyl, and acyl.

12. A pharmaceutical composition according to embodiment 11 wherein in the compound of formula (1) $R^{26}$ and $R^{27}$ are independently selected from the group consisting of H, methyl, ethyl, isopropyl, propyl, butyl, isobutyl, pentyl, hexyl, heptyl, acetyl and 2-methoxy-ethyl.

13. A pharmaceutical composition according to embodiment 2 wherein in the compound of formula (1) $R^1$ is a group of the formula:

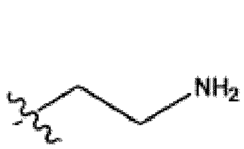

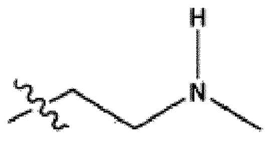

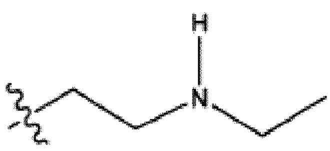

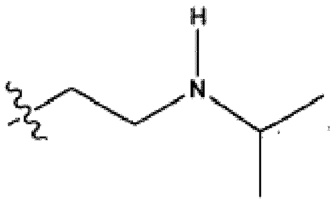
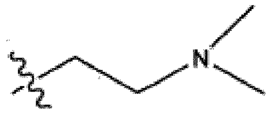
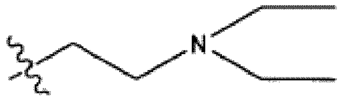

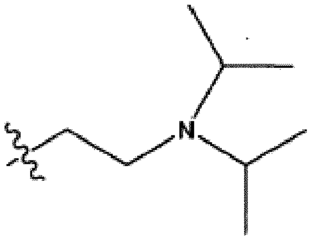
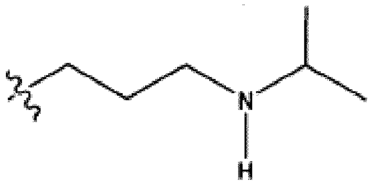
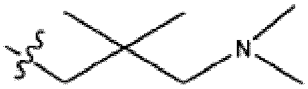

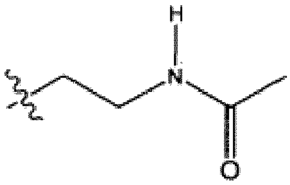
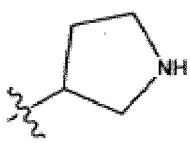
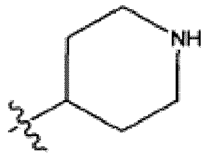
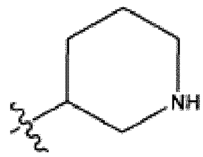

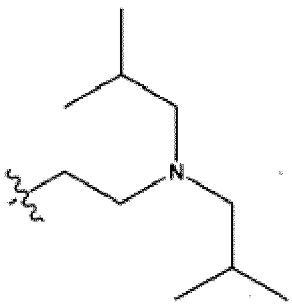
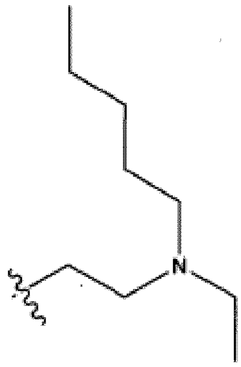
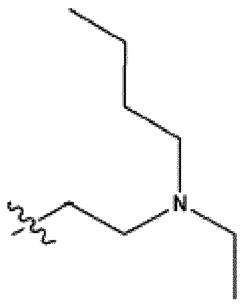

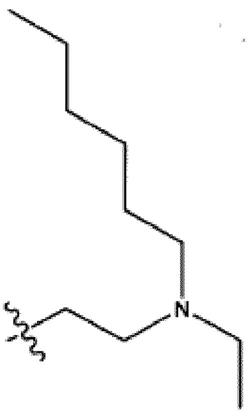
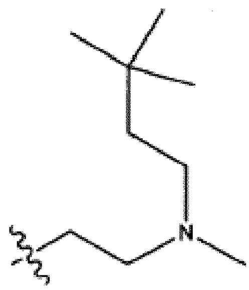
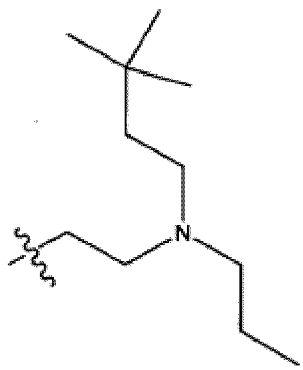

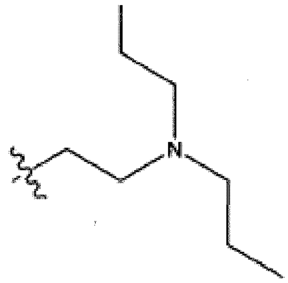 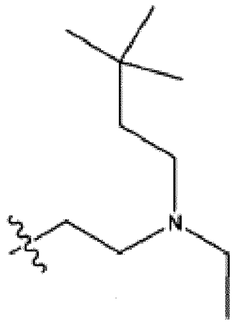 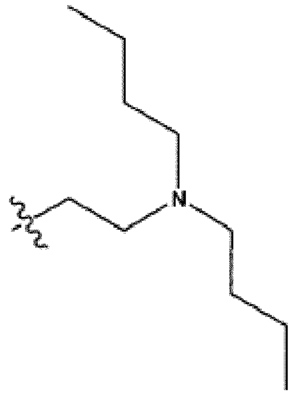

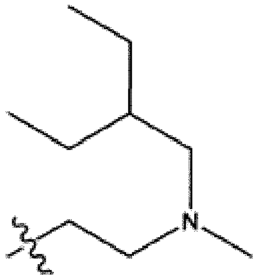 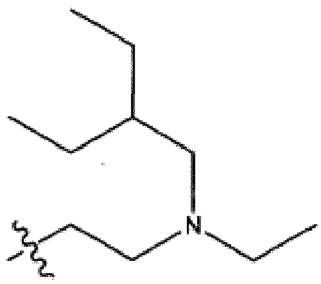 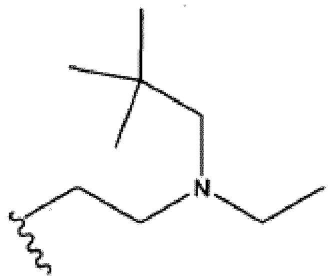

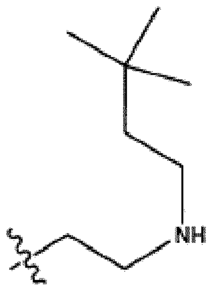 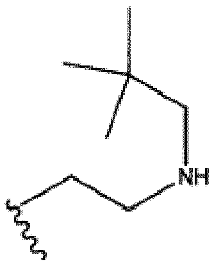 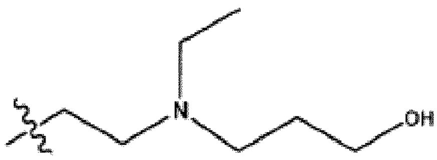

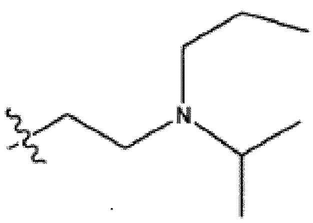 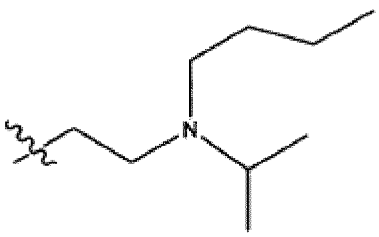 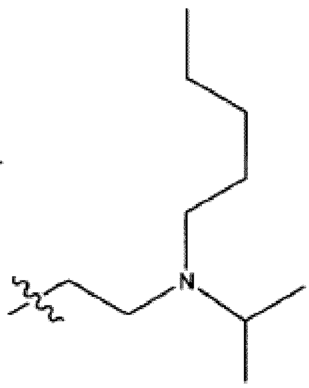

14. A pharmaceutical composition according to any one of embodiments 2 to 13 wherein in the compound of formula (1) $R^2$ is selected from the group consisting of H, alkyl, cycloalkyl, heteroalkyl, alkenyl, alkynyl, alkoxyalkyl and cycloalkylalkyl each of which may be unsubstituted or substituted.

15. A pharmaceutical composition according to embodiment 14 wherein in the compound of formula (1) $R^2$ is selected

from the group consisting of: H; methyl; ethoxymethyl; [Bicyclo[2.2.1]2-ylmethyl; Adamantan-2-ylmethyl; 2-methansulfanyl-ethyl; 2,2,2-triflouro-ethyl; propyl; 2-2-dimethyl-propyl; isopropyl; 3,3,3-triflouro-propyl; butyl; isobutyl; 3,3-dimethyl-butyl; but-3-enyl; but-3-yny; pentyl; 2,4,4-trimethyl-pentyl; Bicyclo[2.2.1]hept-5-en-2yl; hexyl; hex-3-enyl; octyl; non-3-enyl; non-6-enyl; 2-methoxy-nonyl, 2-phenyl-cyclopropyl; cyclohexyl; $(CH_3)_3CCH_2CONH(CH_2)_2$-; $(CH_3)_3CCONH(CH_2)_2$-; $(CH_3)_3CCONH(CH_2)$- and $CH_3(CH_2)_2CONH(CH_2)$-.

16. A pharmaceutical composition according to embodiment 2 wherein the compound of formula (1) is selected from the group consisting of

- (E)-3-[1-(3-Dimethylamino-2,2-dimethyl-propyl)-2-isopropyl-1H-benzimidazol-5-yl]-N-hydroxy-acrylamide
- (E)-3-[2-Butyl-1-(3-dimethylamino-2,2-dimethyl-propyl)-1H-benzimidazol-5-yl]-N-hydroxy-acrylamide
- (E)-3-[1-(3-Dimethylamino-2,2-dimethyl-propyl)-2-(2-methylsulfanyl-ethyl)-1H-benzimidazol-5-yl]-N-hydroxy-acrylamide
- (E)-3-[1-(3-Dimethylamino-2,2-dimethyl-propyl)-2-ethoxymethyl-1H-benzimidazol-5-yl]-N-hydroxy-acrylamide
- (E)-3-[1-(3-Dimethylamino-2,2-dimethyl-propyl)-2-isobutyl-1H-benzimidazol-5-yl]-N-hydroxy-acrylamide
- (E)-3-[1-(2-Diethylamino-ethyl)-2-isobutyl-1H-benzimidazol-5-yl]-N-hydroxy-acrylamide
- (E)-3-[2-Butyl-1-(2-diethylamino-ethyl)-1H-benzimidazol-5-yl]-N-hydroxy-acrylamide
- (E)-3-[2-But-3-ynyl-1-(3-dimethylamino-2,2-dimethyl-propyl)-1H-benzimidazol-5-yl]-N-hydroxy-acrylamide
- (E)-3-[2-But-3-enyl-1-(3-dimethylamino-2,2-dimethyl-propyl)-1H-benzimidazol-5-yl]-N-hydroxy-acrylamide
- (E)-3-[2-But-3-enyl-1-(2-diethylamino-ethyl)-1H-benzimidazol-5-yl]-N-hydroxy-acrylamide
- (E)-3-[2-But-3-ynyl-1-(2-diethylamino-ethyl)-1H-benzimidazol-5-yl]-N-hydroxy-acrylamide
- (E)-3-[1-(3-Dimethylamino-2,2-dimethyl-propyl)-2-(3,3,3-trifluoro-propyl)-1H-benzimidazol-5-yl]-N-hydroxy-acrylamide
- (E)-3-[1-(2-Diethylamino-ethyl)-2-(3,3,3-trifluoro-propyl)-1H-benzimidazol-5-yl]-N-hydroxy-acrylamide
- (E)-3-[1-(2-Diethylamino-ethyl)-2-ethoxymethyl-1H-benzimidazol-5-yl]-N-hydroxy-acrylamide
- (E)-3-[1-(3-Dimethylamino-2,2-dimethyl-propyl)-2-methyl-1H-benzimidazol-5-yl]-N-hydroxy-acrylamide
- (E)-3-[1-(2-Diethylamino-ethyl)-2-(2,2-dimethyl-propyl)-1H-benzimidazol-5-yl]-N-hydroxy-acrylamide
- (E)-N-Hydroxy-3-[1-(3-isopropylamino-propyl)-2-(3,3,3-trifluoro-propyl)-1H-benzimidazol-5-yl]-acrylamide
- (E)-3-[2-(2,2-Dimethyl-propyl)-1-(2-isopropylamino-ethyl)-1H-benzimidazol-5-yl]-N-hydroxy-acrylamide
- (E)-3-[1-(2-Diisopropylamino-ethyl)-2-(2,2-dimethyl-propyl)-1H-benzimidazol-5-yl]-N-hydroxy-acrylamide
- (E)-3-[1-(2-Diisopropylamino-ethyl)-2-isobutyl-1H-benzimidazol-5-yl]-N-hydroxy-acrylamide
- (E)-3-[1-(3-Dimethylamino-2,2-dimethyl-propyl)-2-hex-3-enyl-1H-benzimidazol-5-yl]-N-hydroxy-acrylamide
- (E)-3-[1-(3-Dimethylamino-2,2-dimethyl-propyl)-2-(2,4,4-trimethyl-pentyl)-1H-benzimidazol-5-yl]-N-hydroxy-acrylamide
- (E)-3-[2-Cyclohexyl-1-(3-dimethylamino-2,2-dimethyl-propyl)-1H-benzimidazol-5-yl]-N-hydroxy-acrylamide

- (E)-3-[2-Bicyclo[2.2.1]hept-5-en-2-yl-1-(3-dimethylamino-2,2-dimethyl-propyl)-1H-benzimidazol-5-yl]-N-hydroxy-acrylamide
- (E)-3-[1-(2-Diethylamino-ethyl)-2-hex-3-enyl-1H-benzimidazol-5-yl]-N-hydroxy-acrylamide
- (E)-3-[1-(2-Diisopropylamino-ethyl)-2-hex-3-enyl-1H-benzimidazol-5-yl]-N-hydroxy-acrylamide
- (E)-3-[2-Hex-3-enyl-1-(2-isopropylamino-ethyl)-1H-benzimidazol-5-yl]-N-hydroxy-acrylamide
- (E)-3-[2-Hex-3-enyl-1-(3-isopropylamino-propyl)-1H-benzimidazol-5-yl]-N-hydroxy-acrylamide
- (E)-3-[1-(2-Ethylamino-ethyl)-2-hex-3 -enyl-1H-benzimidazol-5-yl]-N-hydroxy-acrylamide
- (E)-3-[1-(2-Diethylamino-ethyl)-2-hexyl-1H-benzimidazol-5-yl]-N-hydroxy-acrylamide
- (E)-N-Hydroxy-3-[1-(3-isopropylamino-propyl)-2-(2,4,4-trimethyl-pentyl)-1H-benzimidazol-5-yl]-acrylamide
- (E)-3-[2-(2,2-Dimethyl-propyl)-1-(3-isopropylamino-propyl)-1H-benzimidazol-5-yl]-N-hydroxy-acrylamide
- (E)-3-[1-(2-Diisopropylamino-ethyl)-2-(3,3,3-trifluoro-propyl)-1H-benzimidazol-5-yl]-N-hydroxy-acrylamide
- (E)-N-Hydroxy-3-[2-isobutyl-1-(2-isopropylamino-ethyl)-1H-benzimidazol-5-yl]-acrylamide
- (E)-3-[2-(2,2-Dimethyl-propyl)-1-(2-ethylamino-ethyl)-1H-benzimidazol-5-yl]-N-hydroxy-acrylamide
- (E)-3-[1-(2-Ethylamino-ethyl)-2-isobutyl-1H-benzimidazol-5-yl]-N-hydroxy-acrylamide
- (E)-3-[1-(2-Diisopropylamino-ethyl)-2-(2,4,4-trimethyl-pentyl)-1H-benzimidazol-5-yl]-N-hydroxy-acrylamide
- (E)-N-Hydroxy-3-[1-(2-isopropylamino-ethyl)-2-(2,4,4-trimethyl-pentyl)-1H-benzimidazol-5-yl]-acrylamide
- (E)-3-[1-(2-Ethylamino-ethyl)-2-(2,4,4-trimethyl-pentyl)-1H-benzimidazol-5-yl]-N-hydroxy-acrylamide
- (E)-3-[1-(2-Diethylamino-ethyl)-2-(2,4,4-trimethyl-pentyl)-1H-benzimidazol-5-yl]-N-hydroxy-acrylamide
- (E)-3-[1-(2-Diethylamino-ethyl)-2-propyl-1H-benzimidazol-5-yl]-N-hydroxy-acrylamide
- (E)-3-[2-Butyl-1-(2-diisopropylamino-ethyl)-1H-benzimidazol-5-yl]-N-hydroxy-acrylamide
- (E)-3-[2-Butyl-1-(2-ethylamino-ethyl)-1H-benzimidazol-5-yl]-N-hydroxy-acrylamide
- (E)-3-[1-(2-Diethylamino-ethyl)-2-(2-methylsulfanyl-ethyl)-1H-benzimidazol-5-yl]-N-hydroxy-acrylamide
- (E)-3-[2-Butyl-1-(2-isopropylamino-ethyl)-1H-benzimidazol-5-yl]-N-hydroxy-acrylamide
- (E)-3-[2-Butyl-1-(3-isopropylamino-propyl)-1H-benzimidazol-5-yl]-N-hydroxy-acrylamide
- (E)-3-[1-(1-Benzyl-piperidin-4-yl)-2-butyl-1H-benzimidazol-5-yl]-N-hydroxy-acrylamide
- (E)-3-[2-But-3-enyl-1-(2-ethylamino-ethyl)-1H-benzimidazol-5-yl]-N-hydroxy-acrylamide
- (E)-3-[2-Hexyl-1-(2-isopropylamino-ethyl)-1H-benzimidazol-5-yl]-N-hydroxy-acrylamide
- (E)-3-[1-(2-Dimethylamino-ethyl)-2-(2,4,4-trimethyl-pentyl)-1H-benzimidazol-5-yl]-N-hydroxy-acrylamide
- (E)-3-[1-(2-Ethylamino-ethyl)-2-hexyl-1H-benzimidazol-5-yl]-N-hydroxy-acrylamide
- (E)-N-Hydroxy-3-[1-(2-isopropylamino-ethyl)-2-(3,3,3-trifluoro-propyl)-1H-benzimidazol-5-yl]-acrylamide

- (E)-3-[1-(2-Dimethylamino-ethyl)-2-hex-3-enyl-1H-benzimidazol-5-yl]-N-hydroxy-acrylamide
- (E)-3-[1-(2-Amino-ethyl)-2-(2,4,4-trimethyl-pentyl)-1H-benzimidazol-5-yl]-N-hydroxy-acrylamide
- (E)-3-[1-(2-Amino-ethyl)-2-(2-methoxy-nonyl)-1H-benzimidazol-5-yl]-N-hydroxy-acrylamide
- (E)-3-[2-Butyl-1-(2-dimethylamino-ethyl)-1H-benzimidazol-5-yl]-N-hydroxy-acrylamide
- (E)-3-[1-(2-Dimethylamino-ethyl)-2-hexyl-1H-benzimidazol-5-yl]-N-hydroxy-acrylamide
- (E)-N-{2-[1-(2-Diethylamino-ethyl)-5-(2-hydroxycarbamoyl-vinyl)-1H-benzimidazol-2-yl]-ethyl} -3,3-dimethyl-butyramide
- (E)-3-{1-(2-Diethylamino-ethyl)-2-[2-(2,2-dimethyl-propionylamino)-ethyl]-1H-benzimidazol-5-yl}-N-hydroxy-acrylamide
- (E)-3-{1-(2-Diethylamino-ethyl)-2-[(2,2-dimethyl-propionylamino)-methyl]-1H-benzimidazol-5-yl}-N-hydroxy-acrylamide
- (E)-N-[1-(2-Diethylamino-ethyl)-5-(2-hydroxycarbamoyl-vinyl)-1H-benzimidazol-2-ylmethyl]-butyramide
- (E)-3-[1-(2-ethylamino-ethyl)-2-(3,3-dimethyl-butyl)-1H-benzimidazol-5-yl]-N-hydroxy-acrylamide
- (E)-3-[2-(3,3-Dimethyl-butyl)-1-(2-Dimethylamino-ethyl)-1H-benzimidazol-5-yl]-N-hydroxy-acrylamide
- (E)-3-[1-(2-Dimethylamino-ethyl)-2-pentyl-1H-benzimidazol-5-yl]-N-hydroxy-acrylamide
- (E)-3-[1-(2-Dimethylamino-ethyl)-2-(2,2,2-trifluoro-ethyl)-1H-benzimidazol-5-yl]-N-hydroxy-acrylamide
- (E)-N-Hydroxy-3-[1-(5-methyl-1H-pyrazol-3-yl)-2-(2,4,4-trimethyl-pentyl)-1H-benzimidazol-5-yl]-acrylamide
- (E)-3-[1-(2-Ethylamino-ethyl)-2-pentyl-1H-benzimidazol-5-yl]-N-hydroxy-acrylamide
- (E)-3-(2-Butyl-1-pyrrolidin-3-yl-1H-benzimidazol-5-yl)-N-hydroxy-acrylamide
- (E)-3-(2-Butyl-1-piperidin-4-yl-1H-benzimidazol-5-yl)-N-hydroxy-acrylamide
- (E)-N-Hydroxy-3-[1-(2-isopropylamino-ethyl)-2-pentyl-1H-benzimidazol-5-yl]-acrylamide
- (E)-N-Hydroxy-3-[1-(2-methylamino-ethyl)-2-non-3-enyl-1H-benzimidazol-5-yl]-acrylamide
- (E)-N-Hydroxy-3-[1-(2-methylamino-ethyl)-2-non-6-enyl-1H-benzimidazol-5-yl]-acrylamide
- (E)-3-[2-Hexyl-1-(2-methylamino-ethyl)-1H-benzimidazol-5-yl]-N-hydroxy-acrylamide
- (E)-N-Hydroxy-3-[1-(2-methylamino-ethyl)-2-pentyl-1H-benzimidazol-5-yl]-acrylamide
- (E)-N-Hydroxy-3-[1-(2-methylamino-ethyl)-2-octyl-1H-benzimidazol-5-yl]-acrylamide
- (E)-3-[1-(2-Amino-ethyl)-2-octyl-1H-benzimidazol-5-yl]-N-hydroxy-acrylamide
- (E)-3-{2-Butyl-1-[2-(isopropyl-methyl-amino)-ethyl]-1H-benzimidazol-5-yl}-N-hydroxy-acrylamide
- (E)-3-{1-[2-(Ethyl-methyl-amino)-ethyl]-2-pentyl-1H-benzimidazol-5-yl}-N-hydroxy-acrylamide
- (E)-3-(2-Hexyl-1-pyrrolidin-3-yl-1H-benzimidazol-5-yl)-N-hydroxy-acrylamide
- (E)-3-[2-Butyl-1-(1-methyl-pyrrolidin-3-yl)-1H-benzimidazol-5-yl]-N-hydroxy-acrylamide

- (E)-3-(2-Butyl-1-piperidin-3-yl-1H-benzimidazol-5-yl)-N-hydroxy-acrylamide
- (E)-3-(2-Hexyl-1-piperidin-3-yl-1H-benzimidazol-5-yl)-N-hydroxy-acrylamide
- (E)-3-(1-{2-[Ethyl-(2-methoxy-ethyl)-amino]-ethyl}-2-pentyl-1H-benzimidazol-5-yl)-N-hydroxy-acrylamide
- (E)-3-{2-Butyl-1- [2-(ethyl-methyl-amino)-ethyl]-1H-benzimidazol-5-yl}-N-hydroxy-acrylamide
- (E)-N-Hydroxy-3-[1-(1-methyl-piperidin-3-yl)-2-pentyl-1H-benzimidazol-5-yl]-acrylamide
- (E)-3-{1-[2-(Ethyl-hexyl-amino)-ethyl]-1H-benzimidazol-5-yl}-N-hydroxy-acrylamide
- (E)-3-{1-[2-(Ethyl-pentyl-amino)-ethyl]-1H-benzimidazol-5-yl}-N-hydroxy-acrylamide
- (E)-3-{1-[2-(Ethyl-heptyl-amino)-ethyl]-1H-benzimidazol-5-yl}-N-hydroxy-acrylamide
- (E)-3-{2-Hexyl-1-[1-(2-hydroxy-ethyl)-piperidin-3-yl]-1H-benzimidazol-5-yl}-N-hydroxy-acrylamide
- (E)-3-(2-Butyl-1~{2-[ethyl-(3 -hydroxy-propyl)-amino]-ethyl}-1H-benzimidazol-5-yl)-N-hydroxy-acrylamide
- (E)-3-(1-{2-[Ethyl-(3-hydroxy-propyl)-amino]-ethyl}-2-pentyl-1H-benzimidazol-5-yl)-N-hydroxy-acrylamide
- (E)-N-hydroxy-3-(1-(1-phenethylpyrrolidin-3-yl)-1H-benzo[d]imidazol-5-yl)acrylamide
- (E)-N-hydroxy-3-(1-(1-pentylpiperidin-3-yl)-1H-benzo[d]imidazol-5-yl)acrylamide
- (E)-3-{1-[2-(Butyl-ethyl-amino)-ethyl]-1H-benzimidazol-5-yl}-N-hydroxy-acrylamide
- (E)-N-hydroxy-3-(1-(1-phenethylpiperidin-3-yl)-1H-benzo[d]imidazol-5-yl)acrylamide
- (E)-N-hydroxy-3-(1-(1-(3-phenylpropyl)piperidin-3-yl)-1H-benzo[d]imidazol-5-yl)acrylamide
- (E)-N-hydroxy-3-(1-(1-(3-phenylpropyl)pyrrolidin-3-yl)-1H-benzo[d]imidazol-5-yl)acrylamide
- (E)-3-{1-[1-(3,3-Dimethyl-butyl)-pyrrolidin-3-yl]-1H-benzimidazol-5-yl}-N-hydroxy-acrylamide
- (E)-3-(1-(2-(diethylamino)ethyl)-1H-benzo[d]imidazol-5-yl)-N-hydroxyacrylamide
- (E)-3-[2-(4-Cyano-butyl)-1-(2-diethylamino-ethyl)-1H-benzimidazol-5-yl]-N-hydroxy-acrylamide
- (E)-3-(1-(1-butylpiperidin-3-yl)-1H-benzo[d]imidazol-5-yl)-N-hydroxyacrylamide
- (E)-N-hydroxy-3-(1-(1-(pent-4-enyl)piperidin-3-yl)-1H-benzo[d]imidazol-5-yl)acrylamide
- (E)-3-(1-(1-(3,3-dimethylbutyl)piperidin-4-yl)-1H-benzo[d]imidazol-5-yl)-N-hydroxyacrylamide
- (E)-3-[1-(2-Diethylamino-ethyl)-2-propylamino-1H-benzimidazol-5-yl]-N-hydroxy-acrylamide
- (E)-N-hydroxy-3-(1-(2-(isopropyl(propyl)amino)ethyl)-1H-benzo[d]imidazol-5-yl)acrylamide
- (E)-3-{1-[2-(Butyl-isopropyl-amino)-ethyl]-1H-benzimidazol-5-yl}-N-hydroxy-acrylamide
- N-Hydroxy-3-{1-[2-(isopropyl-pentyl-amino)-ethyl]-1H-benzimidazol-5-yl}-acrylamide
- (E)-3-[2-(5-Cyano-pentyl)-1-(2-diethylamino-ethyl)-1H-benzimidazol-5-yl]-N-hydroxy-acrylamide
- (E)-3-(1-{2-[(3,3-Dimethyl-butyl)-ethyl-amino]-ethyl}-1H-benzimidazol-5-yl)-N-hydroxy-acrylamide

- (E)-3-{1-[2-(Ethyl-propyl-amino)-ethyl]-1H-benzimidazol-5-yl}-N-hydroxy-acrylamide
- (E)-N-Hydroxy-3-(1-{2-[isopropyl-(2-methyl-pentyl)-amino]-ethyl}-1H-benzimidazol-5-yl)-acrylamide
- (E)-N-hydroxy-3-(1-(2-(isopropyl(4,4,4-trifluorobutyl)amino)ethyl)-1H-benzo[d]imidazol-5-yl)acrylamide
- (E)-3-[1-(3-Dimethylamino-2,2-dimethyl-propyl)-2-propylamino-1H-benzimidazol-5-yl]-N-hydroxy-acrylamide
- (E)-3-{1-[2-(Ethyl-hexyl-amino)-ethyl]-2-methyl-1H-benzimidazol-5-yl}-N-hydroxy-acrylamide
- (E)-3-{1-[2-(Butyl-ethyl-amino)-ethyl]-2-trifluoromethyl-1H-benzimidazol-5-yl}-N-hydroxy-acrylamide
- (E)-3-{1-[2-(Ethyl-hexyl-amino)-ethyl]-2-trifluoromethyl-1H-benzimidazol-5-yl}-N-hyd roxy-acry lam ide
- (E)-3-(1-(2-(dibutylamino)ethyl)-2-propyl-1H-benzo[d]imidazol-5-yl)-N-hydroxyacrylamide
- (E)-3-[1-(2-Dipropylamino-ethyl)-1H-benzimidazol-5-yl]-N-hydroxy-acrylamide
- N-Hydroxy-3-(1-{2-[isopropyl-(3-methyl-butyl)-amino]-ethyl}-1H-benzimidazol-5-yl)-acrylamide
- (E)-3-(1-{2-[(3,3-Dimethyl-butyl)-methyl-amino]-ethyl}-1H-benzimidazol-5-yl)-N-hydroxy-acrylamide
- (E)-3-(1-{2-[(2-Ethyl-butyl)-methyl-amino]-ethyl}-1H-benzimidazol-5-yl)-N-hydroxy-acrylamide
- (E)-3-(1-(2-(bis(3,3-dimethylbutyl)amino)ethyl)-1H-benzo[d]imidazol-5-yl)-N-hydroxyacrylamide
- (E)-3-(1-(2-(diisobutylamino)ethyl)-1H-benzo[d]imidazol-5-yl)-N-hydroxyacrylamide
- (E)-3-{1-[2-(3,3-Dimethyl-butylamino)-ethyl]-1H-benzimidazol-5-yl}-N-hydroxy-acrylamide
- (E)-N-Hydroxy-3-{1-[2-(methyl-pent-4-enyl-amino)-ethyl]-1H-benzimidazol-5-yl}-acrylamide
- (E)-3-(1-{2-[(3,3-Dimethyl-butyl)-propyl-amino]-ethyl}-1H-benzimidazol-5-yl)-N-hydroxy-acrylamide
- (E)-3-[1-(3-Dimethylamino-2,2-dimethyl-propyl)-2-methylsulfanyl-1H-benzimidazol-5-yl]-N-hydroxy-acrylamide
- (E)-3-{1-[2-(3,3-Dimethyl-butylamino)-ethyl]-2-propyl-1H-benzimidazol-5-yl}-N-hydroxy-acrylamide
- (E)-3-[1-[2-(3,3-Dimethyl-butylamino)-ethyl]-2-(2,2-dimethyl-propyl)-1H-benzimidazol-5-yl]-N-hydroxy-acrylamide
- (E)-3-[1-{2-[Bis-(3,3-dimethyl-butyl)-amino]-ethyl}-2-(2,2-dimethyl-propyl)-1H-benzimidazol-5-yl]-N-hydroxy-acrylamide
- (E)-3-{1-[2-(2,2-Dimethyl-propylamino)-ethyl]-1H-benzimidazol-5-yl}-N-hydroxy-acrylamide
- (E)-3-(1-{2-[(2,2-Dimethyl-propyl)-propyl-amino]-ethyl}-1H-benzimidazol-5-yl)-N-hydroxy-acrylamide
- (E)-3-{1-[2-(3,3-Dimethyl-butylamino)-ethyl]-2-ethyl-1H-benzimidazol-5-yl}-N-hydroxy-acrylamide
- (E)-3-(1-{2-[3,3-Dimethyl-butyl)-methyl-amino]-ethyl}-2-propyl-1H-benzimidazol-5-yl)-N-hydroxy-acrylamide
- (E)-3-(1-{2-[(3,3-Dimethyl-butyl)-(2,2,2-trifluoro-ethyl)-amino]-ethyl}-1H-benzimidazol-5-yl)-N-hydroxy-acrylamide, and
- (E)-3-(H2-[Butyl-(2,2,2-trifluoro-ethyl)-amino]-ethyl}-1H-benzimidazol-5-yl)-N-hydroxy-acrylamide
  or a pharmaceutically acceptable salt thereof.

17. A pharmaceutical composition according to any one of embodiments 1 to 16 wherein the second anti-cancer agent is selected from the group consisting of alkylating agents, antimetabolites, antitumour antibiotics, mitotic spindle agents, topoisomerase inhibitors, steroid and hormonal agents, monoclonal antibodies, proteasome inhibitors, thalidomide and its analogs, death receptor ligands, kinase inhibitors, anti-angiogenesis agents, HDAC inhibitors, synthetic zinc finger transcription factors, interferons, platelet growth factors, photosensitizers, hypomethylating agents, rapalogs, mTor inhibitors, derivatives thereof, analogs thereof and pharmaceutically acceptable salts thereof.

18. A pharmaceutical composition according to any one of embodiments 1 to 17 wherein the second anti-cancer agent is selected from the group consisting of Adriamycin, avastin, Cisplatin, Oxaliplatin, Carboplatin, Satroplatin, Gemcitabine, Dasatinib, decitabine (Dacogen), Docetaxel, Doxorubicin, hydroxydaunarubicin, Dexamethason, Chlorambucil, Fludarabine, Tarceva (Erlotinib), Iressa, Alimta, Melphalan, 5-Azacytidine (Vidaza), 5-FU (5-Fluorouracil), Actinomycin D, Paclitaxel, prednisone, Vinblastine, lenalidomide (Revlimid), Irinotecan, Topotecan, Etoposide, Tamoxifen, Rituximab (Rituxan), Herceptin, Cetuximab, sorafenib, sutent, Angiostatin, Endostatin, temsirolimus, everolimus, Avastin (Bevacizumab) derivatives thereof, analogs thereof and pharmaceutically acceptable salts thereof.

19. A pharmaceutical composition according to any one of embodiments 1 to 18 including a further anti-cancer agent.

20. A pharmaceutical composition according to embodiment 19 wherein the further anticancer agent is selected from the group consisting of Adriamycin, avastin, Cisplatin, Oxaliplatin, Carboplatin, Satroplatin, Gemcitabine, Dasatinib, decitabine (Dacogen), Docetaxel, Doxorubicin, hydroxydaunarubicin, Dexamethasone, Chlorambucil, Fludarabine, Tarceva (Erlotinib), Iressa, Alimta, Melphalan, 5-Azacytidine (Vidaza), 5-FU (5-Fluorouracil), Actinomycin D, Paclitaxel, prednisone, Vinblastine, lenalidomide (Revlimid), Irinotecan, Topotecan, Etoposide, Tamoxifen, Rituximab (Rituxan), Herceptin, Cetuximab, sorafenib, Angiostatin, Endostatin, temsirolemus, everolimus, Avastin (Bevacizumab), derivatives thereof, analogs thereof, pharmaceutically acceptable salts thereof and combinations thereof.

21. A method of treating cancer including administering to a patient in need thereof:

a first amount of a benzimidazole based anti-cancer agent and

a second amount of a second anti-cancer agent;

wherein the first amount and the second amount together comprise a therapeutically effective amount.

22. A method according to embodiment 21 wherein the benzimidazole based anti-cancer agent is a compound of the formula (1):

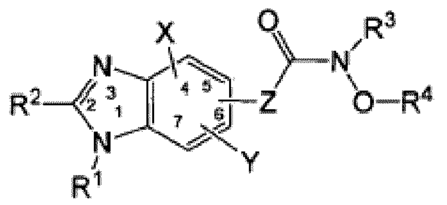

Formula (1)

wherein

$R^1$ is an optionally substituted heteroaryl group, an optionally substituted heterocycloalkyl group or a group of formula:
$-(CR^{20}R^{21})_m-(CR^{22}R^{23})_n-(CR^{24}R^{25})_o-NR^{26}R^{27}$;

$R^2$ is selected from the group consisting of: H, alkyl, alkenyl, alkynyl, heteroalkyl, cycloalkyl, cycloalkenyl, cycloalkylalkyl, alkoxyalkyl, $R^{11}S(O)R^{13}$-, $R^{11}S(O)_2R^{13}$-, $R^{11}C(O)N(R^{12})R^{13}$-, $R^{11}SO_2N(R^{12})R^{13}$-, $R^{11}N(R^{12})C(O)R^{13}$-, $R^{11}N(R^{12})SO_2R^{13}$-, $R^{11}N(R^{12})C(O)N(R^{12})R^{13}$- and acyl, each of which may be optionally substituted;

$R^3$ is selected from the group consisting of H, $C_1$ -$C_6$ alkyl, and acyl, each of which may be optionally substituted;

X and Y are the same or different and are independently selected from the group consisting of: H, halogen, -CN, -$NO_2$, -$CF_3$, -$OCF_3$, alkyl, alkenyl, alkynyl, haloalkyl, haloalkenyl, haloalkynyl, heteroalkyl, cycloalkyl, cycloalkenyl, heterocycloalkyl, heterocycloalkenyl, aryl, heteroaryl, hydroxy, hydroxyalkyl, alkoxy, alkoxyalkyl, alkoxyaryl, alkoxyheteroaryl, alkenyloxy, alkynyloxy, cycloalkyloxy, cycloalkenyloxy, heterocycloalkyloxy, heterocycloalkenyloxy, aryloxy, heteroaryloxy, arylalkyl, heteroarylalkyl, arylalkyloxy, -amino, alkylamino, acylamino, aminoalkyl, arylamino, sulfonyl, alkylsulfonyl, arylsulfonyl, aminosulfonyl, aminoalkyl, alkoxyalky, -COOH -C(O)$OR^5$, -$COR^5$, -SH, -$SR^6$, -$OR^6$ acyl and -$NR^7R^8$, each of which may be optionally substituted;

each $R^4$ is selected from the group consisting of: H, alkyl, alkenyl, alkynyl, haloalkyl, heteroalkyl, cycloalkyl, heterocycloalkyl, aryl, heteroaryl, cycloalkylalkyl, heterocycloalkylalkyl, arylalkyl, heteroarylalkyl and acyl, each of which may be optionally substituted;

each $R^5$ is independently selected from the group consisting of: H, alkyl, alkenyl, alkynyl, haloalkyl, heteroalkyl, cycloalkyl, heterocycloalkyl, aryl, heteroaryl, cycloalkylalkyl, heterocycloalkylalkyl, arylalkyl, heteroarylalkyl and acyl, each of which may be optionally substituted;

each $R^6$ is independently selected from the group consisting of: H, alkyl, alkenyl, alkynyl, haloalkyl, heteroalkyl, cycloalkyl, Heterocycloalkyl, aryl, heteroaryl, cycloalkylalkyl, heterocycloalkylalkyl, arylalkyl, heteroarylalkyl and acyl; each of which may be optionally substituted;

each $R^7$ and $R^8$ is independently selected from the group consisting of: H, alkyl, alkenyl, alkynyl, haloalkyl, heteroalkyl, cycloalkyl, heterocycloalkyl, aryl, heteroaryl, cycloalkylalkyl, heterocycloalkylalkyl, arylalkyl, heteroarylalkyl and acyl, each of which may be optionally substituted;

each $R^{11}$ and $R^{12}$ is independently selected from the group consisting of H, alkyl, alkenyl, and alkynyl, each of which may be optionally substituted;

each $R^{13}$ is a bond or is independently selected from the group consisting of: alkyl, alkenyl, and alkynyl, each of which may be optionally substituted;

each $R^{20}$, $R^{21}$, $R^{22}$, $R^{23}$, $R^{24}$ and $R^{25}$ is independently selected from the group consisting of: H, halogen, -CN, -$NO_2$, -$CF_3$, -$OCF_3$, alkyl, alkenyl, alkynyl, haloalkyl, haloalkenyl, haloalkynyl, heteroalkyl, cycloalkyl, cycloalkenyl, heterocycloalkyl, heterocycloalkenyl, aryl, heteroaryl, cycloalkylalkyl, heterocycloalkylalkyl, arylalkyl, heteroarylalkyl, arylalkenyl, cycloalkylheteroalkyl, heterocycloalkylheteroalkyl, heteroarylheteroalkyl, arylheteroalkyl, hydroxy, hydroxyalkyl, alkoxy, alkoxyalkyl, alkoxyaryl, alkoxyheteroaryl, alkenyloxy, alkynyloxy, cycloalkylkoxy, heterocycloalkyloxy, aryloxy, arylalkyloxy, phenoxy, benzyloxy heteroaryloxy, amino, alkylamino, acylamino, aminoalkyl, arylamino, alkoxycarbonyl, alkylaminocarbonyl, sulfonyl, alkylsulfonyl, aminosulfonyl, arylsulfonyl, arylsulfinyl -COOH, -C(O)$OR^5$, -$COR^5$, -SH, -$SR^6$, -$OR^6$ and acyl, each of which may be optionally substituted; or

$R^{20}$ and $R^{21}$ when taken together may form a group of formula =O or ≈S, and/or

$R^{22}$ and $R^{23}$ when taken together may form a group of formula =O or ≈S, and/or

$R^{24}$ and $R^{25}$ when taken together may form a group of formula =O or ≈S;

each $R^{26}$ and $R^{27}$ is independently selected from the group consisting of is selected from the group consisting of: H, halogen, alkyl, alkenyl, alkynyl, haloalkyl, haloalkenyl, heteroalkyl, cycloalkyl, cycloalkenyl, heterocycloalkyl, heterocycloalkenyl, aryl, heteroaryl, cycloalkylalkyl, heterocycloalkylalkyl, arylalkyl, heteroarylalkyl, arylalkenyl, cycloalkylheteroalkyl, heterocycloalkylheteroalkyl, heteroarylheteroalkyl, arylheteroalkyl, hydroxy, hydroxyalkyl, alkoxy, alkoxyalkyl, alkoxyaryl, alkenyloxy, alkynyloxy, cycloalkylkoxy, heterocycloalkyloxy, aryloxy, arylalkyloxy, heteroaryloxy, amino, alkylamino, aminoalkyl, acylamino, arylamino, phenoxy, benzyloxy, COOH, alkoxycarbonyl, alkylaminocarbonyl, sulfonyl, alkylsulfonyl, alkylsulfinyl, arylsulfonyl, arylsulfinyl, aminosulfonyl, $SR^5$, and acyl, each of which may be optionally substituted, or $R^{26}$ and $R^{27}$ when taken together with the nitrogen atom to which they are attached form an optionally substituted heterocycloalkyl group;

Z is selected from the group consisting of $-CH_2-$, $-CH_2CH_2-$, -CH=CH-, $C_3$-$C_6$ alkylene, $C_3$-$C_6$ alkenylene, $C_3$-$C_6$ alkynylene, $C_3$-$C_6$ cycloalkyl, unsubstituted or substituted with one or more substituents independently selected from the group consisting of $C_1$-$C_4$ alkyl;

m, n and o are integers independently selected from the group consisting of 0, 1, 2, 3 and 4;

or a pharmaceutically acceptable salt or prodrug thereof.

23. A method according to embodiment 22 wherein in the compound of formula (1) Z is -CH=CH-, and is attached at ring position 5.

24. A method according to embodiment 22 or 23 wherein in the compound of formula (1) $R^3$, $R^4$, X and Y = H.

25. A method according to any one of embodiments 22 to 24 wherein in the compound of formula (1) $R^1$ is selected from the group consisting of:

$-(CR^{20}R^{21})_2-NR^{26}R^{27}$;

$-(CR^{22}R^{23})_2-NR^{26}R^{27}$;

$-(CR^{24}R^{25})_2-NR^{26}R^{27}$;

$-(CR^{20}R^{21})-(CR^{22}R^{23})-NR^{26}R^{27}$;

$-(CR^{20}R^{21})-(CR^{24}R^{25})-NR^{26}R^{27}$;

$-(CR^{22}R^{23})-(CR^{24}R^{25})-NR^{26}R^{27}$,

$-(CR^{20}R^{21})_3-NR^{26}R^{27}$;

$-(CR^{22}R^{23})_3-NR^{26}R^{27}$;

$-(CR^{24}R^{25})_3-NR^{26}R^{27}$;

$-(CR^{20}R^{21})_2-(CR^{22}R^{23})-NR^{26}R^{27}$;

$-(CR^{20}R^{21})_2-(CR^{24}R^{25})-NR^{26}R^{27}$;

$-(CR^{20}R^{21})-(CR^{22}R^{23})_2-NR^{26}R^{27}$;

$-(CR^{22}R^{23})_2-(CR^{24}R^{25})-NR^{26}R^{27}$;

$-(CR^{20}R^{21})-(CR^{24}R^{25})_2-NR^{26}R^{27}$;

$-(CR^{22}R^{23})-(CR^{24}R^{25})_2-NR^{26}R^{27}$; and

$-(CR^{20}R^{21})-(CR^{22}R^{23})-(CR^{24}R^{25})-NR^{26}R^{27}$.

26. A method according to embodiment 22 wherein the compound of formula (1) has the formula:

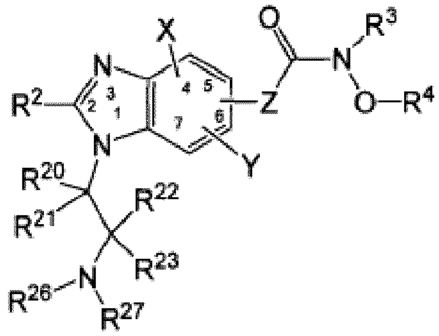

27. A method according to embodiment 22 wherein the compound of formula (1) has the formula:

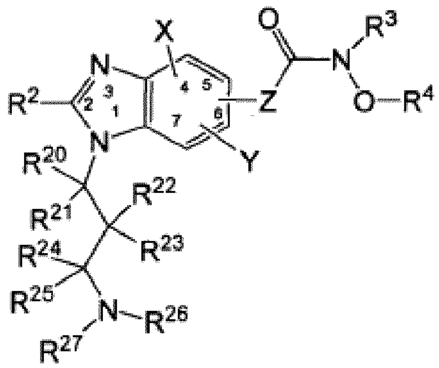

28. A method according to embodiment 26 or 27 wherein in the compound of formula (1) $R^{20}$, $R^{21}$, $R^{22}$, $R^{23}$, $R^{24}$ and $R^{25}$ are each independently selected from the group consisting of H, alkyl, alkenyl and alkynyl.

29. A method according to embodiment 26 wherein in the compound of formula (1) $R^{20}$, $R^{21}$, $R^{22}$, and $R^{23}$, are each H.

30. A method according to embodiment 27 wherein in the compound of formula (1) $R^{20}$, $R^{21}$, and $R^{24}$ and $R^{25}$ are each H, and $R^{22}$ and $R^{23}$ are each methyl.

31. A method according to any one of embodiments 22 to 30 wherein in the compound of formula (1) $R^{26}$ and $R^{27}$ are independently selected from the group consisting of: H, alkyl, alkenyl, alkynyl, alkoxyalkyl, and acyl.

32. A method according to embodiment 31 wherein in the compound of formula (1) $R^{26}$ and $R^{27}$ are independently selected from the group consisting of H, methyl, ethyl, isopropyl, propyl, butyl, isobutyl, pentyl, hexyl, heptyl, acetyl and 2-methoxy-ethyl.

33. A method according to any one of embodiments 22 to 32 wherein in the compound of formula (1) $R^1$ is a group of the formula:

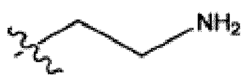 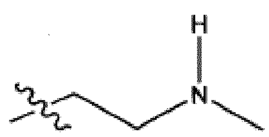 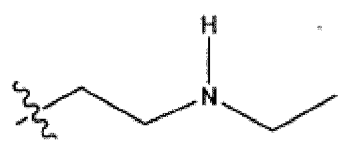

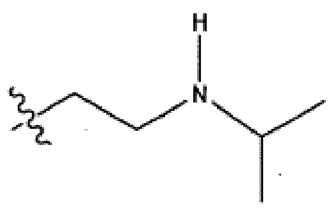 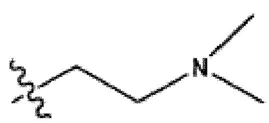 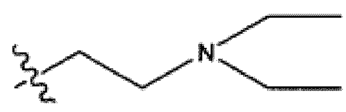

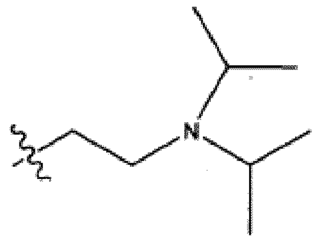
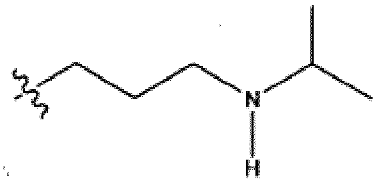
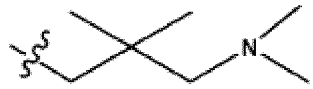

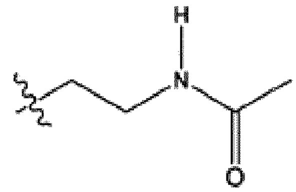
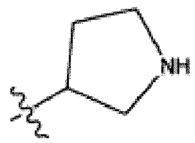
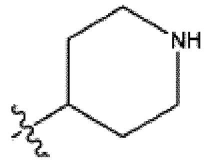
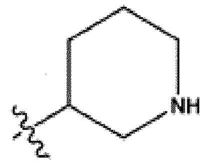

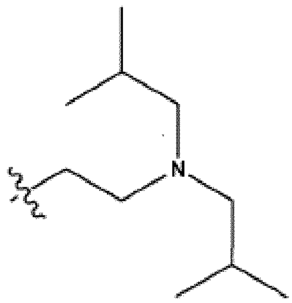
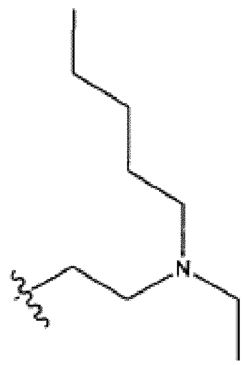
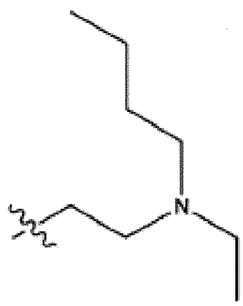

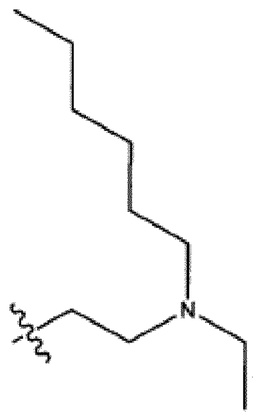
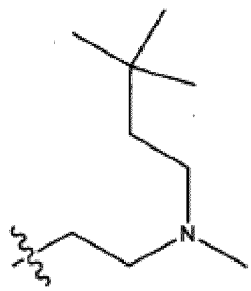
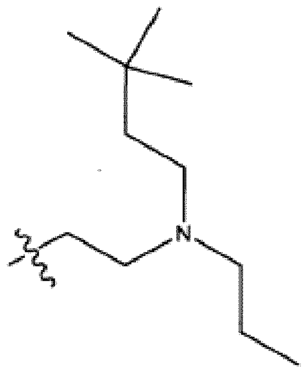

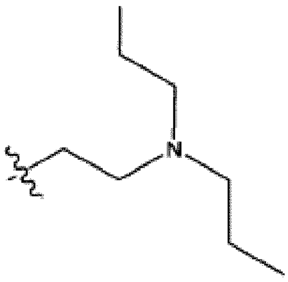
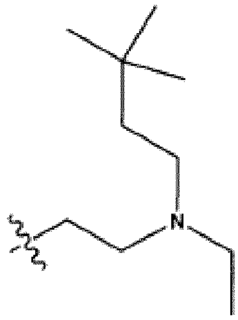
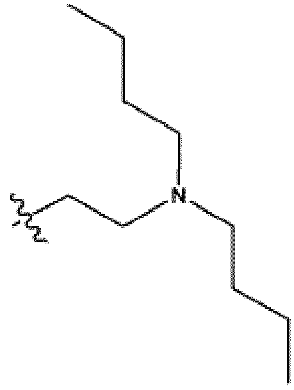

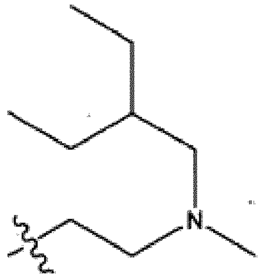
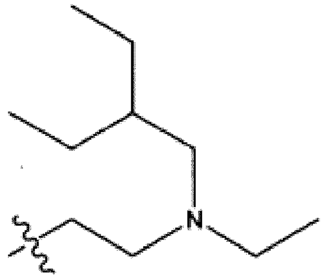
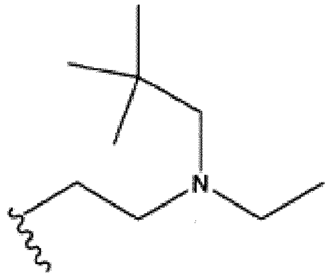

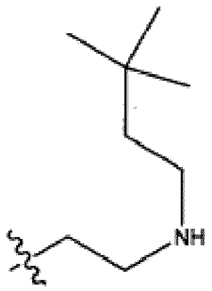
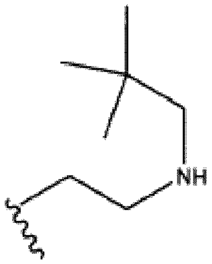
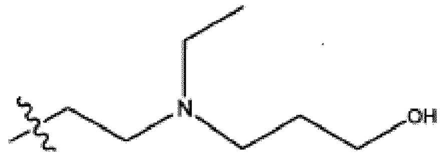

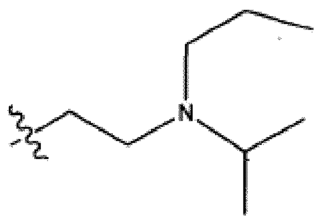
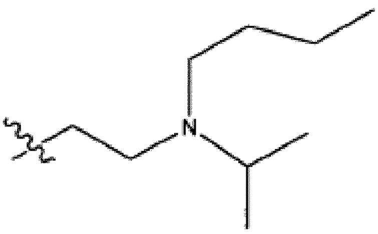
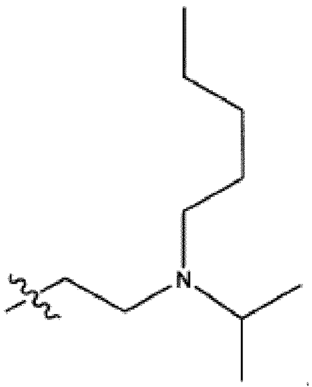

34. A method according to any one of embodiments 22 to 33 wherein in the compound of formula (1) $R^2$ is selected from the group consisting of H, alkyl, cycloalkyl, heteroalkyl, alkenyl, alkynyl, alkoxyalkyl and cycloalkylalkyl each of which may be unsubstituted or substituted.

35. A method according to embodiment 34 wherein in the compound of formula (1) $R^2$ is selected from the group consisting of: H; methyl; ethoxymethyl; [Bicyclo[2.2.1]2-ylmethyl; Adamantan-2-ylmethyl; 2-methansulfanyl-ethyl;

2,2,2-triflouro-ethyl; propyl; 2-2-dimethyl-propyl; isopropyl; 3,3,3-triflouro-propyl; butyl; isobutyl; 3,3-dimethyl-butyl; but-3-enyl; but-3-yny; pentyl; 2,4,4-trimethyl-pentyl; Bicyclo[2.2.1]hept-5-en-2yl; hexyl; hex-3-enyl; octyl; non-3-enyl; non-6-enyl; 2-methoxy-nonyl, 2-phenyl-cyclopropyl; cyclohexyl; $(CH_3)_3CCH_2CONH(CH_2)_2$-; $(CH_3)_3CCONH(CH_2)_2$-; $(CH_3)_3CCONH(CH_2)$- and $CH_3(CH_2)_2CONH(CH_2)$-.

36. A method according to embodiment 22 wherein the compound of formula (1) is selected from the group consisting of

- (E)-3-[1-(3-Dimethylamino-2,2-dimethyl-propyl)-2-isopropyl-1H-benzimidazol-5-yl]-N-hydroxy-acrylamide
- (E)-3-[2-Butyl-1-(3-dimethylamino-2,2-dimethyl-propyl)-1H-benzimidazol-5-yl]-N-hydroxy-acrylamide
- (E)-3-[1-(3-Dimethylamino-2,2-dimethyl-propyl)-2-(2-methylsulfanyl-ethyl)-1H-benzimidazol-5-yl]-N-hydroxy-acrylamide
- (E)-3-[1-(3-Dimethylamino-2,2-dimethyl-propyl)-2-ethoxymethyl-1H-benzimidazol-5-yl]-N-hydroxy-acrylamide
- (E)-3-[1-(3-Dimethylamino-2,2-dimethyl-propyl)-2-isobutyl-1H-benzimidazol-5-yl]-N-hydroxy-acrylamide
- (E)-3-[1-(2-Diethylamino-ethyl)-2-isobutyl-1H-benzimidazol-5-yl]-N-hydroxy-acrylamide
- (E)-3-[2-Butyl-1-(2-diethylamino-ethyl)-1H-benzimidazol-5-yl]-N-hydroxy-acrylamide
- (E)-3-[2-But-3-ynyl-1-(3-dimethylamino-2,2-dimethyl-propyl)-1H-benzimidazol-5-yl]-N-hydroxy-acrylamide
- (E)-3-[2-But-3-enyl-1-(3-dimethylamino-2,2-dimethyl-propyl)-1H-benzimidazol-5-yl]-N-hydroxy-acrylamide
- (E)-3-[2-But-3-enyl-1-(2-diethylamino-ethyl)-1H-benzimidazol-5-yl]-N-hydroxy-acrylamide
- (E)-3-[2-But-3-ynyl-1-(2-diethylamino-ethyl)-1H-benzimidazol-5-yl]-N-hydroxy-acrylamide
- (E)-3-[1-(3-Dimethylamino-2,2-dimethyl-propyl)-2-(3,3,3-trifluoro-propyl)-1H-benzimidazol-5-yl]-N-hydroxy-acrylamide
- (E)-3-[1-(2-Diethylamino-ethyl)-2-(3,3,3-trifluoro-propyl)-1H-benzimidazol-5-yl]-N-hydroxy-acrylamide
- (E)-3-[1-(2-Diethylamino-ethyl)-2-ethoxymethyl-1H-benzimidazol-5-yl]-N-hydroxy-acrylamide
- (E)-3-[1-(3-Dimethylamino-2,2-dimethyl-propyl)-2-methyl-1H-benzimidazol-5-yl]-N-hydroxy-acrylamide
- (E)-3-[1-(2-Dimethylamino-ethyl)-2-(2,2-dimethyl-propyl)-1H-benzimidazol-5-yl]-N-hydroxy-acrylamide
- (E)-N-Hydroxy-3-[1-(3-isopropylamino-propyl)-2-(3,3,3-trifluoro-propyl)-1H-benzimidazol-5-yl]-acrylamide
- (E)-3-[2-(2,2-Dimethyl-propyl)-1-(2-isopropylamino-ethyl)-1H-benzimidazol-5-yl]-N-hydroxy-acrylamide
- (E)-3-[1-(2-Diisopropylamino-ethyl)-2-(2,2-dimethyl-propyl)-1H-benzimidazol-5-yl]-N-hydroxy-acrylamide
- (E)-3-[1-(2-Diisopropylamino-ethyl)-2-isobutyl-1H-benzimidazol-5-yl]-N-hydroxy-acrylamide
- (E)-3-[1-(3-Dimethylamino-2,2-dimethyl-propyl)-2-hex-3-enyl-1H-benzimidazol-5-yl]-N-hydroxy-acrylamide
- (E)-3-[1-(3-Dimethylamino-2,2-dimethyl-propyl)-2-(2,4,4-trimethyl-pentyl)-1H-benzimidazol-5-yl]-N-hydroxy-acrylamide
- (E)-3-[2-Cyclohexyl-1-(3-dimethylamino-2,2-dimethyl-propyl)-1H-benzimidazol-5-yl]-N-hydroxy-acrylamide
- (E)-3-[2-Bicyclo[2.2.1]hept-5-en-2-yl-1-(3-dimethylamino-2,2-dimethyl-propyl)-1H-benzimidazol-5-yl]-N-hydroxy-acrylamide

- (E)-3-[1-(2-Diethylamino-ethyl)-2-hex-3-enyl-1H-benzimidazol-5-yl]-N-hydroxy-acrylamide
- (E)-3-[1-(2-Diisopropylamino-ethyl)-2-hex-3-enyl-1H-benzimidazol-5-yl]-N-hydroxy-acrylamide
- (E)-3-[2-Hex-3-enyl-1-(2-isopropylamino-ethyl)-1H-benzimidazol-5-yl]-N-hydroxy-acrylamide
- (E)-3-[2-Hex-3-enyl-1-(3-isopropylamino-propyl)-1H-benzimidazol-5-yl] -N-hydroxy-acrylamide
- (E)-3-[1-(2-Ethylamino-ethyl)-2-hex-3-enyl-1H-benzimidazol-5-yl]-N-hydroxy-acrylamide
- (E)-3-[1-(2-Diethylamino-ethyl)-2-hexyl-1H-benzimidazol-5-yl]-N-hydroxy-acrylamide
- (E)-N-Hydroxy-3-[1-(3-isopropylamino-propyl)-2-(2,4,4-trimethyl-pentyl)-1H-benzimidazol-5-yl]-acrylamide
- (E)-3-[2-(2,2-Dimethyl-propyl)-1-(3-isopropylamino-propyl)-1H-benzimidazol-5-yl]-N-hydroxy-acrylamide
- (E)-3-[1-(2-Diisopropylamino-ethyl)-2-(3,3,3-trifluoro-propyl)-1H-benzimidazol-5-yl]-N-hydroxy-acrylamide
- (E)-N-Hydroxy-3-[2-isobutyl-1-(2-isopropylamino-ethyl)-1H-benzimidazol-5-yl]-acrylamide
- (E)-3-[2-(2,2-Dimethyl-propyl)-1-(2-ethylamino-ethyl)-1H-benzimidazol-5-yl]-N-hydroxy-acrylamide
- (E)-3-[1-(2-Ethylamino-ethyl)-2-isobutyl-1H-benzimidazol-5-yl]-N-hydroxy-acrylamide
- (E)-3-[1-(2-Diisopropylamino-ethyl)-2-(2,4,4-trimethyl-pentyl)-1H-benzimidazol-5-yl]-N-hydroxy-acrylamide
- (E)-N-Hydroxy-3-[1-(2-isopropylamino-ethyl)-2-(2,4,4-trimethyl-pentyl)-1H-benzimidazol-5-yl]-acrylamide
- (E)-3-[1-(2-Ethylamino-ethyl)-2-(2,4,4-trimethyl-pentyl)-1H-benzimidazol-5-yl]-N-hydroxy-acrylamide
- (E)-3-[1-(2-Diethylamino-ethyl)-2-(2,4,4-trimethyl-pentyl)-1H-benzimidazol-5-yl]-N-hydroxy-acrylamide
- (E)-3-[1-(2-Diethylamino-ethyl)-2-propyl-1H-benzimidazol-5-yl]-N-hydroxy-acrylamide
- (E)-3-[2-Butyl-1-(2-diisopropylamino-ethyl)-1H-benzimidazol-5-yl]-N-hydroxy-acrylamide
- (E)-3-[2-Butyl-1-(2-ethylamino-ethyl)-1H-benzimidazol-5-yl]-N-hydroxy-acrylamide
- (E)-3-[1-(2-Diethylamino-ethyl)-2-(2-methylsulfanyl-ethyl)-1H-benzimidazol-5-yl]-N-hydroxy-acrylamide
- (E)-3-[2-Butyl-1-(2-isopropylamino-ethyl)-1H-benzimidazol-5-yl]-N-hydroxy-acrylamide
- (E)-3-[2-Butyl-1-(3-isopropylamino-propyl)-1H-benzimidazol-5-yl]-N-hydroxy-acrylamide
- (E)-3-[1-(1-Benzyl-piperidin-4-yl)-2-butyl-1H-benzimidazol-5-yl]-N-hydroxy-acrylamide
- (E)-3-[2-But-3-enyl-1-(2-ethylamino-ethyl)-1H-benzimidazol-5-yl]-N-hydroxy-acrylamide
- (E)-3-[2-Hexyl-1-(2-isopropylamino-ethyl)-1H-benzimidazol-5-yl]-N-hydroxy-acrylamide
- (E)-3-[1-(2-Dimethylamino-ethyl)-2-(2,4,4-trimethyl-pentyl)-1H-benzimidazol-5-yl)-N-hydroxy-acrylamide
- (E)-3-[1-(2-Ethylamino-ethyl)-2-hexyl-1H-benzimidazol-5-yl]-N-hydroxy-acrylamide
- (E)-N-Hydroxy-3-[1-(2-isopropylamino-ethyl)-2-(3,3,3-trifluoro-propyl)-1H-benzimidazol-5-yl]-acrylamide

- (E)-3-[1-(2-Dimethylamino-ethyl)-2-hex-3-enyl-1H-benzimidazol-5-yl]-N-hydroxy-acrylamide
- (E)-3-[1-(2-Amino-ethyl)-2-(2,4,4-trimethyl-pentyl)-1H-benzimidazol-5-yl]-N-hydroxy-acrylamide
- (E)-3-[1-(2-Amino-ethyl)-2-(2-methoxy-nonyl)-1H-benzimidazol-5-yl]-N-hydroxy-acrylamide
- (E)-3-[2-Butyl-1-(2-dimethylamino-ethyl)-1H-benzimidazol-5-yl]-N-hydroxy-acrylamide
- (E)-3-[1-(2-Dimethylamino-ethyl)-2-hexyl-1H-benzimidazol-5-yl]-N-hydroxy-acrylamide
- (E)-N-{2-[1-(2-Diethylamino-ethyl)-5-(2-hydroxycarbamoyl-vinyl)-1H-benzimidazol-2-yl]-ethyl}-3,3-dimethyl-butyramide
- (E)-3-{1-(2-Diethylamino-ethyl)-2-[2-(2,2-dimethyl-propionylamino)-ethyl]-1H-benzimidazol-5-yl}-N-hydroxy-acrylamide
- (E)-3-{1-(2-Diethylamino-ethyl)-2-[(2,2-dimethyl-propionylamino)-methyl]-1H-benzimidazol-5-yl}-N-hydroxy-acrylamide
- (E)-N-[1-(2-Diethylamino-ethyl)-5-(2-hydroxycarbamoyl-vinyl)-1H-benzimidazol-2-ylmethyl]-butyramide
- (E)-3-[1-(2-ethylamino-ethyl)-2-(3,3-dimethyl-butyl)-1H-benzimidazol-5-yl]-N-hydroxy-acrylamide
- (E)-3-[2-(3,3-Dimethyl-butyl)-1-(2-Dimethylamino-ethyl)-1H-benzimidazol-5-yl]-N-hydroxy-acrylamide
- (E)-3-[1-(2-Dimethylamino-ethyl)-2-pentyl-1H-benzimidazol-5-yl]-N-hydroxy-acrylamide
- (E)-3-[1-(2-Dimethylamino-ethyl)-2-(2,2,2-trifluoro-ethyl)-1H-benzimidazol-5-yl]-N-hydroxy-acrylamide
- (E)-N-Hydroxy-3-[1-(5-methyl-1H-pyrazol-3-yl)-2-(2,4,4-trimethyl-pentyl)-1H-benzimidazol-5-yl] -acrylamide
- (E)-3-[1-(2-Ethylamino-ethyl)-2-pentyl-1H-benzimidazol-5-yl]-N-hydroxy-acrylamide
- (E)-3-(2-Butyl-1-pyrrolidin-3-yl-1H-benzimidazol-5-yl)-N-hydroxy-acrylamide
- (E)-3-(2-Butyl-1-piperidm-4-yl-1H-benzimidazol-5-yl)-N-hydroxy-acrylamide
- (E)-N-Hydroxy-3-[1-(2-isopropylamino-ethyl)-2-pentyl-1H-benzimidazol-5-yl]-acrylamide
- (E)-N-Hydroxy-3-[1-(2-methylamino-ethyl)-2-non-3-enyl-1H-benzimidazol-5-yl]-acrylamide
- (E)-N-Hydroxy-3-[1-(2-methylamino-ethyl)-2-non-6-enyl-1H-benzimidazol-5-yl]-acrylamide
- (E)-3-[2-Hexyl-1-(2-methylamino-ethyl)-1H-benzimidazol-5-yl]-N-hydroxy-acrylamide
- (E)-N-Hydroxy-3-[1-(2-methylamino-ethyl)-2-pentyl-1H-benzimidazol-5-yl]-acrylamide
- (E)-N-Hydroxy-3-[1-(2-methylamino-ethyl)-2-octyl-1H-benzimidazol-5-yl]-acrylamide
- (E)-3-[1-(2-Amino-ethyl)-2-octyl-1H-benzimidazol-5-yl]-N-hydroxy-acrylamide
- (E)-3-{2-Butyl-1-[2-(isopropyl-methyl-amino)-ethyl]-1H-benzimidazo1-5-yl}-N-hydroxy-acrylamide
- (E)-3-{1-[2-(Ethyl-methyl-amino)-ethyl]-2-pentyl-1H-benzimidazo1-5-yl}-N-hydroxy-acrylamide
- (E)-3-(2-Hexyl-1-pyrrolidin-3-yl-1H-benzimidazol-5-yl)-N-hydroxy-acrylamide
- (E)-3-[2-Butyl-1-(1-methyl-pyrrolidin-3-yl)-1H-benzimidazol-5-yl]-N-hydroxy-acrylamide

- (E)-3-(2-Butyl-1-piperidin-3-yl-1H-benzimidazol-5-yl)-N-hydroxy-acrylamide
- (E)-3-(2-Hexyl-1-piperidin-3-yl-1H-benzimidazol-5-yl)-N-hydroxy-acrylamide
- (E)-3-(1-{2-[Ethyl-(2-methoxy-ethyl)-amino]-ethyl}-2-pentyl-1H-benzimidazol-5-yl)-N-hydroxy-acrylamide
- (E)-3-{2-Butyl-1-[2-(ethyl-methyl-amino)-ethyl]-1H-benzimidazol-5-yl}-N-hydroxy-acrylamide
- (E)-N-Hydroxy-3-[1-(1-methyl-piperidin-3-yl)-2-pentyl-1H-benzimidazol-5-yl]-acrylamide
- (E)-3-{1-[2-(Ethyl-hexyl-amino)-ethyl]-1H-benzimidazol-5-yl}-N-hydroxy-acrylamide
- (E)-3-{1-[2-(Ethyl-pentyl-amino)-ethyl]-1H-benzimidazol-5-yl}-N-hydroxy-acrylamide
- (E)-3-{1-[2-(Ethyl-heptyl-amino)-ethyl]-1H-benzimidazol-5-yl}-N-hydroxy-acrylamide
- (E)-3-{2-Hexyl-1-[1-(2-hydroxy-ethyl)-piperidin-3-yl]-1H-benzimidazol-5-yl}-N-hydroxy-acrylamide
- (E)-3-(2-Butyl-1-{2-[ethyl-(3-hydroxy-propyl)-amino]-ethyl}-1H-benzimidazol-5-yl)-N-hydroxy-acrylamide
- (E)-3-(1-{2-[Ethyl-(3-hydroxy-propyl)-amino]-ethyl}-2-pentyl-1H-benzimidazol-5-yl)-N-hydroxy-acrylamide
- (E)-N-hydroxy-3-(1-(1-phenethylpyrrolidin-3-yl)-1H-benzo[d]imidazol-5-yl)acrylamide
- (E)-N-hydroxy-3-(1-(1-pentylpiperidin-3-yl)-1H-benzo[d]imidazol-5-yl)acrylamide
- (E)-3-{1-[2-(Butyl-ethyl-amino)-ethyl]-1H-benzimidazol-5-yl)-N-hydroxy-acrylamide
- (E)-N-hydroxy-3-(1-(1-phenethylpiperidin-3-yl)-1H-benzo[d]imidazol-5-yl)acrylamide
- (E)-N-hydroxy-3-(1-(1-(3-phenylpropyl)piperidin-3-yl)-1H-benzo[d]imidazol-5-yl)acrylamide
- (E)-N-hydroxy-3-(1-(1-(3-phenylpropyl)pyrrolidin-3-yl)-1H-benzo[d]imidazol-5-yl)acrylamide
- (E)-3-{1-[1-(3,3-Dimethyl-butyl)-pyrrolidin-3-yl]-1H-benzimidazol-5-yl}-N-hydroxy-acrylamide
- (E)-3-(1-(2-(diethylamino)ethyl)-1H-benzo[d]imidazol-5-yl)-N-hydroxyacrylamide
- (E)-3-[2-(4-Cyano-butyl)-1-(2-diethylamino-ethyl)-1H-benzimidazol-5-yl]-N-hydroxy-acrylamide
- (E)-3-(1-(1-butylpiperidin-3-yl)-1H-benzo[d]imidazol-5-yl)-N-hydroxyacrylamide
- (E)-N-hydroxy-3-(1-(1-(pent-4-enyl)piperidin-3-yl)-1H-benzo[d]imidazol-5-yl)acrylamide
- (E)-3-(1-(1-(3,3-dimethylbutyl)piperidin-4-yl)-1H-benzo[d]imidazol-5-yl)-N-hydroxyacrylamide
- (E)-3-[1-(2-Diethylamino-ethyl)-2-propylamino-1H-benzimidazol-5-yl]-N-hydroxy-acrylamide
- (E)-N-hydroxy-3-(1-(2-(isopropyl(propyl)amino)ethyl)-1H-benzo[d]imidazol-5-yl)acrylamide
- (E)-3-{1-[2-(Butyl-isopropyl-amino)-ethyl]-1H-benzimidazo1-5-yl}-N-hydroxy-acrylamide
- (E)-N-Hydroxy-3-{1-[2-(isopropyl-pentyl-amino)-ethyl]-1H-benzimidazol-5-yl}-acrylamide
- (E)-3-[2-(5-Cyano-pentyl)-1-(2-diethylamino-ethyl)-1H-benzimidazol-5-yl]-N-hydroxy-acrylamide
- (E)-3-(1-{2-[(3,3-Dimethyl-butyl)-ethyl-amino]-ethyl}-1H-benzimidazol-5-yl)-N-hydroxy-acrylamide

- (E)-3-{1-[2-(Ethyl-propyl-amino)-ethyl]-1H-benzimidazo1-5-yl}-N-hydroxy-acrylamide
- (E)-N-Hydroxy-3-(1-{2-[isopropyl-(2-methyl-pentyl)-amino]-ethyl}-1H-benzimidazol-5-yl)-acrylamide
- (E)-N-hydroxy-3-(1-(2-(isopropyl(4,4,4-trifluorobutyl)amino)ethyl)-1H-benzo[d]imidazol-5-yl)acrylamide
- (E)-3-[1-(3-Dimethylamino-2,2-dimethyl-propyl)-2-propylamino-1H-benzimidazol-5-yl]-N-hydroxy-acrylamide
- (E)-3-{1-[2-(Ethyl-hexyl-amino)-ethyl]-2-methyl-1H-benzimidazol-5-yl}-N-hydroxy-acrylamide
- (E)-3-{1-[2-(Butyl-ethyl-amino)-ethyl]-2-trifluoromethyl-1H-benzimidazol-5-yl} -N-hydroxy-acrylamide
- (E)-3-{1-[2-(Ethyl-hexyl-amino)-ethyl]-2-trifluoromethyl-1H-benzimidazol-5-yl} -N-hydroxy-acrylamide
- (E)-3-(1-(2-(dibutylamino)ethyl)-2-propyl-1H-benzo[d]imidazol-5-yl)-N-hydroxyacrylamide
- (E)-3-[1-(2-Dipropylamino-ethyl)-1H-benzimidazol-5-yl]-N-hydroxy-acrylamide
- (E)-N-Hydroxy-3-(1-{2-[isopropyl-(3-methyl-butyl)-amino]-ethyl}-1H-benzimidazo1-5-yl)-acrylamide
- (E)-3-(1-{2-[(3,3-Dimethyl-butyl)-methyl-amino]-ethyl}-1H-benzimidazol-5-yl)-N-hydroxy-acrylamide
- (E)-3-(1-{2-[(2-Ethyl-butyl)-methyl-amino]-ethyl}-1H-benzimidazol-5-yl)-N-hydroxy-acrylamide
- (E)-3-(1-(2-(bis(3,3-dimethylbutyl)amino)ethyl)-1H-benzo[d]imidazol-5-yl)-N-hydroxyacrylamide
- (E)-3-(1-(2-(diisobutylamino)ethyl)-1H-benzo[d]imidazol-5-yl)-N-hydroxyacrylamide
- (E)-3-{1-[2-(3,3-Dimethyl-butylamino)-ethyl]-1H-benzimidazol-5-yl}-N-hydroxy-acrylamide
- (E)-N-Hydroxy-3-{1-[2-(methyl-pent-4-enyl-amino)-ethyl]-1H-benzimidazol-5-yl}-acrylamide
- (E)-3-(1-{2-[(3,3-Dimethyl-butyl)-propyl-amino]-ethyl}-1H-benzimidazol-5-yl)-N-hydroxy-acrylamide
- (E)-3-[1-(3-Dimethylamino-2,2-dimethyl-propyl)-2-methylsulfanyl-1H-benzimidazol-5-yl]-N-hydroxy-acrylamide
- (E)-3-{1-[2-(3,3-Dimethyl-butylamino)-ethyl]-2-propyl-1H-benzimidazol-5-yl}-N-hydroxy-acrylamide
- (E)-3-[1-[2-(3,3-Dimethyl-butylamino)-ethyl]-2-(2,2-dimethyl-propyl)-1H-benzimidazol-5-yl]-N-hydroxy-acrylamide
- (E)-3-[1-{2-[Bis-(3,3-dimethyl-butyl)-amino]-ethyl}-2-(2,2-dimethyl-propyl)-1H-benzimidazol-5-yl]-N-hydroxy-acrylamide
- (E)-3-{1-[2-(2,2-Dimethyl-propylamino)-ethyl]-1H-benzimidazol-5-yl}-N-hydroxy-acrylamide
- (E)-3-(1-{2-[(2,2-Dimethyl-propyl)-propyl-amino]-ethyl}-1H-benzimidazol-5-yl)-N-hydroxy-acrylamide
- (E)-3-{1-[2-(3,3-Dimethyl-butylamino)-ethyl]-2-ethyl-1H-benzimidazol-5-yl}-N-hydroxy-acrylamide
- (E)-3-(1-{2-[(3,3-Dimethyl-butyl)-methyl-amino]-ethyl}-2-propyl-1H-benzimidazol-5-yl)-N-hydroxy-acrylamide
- (E)-3-(1-{2-[(3,3-Dimethyl-butyl)-(2,2,2-trifluoro-ethyl)-amino]-ethyl}-1H-benzimidazol-5-yl)-N-hydroxy-acrylamide, and
- (E)-3-(1-{2-[Butyl-(2,2,2-trifluoro-ethyl)-amino]-ethyl}-1H-benzimidazol-5-yl)-N-hydroxy-acrylamide
  or a pharmaceutically acceptable salt thereof.

37. A method according to any one of embodiments 21 to 36 wherein the second anti-cancer agent is selected from the group consisting of alkylating agents, antimetabolites, anti-tumour antibiotics, mitotic spindle agents, topoisomerase inhibitors, steroid and hormonal agents, monoclonal antibodies, proteasome inhibitors, thalidomide and its analogs, death receptor ligands, kinase inhibitors, anti-angiogenesis agents, HDAC inhibitors, synthetic zinc finger transcription factors, interferons, platelet growth factors, photosensitizers, hypomethylating agents, rapalogs, mTor inhibitors, derivatives thereof, analogs thereof and pharmaceutically acceptable salts thereof.

38. A method according to any one of embodiments 21 to 37 wherein the second anti-cancer agent is selected from the group consisting of Adriamycin, avastin, Cisplatin, Oxaliplatin, Carboplatin, Satroplatin, Gemcitabine, Dasatinib, decitabine (Dacogen), Docetaxel, Doxorubicin, hydroxydaunarubicin, Dexamethason, Chlorambucil, Fludarabine, Tarceva (Erlotinib), Iressa, Alimta, Melphalan, 5-Azacytidine (Vidaza), 5-FU (5-Fluorouracil), Actinomycin D, Paclitaxel, prednisone, Vinblastine, lenalidomide (Revlimid), Irinotecan, Topotecan, Etoposide, Tamoxifen, Rituximab (Rituxan), Herceptin, Cetuximab, sorafenib, sutent, Angiostatin, Endostatin, temsirolemus, everolimus, Avastin (Bevacizumab, derivatives thereof, analogs thereof and pharmaceutically acceptable salts thereof.

39. A method according to any one of embodiments 21 to 38 wherein the first amount of a benzimidazole based anti-cancer agent and the second amount of a second anti-cancer agent are administered simultaneously.

40. A method according to any one of embodiments 21 to 38 wherein the first amount of a benzimidazole based anti-cancer agent and the second amount of a second anti-cancer agent are administered sequentially.

41. A method according to embodiment 40 wherein the method includes administration of a first amount of a benzimidazole based anti-cancer agent followed by administration of a second amount of a second anti-cancer agent.

42. A method according to embodiment 40 wherein the method includes administration of a second amount of the second anti-cancer agent followed by administration of a first amount of a benzimidazole based anti-cancer agent.

43. A method according to any one of embodiments 21 to 42 wherein the method includes administration of a further anti-cancer agent.

44. A method according to embodiment 43 wherein the further anti-cancer agent is selected from the group consisting of Adriamycin, avastin, Cisplatin, Oxaliplatin, Carboplatin, Satroplatin, Gemcitabine, Dasatinib, decitabine (Dacogen), Docetaxel, Doxorubicin, hydroxydaunarubicin, Dexamethason, Chlorambucil, Fludarabine, Tarceva (Erlotinib), Iressa, Alimta, Melphalan, 5-Azacytidine (Vidaza), 5-FU (5-Fluorouracil), Actinomycin D, Paclitaxel, prednisone, Vinblastine, lenalidomide (Revlimid), Irinotecan, Topotecan, Etoposide, Tamoxifen, Rituximab (Rituxan), Herceptin, Cetuximab, sorafenib, Angiostatin, Endostatin, temsirolemus, evirolemus, Avastin (Bevacizumab), derivatives thereof, analogs thereof, pharmaceutically acceptable salts thereof and combinations thereof.

45. A method according to embodiment 43 or 44 wherein the further anti-cancer agent is administered either simultaneously or sequentially with either the benzimidazole based anti-cancer agent and/or the second anti-cancer agent.

46. A method according to any one of embodiments 22 to 45 wherein the cancer is selected from the group consisting of colon cancer, gastric cancer, hepatocarcinoma, leukaemia, lymphoma, lung cancer, liver cancer, myelodysplastic syndrome, pancreatic cancer, prostate cancer, breast cancer, hepatoma, renal cell carcinoma, and ovarian cancer.

47. Use of a benzimidazole based anti-cancer agent in the manufacture of a medicament for the treatment of cancer by administration of (i) the benzimidazole based ant-cancer agent and (ii) a second anti-cancer agent.

48. A use according to embodiment 47 wherein the anti-cancer agent is a compound of the formula (1):

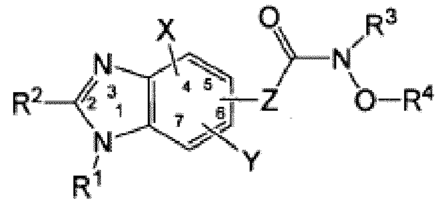

Formula (1)

wherein

$R^1$ is an optionally substituted heteroaryl group, an optionally substituted heterocycloalkyl group or a group of formula:
$-(CR^{20}R^{21})_m-(CR^{22}R^{23})_n-(CR^{24}R^{25})_o-NR^{26}R^{27}$;

$R^2$ is selected from the group consisting of: H, alkyl, alkenyl, alkynyl, heteroalkyl, cycloalkyl, cycloalkenyl, cycloalkylalkyl, alkoxyalkyl, $R^{11}S(O)R^{13}$-, $R^{11}S(O)_2R^{13}$-, $R^{11}C(O)N(R^{12})R^{13}$-, $R^{11}SO_2N(R^{12})R^{13}$-, $R^{11}N(R^{12})C(O)R^{13}$-, $R^{11}N(R^{12})SO_2R^{13}$-, $R^{11}N(R^{12})C(O)N(R^{12})R^{13}$- and acyl, each of which may be optionally substituted;

$R^3$ is selected from the group consisting of H, $C_1$-$C_6$ alkyl, and acyl, each of which may be optionally substituted;

X and Y are the same or different and are independently selected from the group consisting of: H, halogen, -CN, $-NO_2$, $-CF_3$, $-OCF_3$, alkyl, alkenyl, alkynyl, haloalkyl, haloalkenyl, haloalkynyl, heteroalkyl, cycloalkyl, cycloalkenyl, heterocycloalkyl, heterocycloalkenyl, aryl, heteroaryl, hydroxy, hydroxyalkyl, alkoxy, alkoxyalkyl, alkoxyaryl, alkoxyheteroaryl, alkenyloxy, alkynyloxy, cycloalkyloxy, cycloalkenyloxy, heterocycloalkyloxy, heterocycloalkenyloxy, aryloxy, heteroaryloxy, arylalkyl, heteroarylalkyl, arylalkyloxy, -amino, alkylamino, acylamino, aminoalkyl, arylamino, sulfonyl, alkylsulfonyl, arylsulfonyl, aminosulfonyl, aminoalkyl, alkoxyalky, -COOH -C(O)OR$^5$, -COR$^5$, -SH, -SR$^6$, -OR$^6$ acyl and -NR$^7$R$^8$, each of which may be optionally substituted;

each $R^4$ is selected from the group consisting of: H, alkyl, alkenyl, alkynyl, haloalkyl, heteroalkyl, cycloalkyl, heterocycloalkyl, aryl, heteroaryl, cycloalkylalkyl, heterocycloalkylalkyl, arylalkyl, heteroarylalkyl and acyl, each of which may be optionally substituted;

each $R^5$ is independently selected from the group consisting of: H, alkyl, alkenyl, alkynyl, haloalkyl, heteroalkyl, cycloalkyl, heterocycloalkyl, aryl, heteroaryl, cycloalkylalkyl, heterocycloalkylalkyl, arylalkyl, heteroarylalkyl and acyl, each of which may be optionally substituted;

each $R^6$ is independently selected from the group consisting of: H, alkyl, alkenyl, alkynyl, haloalkyl, heteroalkyl, cycloalkyl, heterocycloalkyl, aryl, heteroaryl, cycloalkylalkyl, heterocycloalkylalkyl, arylalkyl, heteroarylalkyl and acyl; each of which may be optionally substituted;

each $R^7$ and $R^8$ is independently selected from the group consisting of: H, alkyl, alkenyl, alkynyl, haloalkyl, heteroalkyl, cycloalkyl, heterocycloalkyl, aryl, heteroaryl, cycloalkylalkyl, heterocycloalkylalkyl, arylalkyl, heteroarylalkyl and acyl, each of which may be optionally substituted;

each $R^{11}$ and $R^{12}$ is independently selected from the group consisting of H, alkyl, alkenyl, and alkynyl, each of which may be optionally substituted;

each $R^{13}$ is a bond or is independently selected from the group consisting of: alkyl, alkenyl, and alkynyl, each of which may be optionally substituted;

each $R^{20}$, $R^{21}$, $R^{22}$, $R^{23}$, $R^{24}$ and $R^{25}$ is independently selected from the group consisting of: H, halogen, $-CN_1$ $-NO_2$, $-CF_3$, $-OCF_3$, alkyl, alkenyl, alkynyl, haloalkyl, haloalkenyl, haloalkynyl, heteroalkyl, cycloalkyl, cycloalkenyl, heterocycloalkyl, heterocycloalkenyl, aryl, heteroaryl, cycloalkylalkyl, heterocycloalkylalkyl, arylalkyl, heteroarylalkyl, arylalkenyl, cycloalkylheteroalkyl, heterocycloalkylheteroalkyl, heteroarylheteroalkyl, arylheteroalkyl, hydroxy, hydroxyalkyl, alkoxy, alkoxyalkyl, alkoxyaryl, ajkoxyheteroaryl, alkenyloxy, alkynyloxy, cycloalkylkoxy, heterocycloalkyloxy, aryloxy, arylalkyloxy, phenoxy, benzyloxy heteroaryloxy, amino, alkylamino, acylamino, aminoalkyl, arylamino, alkoxycarbonyl, alkylaminocarbonyl, sulfonyl, alkylsulfonyl, aminosulfonyl, arylsulfonyl, arylsulfinyl -COOH, -C(O)OR$^5$, -COR$^5$, -SH, -SR$^6$, -OR$^6$ and acyl, each of which may be optionally substituted; or

$R^{20}$ and $R^{21}$ when taken together may form a group of formula =O or =S, and/or

$R^{22}$ and $R^{23}$ when taken together may form a group of formula =O or =S, and/or

$R^{24}$ and $R^{25}$ when taken together may form a group of formula =O or =S;

each $R^{26}$ and $R^{27}$ is independently selected from the group consisting of is selected from the group consisting of: H, halogen, alkyl, alkenyl, alkynyl, haloalkyl, haloalkenyl, heteroalkyl, cycloalkyl, cycloalkenyl, heterocycloalkyl, heterocycloalkenyl, aryl, heteroaryl, cycloalkylalkyl, heterocycloalkylalkyl, arylalkyl, heteroarylalkyl, arylalkenyl, cycloalkylheteroalkyl, heterocycloalkylheteroalkyl, heteroarylheteroalkyl, arylheteroalkyl, hydroxy, hydroxyalkyl, alkoxy, alkoxyalkyl, alkoxyaryl, alkenyloxy, alkynyloxy, cycloalkylkoxy, heterocycloalkyloxy, aryloxy, arylalkyloxy, heteroaryloxy, amino, alkylamino, aminoalkyl, acylamino, arylamino, phenoxy, benzyloxy, COOH, alkoxycarbonyl, alkylaminocarbonyl, sulfonyl, alkylsulfonyl, alkylsulfinyl, arylsulfonyl, arylsulfinyl, aminosulfonyl, SR$^5$, and acyl, each of which may be optionally substituted, or $R^{26}$ and $R^{27}$ when taken together with the nitrogen atom to which they are attached form an optionally substituted heterocycloalkyl group;

Z is selected from the group consisting of $-CH_2-$, $-CH_2CH_2-$, -CH=CH-, $C_3$-$C_6$ alkylene, $C_3$-$C_6$ alkenylene, $C_3$-$C_6$ alkynylene, $C_3$-$C_6$ cycloalkyl, unsubstituted or substituted with one or more substituents independently selected from the group consisting of $C_1$-$C_4$ alkyl;

m, n and o are integers independently selected from the group consisting of 0, 1, 2, 3 and 4;

or a pharmaceutically acceptable salt or prodrug thereof.

49. A use according to embodiment 48 wherein in the compound of formula (1) Z is -CH=CH-, and is attached at ring position 5.

50. A use according to embodiment 48 or 49 wherein in the compound of formula (1) $R^3$, $R^4$, X and Y = H.

51. A use according to any one of embodiments 48 to 50 wherein in the compound of formula (1) $R^1$ is selected from the group consisting of:

$-(CR^{20}R^{21})_2-NR^{26}R^{27}$;

$-(CR^{22}R^{23})_2-NR^{26}R^{27}$;

$-(CR^{24}R^{25})_2-NR^{26}R^{27}$;

$-(CR^{20}R^{21})-(CR^{22}R^{23})-NR^{26}R^{27}$;

$-(CR^{20}R^{21})-(CR^{24}R^{25})-NR^{26}R^{27}$,

$-(CR^{22}R^{23})-(CR^{24}R^{25})-NR^{26}R^{27}$,

$-(CR^{20}R^{21})_3-NR^{26}R^{27}$;

$-(CR^{22}R^{23})_3-NR^{26}R^{27}$;

$-(CR^{24}R^{25})_3-NR^{26}R^{27}$;

$-(CR^{20}R^{21})_2-(CR^{22}R^{23})-NR^{26}R^{27}$;

$-(CR^{20}R^{21})_2-(CR^{24}R^{25})-NR^{26}R^{27}$;

$-(CR^{20}R^{21})-(CR^{22}R^{23})_2-NR^{26}R^{27}$;

$-(CR^{22}R^{23})_2-(CR^{24}R^{25})-NR^{26}R^{27}$;

$-(CR^{20}R^{21})-(CR^{24}R^{25})_2-NR^{26}R^{27}$;

$-(CR^{22}R^{23})-(CR^{24}R^{25})_2-NR^{26}R^{27}$; and

$-(CR^{20}R^{21})-(CR^{22}R^{23})-(CR^{24}R^{25})-NR^{26}R^{27}$.

52. A use according to embodiment 48 wherein the compound of formula (1) has the formula:

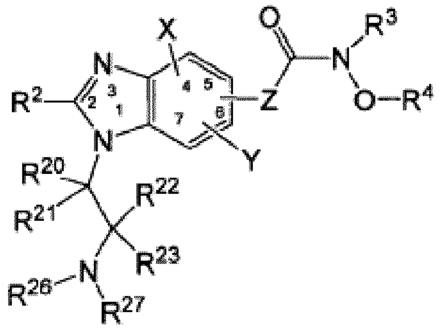

53. A use according to embodiment 48 wherein the compound of formula (1) has the formula:

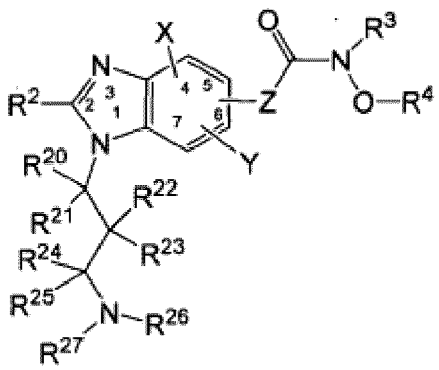

54. A use according to embodiment 52 or 53 wherein in the compound of formula (1) $R^{20}$, $R^{21}$, $R^{22}$, $R^{23}$, $R^{24}$ and $R^{25}$ are each independently selected from the group consisting of H, alkyl, alkenyl and alkynyl.

55. A use according to embodiment 51 wherein in the compound of formula (1) $R^{20}$, $R^{21}$, $R^{22}$, and $R^{23}$, are each H.

56. A use according to embodiment 51 wherein in the compound of formula (1) $R^{20}$, $R^{21}$, and $R^{24}$ and $R^{25}$ are each H, and $R^{22}$ and $R^{23}$ are each methyl.

57. A use according to any one of embodiments 48 to 56 wherein in the compound of formula (1) $R^{26}$ and $R^{27}$ are independently selected from the group consisting of: H, alkyl, alkenyl, alkynyl, alkoxyalkyl, and acyl.

58. A use according to embodiment 57 wherein in the compound of formula (1) $R^{26}$ and $R^{27}$ are independently selected from the group consisting of H, methyl, ethyl, isopropyl, propyl, butyl, isobutyl, pentyl, hexyl, heptyl, acetyl and 2-methoxy-ethyl.

59. A use according to any one of embodiments 48 to 58 wherein in the compound of formula (1) $R^1$ is a group of the formula:

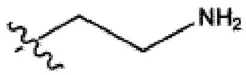
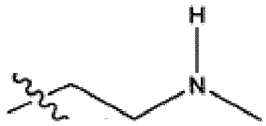
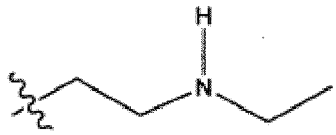
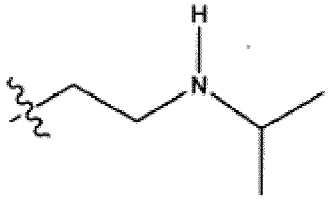
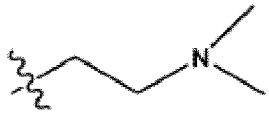
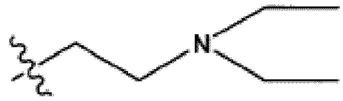
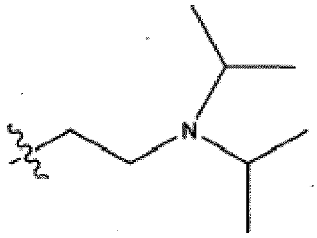
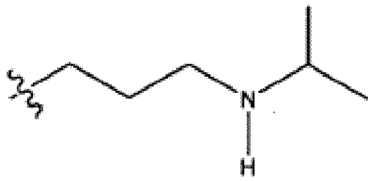
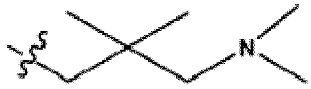
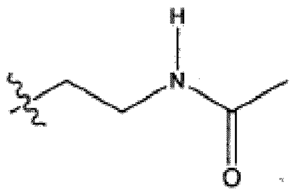
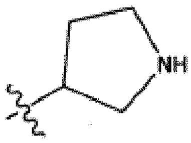
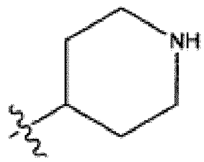
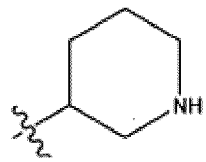
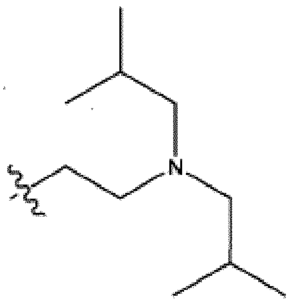
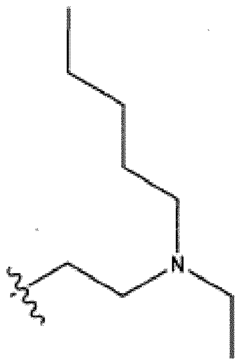
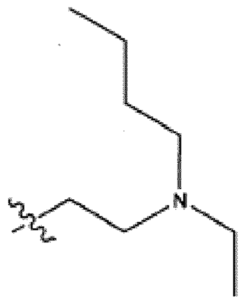

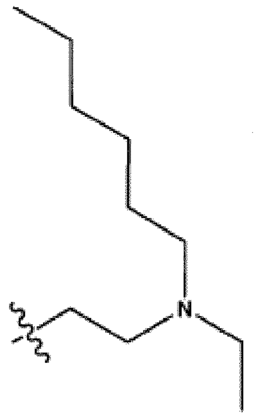
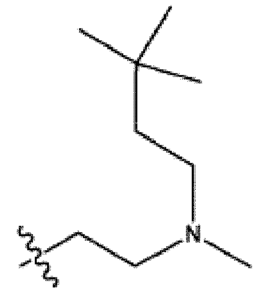
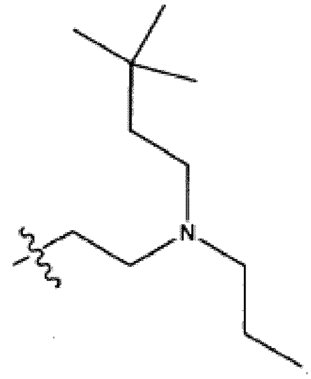

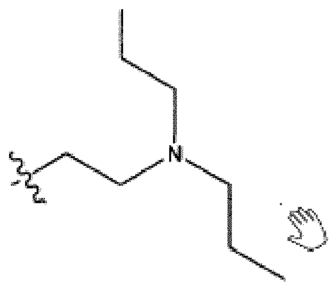
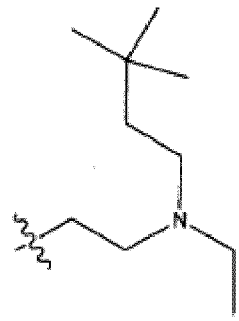
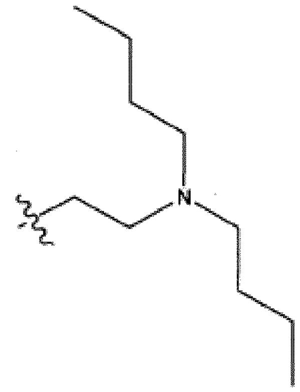

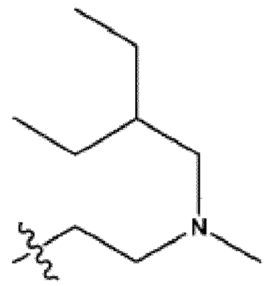
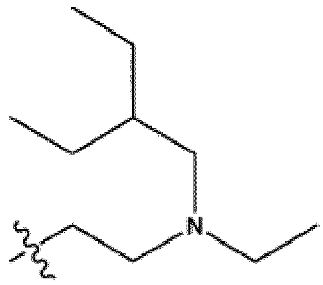
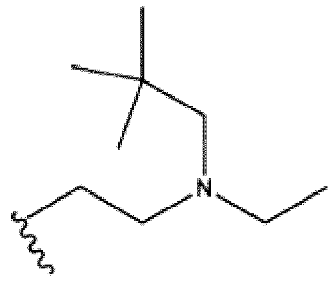

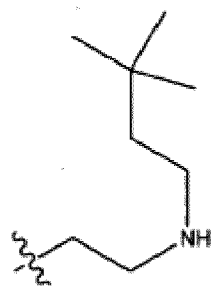
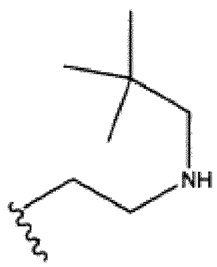
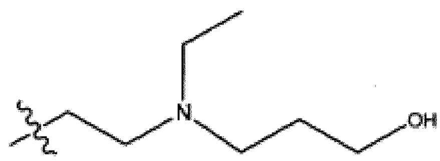

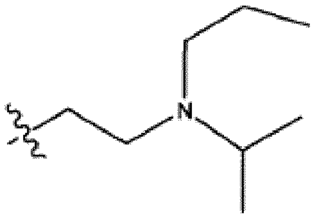
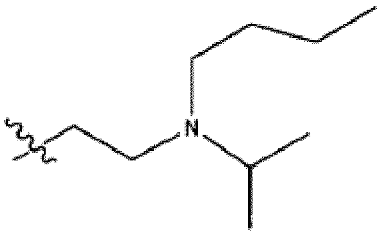
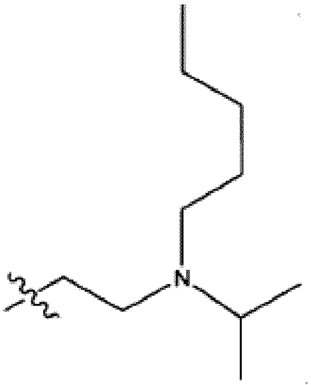

60. A use according to any one of embodiments 48 to 59 wherein in the compound of formula (1) $R^2$ is selected from the group consisting of H, alkyl, cycloalkyl, heteroalkyl, alkenyl, alkynyl, alkoxyalkyl and cycloalkylalkyl each of which may be unsubstituted or substituted.

61. A use according to embodiment 60 wherein in the compound of formula (1) $R^2$ is selected from the group consisting of: H; methyl; ethoxymethyl; [Bicylco[2.2.1]2-ylmethyl; Adamantan-2-ylmethyl; 2-methansulfanyl-ethyl; 2,2,2-triflouro-ethyl; propyl; 2-2-dimethyl-propyl; isopropyl; 3,3,3-triflouro-propyl; butyl; isobutyl; 3,3-dimethyl-butyl; but-3-enyl; but-3-yny; pentyl; 2,4,4-trimethyl-pentyl; Bicyclo[2.2.1]hept-5-en-2yl; hexyl; hex-3-enyl; octyl; non-3-enyl; non-6-enyl; 2-methoxy-nonyl, 2-phenyl-cyclopropyl; cyclohexyl; $(CH_3)_3CCH_2CONH(CH_2)_2$-; $(CH_3)_3CCONH(CH_2)_2$-; $(CH_3)_3CCONH(CH_2)$- and $CH_3(CH_2)_2CONH(CH_2)$-.

62. A use according to embodiment 48 wherein the compound of formula (1) is selected from the group consisting of

- (E)-3-[1-(3-Dimethylamino-2,2-dimethyl-propyl)-2-isopropyl-1H-benzimidazol-5-yl]-N-hydroxy-acrylamide
- (E)-3-[2-Butyl-1-(3-dimethylamino-2,2-dimethyl-propyl)-1H-benzimidazol-5-yl]-N-hydroxy-acrylamide
- (E)-3-[1-(3-Dimethylamino-2,2-dimethyl-propyl)-2-(2-methylsulfanyl-ethyl)-1H-benzimidazol-5-yl]-N-hydroxy-acrylamide
- (E)-3-[1-(3-Dimethylamino-2,2-dimethyl-propyl)-2-ethoxymethyl-1H-benzimidazol-5-yl]-N-hydroxy-acrylamide
- (E)-3-[1-(3-Dimethylamino-2,2-dimethyl-propyl)-2-isobutyl-1H-benzimidazol-5-yl]-N-hydroxy-acrylamide
- (E)-3-[1-(2-Diethylamino-ethyl)-2-isobutyl-1H-benzimidazol-5-yl]-N-hydroxy-acrylamide
- (E)-3-[2-Butyl-1-(2-diethylamino-ethyl)-1H-benzimidazol-5-yl]-N-hydroxy-acrylamide
- (E)-3-[2-But-3-ynyl-1-(3-dimethylamino-2,2-dimethyl-propyl)-1H-benzimidazol-5-yl]-N-hydroxy-acrylamide
- (E)-3-[2-But-3-enyl-1-(3-dimethylamino-2,2-dimethyl-propyl)-1H-benzimidazol-5-yl]-N-hydroxy-acrylamide
- (E)-3-[2-But-3-enyl-1-(2-diethylamino-ethyl)-1H-benzimidazol-5-yl]-N-hydroxy-acrylamide
- (E)-3-[2-But-3 -ynyl-1-(2-diethylamino-ethyl)-1H-benzimidazol-5-yl]-N-hydroxy-acrylamide
- (E)-3-[1-(3-Dimethylamino-2,2-dimethyl-propyl)-2-(3,3,3-trifluoro-propyl)-1H-benzimidazol-5-yl]-N-hydroxy-acrylamide
- (E)-3-[1-(2-Diethylamino-ethyl)-2-(3,3,3-trifluoro-propyl)-1H-benzimidazol-5-yl]-N-hydroxy-acrylamide
- (E)-3-[1-(2-Diethylamino-ethyl)-2-ethoxymethyl-1H-benzimidazol-5-yl]-N-hydroxy-acrylamide
- (E)-3-[1-(3-Dimethylamino-2,2-dimethyl-propyl)-2-methyl-1H-benzimidazol-5-yl]-N-hydroxy-acrylamide

- (E)-3-[1-(2-Diethylamino-ethyl)-2-(2,2-dimethyl-propyl)-1H-benzimidazol-5-yl]-N-hydroxy-acrylamide
- (E)-N-Hydroxy-3-[1-(3-isopropylamino-propyl)-2-(3,3,3-trifluoro-propyl)-1H-benzimidazol-5-yl]-acrylamide
- (E)-3-[2-(2,2-Dimethyl-propyl)-1-(2-isopropylamino-ethyl)-1H-benzimidazol-5-yl]-N-hydroxy-acrylamide
- (E)-3-[1-(2-Diisopropylamino-ethyl)-2-(2,2-dimethyl-propyl)-1H-benzimidazol-5-yl]-N-hydroxy-acrylamide
- (E)-3-[1-(2-Diisopropylamino-ethyl)-2-isobutyl-1H-benzimidazol-5-yl]-N-hydroxy-acrylamide
- (E)-3-[1-(3-Dimethylamino-2,2-dimethyl-propyl)-2-hex-3-enyl-1H-benzimidazol-5-yl]-N-hydroxy-acrylamide
- (E)-3-[1-(3-Dimethylamino-2,2-dimethyl-propyl)-2-(2,4,4-trimethyl-pentyl)-1H-benzimidazol-5-yl]-N-hydroxy-acrylamide
- (E)-3-[2-Cyclohexyl-1-(3-dimethylamino-2,2-dimethyl-propyl)-1H-benzimidazol-5-yl]-N-hydroxy-acrylamide
- (E)-3-[2-Bicyclo[2.2.1]hept-5-en-2-yl-1-(3-dimethylamino-2,2-dimethyl-propyl)-1H-benzimidazol-5-yl]-N-hydroxy-acrylamide
- (E)-3-[1-(2-Diethylamino-ethyl)-2-hex-3-enyl-1H-benzimidazo1-5-yl]-N-hydroxy-acrylamide
- (E)-3-[1-(2-Diisopropylamino-ethyl)-2-hex-3-enyl-1H-benzimidazol-5-yl]-N-hydroxy-acrylamide
- (E)-3-[2-Hex-3-enyl-1-(2-isopropylamino-ethyl)-1H-benzimidazo1-5-yl]-N-hydroxy-acrylamide
- (E)-3-[2-Hex-3-enyl-1-(3-isopropylamino-propyl)-1H-benzimidazol-5-yl]-N-hydroxy-acrylamide
- (E)-3-[1-(2-Ethylamino-ethyl)-2-hex-3-enyl-1H-benzimidazo1-5-yl]-N-hydroxy-acrylamide
- (E)-3-[1-(2-Diethylamino-ethyl)-2-hexyl-1H-benzimidazol-5-yl]-N-hydroxy-acrylamide
- (E)-N-Hydroxy-3-[1-(3-isopropylamino-propyl)-2-(2,4,4-trimethyl-pentyl)-1H-benzimidazol-5-yl]-acrylamide
- (E)-3-[2-(2,2-Dimethyl-propyl)-1-(3-isopropylamino-propyl)-1H-benzimidazol-5-yl]-N-hydroxy-acrylamide
- (E)-3-[1-(2-Diisopropylamino-ethyl)-2-(3,3,3-trifluoro-propyl)-1H-benzimidazol-5-yl]-N-hydroxy-acrylamide
- (E)-N-Hydroxy-3-[2-isobutyl-1-(2-isopropylamino-ethyl)-1H-benzimidazol-5-yl]-acrylamide
- (E)-3-[2-(2,2-Dimethyl-propyl)-1-(2-ethylamino-ethyl)-1H-benzimidazol-5-yl]-N-hydroxy-acrylamide
- (E)-3-[1-(2-Ethylamino-ethyl)-2-isobutyl-1H-benzimidazol-5-yl]-N-hydroxy-acrylamide
- (E)-3-[1-(2-Diisopropylamino-ethyl)-2-(2,4,4-trimethyl-pentyl)-1H-benzimidazol-5-yl]-N-hydroxy-acrylamide
- (E)-N-Hydroxy-3-[1-(2-isopropylamino-ethyl)-2-(2,4,4-trimethyl-pentyl)-1H-benzimidazol-5-yl]-acrylamide
- (E)-3-[1-(2-Ethylamino-ethyl)-2-(2,4,4-trimethyl-pentyl)-1H-benzimidazol-5-yl]-N-hydroxy-acrylamide
- (E)-3-[1-(2-Diethylamino-ethyl)-2-(2,4,4-trimethyl-pentyl)-1H-benzimidazol-5-yl]-N-hydroxy-acrylamide
- (E)-3-[1-(2-Diethylamino-ethyl)-2-propyl-1H-benzimidazol-5-yl]-N-hydroxy-acrylamide
- (E)-3-[2-Butyl-1-(2-diisopropylamino-ethyl)-1H-benzimidazol-5-yl]-N-hydroxy-acrylamide
- (E)-3-[2-Butyl-1-(2-ethylamino-ethyl)-1H-benzimidazol-5-yl]-N-hydroxy-acrylamide

- (E)-3-[1-(2-Diethylamino-ethyl)-2-(2-methylsulfanyl-ethyl)-1H-benzimidazol-5-yl]-N-hydroxy-acrylamide
- (E)-3-[2-Butyl-1-(2-isopropylamino-ethyl)-1H-benzimidazol-5-yl]-N-hydroxy-acrylamide
- (E)-3-[2-Butyl-1-(3-isopropylamino-propyl)-1H-benzimidazol-5-yl]-N-hydroxy-acrylamide
- (E)-3-[1-(1-Benzyl-piperidin-4-yl)-2-butyl-1H-benzimidazol-5-yl]-N-hydroxy-acrylamide
- (E)-3-[2-But-3-enyl-1-(2-ethylamino-ethyl)-1H-benzimidazol-5-yl]-N-hydroxy-acrylamide
- (E)-3-[2-Hexyl-1-(2-isopropylamino-ethyl)-1H-benzimidazol-5-yl]-N-hydroxy-acrylamide
- (E)-3-[1-(2-Dimethylamino-ethyl)-2-(2,4,4-trimethyl-pentyl)-1H-benzimidazol-5-yl]-N-hydroxy-acrylamide
- (E)-3-[1-(2-Ethylamino-ethyl)-2-hexyl-1H-benzimidazol-5-yl]-N-hydroxy-acrylamide
- (E)-N-Hydroxy-3-[1-(2-isopropylamino-ethyl)-2-(3,3,3-trifluoro-propyl)-1H-benzimidazol-5-yl]-acrylamide
- (E)-3-[1-(2-Dimethylamino-ethyl)-2-hex-3-enyl-1H-benzimidazol-5-yl]-N-hydroxy-acrylamide
- (E)-3-[1-(2-Amino-ethyl)-2-(2,4,4-trimethyl-pentyl)-1H-benzimidazol-5-yl]-N-hydroxy-acrylamide
- (E)-3-[1-(2-Amino-ethyl)-2-(2-methoxy-nonyl)-1H-benzimidazol-5-yl]-N-hydroxy-acrylamide
- (E)-3-[2-Butyl-1-(2-dimethylamino-ethyl)-1H-benzimidazol-5-yl]-N-hydroxy-acrylamide
- (E)-3-[1-(2-Dimethylamino-ethyl)-2-hexyl-1H-benzimidazol-5-yl]-N-hydroxy-acrylamide
- (E)-N-{2-[-(2-Diethylamino-ethyl)-5-(2-hydroxycarbamoyl-vinyl)-1H-benzimidazol-2-yl]-ethyl} -3,3-dimethyl-butyramide
- (E)-3-{1-(2-Diethylamino-ethyl)-2-[2-(2,2-dimethyl-propionylamino)-ethyl]-1H-benzimidazol-5-yl}-N-hydroxy-acrylamide
- (E)-3-{1-(2-Diethylamino-ethyl)-2-[(2,2-dimethyl-propionylamino)-methyl]-1H-benzimidazol-5-yl}-N-hydroxy-acrylamide
- (E)-N-[1-(2-Diethylamino-ethyl)-5-(2-hydroxycarbamoyl-vinyl)-1H-benzimidazo1-2-ylmethyl]-butyramide
- (E)-3-[1-(2-ethylamino-ethyl)-2-(3,3-dimethyl-butyl)-1H-benzimidazo1-5-yl]-N-hydroxy-acrylamide
- (E)-3-[2-(3,3-Dimethyl-butyl)-1-(2-Dimethylamino-ethyl)-1H-benzimidazol-5-yl]-N-hydroxy-acrylamide
- (E)-3-[1-(2-Dimethylamino-ethyl)-2-pentyl-1H-benzimidazol-5-yl]-N-hydroxy-acrylamide
- (E)-3-[1-(2-Dimethylamino-ethyl)-2-(2,2,2-trifluoro-ethyl)-1H-benzimidazol-5-yl]-N-hydroxy-acrylamide
- (E)-N-Hydroxy-3-[1-(5-methyl-1H-pyrazol-3-yl)-2-(2,4,4-trimethyl-pentyl)-1H-benzimidazol-5-yl]-acrylamide
- (E)-3-[1-(2-Ethylamino-ethyl)-2-pentyl-1H-benzimidazol-5-yl]-N-hydroxy-acrylamide
- (E)-3-(2-Butyl-1-pyrrolidin-3-yl-1H-benzimidazol-5-yl)-N-hydroxy-acrylamide
- (E)-3-(2-Butyl-1-piperidin-4-yl-1H-benzimidazol-5-yl)-N-hydroxy-acrylamide
- (E)-N-Hydroxy-3-[1-(2-isopropylamino-ethyl)-2-pentyl-1H-benzimidazol-5-yl]-acrylamide
- (E)-N-Hydroxy-3-[1-(2-methylamino-ethyl)-2-non-3-enyl-1H-benzimidazol-5-yl]-acrylamide

- (E)-N-Hydroxy-3-[1-(2-methylamino-ethyl)-2-non-6-enyl-1H-benzimidazol-5-yl]-acrylamide
- (E)-3-[2-Hexyl-1-(2-methylamino-ethyl)-1H-benzimidazol-5-yl] -N-hydroxy-acrylamide
- (E)-N-Hydroxy-3-[1-(2-methylamino-ethyl)-2-pentyl-1H-benzimidazol-5-yl]-acrylamide
- (E)-N-Hydroxy-3-[1-(2-methylamino-ethyl)-2-octyl-1H-benzimidazol-5-yl]-acrylamide
- (E)-3-[1-(2-Amino-ethyl)-2-octyl-1H-benzimidazol-5-yl]-N-hydroxy-acrylamide
- (E)-3-{2-Butyl-1-[2-(isopropyl-methyl-amino)-ethyl]-1H-benzimidazo1-5-yl}-N-hydroxy-acrylamide
- (E)-3-{1-[2-(Ethyl-methyl-amino)-ethyl]-2-pentyl-1H-benzimidazol-5-yl}-N-hydroxy-acrylamide
- (E)-3-(2-Hexyl-1-pyrrolidin-3-yl-1H-benzimidazol-5-yl)-N-hydroxy-acrylamide
- (E)-3-[2-Butyl-1-(1-methyl-pyrrolidin-3-yl)-1H-benzimidazol-5-yl]-N-hydroxy-acrylamide
- (E)-3-(2-Butyl-1-piperidin-3-yl-1H-benzimidazol-5-yl)-N-hydroxy-acrylamide
- (E)-3-(2-Hexyl-1-piperidin-3-yl-1H-benzimidazol-5-yl)-N-hydroxy-acrylamide
- (E)-3-(1-{2-[Ethyl-(2-methoxy-ethyl)-amino]-ethyl}-2-pentyl-1H-benzimidazol-S-yl)-N-hydroxy-acrylamide
- (E)-3-{2-Butyl-1-[2-(ethyl-methyl-amino)-ethyl]-1H-benzimidazol-5-yl}-N-hydroxy-acrylamide
- (E)-N-Hydroxy-3-[1-(1-methyl-piperidin-3-yl)-2-pentyl-1H-benzimidazol-5-yl]-acrylamide
- (E)-3-{1-[2-(Ethyl-hexyl-amino)-ethyl]-1H-benzimidazol-5-yl}-N-hydroxy-acrylamide
- (E)-3-{1-[2-(Ethyl-pentyl-amino)-ethyl]-1H-benzimidazol-5-yl}-N-hydroxy-acrylamide
- (E)-3-{1-[2-(Ethyl-heptyl-amino)-ethyl]-1H-benzimidazol-5-yl}-N-hydroxy-acrylamide
- (E)-3-{2-Hexyl-1-[1-(2-hydroxy-ethyl)-piperidin-3-yl]-1H-benzimidazol-5-yl}-N-hydroxy-acrylamide
- (E)-3-(2-Butyl-1-{2-[ethyl-(3-hydroxy-propyl)-amino]-ethyl}-1H-benzimidazol-5-yl)-N-hydroxy-acrylamide
- (E)-3-(1-{2-[Ethyl-(3-hydroxy-propyl)-amino]-ethyl}-2-pentyl-1H-benzimidazol-5-yl)-N-hydroxy-acrylamide
- (E)-N-hydroxy-3-(1-(1-phenethylpyrrolidin-3-yl)-1H-benzo[d]imidazol-5-yl)acrylamide
- (E)-N-hydroxy-3-(1-(1-pentylpiperidin-3-yl)-1H-benzo[d]imidazol-5-yl)acrylamide
- (E)-3-{1-[2-(Butyl-ethyl-amino)-ethyl]-1H-benzimidazol-5-yl}-N-hydroxy-acrylamide
- (E)-N-hydroxy-3-(1-(1-phenethylpiperidin-3-yl)-1H-benzo[d]imidazol-5-yl)acrylamide
- (E)-N-hydroxy-3-(1-(1-(3-phenylpropyl)piperidin-3-yl)-1H-benzo[d]imidazol-5-yl)acrylamide
- (E)-N-hydroxy-3-(1-(1-(3-phenylpropyl)pyrrolidin-3-yl)-1H-benzo[d]imidazol-5-yl)acrylamide
- (E)-3-{1-[1-(3,3-Dimethyl-butyl)-pyrrolidin-3-yl]-1H-benzimidazol-5-yl}-N-hydroxy-acrylamide
- (E)-3-(1-(2-(diethylamino)ethyl)-1H-benzo[d]imidazol-5-yl)-N-hydroxyacrylamide
- (E)-3-[2-(4-Cyano-butyl)-1-(2-diethylamino-ethyl)-1H-benzimidazol-5-yl]-N-hydroxy-acrylamide

- (E)-3-(1-(1-butylpiperidin-3-yl)-1H-benzo[d]imidazol-5-yl)-N-hydroxyacrylamide
- (E)-N-hydroxy-3-(1-(1-(pent-4-enyl)piperidin-3-yl)-1H-benzo[d]imidazol-5-yl)acrylamide
- (E)-3-(1-(1-(3,3-dimethylbutyl)piperidin-4-yl)-1H-benzo[d]imidazol-5-yl)-N-hydroxyacrylamide
- (E)-3-[1-(2-Diethylamino-ethyl)-2-propylamino-1H-benzimidazol-5-yl]-N-hydroxy-acrylamide
- (E)-N-hydroxy-3-(1-(2-(isopropyl(propyl)amino)ethyl)-1H-benzo[d]imidazol-5-yl)acrylamide
- (E)-3-{1-[2-(Butyl-isopropyl-amino)-ethyl]-1H-benzimidazol-5-yl}-N-hydroxy-acrylamide
- N-Hydroxy-3-{1-[2-(isopropyl-pentyl-amino)-ethyl]-1H-benzimidazol-5-yl}-acrylamide
- (E)-3-[2-(5-Cyano-pentyl)-1-(2-diethylamino-ethyl)-1H-benzimidazol-5-yl]-N-hydroxy-acrylamide
- (E)-3-(1-{2-[(3,3-Dimethyl-butyl)-ethyl-amino]-ethyl}-1H-benzimidazol-5-yl)-N-hydroxy-acrylamide
- (E)-3-{1-[2-(Ethyl-propyl-amino)-ethyl]-1H-benzimidazol-5-yl}-N-hydroxy-acrylamide
- (E)-N-Hydroxy-3-(1-{2-[isopropyl-(2-methyl-pentyl)-amino]-ethyl}-1H-benzimidazol-5-yl)-acrylamide
- (E)-N-hydroxy-3-(1-(2-(isopropyl(4,4,4-trifluorobutyl)amino)ethyl)-1H-benzo[d]imidazol-5-yl)acrylamide
- (E)-3-[1-(3-Dimethylamino-2,2-dimethyl-propyl)-2-propylamino-1H-benzimidazol-5-yl]-N-hydroxy-acrylamide
- (E)-3-{1-[2-(Ethyl-hexyl-amino)-ethyl]-2-methyl-1H-benzimidazol-5-yl}-N-hydroxy-acrylamide
- (E)-3-{1-[2-(Butyl-ethyl-amino)-ethyl]-2-trifluoromethyl-1H-benzimidazol-5-yl}-N-hydroxy-acrylamide
- (E)-3-{1-[2-(Ethyl-hexyl-amino)-ethyl]-2-trifluoromethyl-1H-benzimidazol-5-yl}-N-hydroxy-acrylamide
- (E)-3-(1-(2-(dibutylamino)ethyl)-2-propyl-1H-benzo[d]imidazol-5-yl)-N-hydroxyacrylamide
- (E)-3-[1-(2-Dipropylamino-ethyl)-1H-benzimidazol-5-yl]-N-hydroxy-acrylamide
- (E)-N-Hydroxy-3-(1-{2-[isopropyl-(3-methyl-butyl)-amino]-ethyl}-1H-benzimidazol-5-yl)-acrylamide
- (E)-3-(1-{2-[(3,3-Dimethyl-butyl)-methyl-amino]-ethyl}-1H-benzimidazol-5-yl)-N-hydroxy-acrylamide
- (E)-3-(1-{2-[(2-Ethyl-butyl)-methyl-amino]-ethyl}-1H-benzimidazol-5-yl)-N-hydroxy-acrylamide
- (E)-3-(1-(2-(bis(3,3-dimethylbutyl)amino)ethyl)-1H-benzo[d]imidazol-5-yl)-N-hydroxyacrylamide
- (E)-3-(1-(2-(diisobutylamino)ethyl)-1H-benzo[d]imidazol-5-yl)-N-hydroxyacrylamide
- (E)-3-{1-[2-(3,3-Dimethyl-butylamino)-ethyl]-1H-benzimidazol-5-yl}-N-hydroxy-acrylamide
- (E)-N-Hydroxy-3-{1-[2-(methyl-pent-4-enyl-amino)-ethyl]-1H-benzimidazol-5-yl}-acrylamide
- (E)-3-(1-{2-[(3,3-Dimethyl-butyl)-propyl-amino]-ethyl}-1H-benzimidazol-5-yl)-N-hydroxy-acrylamide
- (E)-3-[1-(3-Dimethylamino-2,2-dimethyl-propyl)-2-methylsulfanyl-1H-benzimidazol-5-yl]-N-hydroxy-acrylamide
- (E)-3-{1-[2-(3,3-Dimethyl-butylamino)-ethyl]-2-propyl-1H-benzimidazol-5-yl}-N-hydroxy-acrylamide
- (E)-3-[1-[2-(3,3-Dimethyl-butylamino)-ethyl]-2-(2,2-dimethyl-propyl)-1H-benzimidazol-5-yl]-N-hydroxy-acrylamide

- (E)-3-[1-{2-[Bis-(3,3-dimethyl-butyl)-amino]-ethyl}-2-(2,2-dimethyl-propyl)-1H-benzimidazol-5-yl]-N-hydroxy-acrylamide
- (E)-3-{1-[2-(2,2-Dimethyl-propylamino)-ethyl]-1H-benzimidazol-5-yl}-N-hydroxy-acrylamide
- (E)-3-(1-{2-[(2,2-Dimethyl-propyl)-propyl-amino]-ethyl}-1H-benzimidazol-5-yl)-N-hydroxy-acrylamide
- (E)-3-{1-[2-(3,3-Dimethyl-butylamino)-ethyl]-2-ethyl-1H-benzimidazol-5-yl}-N-hydroxy-acrylamide
- (E)-3-(1-{2-[(3,3-Dimethyl-butyl)-methyl-amino]-ethyl}-2-propyl-1H-benzimidazol-5-yl)-N-hydroxy-acrylamide
- (E)-3-(1-{2-[(3,3-Dimethyl-butyl)-(2,2,2-trifluoro-ethyl)-amino]-ethyl}-1H-benzimidazol-5-yl)-N-hydroxy-acrylamide,and
- (E)-3-(1-{2-[Butyl-(2,2,2-trifluoro-ethyl)-amino]-ethyl}-1H-benzimidazol-5-yl)-N-hydroxy-acrylamide
  or a pharmaceutically acceptable salt thereof.

63. A use according to any one of embodiments 47 to 62 wherein the second anti-cancer agent is selected from the group consisting of alkylating agents, antimetabolites, anti-tumour antibiotics, mitotic spindle agents, topoisomerase inhibitors, steroid and hormonal agents, monoclonal antibodies, proteasome inhibitors, thalidomide and its analogs, death receptor ligands, kinase inhibitors, anti-angiogenesis agents, HDAC inhibitors, synthetic zinc finger transcription factors, interferons, platelet growth factors, photosensitizers, hypomethylating agents, rapalogs, mTor inhibitors, derivatives thereof, analogs thereof and pharmaceutically acceptable salts thereof.

64. A use according to any one of embodiments 47 to 63 wherein the second anti-cancer agent is selected from the group consisting of Adriamycin, avastin, Cisplatin, Oxaliplatin, Carboplatin, Satroplatin, Gemcitabine, Dasatinib, decitabine (Dacogen), Docetaxel, Doxorubicin, hydroxydaunarubicin, Dexamethason, Chlorambucil, Fludarabine, Tarceva (Erlotinib), Iressa, Alimta, Melphalan, 5-Azacytidine (Vidaza), 5-FU (5-Fluorouracil), Actinomycin D, Paclitaxel, prednisone, Vinblastine, lenalidomide (Revlimid), Irinotecan, Topotecan, Etoposide, Tamoxifen, Rituximab (Rituxan), Herceptin, Cetuximab, sorafenib, sutent, Angiostatin, Endostatin, temsirolemus, everolimus, Avastin (Bevacizumab), derivatives thereof, analogs thereof and pharmaceutically acceptable salts thereof.

65. A use according to any one of embodiments 47 to 64 wherein the medicament includes a further anti-cancer agent.

66. A use according to embodiment 65 wherein the further anti-cancer agent is selected from the group consisting of Adriamycin, avastin, Cisplatin, Oxaliplatin, Carboplatin, Satroplatin, Gemcitabine, Dasatinib, decitabine (Dacogen), Docetaxel, Doxorubicin, hydroxydaunarubicin, Dexamethason, Chlorambucil, Fludarabine, Tarceva (Erlotinib), Iressa, Alimta, Melphalan, 5-Azacytidine (Vidaza), 5-FU (5-Fluorouracil), Actinomycin D, Paclitaxel, prednisone, Vinblastine, lenalidomide (Revlimid), Irinotecan, Topotecan, Etoposide, Tamoxifen, Rituximab (Rituxan), Herceptin, Cetuximab, sorafenib, Angiostatin, Endostatin, temsirolemus, evirolemus, Avastin (Bevacizumab), derivatives thereof, analogs thereof and pharmaceutically acceptable salts thereof and combinations thereof.

67. A use according to any one of embodiments 47 to 66 wherein the cancer is selected from the group consisting of colon cancer, gastric cancer, hepatocarcinoma, leukemia, lymphoma, lung cancer, liver cancer, myelodysplastic syndrome, pancreatic cancer, prostate cancer, breast cancer, hepatoma, renal cell carcinoma, and ovarian cancer.

68. A kit or kit-of-parts including

(i) a benzimidazole based anti-cancer agent; and

(ii) a second anti-cancer agent,
wherein said kit or kit-of-parts is suitable for use in a method for treating cancer.

69. A kit or kit of parts according to embodiment 68 wherein the benzimidazole based anti-cancer agent is a benzimidazole based anti-cancer agent as described in any one of embodiments 2 to 16.

70. A kit or kit of parts according to embodiment 68 wherein the second anti-cancer agent is an anti-cancer agent as described in embodiment 18.

71. A kit or kit of parts according any one of embodiments 68 to 70 wherein the kit includes a further anti-cancer agent.

**References:**

1. Chou, T.C., and Talalay, P. (1984). Quantitative analysis of dose-effect realationships: the combined effects of multiple drugs or enzyme inhibitors. Adv Enzyme Regul 22, 27-55
2. Chou, T. C. and Hayball, M. P. (1996) CalcuSyn Windows Software for Dose Effect Analysis. Biosoft, Cambridge, MA.
3. Idziorek, T, Estaquier, J., de Bels, F., and Ameisen, J.C. (1995) YOPRO-1 permits cytofluorometric analysis of programmed cell death (apoptosis) without interfering with cell viability. J Immunol Methods 185:249-258


**Claims**

**1.** A pharmaceutical composition suitable for the treatment of cancer, the composition including:

(i) azacitidine; and
(ii) a benzimidazole based anti-cancer agent of formula (1):

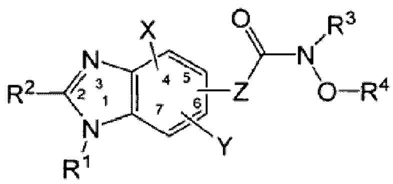

formula (1)

wherein

$R^1$ is a group of formula:
$-(CR^{20}R^{21})_m-(CR^{22}R^{23})_n-(CR^{24}R^{25})_o-NR^{26}R^{27}$;
$R^2$ is $C_1$-$C_6$ alkyl;
$R^3$ is H;
X and Y are each independently H;
$R^4$ is H;
Z is -CH=CH- in the "E" configuration at the 5 or 6 position;
each m, n and o are integers independently selected from the group consisting of 0, 1, 2, 3, and 4
each $R^{20}$, $R^{21}$, $R^{22}$, $R^{23}$, $R^{24}$ and $R^{25}$ is independently selected from the group consisting of H and methyl;
each $R^{26}$ and $R^{27}$ is independently selected from the group consisting of H and $C_1$-$C_6$ alkyl;
or a pharmaceutically acceptable salt or prodrug thereof.

**2.** The pharmaceutical composition according to claim 1, wherein Z is attached at ring position 5.

**3.** The pharmaceutical composition according to either of claim 1 or claim 2, wherein $R^1$ is selected from the group consisting of:

$-(CR^{20}R^{21})_2-NR^{26}R^{27}$;
$-(CR^{22}R^{23})_2-NR^{26}R^{27}$;
$-(CR^{24}R^{25})_2-NR^{26}R^{27}$;
$-(CR^{20}R^{21})-(CR^{22}R^{23})-NR^{26}R^{27}$;
$-(CR^{20}R^{21})(CR^{24}R^{25})-NR^{26}R^{27}$;
$-(CR^{22}R^{23})-(CR^{24}R^{25})-NR^{26}R^{27}$,

$-(CR^{20}R^{21})_3-NR^{26}R^{27}$;
$-(CR^{22}R^{23})_3-NR^{26}R^{27}$;
$-(CR^{24}R^{25})_3-NR^{26}R^{27}$;
$-(CR^{20}R^{21})_2-(CR^{22}R^{23})-NR^{26}R^{27}$
$-(CR^{20}R^{21})_2-(CR^{24}R^{25})-NR^{26}R^{27}$
$-(CR^{20}R^{21})-(CR^{22}R^{23})_2-NR^{26}R^{27}$;
$-(CR^{22}R^{23})_2-(CR^{24}R^{25})-NR^{26}R^{27}$;
$-(CR^{20}R^{21})-(CR^{24}R^{25})_2-NR^{26}R^{27}$;
$-(CR^{22}R^{23})-(CR^{24}R^{25})_2-NR^{26}R^{27}$; and
$-(CR^{20}R^{21})-(CR^{22}R^{23})-(CR^{24}R^{25})-NR^{26}R^{27}$;

**4.** The pharmaceutical composition according to claim 1, wherein the compound of formula (1) has the formula:

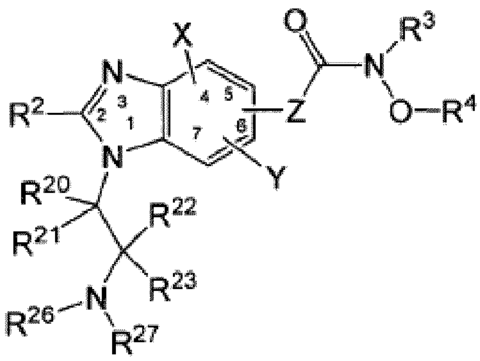

or

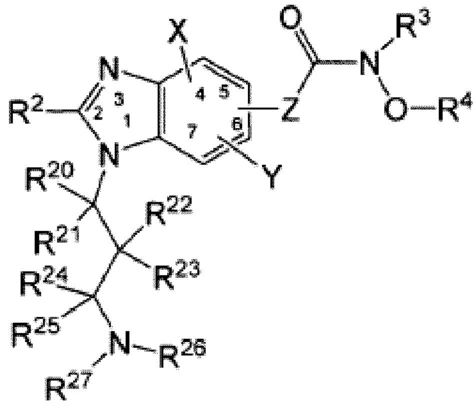

**5.** The pharmaceutical composition according to claim 4, wherein $R^{20}$, $R^{21}$, and $R^{24}$ and $R^{25}$ are each H, and $R^{22}$ and $R^{23}$ are each methyl.

**6.** The pharmaceutical composition according to any one of claims 1 to 5, wherein $R^{26}$ and $R^{27}$ are independently selected from the group consisting of H, methyl, ethyl, isopropyl, propyl, butyl, isobutyl, pentyl, and hexyl.

**7.** The pharmaceutical composition according to claim 1, wherein $R^1$ is a group of the formula:

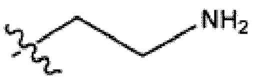
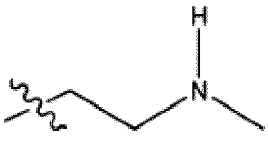
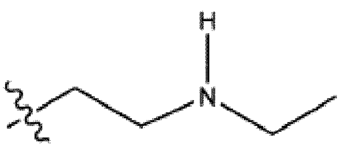

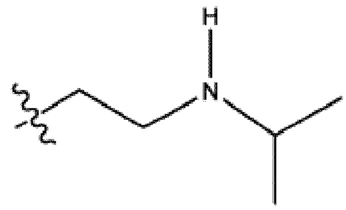
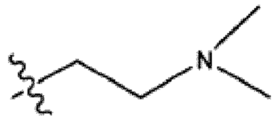
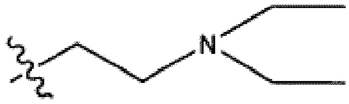

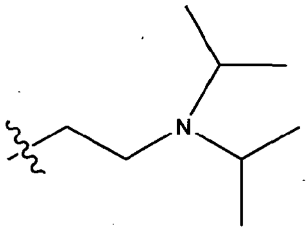
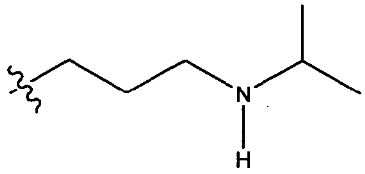
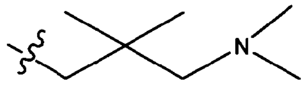

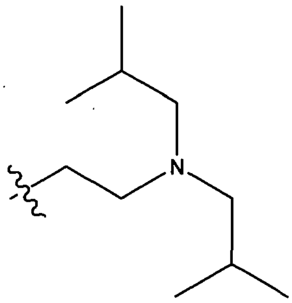
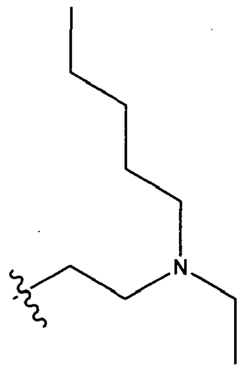
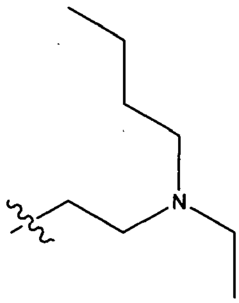

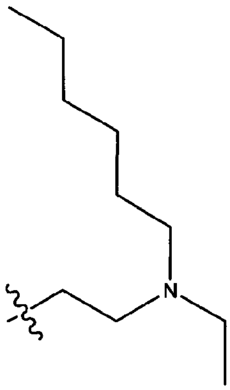
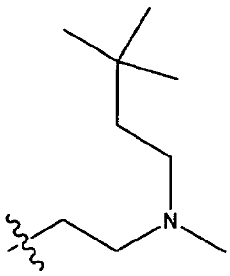
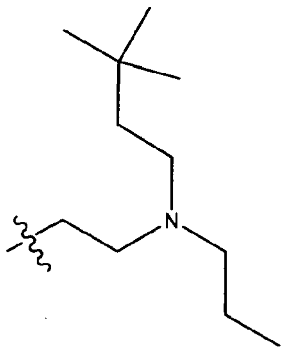

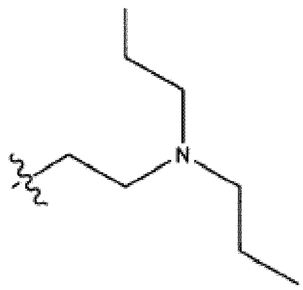
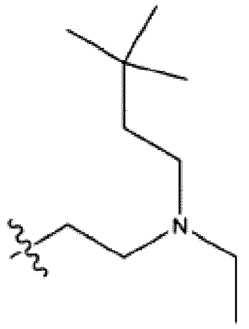
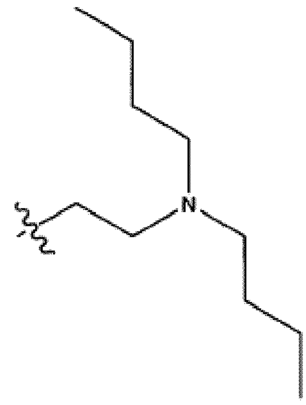

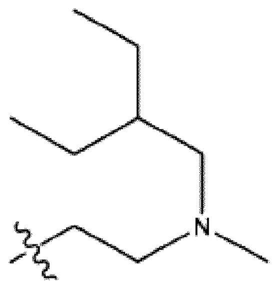
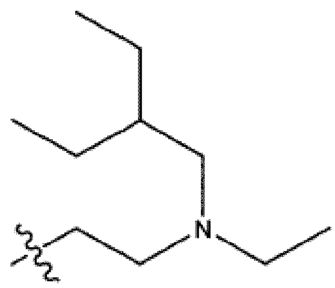
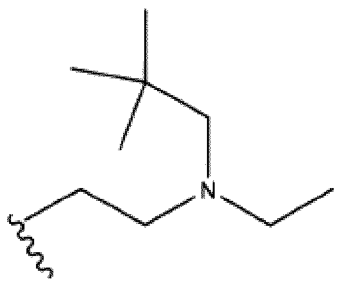

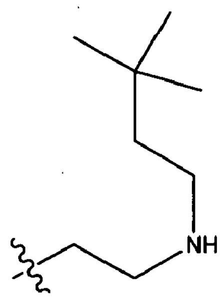
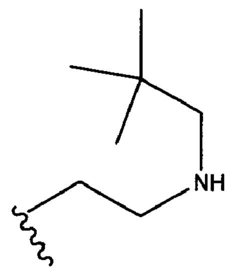

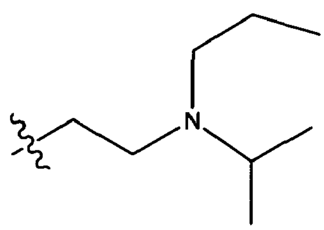
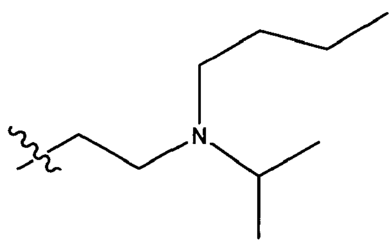
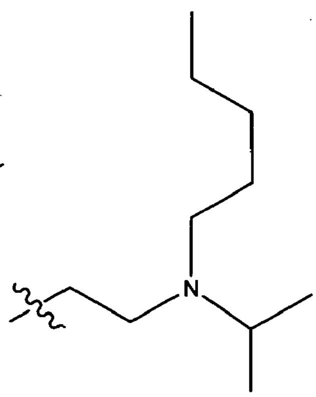

**8.** The pharmaceutical composition according to claim 1, wherein $R^2$ is selected from the group consisting of: H; methyl; propyl; 2-2-dimethyl-propyl; isopropyl; butyl; isobutyl; 3,3-dimethyl-butyl; pentyl; and hexyl.

**9.** The pharmaceutical composition according to claim 1, wherein the compound of formula (1) is selected from the

group consisting of

- (E)-3-[1-(3-Dimethylamino-2,2-dimethyl-propyl)-2-isopropyl-1H-benzimidazol-5-yl]-N-hydroxy-acrylamide
- (E)-3-[2-Butyl-1-(3-dimethylamino-2,2-dimethyl-propyl)-1H-benzimidazol-5-yl]-N-hydroxy-acrylamide
- (E)-3-[1-(3-Dimethylamino-2,2-dimethyl-propyl)-2-isobutyl-1H-benzimidazol-5-yl]-N-hydroxy-acrylamide
- (E)-3-[1-(2-Diethylamino-ethyl)-2-isobutyl-1H-benzimidazol-5-yl]-N-hydroxy-acrylamide
- (E)-3-[2-Butyl-1-(2-diethylamino-ethyl)-1H-benzimidazol-5-yl]-N-hydroxy-acrylamide
- (E)-3-[1-(3-Dimethylamino-2,2-dimethyl-propyl)-2-methyl-1H-benzimidazol-5-yl]-N-hydroxy-acrylamide
- (E)-3-[1-(2-Diethylamino-ethyl)-2-(2,2-dimethyl-propyl)-1H-benzimidazol-5-yl]-N-hydroxy-acrylamide
- (E)-3-[2-(2,2-Dimethyl-propyl)-1-(2-isopropylamino-ethyl)-1H-benzimidazol-5-yl]-N-hydroxy-acrylamide
- (E)-3-[1-(2-Diisopropylamino-ethyl)-2-(2,2-dimethyl-propyl)-1H-benzimidazol-5-yl]-N-hydroxy-acrylamide
- (E)-3-[1-(2-Diisopropylamino-ethyl)-2-isobutyl-1H-benzimidazol-5-yl]-N-hydroxy-acrylamide
- (E)-3-[1-(2-Diethylamino-ethyl)-2-hexyl-1H-benzimidazol-5-yl]-N-hydroxy-acrylamide
- (E)-3-[2-(2,2-Dimethyl-propyl)-1-(3-isopropylamino-propyl)-1H-benzimidazol-5-yl]-N-hydroxy-acrylamide
- (E)-N-Hydroxy-3-[2-isobutyl-1-(2-isopropylamino-ethyl)-1H-benzimidazol-5-yl]-acrylamide
- (E)-3-[2-(2,2-Dimethyl-propyl)-1-(2-ethylamino-ethyl)-1H-benzimidazol-5-yl]-N-hydroxy-acrylamide
- (E)-3-[1-(2-Ethylamino-ethyl)-2-isobutyl-1H-benzimidazol-5-yl]-N-hydroxy-acrylamide
- (E)-3-[1-(2-Diethylamino-ethyl)-2-propyl-1H-benzimidazol-5-yl]-N-hydroxy-acrylamide
- (E)-3-[2-Butyl-1-(2-diisopropylamino-ethyl)-1H-benzimidazol-5-yl]-N-hydroxy-acrylamide
- (E)-3-[2-Butyl-1-(2-ethylamino-ethyl)-1H-benzimidazol-5-yl]-N-hydroxy-acrylamide
- (E)-3-[2-Butyl-1-(2-isopropylamino-ethyl)-1H-benzimidazol-5-yl]-N-hydroxy-acrylamide
- (E)-3-[2-Butyl-1-(3-isopropylamino-propyl)-1H-benzimidazol-5-yl]-N-hydroxy-acrylamide
- (E)-3-[2-Hexyl-1-(2-isopropylamino-ethyl)-1H-benzimidazol-5-yl]-N-hydroxy-acrylamide
- (E)-3-[1-(2-Ethylamino-ethyl)-2-hexyl-1H-benzimidazol-5-yl]-N-hydroxy-acrylamide
- (E)-3-[2-Butyl-1-(2-dimethylamino-ethyl)-1H-benzimidazol-5-yl]-N-hydroxy-acrylamide
- (E)-3-[2-(2-Dimethylamino-ethyl)-2-hexyl-1H-benzimidazol-5-yl]-N-hydroxy-acrylamide
- (E)-3-[1-(2-ethylamino-ethyl)-2-(3,3-dimethyl-butyl)-1H-benzimidazol-5-yl]-N-hydroxy-acrylamide
- (E)-3-[2-(3,3-Dimethyl-butyl)-1-(2-Dimethylamino-ethyl)-1H-benzimidazol-5-yl]-N-hydroxy-acrylamide,
- (E)-3-[1-(2-Dimethylamino-ethyl)-2-pentyl-1H-benzimidazol-5-yl]-N-hydroxy-acrylamide
- (E)-3-[1-(2-Ethylamino-ethyl)-2-pentyl-1H-benzimidazol-5-yl]-N-hydroxy-acrylamide
- (E)-N-Hydroxy-3-[1-(2-isopropylamino-ethyl)-2-pentyl-1H-benzimidazol-5-yl]-acrylamide
- (E)-3-[2-Hexyl-1-(2-methylamino-ethyl)-1H-benzimidazol-5-yl]-N-hydroxy-acrylamide
- (E)-N-Hydroxy-3-[1-(2-methylamino-ethyl)-2-pentyl-1H-benzimidazol-5-yl]-acrylamide
- (E)-3-{2-Butyl-1[2-(isopropyl-methyl-amino)-ethyl]-1H-benzimidazol-5-yl}-N-hydroxy-acrylamide
- (E)-3-{1-[2-(Ethyl-methyl-amino)-ethyl]-2-pentyl-1H-benzimidazol-5-yl}-N-hydroxy-acrylamide
- (E)-3-{2-Butyl-1-[2-(ethyl-methyl-amino)-ethyl]-1H-benzimidazol-5-yl}-N-hydroxy-acrylamide
- (E)-3-{1-[2-(Ethyl-hexyl-amino)-ethyl]-2-methyl-1H-benzimidazol-5-yl}-N-hydroxy-acrylamide
- (E)-3-(1-(2-(dibutylamino)ethyl)-2-propyl-1H-benzo[d]imidazol-5-yl)-N-hydroxyacrylamide
- (E)-3-{1-[2-(3,3-Dimethyl-butylamino)-ethyl]-2-propyl-1H-benzimidazol-5-yl}-N-hydroxy-acrylamide
- (E)-3-{1-[2-(3,3-Dimethyl-butylamino)-ethyl}-2-(2,2-dimethyl-propyl)-1H-benzimidazol-5-yl]-N-hydroxy-acrylamide
- (E)-3-[1-{2-[Bis-(3,3-dimethyl-butyl)-amino]-ethyl}-2-(2,2-dimethyl-propyl)-1H-benzimidazol-5-yl]-N-hydroxy-acrylamide
- (E)-3-{1-[2-(3,3-Dimethyl-butylamino)-ethyl]-2-ethyl-1H-benzimidazol-5-yl}-N-hydroxy-acrylamide
- (E)-3-(1-{2-[(3,3-Dimethyl-butyl)-methyl-amino]-ethyl)-2-propyl-1H-benzimidazol-5-yl}-N-hydroxy-acrylamide

or a pharmaceutically acceptable salt thereof.

**10.** The pharmaceutical composition according to claim 1, wherein the compound of formula (1) is

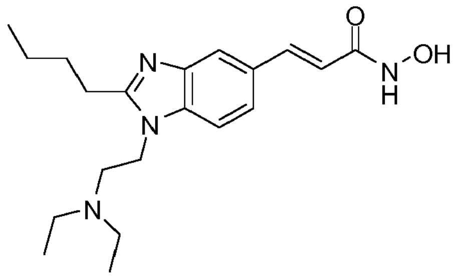

or a pharmaceutically acceptable salt thereof.

**11.** A pharmaceutical composition of any one of claims 1-10 for use in a method for the treatment of cancer.

**12.** The pharmaceutical composition for the use according to claim 11, wherein the cancer is selected from the group consisting of colon cancer, gastric cancer, hepatocarcinoma, leukemia, lymphoma, lung cancer, liver cancer, myelodysplastic syndrome, pancreatic cancer, prostate cancer, breast cancer, hepatoma, renal cell carcinoma, and ovarian cancer.

**13.** The pharmaceutical composition for the use according to claim 12, wherein the leukemia is selected from the group consisting of erythroleukemia, acute promyelocytic leukemia, acute myeloid leukemia (AML), acute lymophocytic leukemia, and acute T-cell leukemia.

**14.** The pharmaceutical composition for the use according to claim 12, wherein the lymphoma is selected from the group consisting of B-cell lymphoma (e.g. Burkitt's lymphoma), mantle cell lymphoma, cutaneous T-cell lymphoma (CTCL), peripheral T-cell lymphoma, Hodgkin's lymphoma, and non-Hodgkin's lymphoma.

Europäisches Patentamt
European Patent Office
Office européen des brevets

# EUROPEAN SEARCH REPORT

Application Number
EP 18 19 8417

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| Y | EP 1 673 349 A1 (S BIO PTE LTD [SG]) 28 June 2006 (2006-06-28) * claim 1 * ----- | 1-14 | INV. A61K31/4184 |
| Y | WO 2005/028447 A (S BIO PTE LTD [SG]; CHEN DIZHONG [SG]; DENG WEIPING [SG]; SANGTHONGPIT) 31 March 2005 (2005-03-31) * page 2, line 20 - page 6, line 19 * * page 34, lines 6-12 * ----- | 1-14 | |
| Y | US 2003/100592 A1 (CAMDEN JAMES BERGER [US] ET AL) 29 May 2003 (2003-05-29) * column 13, paragraph 1; claim 1; table 3 * ----- | 1-14 | |
| Y | WO 98/32440 A (PROCTER & GAMBLE [US]) 30 July 1998 (1998-07-30) * claims 1,6 * ----- | 1-14 | |
| | | | TECHNICAL FIELDS SEARCHED (IPC) |
| | | | A61K |

The present search report has been drawn up for all claims

1

| Place of search | Date of completion of the search | Examiner |
|---|---|---|
| Munich | 10 January 2019 | Bareyt, Sébastian |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document

EPO FORM 1503 03.82 (P04C01)

## ANNEX TO THE EUROPEAN SEARCH REPORT ON EUROPEAN PATENT APPLICATION NO. EP 18 19 8417

This annex lists the patent family members relating to the patent documents cited in the above-mentioned European search report. The members are as contained in the European Patent Office EDP file on
The European Patent Office is in no way liable for these particulars which are merely given for the purpose of information.

10-01-2019

| Patent document cited in search report | Publication date | Patent family member(s) | Publication date |
|---|---|---|---|
| EP 1673349 A1 | 28-06-2006 | EP 1673349 A1 | 28-06-2006 |
| | | SI 1673349 T1 | 29-10-2010 |
| WO 2005028447 A | 31-03-2005 | NONE | |
| US 2003100592 A1 | 29-05-2003 | US 6462062 B1 | 08-10-2002 |
| | | US 2003100592 A1 | 29-05-2003 |
| | | US 2005288348 A1 | 29-12-2005 |
| WO 9832440 A | 30-07-1998 | NONE | |

EPO FORM P0459

For more details about this annex : see Official Journal of the European Patent Office, No. 12/82

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Non-patent literature cited in the description


- Remington's Pharmaceutical Sciences. Mack Publishing Co, 1995
- Remingtons Pharmaceutical Sciences. Mack Publishing Co, 1995
- **T.W. GREENE.** Protective Groups in Organic Synthesis. John Wiley & Sons, 1991
- **STILL et al.** *J. Org. Chem.,* 1978, vol. 43, 2923
- *J. Med. Chem.,* 2001, vol. 44, 1516-1529
- *J. Med. Chem.,* 2002, vol. 45, 753-757
- *Tetrahedron Letters,* 2000, vol. 41, 9871-9874
- **CHOU, T.C. ; TALALAY, P.** Quantitative analysis of dose-effect realationships: the combined effects of multiple drugs or enzyme inhibitors. *Adv Enzyme Regul,* 1984, vol. 22, 27-55
- **CHOU, T. C. ; HAYBALL, M. P.** CalcuSyn Windows Software for Dose Effect Analysis. *Biosoft,* 1996
- **IDZIOREK, T ; ESTAQUIER, J. ; DE BELS, F. ; AMEISEN, J.C.** YOPRO-1 permits cytofluorometric analysis of programmed cell death (apoptosis) without interfering with cell viability. *J Immunol Methods,* 1995, vol. 185, 249-258